(19) Europäisches Patentamt
European Patent Office
Office européen des brevets

(11) EP 2 841 575 B1

(12) **EUROPEAN PATENT SPECIFICATION**

(45) Date of publication and mention of the grant of the patent:
**26.06.2019 Bulletin 2019/26**

(21) Application number: **13782061.9**

(22) Date of filing: **27.04.2013**

(51) Int Cl.:
***C07K 16/40*** *(2006.01)* ***G01N 33/574*** *(2006.01)*

(86) International application number:
**PCT/US2013/038542**

(87) International publication number:
**WO 2013/163633 (31.10.2013 Gazette 2013/44)**

(54) **ANTI-GCC ANTIBODY MOLECULES AND USE OF SAME TO TEST FOR SUSCEPTIBILITY TO GCC-TARGETED THERAPY**

ANTI-GCC-ANTIKÖRPERMOLEKÜLE UND VERWENDUNG DAVON FÜR EINEN TEST AUF EMPFÄNGLICHKEIT FÜR EINE GCC-GEZIELTE THERAPIE

MOLÉCULES D'ANTICORPS ANTI-GCC ET LEUR UTILISATION EN VUE DE TESTER LA SENSIBILITÉ À UNE THÉRAPIE CIBLÉE SUR LA GCC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.04.2012 US 201261639376 P**

(43) Date of publication of application:
**04.03.2015 Bulletin 2015/10**

(73) Proprietor: **Millennium Pharmaceuticals, Inc.**
**Cambridge, MA 02139 (US)**

(72) Inventors:
- **FRANK, Helen, Alison**
**Sharon, MA 02067 (US)**
- **MCDONALD, Alice, A.**
**Swampscott, MA 01907 (US)**
- **O'KEEFE, Theresa, L.**
**Waltham, MA 02453 (US)**

(74) Representative: **Lau, Sarah Jane et al**
**Kilburn & Strode LLP**
**Lacon London**
**84 Theobalds Road**
**London WC1X 8NL (GB)**

(56) References cited:
**WO-A1-2010/065293 WO-A1-2011/050242**
**WO-A1-2014/134311 US-A1- 2011 110 936**

- **C. P. SWAMINATHAN: "Thermodynamic Analyses Reveal Role of Water Release in Epitope Recognition by a Monoclonal Antibody against the Human Guanylyl Cyclase C Receptor", JOURNAL OF BIOLOGICAL CHEMISTRY, vol. 274, no. 44, 29 October 1999 (1999-10-29), pages 31272-31278, XP055060704, ISSN: 0021-9258, DOI: 10.1074/jbc.274.44.31272**
- **Glenn E. Morris: "Epitope Mapping of Protein Antigens by Competition ELISA" In: "The Protein Protocols Handbook", 1 January 1996 (1996-01-01), Humana Press, Totowa, NJ, XP055007939, ISBN: 978-1-60-327259-9 pages 595-600, DOI: 10.1007/978-1-60327-259-9_96, * page 595 ***
- **P VEIBY ET AL: "The Investigational Drug MLN0264 First-in-human, First in Class ADC Targeting GCC: Phase I Dose-escalation Study and Supportive Scientific Rationale", EUROPEAN JOURNAL OF CANCER, vol. 48, Suppl. 6, 8 November 2012 (2012-11-08), page 101, XP055222847,**
- **BHANDARI ET AL.: 'Functional Inactivation of the Human Guanylyl Cyclase C Receptor: Modeling and Mutation of the Protein Kinase-like Domain' BIOCHEMISTRY vol. 40, no. 31, 07 August 2001, pages 9196 - 9206, XP055171134**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **GERSHONI JONATHAN M ET AL: "Epitope mapping - The first step in developing epitope-based vaccines", BIODRUGS, ADIS INTERNATIONAL LTD, NZ, vol. 21, no. 3, 1 January 2007 (2007-01-01), pages 145-156, XP009103541, ISSN: 1173-8804, DOI: 10.2165/00063030-200721030-00002**
- **BIGOTT-HENNKENS H M ET AL: "In vitro receptor binding assays: general methods and considerations.", THE QUARTERLY JOURNAL OF NUCLEAR MEDICINE AND MOLECULAR IMAGING : OFFICIAL PUBLICATION OF THE ITALIAN ASSOCIATION OF NUCLEAR MEDICINE (AIMN) [AND] THE INTERNATIONAL ASSOCIATION OF RADIOPHARMACOLOGY (IAR), [AND] SECTION OF THE SOCIETY OF... SEP 2008, vol. 52, no. 3, September 2008 (2008-09), pages 245-253, ISSN: 1824-4785**
- **W. Sawyer and D. Winzor: "Biophysical Methods: Data analysis" In: "Current Protocols in Protein Science", 2001 vol. Appendix, pages A.5A.1-A.5A.40,**
- **FREEMAN J W ET AL: "DISCRIMINATION OF EPITOPES IDENTIFIED BY MONOCLONAL ANTIBODIES BY COMPETITIVE BINDING TO NITROCELLULOSE BOUND ANTIGENS", JOURNAL OF IMMUNOLOGICAL METHODS, vol. 78, no. 2, 1985, pages 259-266, ISSN: 0022-1759**


Remarks:

The file contains technical information submitted after the application was filed and not included in this specification

## Description

### Related Applications

The present application claims the benefit of and priority to U.S. Provisional Application Serial No. 61/639,376, filed April 27, 2012.

### Field of the Invention

The invention relates to antibody molecules which bind GCC, and methods for using the same to select patients for treatment with a GCC-targeted therapy, such as an immunoconjugate containing an anti-GCC antibody molecule, or fragment thereof, conjugated to an agent such as a therapeutic agent.

### Background

Guanylyl cyclase C ("GCC") is a transmembrane cell surface receptor that functions in the maintenance of intestinal fluid, electrolyte homeostasis and cell proliferation, see, e.g., Carrithers et al., Proc. Natl. Acad. Sci. USA 100:3018-3020 (2003). GCC is expressed at the mucosal cells lining the small intestine, large intestine and rectum (Carrithers et al., Dis Colon Rectum 39: 171-181 (1996)). GCC expression is maintained upon neoplastic transformation of intestinal epithelial cells, with expression in all primary and metastatic colorectal tumors (Carrithers et al., Dis Colon Rectum 39: 171-181 (1996); Buc et al. Eur J Cancer 41: 1618-1627 (2005); Carrithers et al., Gastroenterology 107: 1653-1661 (1994)). Antibodies and antigen-binding fragments of antibodies that bind GCC are disclosed in WO 2011/050242.

### Summary

The subject matter for which protection is sought is as defined in the claims. The disclosure is based, at least in part, on the discovery of novel anti-GCC antibodies. Accordingly, in one aspect, the invention features an anti-GCC antibody molecule, as disclosed herein. The anti-GCC antibody molecules are useful as naked antibody molecules and as components of immunoconjugates. Accordingly, in another aspect, the invention features immunoconjugates comprising an anti-GCC antibody molecule describd herein and a therapeutic agent or label. The invention also features methods of using the anti-GCC antibody molecules and immunoconjucates described herein, e.g., for detection of GCC and of cells or tissues that express GCC. Such methods are useful, *inter alia,* for diagnosis, prognosis, imaging, or staging of a GCC-mediated disease. Accordingly, in some aspects, the invention features methods of identifying a subject for treatment with a GCC- targeted therapy, e.g., an anti-GCC antibody therapy, e.g., an immunoconjugate comprising an anti-GCC antibody conjugated with a therapeutic agent. The invention also features an *in vitro* or *in vivo* method of determining of a subject having cancer is a potential candidate for a GCC-targeted therapy, e.g., a GCC-targeted therapy described herein.

Anti-GCC antibodies, e.g., the anti-GCC antibodies described herein, are also useful e.g., for modulating an activity or function of a GCC protein; and for treatment of a GCC-mediated disease, as described herein. In some aspects, the treatment includes acquiring knowledge and/or evaluating a sample or subject to determined GCC expression levels, and if the subject expresses GCC, then administering a GCC-targeted therapy, e.g., a GCC targeted therapy described herein. In other aspects, the method features generating a personalized treatment report, e.g., a GCC targeted treatment report, by obtaining a sample from a subject and determining GCC expression levels, e.g., by a detection method described herein, e.g., using an anti-GCC antibody described herein, and based upon the determination, selecting a targeted treatment report for the subject.

In another aspect, the invention also features isolated and/or recombinant nucleic acids encoding anti-GCC antibody molecule amino acid sequences, as well as vectors and host cells comprising such nucleic acids, and methods for producing anti-GCC antibody molecules. Also featured herein are reaction mixtures and kits comprising the anti-GCC antibodies, e.g., an immunocnjugate, described herein, as well as *in vitro* assays, e.g., comprising an anti-GCC antibody described herein, to detect GCC expression.

Other features, objects, and advantages of the invention(s) disclosed herein will be apparent from the description and drawings, and from the claims.

### Brief Description of the Drawings

Figure 1 is a circular map of a protein expression vector used to generate a human GCC (hGCC) extracellular domain (ECD) mouse Fc (mFc) fusion protein (hGCC-ECD-mFc) of the invention.

Figures 2A-2D depict bar graphs summarizing the combined/aggregate (apical and cytoplasmic) H score distribution of GCC expression across tumor types from cancer patients screened for enrollment into the C26001 Study, a Phase I clinical trial of a GCC-targeted immunoconjugate. Figure 2A depicts the combined aggregate H score distribution across colorectal cancer patients screened; Figure 2B depicts the combined aggregate H score distribution across gastric cancer patients screened; Figure 2C depicts the combined aggregate H score distribution across pancreatic cancer patients screened; Figure 2D depicts the combined aggregate H score distribution across esophageal cancer patients screened.

Figures 3A-3C depict bar graphs summarizing the combined/aggregate (apical and cytoplasmic) H score distribution of GCC expression across various tumor specific microarrays that were screened. Figure 3A depicts the combined/aggregate H score distribution across samples on various colorectal tumor microarrays screened for GCC expression; Figure 3B depicts the combined/aggregate H score distribution across samples on a gastric tumor microarray screened for GCC expression; Figure 3C depicts the combined/aggregate H score distribution across samples on various pancreatic tumor microarrays screened for GCC expression.

**Detailed Description**

Guanylyl cyclase C (GCC) (also known as STAR, ST Receptor, GUC2C, and GUCY2C) is a transmembrane cell surface receptor that functions in the maintenance of intestinal fluid, electrolyte homeostasis and cell proliferation (Carrithers et al., Proc Natl Acad Sci USA 100: 3018-3020 (2003); Mann et al., Biochem Biophys Res Commun 239: 463-466 (1997); Pitari et al., Proc Natl Acad Sci USA 100: 2695-2699 (2003)); GenBank Accession No. NM_004963. This function is mediated through binding of guanylin (Wiegand et al. FEBS Lett. 311:150-154 (1992)). GCC also is a receptor for heat-stable enterotoxin (ST, e.g., having an amino acid sequence of NTFYCCELCCNPACAGCY, SEQ ID NO: 1) which is a peptide produced by E. coli, as well as other infectious organisms (Rao, M. C. Ciba Found. Symp. 112:74-93 (1985); Knoop F. C. and Owens, M. J. Pharmacol. Toxicol. Methods 28:67-72 (1992)). Binding of ST to GCC activates a signal cascade that results in enteric disease, e.g., diarrhea.

Nucleotide sequence for human GCC (GenBank Accession No. NM_004963; SEQ ID NO: 2):

```
  1 gaccagagag aagcgtgggg aagagtgggc tgagggactc cactagaggc tgtccatctg
 61 gattccctgc ctccctagga gcccaacaga gcaaagcaag tgggcacaag gagtatggtt
121 ctaacgtgat tggggtcatg aagacgttgc tgttggactt ggctttgtgg tcactgctct
181 tccagcccgg gtggctgtcc tttagttccc aggtgagtca gaactgccac aatggcagct
241 atgaaatcag cgtcctgatg atgggcaact cagcctttgc agagcccctg aaaaacttgg
301 aagatgcggt gaatgagggg ctggaaatag tgagaggacg tctgcaaaat gctggcctaa
361 atgtgactgt gaacgctact ttcatgtatt cggatggtct gattcataac tcaggcgact
421 gccggagtag cacctgtgaa ggcctcgacc tactcaggaa aatttcaaat gcacaacgga
481 tgggctgtgt cctcataggg ccctcatgta catactccac cttccagatg taccttgaca
541 cagaattgag ctaccccatg atctcagctg gaagttttgg attgtcatgt gactataaag
601 aaaccttaac caggctgatg tctccagcta gaaagttgat gtacttcttg gttaactttt
661 ggaaaaccaa cgatctgccc ttcaaaactt attcctggag cacttcgtat gtttacaaga
721 atggtacaga aactgaggac tgtttctggt accttaatgc tctggaggct agcgtttcct
781 atttctccca cgaactcggc tttaaggtgg tgttaagaca agataaggag tttcaggata
841 tcttaatgga ccacaacagg aaaagcaatg tgattattat gtgtggtggt ccagagttcc
901 tctacaagct gaagggtgac cgagcagtgg ctgaagacat tgtcattatt ctagtggatc
```

961 ttttcaatga ccagtacttt gaggacaatg tcacagcccc tgactatatg aaaaatgtcc
1021 ttgttctgac gctgtctcct gggaattccc ttctaaatag ctctttctcc aggaatctat
1081 caccaacaaa acgagacttt gctcttgcct atttgaatgg aatcctgctc tttggacata
1141 tgctgaagat atttcttgaa aatggagaaa atattaccac ccccaaattt gctcatgctt
1201 tcaggaatct cacttttgaa gggtatgacg gtccagtgac cttggatgac tgggggggatg
1261 ttgacagtac catggtgctt ctgtatacct ctgtggacac caagaaatac aaggttcttt
1321 tgacctatga tacccacgta aataagacct atcctgtgga tatgagcccc acattcactt
1381 ggaagaactc taaacttcct aatgatatta caggccgggg ccctcagatc ctgatgattg
1441 cagtcttcac cctcactgga gctgtggtgc tgctcctgct cgtcgctctc ctgatgctca
1501 gaaaatatag aaaagattat gaacttcgtc agaaaaaatg gtcccacatt cctcctgaaa
1561 atatctttcc tctggagacc aatgagacca atcatgttag cctcaagatc gatgatgaca
1621 aaagacgaga tacaatccag agactacgac agtgcaaata cgacaaaaag cgagtgattc
1681 tcaaagatct caagcacaat gatggtaatt tcactgaaaa acagaagata gaattgaaca
1741 agttgcttca gattgactat tacaacctga ccaagttcta cggcacagtg aaacttgata
1801 ccatgatctt cggggtgata gaatactgtg agagaggatc cctccgggaa gttttaaatg
1861 acacaatttc ctaccctgat ggcacattca tggattggga gtttaagatc tctgtcttgt
1921 atgacattgc taagggaatg tcatatctgc actccagtaa gacagaagtc catggtcgtc
1981 tgaaatctac caactgcgta gtggacagta gaatggtggt gaagatcact gattttggct
2041 gcaattccat tttacctcca aaaaaggacc tgtggacagc tccagagcac ctccgccaag
2101 ccaacatctc tcagaaagga gatgtgtaca gctatgggat catcgcacag gagatcatcc
2161 tgcggaaaga aaccttctac actttgagct gtcgggaccg gaatgagaag attttcagag
2221 tggaaaattc caatggaatg aaacccttcc gcccagattt attcttggaa acagcagagg
2281 aaaaagagct agaagtgtac ctacttgtaa aaaactgttg ggaggaagat ccagaaaaga
2341 gaccagattt caaaaaaatt gagactacac ttgccaagat atttggactt tttcatgacc
2401 aaaaaaatga aagctatatg gataccttga tccgacgtct acagctatat tctcgaaacc
2461 tggaacatct ggtagaggaa aggacacagc tgtacaaggc agagagggac agggctgaca
2521 gacttaactt tatgttgctt ccaaggctag tggtaaagtc tctgaaggag aaaggctttg
2581 tggagccgga actatatgag gaagttacaa tctacttcag tgacattgta ggtttcacta
2641 ctatctgcaa atacagcacc cccatggaag tggtggacat gcttaatgac atctataaga
2701 gttttgacca cattgttgat catcatgatg tctacaaggt ggaaaccatc ggtgatgcgt
2761 acatggtggc tagtggtttg cctaagagaa atggcaatcg gcatgcaata gacattgcca
2821 agatggcctt ggaaatcctc agcttcatgg ggacctttga gctggagcat cttcctggcc
2881 tcccaatatg gattcgcatt ggagttcact ctggtccctg tgctgctgga gttgtgggaa
2941 tcaagatgcc tcgttattgt ctatttggag atacggtcaa cacagcctct aggatggaat
3001 ccactggcct ccctttgaga attcacgtga gtggctccac catagccatc ctgaagagaa

3061 ctgagtgcca gttcctttat gaagtgagag gagaaacata cttaaaggga agaggaaatg
3121 agactaccta ctggctgact gggatgaagg accagaaatt caacctgcca acccctccta
3181 ctgtggagaa tcaacagcgt ttgcaagcag aattttcaga catgattgcc aactctttac
3241 agaaaagaca ggcagcaggg ataagaagcc aaaaacccag acgggtagcc agctataaaa
3301 aaggcactct ggaatacttg cagctgaata ccacagacaa ggagagcacc tatttttaaa

Amino acid sequence for human GCC (GenPept Accession No. NP_004954; SEQ ID NO: 3):

```
   1 mktllldlal wsllfqpgwl sfssqvsqnc hngsyeisvl mmgnsafaep lknledavne
  61 gleivrgrlq naglnvtvna tfmysdglih nsgdcrsstc egldllrkis naqrmgcvli
 121 gpsctystfq myldtelsyp misagsfgls cdyketltrl msparklmyf lvnfwktndl
 181 pfktyswsts yvykngtete dcfwylnale asvsyfshel gfkvvlrqdk efqdilmdhn
 241 rksnviimcg gpeflyklkg dravaedivi ilvdlfndqy fednvtapdy mknvlvltls
 301 pgnsllnssf srnlsptkrd falaylngil lfghmlkifl engenittpk fahafrnltf
 361 egydgpvtld dwgdvdstmv llytsvdtkk ykvlltydth vnktypvdms ptftwknskl
 421 pnditgrgpq ilmiavftlt gavvllllva llmlrkyrkd yelrqkkwsh ippenifple
 481 tnetnhvslk idddkrrdti qrlrqckydk krvilkdlkh ndgnftekqk ielnkllqid
 541 yynltkfygt vkldtmifgv ieycergslr evlndtisyp dgtfmdwefk isvlydiakg
 601 msylhsskte vhgrlkstnc vvdsrmvvki tdfgcnsilp pkkdlwtape hlrqanisqk
 661 gdvysygiia qeiilrketf ytlscrdrne kifrvensng mkpfrpdlfl etaeekelev
 721 yllvkncwee dpekrpdfkk iettlakifg lfhdqknesy mdtlirrlql ysrnlehlve
 781 ertqlykaer dradrlnfml lprlvvkslk ekgfvepely eevtiyfsdi vgfttickys
 841 tpmevvdmln diyksfdhiv dhhdvykvet igdaymvasg lpkrngnrha idiakmalei
 901 lsfmgtfele hlpglpiwir igvhsgpcaa gvvgikmpry clfgdtvnta srmestglpl
 961 rihvsgstia ilkrtecqfl yevrgetylk grgnettywl tgmkdqkfnl ptpptvenqq
1021 rlqaefsdmi anslqkrqaa girsqkprrv asykkgtley lqlnttdkes tyf
```

The GCC protein has some generally accepted domains each of which contributes a separable function to the GCC molecule. The portions of GCC include a signal sequence (for directing the protein to the cell surface) from amino acid residue 1 to about residue 23, or residue 1 to about residue 21 of SEQ ID NO: 3 (excised for maturation to yield functional mature protein from about amino acid residues 22 or 24 to 1073 of SEQ ID NO: 3), an extracellular domain for ligand, e.g., guanylin or ST, binding from about amino acid residue 24 to about residue 420, or about residue 54 to about residue 384 of SEQ ID NO: 3, a transmembrane domain from about amino acid residue 431 to about residue 454, or about residue 436 to about residue 452 of SEQ ID NO: 3, a kinase homology domain, predicted to have tyrosine kinase activity from about amino acid residue 489 to about residue 749, or about residue 508 to about residue 745 of SEQ ID NO: 3 and a guanylyl cyclase catalytic domain from about residue 750 to about residue 1007, or about residue 816 to about residue 1002 of SEQ ID NO: 3.

In normal human tissues, GCC is expressed at the mucosal cells, e.g., at the apical brush border membranes, lining the small intestine, large intestine and rectum (Carrithers et al., Dis Colon Rectum 39: 171-181 (1996)). GCC expression is maintained upon neoplastic transformation of intestinal epithelial cells, with expression in all primary and metastatic colorectal tumors (Carrithers et al., Dis Colon Rectum 39: 171-181 (1996); Buc et al. Eur J Cancer 41: 1618-1627 (2005); Carrithers et al., Gastroenterology 107: 1653-1661 (1994)). Neoplastic cells from the stomach, esophagus and the gastroesophageal junction also express GCC (see, e.g., U.S. Pat. No. 6,767,704; Debruyne et al. Gastroenterology 130:1191-1206 (2006)). The tissue-specific expression and association with cancer, e.g., of gastrointestinal origin, (e.g., colorectal cancer, stomach cancer, esophageal cancer or small intestine cancer), can be exploited for the use of GCC as a diagnostic marker for this disease (Carrithers et al., Dis Colon Rectum 39: 171-181 (1996); Buc et al. Eur J Cancer 41: 1618-1627 (2005)). Additionally, as demonstrated in the Examples herein, several different types of cancer of non-gastrointestinal origin have been shown to express GCC, including but not limited to pancreatic cancer, lung cancer, soft-tissue sarcomas (e.g., leiomyosarcoma and rhabdomyosarcoma) gastrointestinal or bronchopulmonary neuroendocrine tumors, and neuroectodermal tumors.

As a cell surface protein, GCC can also serve as a diagnostic or therapeutic target for receptor binding proteins such as antibodies or ligands. In normal intestinal tissue, GCC is expressed on the apical side of epithelial cell tight junctions that form an impermeable barrier between the luminal environment and vascular compartment (Almenoff et al., Mol Microbiol 8: 865-873); Guarino et al., Dig Dis Sci 32: 1017-1026 (1987)). As such, systemic intravenous administration of a GCC-binding protein therapeutic will have minimal effect on intestinal GCC receptors, while having access to neoplastic cells of the gastrointestinal system, including invasive or metastatic colon cancer cells, extraintestinal or metastatic colon tumors, esophageal tumors or stomach tumors, adenocarcinoma at the gastroesophageal junction.

Additionally, GCC internalizes through receptor mediated endocytosis upon ligand binding (Buc et al. Eur J Cancer 41: 1618-1627 (2005); Urbanski et al., Biochem Biophys Acta 1245: 29-36 (1995)).

Polyclonal antibodies raised against the extracellular domain of GCC (Nandi et al. Protein Expr. Purif. 8:151-159 (1996)) were able to inhibit the ST peptide binding to human and rat GCC and inhibit ST-mediated cGMP production by human GCC.

GCC has been characterized as a protein involved in cancers, including colon cancers. See also, Carrithers et al., Dis Colon Rectum 39: 171-181 (1996); Buc et al. Eur J Cancer 41: 1618-1627 (2005); Carrithers et al., Gastroenterology 107: 1653-1661 (1994); Urbanski et al., Biochem Biophys Acta 1245: 29-36 (1995). Antibody molecules directed to GCC can thus be used alone in unconjugated form to inhibit the GCC-expressing cancerous cells. Additionally, antibody molecules directed to GCC can be used in naked or labeled form, to detect GCC-expressing cancerous cells. Anti-GCC antibody molecules of the invention can bind human GCC. In some embodiments, an anti-GCC antibody molecule of the invention can inhibit the binding of a ligand, e.g., guanylin or heat-stable enterotoxin to GCC. In other embodiments, an anti-GCC antibody molecule of the invention does not inhibit the binding of a ligand, e.g., guanylin or heat-stable enterotoxin to GCC.

Monoclonal antibodies specific for GCC include GCC:B10 (Nandi et al., J. Cell. Biochem. 66:500-511 (1997)), GCC:4D7 (Vijayachandra et al. Biochemistry 39:16075-16083 (2000)) and GCC:C8 (Bakre et al. Eur. J. Biochem. 267:179-187 (2000)). GCC:B10 has a kappa light chain and an IgG2a isotype and cross-reacts to rat, pig and monkey GCC. GCC:B10 binds to the first 63 amino acids of the intracellular domain of GCC, specifically to residues 470-480 of SEQ ID NO: 3 (Nandi et al. Protein Sci. 7:2175-2183 (1998)). GCC:4D7 binds to the kinase homology domain, within residues 491-568 of GCC (Bhandari et al. Biochemistry 40:9196-9206 (2001)). GCC:C8 binds to the protein kinase-like domain in the cytoplasmic portion of GCC.

## Definitions

Unless otherwise defined herein, scientific and technical terms used in connection with the present invention have the meanings that are commonly understood by those of ordinary skill in the art. Generally, nomenclature utilized in connection with, and techniques of, cell and tissue culture, molecular biology, and protein and oligo- or polynucleotide chemistry and hybridization described herein are those known in the art. GenBank or GenPept accession numbers and useful nucleic acid and peptide sequences can be found at the website maintained by the National Center for Biotechnological Information, Bethesda Md. Standard techniques are used for recombinant DNA, oligonucleotide synthesis, and tissue culture and transformation and transfection (e.g., electroporation, lipofection). Enzymatic reactions and purification techniques are performed according to manufacturer's specifications or as commonly accomplished in the art or as described herein. The foregoing techniques and procedures are generally performed according to methods known in the art, e.g., as described in various general and more specific references that are cited and discussed throughout the present specification. See e.g., Sambrook et al. Molecular Cloning: A Laboratory Manual (3rd ed., Cold Spring Harbor Laboratory Press, Cold Spring Harbor, N.Y. (2000)) or see generally, Harlow, E. and Lane, D. (1988) Antibodies: A Laboratory Manual, Cold Spring Harbor Laboratory Press, Cold Spring Harbor, N.Y. The nomenclatures utilized in connection with, and the laboratory procedures and techniques of, described herein are known in the art. Furthermore, unless otherwise required by context, singular terms shall include pluralities and plural terms shall include the singular.

As used herein, the term "antibody molecule" refers to an antibody, antibody peptide(s) or immunoglobulin, or an antigen binding fragment of any of the foregoing, e.g., of an antibody. Antibody molecules include single chain antibody molecules, e.g., scFv, see. e.g., Bird et al. (1988) Science 242:423-426; and Huston et al. (1988) Proc. Natl. Acad. Sci. USA 85:5879-5883), and single domain antibody molecules, see, e.g., WO9404678. Although not within the term "antibody molecules," the invention also includes "antibody analog(s)," other non-antibody molecule protein-based scaffolds, e.g., fusion proteins and/or immunoconjugates that use CDRs to provide specific antigen binding.

An "anti-GCC antibody molecule" refers to an antibody molecule (i.e., an antibody, antigen-binding fragment of an antibody or antibody analog) which interacts with or recognizes, e.g., binds (e.g., binds specifically) to GCC, e.g., human GCC. Exemplary anti-GCC antibody molecules are such as those summarized in Tables 1 and 2.

As used herein, the term "antibody," "antibody peptide(s)" or "immunoglobulin" refers to single chain, two-chain, and multi-chain proteins and glycoproteins. The term antibody includes polyclonal, monoclonal, chimeric, CDR-grafted and human or humanized antibodies, all of which are discussed in more detail elsewhere herein. Also included within the term are camelid antibodies, see, e.g., US2005/0037421, and nanobodies, e.g., IgNARs (shark antibodies), see, e.g., WO03/014161. The term "antibody" also includes synthetic and genetically engineered variants.

As used herein, the term "antibody fragment" or "antigen binding fragment" of an antibody refers, e.g., to Fab, Fab', $F(ab')_2$, and Fv fragments, single chain antibodies, functional heavy chain antibodies (nanobodies), as well as any portion of an antibody having specificity toward at least one desired epitope, that competes with the intact antibody for specific binding (e.g., a fragment having sufficient CDR sequences and having sufficient framework sequences so as to bind specifically to an epitope). E.g., an antigen binding fragment can compete for binding to an epitope which binds

the antibody from which the fragment was derived. Derived, as used in this and similar contexts, does not imply any particular method or process of derivation, but can refer merely to sequence similarity. Antigen binding fragments can be produced by recombinant techniques, or by enzymatic or chemical cleavage of an intact antibody. The term, antigen binding fragment, when used with a single chain, e.g., a heavy chain, of an antibody having a light and heavy chain means that the fragment of the chain is sufficient such that when paired with a complete variable region of the other chain, e.g., the light chain, it will allow binding of at least 25, 50, 75, 85 or 90% of that seen with the whole heavy and light variable region.

The term, "antigen binding constellation of CDRs" or "a number of CDRs sufficient to allow binding" (and similar language), as used herein, refers to sufficient CDRs of a chain, e.g., the heavy chain, such that when placed in a framework and paired with a complete variable region of the other chain, or with a portion of the other chain's variable region of similar length and having the same number of CDRs, e.g., the light chain, will allow binding, e.g., of at least 25, 50, 75, 85 or 90% of that seen with the whole heavy and light variable region.

As used herein, the term "humanized antibody" refers to an antibody that is derived from a non-human antibody e.g., rabbit, rodent (e.g., murine), sheep or goat, that retains or substantially retains the antigen-binding properties of the parent antibody but is less immunogenic in humans. Humanized as used herein is intended to include deimmunized antibodies. Typically, humanized antibodies include non-human CDRs and human or human derived framework and constant regions.

The term "modified" antibody, as used herein, refers to antibodies that are prepared, expressed, created or isolated by recombinant means, such as antibodies expressed using a recombinant expression vector transfected into a host cell, antibodies isolated from a recombinant, combinatorial antibody library, antibodies isolated from a non-human animal (e.g., a rabbit, mouse, rat, sheep or goat) that is transgenic for human immunoglobulin genes or antibodies prepared, expressed, created or isolated by any other means that involves splicing of human immunoglobulin gene sequences to other DNA sequences. Such modified antibodies include humanized, CDR grafted (e.g., an antibody having CDRs from a first antibody and a framework region from a different source, e.g., a second antibody or a consensus framework), chimeric, *in vitro* generated (e.g., by phage display) antibodies, and may optionally include variable or constant regions derived from human germline immunoglobulin sequences or human immunoglobulin genes or antibodies which have been prepared, expressed, created or isolated by any means that involves splicing of human immunoglobulin gene sequences to alternative immunoglobulin sequences. In embodiments a modified antibody molecule includes an antibody molecule having a sequence change from a reference antibody.

The term "monospecific antibody" refers to an antibody or antibody preparation that displays a single binding specificity and affinity for a particular epitope. This term includes a "monoclonal antibody" or "monoclonal antibody composition."

The term "bispecific antibody" or "bifunctional antibody" refers to an antibody that displays dual binding specificity for two epitopes, where each binding site differs and recognizes a different epitope.

The terms "non-conjugated antibody" and "naked antibody" are used interchangeably to refer to an antibody molecule that is not conjugated to a non-antibody moiety, e.g., an agent or a label.

Each of the terms "immunoconjugate", "antibody-drug conjugate" and "antibody conjugate", are used interchangeably and refer to an antibody that is conjugated to a non-antibody moiety, e.g., an agent or a label. The term "agent" is used herein to denote a chemical compound, a mixture of chemical compounds, a biological macromolecule, or an extract made from biological materials. The term "therapeutic agent" refers to an agent that has biological activity. Exemplary therapeutic agents are chemotherapeutic agents.

The term "anti-cancer agent" or "chemotherapeutic agent" is used herein to refer to agents that have the functional property of inhibiting a development or progression of a neoplasm in a human, particularly a malignant (cancerous) lesion, such as a carcinoma, sarcoma, lymphoma, or leukemia Inhibition of metastasis or angiogenesis is frequently a property of anti-cancer or chemotherapeutic agents. A chemotherapeutic agent may be a cytotoxic or cytostatic agent. The term "cytostatic agent" refers to an agent which inhibits or suppresses cell growth and/or multiplication of cells.

"Cytotoxic agents" refer to compounds which cause cell death primarily by interfering directly with the cell's functioning, including, but not limited to, alkylating agents, tumor necrosis factor inhibitors, intercalators, microtubule inhibitors, kinase inhibitors, proteasome inhibitors and topoisomerase inhibitors. A "toxic payload" as used herein refers to a sufficient amount of cytotoxic agent which, when delivered to a cell results in cell death. Delivery of a toxic payload may be accomplished by administration of a sufficient amount of immunoconjugate comprising an antibody or antigen binding fragment of the invention and a cytotoxic agent. Delivery of a toxic payload may also be accomplished by administration of a sufficient amount of an immunoconjugate comprising a cytotoxic agent, wherein the immunoconjugate comprises a secondary antibody or antigen binding fragment thereof which recognizes and binds an antibody or antigen binding fragment of the invention.

As used herein the phrase, a sequence "derived from" or "specific for a designated sequence" refers to a sequence that comprises a contiguous sequence of approximately at least 6 nucleotides or at least 2 amino acids, at

least about 9 nucleotides or at least 3 amino acids, at least about 10-12 nucleotides or 4 amino acids, or at least about 15-21 nucleotides or 5-7 amino acids corresponding, i.e., identical or complementary to, e.g., a contiguous region of the designated sequence. In certain embodiments, the sequence comprises all of a designated nucleotide or amino acid sequence. The sequence may be complementary (in the case of a polynucleotide sequence) or identical to a sequence region that is unique to a particular sequence as determined by techniques known in the art. Regions from which sequences may be derived, include but are not limited to, regions encoding specific epitopes, regions encoding CDRs, regions encoding framework sequences, regions encoding constant domain regions, regions encoding variable domain regions, as well as non-translated and/or non-transcribed regions. The derived sequence will not necessarily be derived physically from the sequence of interest under study, but may be generated in any manner, including, but not limited to, chemical synthesis, replication, reverse transcription or transcription, that is based on the information provided by the sequence of bases in the region(s) from which the polynucleotide is derived. As such, it may represent either a sense or an antisense orientation of the original polynucleotide. In addition, combinations of regions corresponding to that of the designated sequence may be modified or combined in ways known in the art to be consistent with the intended use. For example, a sequence may comprise two or more contiguous sequences which each comprise part of a designated sequence, and are interrupted with a region which is not identical to the designated sequence but is intended to represent a sequence derived from the designated sequence. With regard to antibody molecules, "derived therefrom" includes an antibody molecule which is functionally or structurally related to a comparison antibody, e.g., "derived therefrom" includes an antibody molecule having similar or substantially the same sequence or structure, e.g., having the same or similar CDRs, framework or variable regions. "Derived therefrom" for an antibody also includes residues, e.g., one or more, e.g., 2, 3, 4, 5, 6 or more residues, which may or may not be contiguous, but are defined or identified according to a numbering scheme or homology to general antibody structure or three-dimensional proximity, i.e., within a CDR or a framework region, of a comparison sequence. The term "derived therefrom" is not limited to physically derived therefrom but includes generation by any manner, e.g., by use of sequence information from a comparison antibody to design another antibody.

As used herein, the phrase "encoded by" refers to a nucleic acid sequence that codes for a polypeptide sequence, wherein the polypeptide sequence or a portion thereof contains an amino acid sequence of at least 3 to 5 amino acids, at least 8 to 10 amino acids, or at least 15 to 20 amino acids from a polypeptide encoded by the nucleic acid sequence.

As used herein, the term "colorectal cancer", also commonly known as colon cancer or bowel cancer, refers to cancer from uncontrolled cell growth in the colon or rectum (parts of the large intestine), or in the appendix.

The terms "stomach cancer" and "gastric cancer" are used herein interchangeably.

Calculationsof "homology" between two sequences can be performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The length of a reference sequence aligned for comparison purposes is at least 30%, 40%, or 50%, at least 60%, or at least 70%, 80%, 90%, 95%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent homology between two sequences can be accomplished using a mathematical algorithm. The percent homology between two amino acid sequences can be determined using any method known in the art. For example, the Needleman and Wunsch, J. Mol. Biol. 48:444-453 (1970), algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6. The percent homology between two nucleotide sequences can also be determined using the GAP program in the GCG software package (Accelerys, Inc. San Diego, Calif.), using an NWSgapdna.CMP matrix and a gap weight of 40, 50, 60, 70, or 80 and a length weight of 1, 2, 3, 4, 5, or 6. An exemplary set of parameters for determination of homology are a Blossum 62 scoring matrix with a gap penalty of 12, a gap extend penalty of 4, and a frameshift gap penalty of 5.

As used herein, the term "hybridizes under stringent conditions" describes conditions for hybridization and washing. Guidance for performing hybridization reactions can be found in Current Protocols in Molecular Biology, John Wiley & Sons, N.Y. (1989), 6.3.1-6.3.6. Aqueous and nonaqueous methods are described in that reference and either can be used. Specific hybridization conditions referred to herein are as follows: 1) low stringency hybridization conditions in 6X sodium chloride/sodium citrate (SSC) at about 45° C, followed by two washes in 0.2X SSC, 0.1% SDS at least at 50° C. (the temperature of the washes can be increased to 55° C for low stringency conditions); 2) medium stringency hybridization conditions in 6X SSC at about 45° C, followed by one or more washes in 0.2X SSC, 0.1% SDS at 60° C; 3) high stringency hybridization conditions in 6X SSC at about 45° C, followed by one or more washes in 0.2X SSC,

0.1% SDS at 65° C; and 4) very high stringency hybridization conditions are 0.5M sodium phosphate, 7% SDS at 65° C, followed by one or more washes at 0.2X SSC, 1% SDS at 65° C. Very high stringency conditions (4) are often the preferred conditions and the ones that should be used unless otherwise specified.

It is understood that the antibodies and antigen binding fragment thereof of the invention may have additional conservative or non-essential amino acid substitutions, which do not have a substantial effect on the polypeptide functions. Whether or not a particular substitution will be tolerated, i.e., will not adversely affect desired biological properties, such as binding activity, can be determined as described in Bowie, J U et al. Science 247:1306-1310 (1990) or Padlan et al. FASEB J. 9:133-139 (1995). A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., asparagine, glutamine, serine, threonine, tyrosine, cysteine), nonpolar side chains (e.g., glycine, alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

A "non-essential" amino acid residue is a residue that can be altered from the wild-type sequence of the binding agent, e.g., the antibody, without abolishing or, without substantially altering a biological activity, whereas an "essential" amino acid residue results in such a change. In an antibody, an essential amino acid residue can be a specificity determining residue (SDR).

As used herein, the term "isolated" refers to material that is removed from its original environment (e.g., the natural environment if it is naturally occurring). For example, a naturally occurring polynucleotide or polypeptide present in a living animal is not isolated, but the same polynucleotide or DNA or polypeptide, separated from some or all of the coexisting materials in the natural system, is isolated. Such polynucleotide or polypeptide could be part of a vector and/or such polynucleotide or polypeptide could be part of a composition, e.g., a mixture, solution or suspension or comprising an isolated cell or a cultured cell which comprises the polynucleotide or polypeptide, and still be isolated in that the vector or composition is not part of its natural environment.

As used herein, the term "replicon" refers to any genetic element, such as a plasmid, a chromosome or a virus, that behaves as an autonomous unit of polynucleotide replication within a cell.

As used herein, the term "operably linked" refers to a situation wherein the components described are in a relationship permitting them to function in their intended manner. Thus, for example, a control sequence "operably linked" to a coding sequence is ligated in such a manner that expression of the coding sequence is achieved under conditions compatible with the control sequence.

As used herein, the term "vector" refers to a replicon in which another polynucleotide segment is attached, such as to bring about the replication and/or expression of the attached segment.

As used herein, the term "control sequence" refers to a polynucleotide sequence that is necessary to effect the expression of a coding sequence to which it is ligated. The nature of such control sequences differs depending upon the host organism. In prokaryotes, such control sequences generally include a promoter, a ribosomal binding site and terminators and, in some instances, enhancers. The term "control sequence" thus is intended to include at a minimum all components whose presence is necessary for expression, and also may include additional components whose presence is advantageous, for example, leader sequences.

As used herein, the term "purified product" refers to a preparation of the product which has been isolated from the cellular constituents with which the product is normally associated and/or from other types of cells that may be present in the sample of interest.

As used herein, the term "epitope" refers to a protein determinate capable of binding specifically to an antibody. Epitopic determinants usually consist of chemically active surface groupings of molecules such as amino acids or sugar side chains and usually have specific three dimensional structural characteristics, as well as specific charge characteristics. Some epitopes are linear epitopes while others are conformational epitopes. A linear epitope is an epitope wherein a contiguous amino acid primary sequence comprises the epitope recognized. A linear epitope typically includes at least 3, and more usually, at least 5, for example, about 8 to about 10 contiguous amino acids. A conformational epitope can result from at least two situations, such as: a) a linear sequence which is only exposed to antibody binding in certain protein conformations, e.g., dependent on ligand binding, or dependent on modification (e.g., phosphorylation) by signaling molecules; or b) a combination of structural features from more than one part of the protein, or in multisubunit proteins, from more than one subunit, wherein the features are in sufficiently close proximity in 3-dimensional space to participate in binding.

As used herein, "isotype" refers to the antibody class (e.g., IgM, IgA, IgE or IgG) that is encoded by heavy chain constant region genes.

As used herein, the terms "detectable agent," "label" or "labeled" are used to refer to incorporation of a detectable marker on a polypeptide or glycoprotein. Various methods of labeling polypeptides and glycoproteins are known in the art and may be used. Examples of labels for polypeptides include, but are not limited to, the following: radioisotopes or

radionuclides (e.g., indium ($^{111}In$), iodine ($^{131}I$ or $^{125}I$), yttrium ($^{90}Y$), lutetium ($^{177}Lu$), actinium ($^{225}Ac$), bismuth ($^{212}Bi$ or $^{213}Bi$), sulfur ($^{35}S$), carbon ($^{14}C$), tritium ($^{3}H$), rhodium ($^{188}Rh$), technetium ($^{99}$ mTc), praseodymium, or phosphorous ($^{32}P$) or a positron-emitting radionuclide, e.g., carbon-11 ($^{11}C$), potassium-40 ($^{40}K$), nitrogen-13 ($^{13}N$), oxygen-15 ($^{15}O)_9$, fluorine-18 ($^{18}F$), gallium-68 ($^{68}Ga$), and iodine-121 ($^{121}I$))), fluorescent labels (e.g., FITC, rhodamine, lanthanide phosphors), enzymatic labels (e.g., horseradish peroxidase, beta-galactosidase, luciferase, alkaline phosphatase), chemiluminescent, biotinyl groups (which can be detected by a marked avidin, e.g., a molecule containing a streptavidin moiety and a fluorescent marker or an enzymatic activity that can be detected by optical or calorimetric methods), and predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

As used herein, "specific binding," "bind(s) specifically" or "binding specificity" means, for an anti-GCC antibody molecule, that the antibody molecule binds to GCC, e.g., human GCC protein, with greater affinity than it does to a non-GCC protein, e.g., BSA. Typically an anti-GCC molecule will have a $K_d$ for the non-GCC protein, e.g., BSA, which is greater than 2, greater than 10, greater than 100, greater than 1,000 times, greater than $10^4$, greater than $10^5$, or greater than $10^6$ times its $K_d$ for GCC, e.g., human GCC protein. In determination of $K_d$, the K $K_d$ for GCC and the non-GCC protein, e.g., BSA, should be done under the same conditions.

The term "affinity" or "binding affinity" refers to the apparent association constant or $K_a$. The $K_a$ is the reciprocal of the dissociation constant ($K_d$). An antibody may, for example, have a binding affinity of at least $10^5$, $10^6$, $10^7$, $10^8$, $10^9$, $10^{10}$ and $10^{11}$ $M^{-1}$ for a particular target molecule. Higher affinity binding of an antibody to a first target relative to a second target can be indicated by a higher $K_A$ (or a smaller numerical value $K_D$) for binding the first target than the $K_A$ (or numerical value $K_D$) for binding the second target. In such cases, the antibody has specificity for the first target (e.g., a protein in a first conformation or mimic thereof) relative to the second target (e.g., the same protein in a second conformation or mimic thereof; or a second protein). Differences in binding affinity (e.g., for specificity or other comparisons) can be at least 1.5, 2, 3, 4, 5, 10, 15, 20, 37.5, 50, 70, 80, 91, 100, 500, 1000, or $10^5$ fold.

Binding affinity can be determined by a variety of methods including equilibrium dialysis, equilibrium binding, gel filtration, ELISA, surface 19act cc resonance, or spectroscopy (e.g., using a fluorescence assay). For example, relative affinity of an anti-GCC antibody molecule can be measured from ELISA measurements against GCC protein (e.g., the immunogen used to raise anti-GCC antibody molecules), by FACS measurements with GCC expressing cells.

Exemplary conditions for evaluating binding affinity are in TRIS-buffer (50mM TRIS, 150mM NaCl, 5mM $CaCl_2$ at pH7.5). These techniques can be used to measure the concentration of bound and free binding protein as a function of binding protein (or target) concentration. The concentration of bound binding protein ([Bound]) is related to the concentration of free binding protein ([Free]) and the concentration of binding sites for the binding protein on the target where (N) is the number of binding sites per target molecule by the following equation:

$$[\mathrm{Bound}] = \mathrm{N} \cdot [\mathrm{Free}]/((1/K_A) + [\mathrm{Free}]).$$

It is not always necessary to make an exact determination of $K_A$, though, since sometimes it is sufficient to obtain a quantitative measurement of affinity, e.g., determined using a method such as ELISA or FACS analysis, is proportional to $K_A$, and thus can be used for comparisons, such as determining whether a higher affinity is, e.g., 2-fold higher, to obtain a qualitative measurement of affinity, or to obtain an inference of affinity, e.g., by activity in a functional assay, e.g., an *in vitro* or *in vivo* assay. Affinity of anti-GCC antibody molecules can also be measured using a technology such as real-time Biomolecular Interaction Analysis (BIA) (see, e.g., Sjolander, S, and Urbaniczky, C., 1991, Anal. Chem. 63:2338-2345 and Szabo et al., 1995, Curr. Opin. Struct. Biol. 5:699-705). As used herein, "BIA" or "surface plasmon resonance" is a technology for studying biospecific interactions in real time, without labeling any of the interactants (e.g., BIACORE™). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

The measurement of affinity of anti-GCC antibody molecules using a BIACORE™ T100 system (GE Healthcare, Piscataway, N.J.) is described in Example 1 of U.S. Patent Application Publication No. US2011/0110936. Briefly, an anti-GCC antibody (Prep A) is diluted to an appropriate concentration (e.g., 20 μg/mL) 10 mM sodium acetate, pH 4.0 and a referenc/control antibody (Prep B) is diluted to an appropriate concentration (e.g., 10 μg/mL) in 10 mM sodium acetate, pH 4.0. Each antibody is covalently immobilized to several CM4 BIACORE chips using standard amine coupling. For each CM4 chip prepared, Prep A antibody is immobilized over two flow cells at around 75-100 RU while Prep B antibody is immobilized to one flow cell at around 70-80 RU. The remaining fourth flow cell of each CM4 chip is used as the reference flow cell. The concentration of GCC protein can be determined using the methods detailed by Pace et al. in Protein Science, 4:2411 (1995), and Pace and Grimsley in Current Protocols in Protein Science 3.1.1-3.1.9 (2003). For each prepared CM4 chip, GCC protein is injected for 2 minutes at a concentration range of 202 nM -1.6 nM (2.times.

serial dilution) followed by a 7 minute dissociation. Samples are randomly injected in triplicate with several buffer inject cycles interspersed for double referencing. To obtain more significant off-rate decay data, three additional 101 nM GCC protein injections and three additional buffer injections are performed with a 2 minute injection and a 4 hour dissociation time. A flow rate of 100 μL/min is used for all experiments and all surfaces are regenerated with a 20 second pulse of 10 mM Glycine-HCl (pH 2.0). All samples are prepared in the running buffer (e.g., Hepes-buffered saline, 0.005% polysorbate 20, pH 7.4 (HBS-P)) with 100 μg/mL of BSA added. All sensorgram (plot of surface plasmon resonance vs time) data can be processed with Scrubber 2.0 software (BioLogic Software, Campbell, Australia) and globally fit to a 1:1 interaction model including a term for the mass transport constant $k_m$ using CLAMP™ software (Myszka and Morton Trends Biochem. Sci. 23:149-150 (1998)).

As used herein, the term "treat" or "treatment" is defined as the administration of an anti-GCC antibody molecule to a subject, e.g., a patient, or administration, e.g., by application, to an isolated tissue or cell from a subject which is returned to the subject. The anti-GCC antibody molecule can be administered alone or in combination with a second agent. The treatment can be to cure, heal, alleviate, relieve, alter, remedy, ameliorate, palliate, improve or affect the disorder, the symptoms of the disorder or the predisposition toward the disorder, e.g., a cancer. While not wishing to be bound by theory, treating is believed to cause the inhibition, ablation, or killing of a cell *in vitro* or *in vivo,* or otherwise reducing capacity of a cell, e.g., an aberrant cell, to mediate a disorder, e.g., a disorder as described herein (e.g., a cancer).

As used herein, the term "subject" is intended to include mammals, primates, humans and non-human animals. For example, a subject can be a patient (e.g., a human patient or a veterinary patient), having a cancer in which at least some of the cells express GCC, such as a cancer of gastrointestinal origin (e.g., colorectal cancer, stomach cancer, small intestine cancer, or esophageal cancer), pancreatic cancer, lung cancer (e.g., squamous cell carcinoma, adenosquamous carcinoma, adenocarcinoma), soft-tissue sarcoma (e.g., leiosacroma or rhabdomyosarcoma), neuroendocrine tumors (e.g., gastrointestinal or bronchopulmonary), or neuroectodermal tumors; a symptom of such GCC expressing cancers; or a predisposition toward such GCC-expressing cancers. As another example, the subject can be a patient having a gastrointestinal disorder in which at least some of the cells of the gastrointestinal system express GCC, such in as inflammatory bowel syndrome, Crohn's disease and constipation. As yet another example, the subject can be a patient having a neurological disorder in which at least some neurons within the patients central nervous system express GCC, such as Parkinson's Disease. The term "non-human animals" of the invention includes all non-human vertebrates, e.g., non-human mammals and non-mammals, such as non-human primates, sheep, dog, cow, chickens, amphibians, reptiles, mouse, rat, rabbit or goat etc., unless otherwise noted. In an embodiment, "subject" excludes one or more or all of a mouse, rat, rabbit or goat.

As used herein, an amount of an anti-GCC antibody molecule "effective" or "sufficient" to treat a disorder, or a "therapeutically effective amount" or "therapeutically sufficient amount" refers to an amount of the antibody molecule which is effective, upon single or multiple dose administration to a subject, in treating a cell, e.g., cancer cell (e.g., a GCC-expressing tumor cell), or in prolonging curing, alleviating, relieving or improving a subject with a disorder as described herein beyond that expected in the absence of such treatment. As used herein, "inhibiting the growth" of the tumor or cancer refers to slowing, interrupting, arresting or stopping its growth and/or metastases and does not necessarily indicate a total elimination of the tumor growth.

As used herein, "GCC," also known as "STAR", "GUC2C", "GUCY2C" or "ST receptor" protein refers to mammalian GCC, preferably human GCC protein. Human GCC refers to the protein shown in SEQ ID NO: 3 and naturally occurring allelic protein variants thereof. The allele in SEQ ID NO: 3 can be encoded by the nucleic acid sequence of GCC shown in SEQ ID NO: 2. Other variants are known in the art. See, e.g., accession number Ensp0000261170, Ensembl Database, European Bioinformatics Institute and Wellcome Trust Sanger Institute, which has a leucine at residue 281; SEQ ID NO: 14 of published US patent application number 20060035852; or GenBank accession number AAB 19934. Typically, a naturally occurring allelic variant has an amino acid sequence at least 95%, 97% or 99% identical to the GCC sequence of SEQ ID NO: 3. The transcript encodes a protein product of 1073 amino acids, and is described in GenBank accession no.: NM_004963. GCC protein is characterized as a transmembrane cell surface receptor protein, and is believed to play a critical role in the maintenance of intestinal fluid, electrolyte homeostasis and cell proliferation.

## Anti-GCC Antibodies

Described herein are anti-GCC antibody molecules useful, *inter alia* to detect GCC expression. The anti-GCC antibody molecules, e.g., useful for GCC detection, can include non-human anti-GCC antibody molecules (e.g., non-human and non-murine antibody molecules) that specifically bind to GCC, e.g., with a binding affinity at least $10^3$, $10^4$, $10^5$, $10^6$, $10^7$,$10^8$, $10^9$, $10^{10}$ and $10^{11}$ $M^{-1}$ for GCC. The anti-GCC antibody molecule can be a non-human, non-murine and non-rat antibody molecule, e.g., a rabbit anti-GCC antibody molecule, e.g., as described herein.

In certain aspects, the disclosure relates to anti-GCC antibody molecules that include features such as those summarized in Tables 1 and 2. In other aspects, the disclosure relates to anti-GCC antibody molecules that include features such as those summarized in Tables 3, 4, 5 and/or 6.

In the present disclosure, the anti-GCC antibody molecule is a rabbit hybridoma antibody and is one of antibody MIL-44-148-2 or MIL-44-67-4. In the present disclosure, the anti-GCC antibody molecule is derived from antibody MIL-44-148-2 or MIL-44-67-4.

In an embodiment of the invention an anti-GCC antibody molecule will have an affinity for GCC, e.g., as measured by direct binding or competition binding assays. In the present disclosure the anti-GCC antibody molecule has a $K_d$ of less than $1x10^{-6}$ M, less than $1x10^{-7}$ M, less than $1x10^{-8}$ M, less than $1x10^{-9}$ M, less than $1x10^{-10}$ M, less than $1x10^{-11}$ M, less than $1x10^{-12\ 12}$ M, or less than $1x10^{-13}$ M. In the present disclosure the antibody molecule is an IgG, or antigen-binding fragment thereof, and has a $K_d$ of less than $1x10^{-6}$ M, less than $1x10^{-7}$ M, less than $1x10^{-8}$ M, or less than $1x10^{-9}$ M. In the present disclosure, an anti-GCC antibody molecule, e.g., a MIL-44-148-2 antibody or antibody derived therefrom has a $K_d$ of about 80 to about 200 pM, preferably about 100 to about 150 pM or about 120 pM. In the present disclosure, an anti-GCC antibody molecule, e.g., a MIL-44-148-2 antibody or antibody derived therefrom has a $k_a$ of about 0.9 to about $1.25x10^5$ $M^{-1}$ $s^{-1}$, preferably about $1.1x10^5$ $M^{-1}$ $s^{-1}$. In the present disclosure the antibody molecule is an ScFv and has a $K_d$ of less than $1x10^{-6}$ M, less than $1x10^{-7}$ M, less than $1x10^{-8}$ M, less than $1x10^{-9}$ M, less than $1x10^{-10}$ M, $1x10^{-11}$ M, less than $1x10^{-12}$ M, or less than $1x10^{-13}$ M.

In the present disclosure, the antibody molecules are not immunoconjugates, i.e., are "naked" and in the present disclosurecause a cellular reaction upon binding to GCC. In related the present disclosure, the cellular reaction is performed by the GCC-expressing cell to which the antibody binds. Such a cellular reaction can be signal transduction mediated by GCC, e.g., if the antibody molecule is an agonist of GCC (see, e.g., US Patent Application publication no. US20040258687). In the present disclosure, the cellular reaction is performed by a second cell, e.g., an immune effector cell (e.g., a natural killer cell) which recognizes the antibody molecule bound to GCC on the first cell. In the present disclosure, surveillance molecules, e.g., complement molecules, contact the GCC-bound antibody molecule prior to the cellular reaction. The cellular reactions in the disclosure can cause death of the GCC-expressing cell.

In the present disclosure, antibody molecules which are immunoconjugates can both cause a cellular reaction upon binding to GCC and internalize to deliver an agent to the GCC-expressing cell to which it binds.

In the present disclosure, an anti-GCC antibody molecule of the disclosure can block ligand binding to GCC.

In the present disclosure, the antibody molecule is not GCC:B10, GCC:4D7 or GCC:C8. In the present disclosure, an anti-GCC antibody molecule does not bind an intracellular domain of GCC, about amino acid residue 455 to 1073 of SEQ ID NO: 3. For example, in the present disclosure, an anti-GCC antibody molecule does not bind the kinase homology domain or the guanylyl cyclase domain of GCC.

The naturally occurring mammalian antibody structural unit is typified by a tetramer. Each tetramer is composed of two pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains can be classified as kappa and lambda light chains. Heavy chains can be classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989)). The variable regions of each light/heavy chain pair form the antibody binding site. Preferred isotypes for the anti-GCC antibody molecules are IgG immunoglobulins, which can be classified into four subclasses, IgG1, IgG2, IgG3 and IgG4, having different gamma heavy chains. Most therapeutic antibodies are human, chimeric, or humanized antibodies of the IgG1 isotype. In the present disclosure, the anti-GCC antibody molecule is a rabbit IgG antibody.

The variable regions of each heavy and light chain pair form the antigen binding site. Thus, an intact IgG antibody has two binding sites which are the same. However, bifunctional or bispecific antibodies are artificial hybrid constructs which have two different heavy/light chain pairs, resulting in two different binding sites.

The chains all exhibit the same general structure of relatively conserved framework regions (FR) joined by three hypervariable regions, also called complementarity determining regions or CDRs. The CDRs from the two chains of each pair are aligned by the framework regions, enabling binding to a specific epitope. From N-terminal to C-terminal, both light and heavy chains comprise the domains FR1, CDR1, FR2, CDR2, FR3, CDR3 and FR4. The assignment of amino acids to each domain is in accordance with the definitions of Kabat Sequences of Proteins of Immunological Interest (National Institutes of Health, Bethesda, Md. (1987 and 1991)), or Chothia & Lesk J. Mol. Biol. 196:901-917 (1987); Chothia et al. Nature 342:878-883 (1989). As used herein, CDRs are referred to for each of the heavy (HCDR1, HCDR2, HCDR3) and light (LCDR1, LCDR2, LCDR3) chains.

An anti-GCC antibody molecule can comprise all, or an antigen binding subset of the CDRs, of one or both, the heavy and light chain, of one of the above-referenced rabbit antibodies. Amino acid sequences of rabbit hybridoma antibodies, including variable regions and CDRs, can be found in Table 3 and Table 5.

Thus, the present disclosure the antibody molecule includes one or both of:

(a) one, two, three, or an antigen binding number of, light chain CDRs (LCDR1, LCDR2 and/or LCDR3) of one of the above-referenced rabbit hybridoma antibodies. In the present disclosure the CDR(s) may comprise an amino acid sequence of one or more or all of LCDR1-3 as follows: LCDR1, or modified LCDR1 wherein one to seven amino acids are conservatively substituted) LCDR2, or modified LCDR2 wherein one or two amino acids are conservatively substituted); or LCDR3, or modified LCDR3 wherein one or two amino acids are conservatively substituted; and

(b) one, two, three, or an antigen binding number of, heavy chain CDRs (HCDR1, HCDR2 and/or HCDR3) of one of the above-referenced rabbit hybridoma antibodies. In the present disclosure the CDR(s) may comprise an amino acid sequence of one or more or all of HCDR1-3 as follows: HCDR1, or modified HCDR1 wherein one or two amino acids are conservatively substituted; HCDR2, or modified HCDR2 wherein one to four amino acids are conservatively substituted; or HCDR3, or modified HCDR3 wherein one or two amino acids are conservatively substituted.

Useful immunogens for production of anti-GCC antibody molecules include GCC e.g., human GCC-expressing cells (e.g., a tumor cell line, e.g., T84 cells, or fresh or frozen colon tumor cells, recombinant cells expressing GCC); membrane fractions of GCC-expressing cells (e.g., a colon tumor cell line, e.g., T84 cells), or fresh or frozen colonic tumor cells; recombinant cells expressing GCC; isolated or purified GCC, e.g., human GCC protein (e.g., biochemically isolated GCC, e.g., isolated from gastrointestinal tumor cells or recombinant cells expressing GCC or a variant thereof), or a portion thereof (e.g., the extracellular domain of GCC, the kinase homology domain of GCC or the guanylyl cyclase catalytic domain of GCC or peptide corresponding to a portion thereof, e.g., comprising at least about 8, 10, 12, 14, 16, 20, 24, 28 or 32 amino acid residues of SEQ ID NO: 3); or an immunogen comprising SEQ ID NO: 46 or comprising a mature portion thereof without the signal sequence (i.e., without amino acid residues 1 to about 21 or 23 of SEQ ID NO: 46), e.g., the mature hGCC(ECD)-mIgG2a FcR r-mutII (also referred to herein as "pLKTOK108") protein, SEQ ID NO: 48.

Immunogens can be fused to heterologous sequences to aid in biochemical manipulation, purification, immunization or antibody titer measurement. Such immunogens can comprise a portion of GCC, e.g., the extracellular domain, and a portion comprising a non-GCC polypeptide. Many options exist for constructing a fusion protein for ease of purification or immobilization onto a solid support, e.g., an affinity column or a microtiter plate or other suitable assay substrate/chip. For example, a fusion moiety can add a domain, e.g., glutathione-S-transferase/kinase (GST), which can bind glutathione; an Fc region of an immunoglobulin, which can bind to protein A or protein G; amino acid residues, e.g., two, three, four, five, preferably six histidine residues which can bind nickel or cobalt on an affinity column; an epitope tag, e.g., a portion of c-myc oncogene (myc-tag), a FLAG tag (U.S. Pat. No. 4,703,004), a hemagglutinin (HA) tag, a T7 gene 10 tag, a V5 tag, an HSV tag, or a VSV-G tag which can bind a tag-specific antibody; or a cofactor, e.g., biotin, which can bind streptavidin.

Immunogens which comprise the Fc portion of an immunoglobulin can hold the GCC, either in solution or attached to a cell, in a configuration which allows structural access to GCC epitopes by the host immune surveillance components for efficient antibody generation. Because immunoglobulin heavy chains comprising the Fc regions associate into dimers through interchain disulfide bonds, immunogens resulting from fusion with Fc regions are dimers. Valency of fusion proteins can reflect the type of immunoglobulin contributing an Fc region. For example, fusions with IgG proteins can be dimers, IgA fusions can make tetrameric immunogens, and IgM fusions can make decameric immunogens, the latter two is facilitated with co-transfection of the J chain. An exemplary immunoglobulin for an Fc fusion protein is IgG1. The portion used typically has the IgG1 hinge, CH2 and CH3 domains encoded by a single exon. Because this exon also has a portion of the CH1 region, which has a cysteine oriented to disulfide bond with a cysteine from the light chain, a useful modification is to mutate the CH1 cysteine, e.g., to a serine, to ensure there is no unpaired cysteine in the fusion protein. Such a mutation also increases flexibility of the hinge.

An Fc portion derived from a non-host species, e.g., human Ig Fc region, for fusing to an immunogen for immunization in a host species, e.g., mouse, rat, rabbit, goat, acts as an adjuvant. This adjuvant function can trigger specific antibodies against both Fc and GCC epitopes. Fc-reactive antibodies can be identified and discarded during screening. The Fc portion can have a wild type sequence or a sequence which is mutated to modify effector function. For example, a mutated constant region (variant) can be incorporated into a fusion protein to minimize binding to Fc receptors and/or ability to fix complement (see e.g. Winter et al, GB 2,209,757 B; Morrison et al., WO 89/07142; Morgan et al., WO 94/29351). In a preferred example, lysine 235 and glycine 237, numbered according to Fc region standards, are mutated, e.g., to alanine. An immunogen/fusion protein with Fc-mutated IgG can have reduced interaction with Fc receptors in the host. A preferred soluble immunogen fusion protein (after maturation to cleave the signal peptide and secretion) is hGCC(ECD)-mIgG2a FcR r-mutII (pLKTOK108), which consists of amino acid residues 24 to 430 of SEQ ID NO: 3 fused to mutated mouse IgG2a immunoglobulin Fc (collectively SEQ ID NO:48).

To prepare a cell-expressed immunogen, the immunoglobulin portion can be structured to mimic an immunoglobulin portion of the B cell receptor. For example, the immunoglobulin Fc region can be further fused to a polypeptide comprising a transmembrane region from an immune receptor, such as Fcγ receptors, Fcα receptors, Fcα/μ receptor or Fcε receptors. Proper orientation of such an Fc receptor cell-bound immunogen with adequate exposure on the cell

surface may be improved if the cell expressing the immunogen fusion protein further comprises additional components of the antigen receptor complex, e.g., B cell IgM receptor or IgD receptor. Suitable components of the complex include immunoglobulin (Ig) sheath proteins, such as MB-1 and B29 (CD79A and CD79B; Hombach et al. Eur. J. Immunol. 20:2795-2799 (1990) for IgM receptor), which form a heterodimer. The Ig sheath proteins can be provided endogenously by the transfected cell, e.g., if transfecting a B cell lymphoma cell line; or by co-transfection of the immunogen with sheath proteins, e.g., in a separate vector or in the same vector.

Useful epitopes, e.g., reference epitopes, from the GCC molecule, to which the anti-GCC antibody molecules, e.g., rabbit monoclonal antibodies, or humanized versions thereof, as described herein, can bind, can be found on the extracellular portion of GCC. Such GCC epitopes can bind antibody molecules on the surface of cells, e.g., on the cell exterior.

For example, an epitope for an anti-GCC antibody molecule can reside within, or include a residue(s) from, residues 1-50 of SEQ ID NO: 3, or a fragment thereof that binds an anti-GCC antibody molecule of the disclosure, e.g., a MIL-44-148-2-binding fragment thereof. Such fragments can comprise residues 1-25, 5-30, 10-35, 15-40, 20-45, 25-50, 5-45, 10-40, 15-35, 20-30 or 33-50 of SEQ ID NO: 3. In the present disclosure, an epitope for an anti-GCC antibody molecule, e.g., a MIL-44-148-2 antibody, is a conformational epitope further comprising one or more additional amino acid residues in the GCC amino acid sequence beyond residue 50, i.e., selected from about residue 50 to 1073 of SEQ ID NO: 3.

Antibodies raised against such epitopes or the extracellular domain, e.g., epitopes that reside within, or include a residue(s) from amino acid residues 24 to 420 of SEQ ID NO: 3, or a reference portion thereof, e.g., residues 24 to 75, 75 to 150, 150 to 225, 225 to 300, 300 to 375 or 375 to 420 of GCC, or antibody molecules derived therefrom, can be useful as therapeutic or diagnostic antibodies, as described herein.

In the present disclosure, the anti-GCC antibody molecule has one or more of the following properties:

a) it competes for binding, e.g., binding to cell surface GCC or purified GCC, with one of the above-referenced anti-GCC antibody molecules summarized in Tables 1 and 2 e.g., rabbit hybridoma antibodies (e.g., MIL-44-148-2);

b) it binds to the same, or substantially the same, epitope on GCC as one of the above-referenced anti-GCC antibody molecules summarized in Tables 1 and 2, e.g., e.g., rabbit hybridoma antibodies (e.g., MIL-44-148-2). In the disclosure, the antibody binds the same epitope, as determined by one or more of a peptide array assay or by binding to truncation mutants, chimeras or point mutants expressed on the cell surface or membrane preparations, e.g., as those assays are described herein;

c) it binds to an epitope which has at least 1, 2, 3, 4, 5, 8, 10, 15 or 20 contiguous amino acid residues in common with the epitope of one of the above-referenced anti-GCC antibody molecules summarized in Tables 1 and 2, e.g., rabbit hybridoma antibodies (e.g., MIL-44-148-2);

d) it binds a region of human GCC that is bound by an anti-GCC antibody of the invention, wherein the region e.g., an extracellular or cytoplasmic region, is 10-15, 10-20, 20-30, or 20-40 residues in length, and binding is determined, e.g., by binding to truncation mutants; In the present disclosure the anti-GCC antibody molecule binds the extracellular region of human GCC. In the present disclosure an anti-GCC antibody molecule can bind the human GCC portion of the extracellular domain defined by amino acid residues 24 to 420 of SEQ ID NO: 3. In the present disclosure an anti-GCC antibody molecule can bind the guanylate cyclase signature site at amino acid residues 931 to 954 of SEQ ID NO: 3; or

e) it binds to a reference epitope described herein.

In the present disclosure the anti-GCC antibody molecule binds the GCC sequence ILVDLFNDQYFEDNVTAPDYMKNVLVLTLS (SEQ ID NO: 8).

In the present disclosure the anti-GCC antibody molecule binds the GCC sequence FAHAFRNLTFEGYDGPVTLDDWGDV (SEQ ID NO: 9).

In the present disclosure the antibody molecule binds a conformational epitope. In other the present disclosure an antibody molecule binds a linear epitope.

The anti-GCC antibody molecules can be polyclonal antibodies, monoclonal antibodies, monospecific antibodies, chimeric antibodies (See U.S. Pat. No. 6,020,153) or humanized antibodies or antibody fragments or derivatives thereof. Synthetic and genetically engineered variants (See U.S. Pat. No. 6,331,415) of any of the foregoing are also contemplated by the present disclosure. Monoclonal antibodies can be produced by a variety of techniques, including conventional murine monoclonal antibody methodology (e.g., the standard somatic cell hybridization technique of Kohler and Milstein, Nature 256: 495 (1975); see generally, Harlow, E. and Lane, D. (1988) Antibodies: A Laboratory Manual,

Cold Spring Harbor Laboratory Press, Cold Spring Harbor, N.Y), and the rabbit monoclonal antibody technology and services provided by Epitomics (Burlingame, CA) which produces custom rabbit monoclonal antibodies (RabMAbs®) using rabbit-rabbit hybridomas generated by fusing isolated B-cells from an immunized rabbit with Epitomics' proprietary fusion partner cell line, as described in U.S. Patents 7,402,409, 7,429,487, 7,462,697, 7,575,896, 7,732,168, and 8,062,867.

Immunization with protein, e.g., GCC or a soluble portion, or fusion protein comprising a portion of GCC (e.g., hGCC(ECD)-mIgG2a FcRbr-mutII (pLKTOK108), or cells or membrane fractions therefrom, e.g., cells expressing surface-exposed GCC or a portion thereof (e.g., the pLKTOK4 product), can be performed with the immunogen prepared for injection in a manner to induce a response, e.g., with adjuvant, e.g., complete Freund's adjuvant. Other suitable adjuvants include, Titermax Gold® adjuvant (CYTRX Corporation, Los Angeles, Calif.) and alum. Small peptide immunogens can be linked to a larger molecule, such as keyhole limpet hemocyanin. Mice or rabbits can be injected in a number of manners, e.g., subcutaneous, intravenous or intramuscular at a number of sites, e.g., in the peritoneum (i.p.), base of the tail, or foot pad, or a combination of sites, e.g., iP and base of tail (BIP). Booster injections can include the same or a different immunogen and can additionally include adjuvant, e.g., incomplete Freund's adjuvant. Immunization with DNA, e.g., DNA encoding GCC or a portion thereof or fusion protein comprising GCC or a portion thereof (e.g., encoding hGCC(ECD)-mIgG2a FcRbr-mutII) can be injected using gene gun technology. For example, DNA is loaded onto microscopic gold particles and injected into mice or rabbits at frequent intervals over a brief period.

Generally, where a monoclonal antibody is desired, a hybridoma is produced by fusing a suitable cell from an immortal cell line (e.g., a myeloma cell line such as SP2/0, P3X63Ag8.653 or a heteromyeloma) with antibody-producing cells. Antibody-producing cells can be obtained from the peripheral blood or, preferably the spleen or lymph nodes, of humans, human-antibody transgenic animals or other suitable animals (e.g., rabbits) immunized with the antigen of interest. Cells that produce antibodies of human origin (e.g., a human antibody) can be produced using suitable methods, for example, fusion of a human antibody-producing cell and a heteromyeloma or trioma, or immortalization of an activated human B cell via infection with Epstein Barr virus. (See, e.g., U.S. Pat. No. 6,197,582 (Trakht); Niedbala et al., Hybridoma, 17:299-304 (1998); Zanella et al., J Immunol Methods, 156:205-215 (1992); Gustafsson et al., Hum Antibodies Hybridomas, 2:26-32 (1991).) The fused or immortalized antibody-producing cells (hybridomas) can be isolated using selective culture conditions, and cloned by limiting dilution. Cells which produce antibodies with the desired specificity can be identified using a suitable assay (e.g., ELISA (e.g., with immunogen, e.g., hGCC(ECD)-mIgG2a FcRbr-mutII, immobilized on the microtiter well) or by FACS on a cell expressing GCC or a portion thereof, e.g., a cell expressing the pLKTOK4 product). For example, if the GCC-immunogen comprises a fusion moiety that is an affinity reagent, this moiety can allow the fusion protein comprising GCC or a portion thereof to be bound to a matrix, e.g., protein G-coated, streptavidin-coated, glutathione-derivatized or antibody-coated microtitre plates or assay chips, which are then combined with the immune serum or conditioned medium from a hybridoma or antibody-expressing recombinant cell, and the mixture incubated under conditions conducive to complex formation (e.g., at physiological conditions for salt and pH). Following incubation, the microtitre plate wells or chip cells are washed to remove any unbound components and binding by anti-GCC antibody is measured.

In the present disclosure, for therapeutic applications, the antibodies of the present disclosure are humanized antibodies. The advantage of humanized antibodies is that they potentially decrease or eliminate the immunogenicity of the antibody in a host recipient, thereby permitting an increase in the bioavailability and a reduction in the possibility of adverse immune reaction, thus potentially enabling multiple antibody administrations.

Modified antibodies include humanized, chimeric or CDR-grafted antibodies. Human anti-mouse antibody (HAMA) responses have led to development of chimeric or otherwise humanized antibodies. While chimeric antibodies have a human constant region and a non-human variable region, it is expected that certain human anti-chimeric antibody (HACA) responses will be observed, particularly in chronic or multi-dose utilizations of the antibody. The presence of such non-human (e.g., murine, rat, rabbit, sheep or goat) derived proteins can lead to the rapid clearance of the antibodies or can lead to the generation of an immune response against the antibody by a patient. In order to avoid the utilization of non-human derived antibodies, humanized antibodies where sequences are introduced to an antibody sequence to make it closer to human antibody sequence, or fully human antibodies generated by the introduction of human antibody function into a non-human species, such as a mouse, rat, rabbit, sheep or goat, have been developed so that the non-human species would produce antibodies having fully human sequences. Human antibodies avoid certain of the problems associated with antibodies that possess rabbit, rodent, sheep or goat variable and/or constant regions.

**Humanization and Display Technologies and Modifications to Antibodies**

Humanized antibody molecules can minimize the immunogenic and allergic responses intrinsic to non-human or non-human-derivatized mAbs and thus to increase the efficacy and safety of the administered antibodies. The use of humanized antibody molecules can provide a substantial advantage in the treatment of chronic and recurring human diseases, such as inflammation, autoimmunity, and cancer, which require repeated antibody administrations.

The production of humanized antibodies with reduced immunogenicity can be accomplished in connection with techniques of humanization and display techniques using appropriate libraries. It will be appreciated that antibodies from non-human species, such as mice, rats, rabbits, sheep, goats, etc., can be humanized or primatized using techniques known in the art. See e.g., Winter and Harris Immunol Today 14:43-46 (1993) and Wright et al. Crit. Reviews in Immunol. 12125-168 (1992). The antibody of interest may be engineered by recombinant DNA techniques to substitute the CH1, CH2, CH3, hinge domains, and/or the framework domain with the corresponding human sequence (see WO 92/02190 and U.S. Pat. Nos. 5,530,101, 5,585,089, 5,693,761, 5,693,792, 5,714,350, and 5,777,085). Also, the use of Ig cDNA for construction of chimeric immunoglobulin genes is known in the art (Liu et al. Proc Natl Acad Sci USA. 84:3439 (1987) and J. Immunol. 139:3521 (1987)). mRNA is isolated from a hybridoma or other cell producing the antibody and used to produce cDNA: The cDNA of interest may be amplified by the polymerase chain reaction using specific primers (U.S. Pat. Nos. 4,683,195 and 4,683,202).

Alternatively, phage display technology (see, e.g., McCafferty et al, Nature, 348:552-553 (1990)) can be used to produce human antibodies or antibodies from other species, as well as antibody fragments *in vitro,* from immunoglobulin variable (V) domain genes, e.g., from repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review see, e.g., Johnson and Chiswell, Current Opinion in Structural Biology, 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol., 222:581-597 (1991), or Griffith et al, EMBO J., 12:725-734 (1993). See, also, U.S. Pat. Nos. 5,565,332 and 5,573,905. Display libraries can contain antibodies or antigen-binding fragments of antibodies that contain artificial amino acid sequences. For example, the library can contain Fab fragments which contain artificial CDRs (e.g., random amino acid sequences) and human framework regions. (See, for example, U.S. Pat. No. 6,300,064 (Knappik, et al.).)

The sequences of human constant region genes may be found in Kabat et al. (1991) Sequences of Proteins of Immunological Interest, N.I.H. publication no. 91-3242. Human C region genes are readily available from known clones. The choice of isotype will be guided by the desired effector functions, such as complement fixation, or activity in antibody-dependent cellular cytotoxicity. Isotypes can be IgG1, IgG2, IgG3 or IgG4. In the present disclosure, antibody molecules of the disclosure are IgG1 and IgG2. Either of the human light chain constant regions, kappa or lambda, may be used. The chimeric, humanized antibody is then expressed by conventional methods.

In the present disclosure, an anti-GCC antibody molecule of the disclosure can draw antibody-dependent cellular cytotoxicity (ADCC) to a cell expressing GCC, e.g., a tumor cell. Antibodies with the IgG1 and IgG3 isotypes are useful for eliciting effector function in an antibody-dependent cytotoxic capacity, due to their ability to bind the Fc receptor. Antibodies with the IgG2 and IgG4 isotypes are useful to minimize an ADCC response because of their low ability to bind the Fc receptor. In the present disclosure substitutions in the Fc region or changes in the glycosylation composition of an antibody, e.g., by growth in a modified eukaryotic cell line, can be made to enhance the ability of Fc receptors to recognize, bind, and/or mediate cytotoxicity of cells to which anti-GCC antibodies bind (see, e.g., U.S. Pat. Nos. 7,317,091, 5,624,821 and publications including WO 00/42072, Shields, et al. J. Biol. Chem. 276:6591-6604 (2001), Lazar et al. Proc. Natl. Acad. Sci. U.S.A. 103:4005-4010 (2006), Satoh et al. Expert Opin Biol. Ther. 6:1161-1173 (2006)). In the present disclosure, the antibody or antigen-binding fragment (e.g., antibody of human origin, human antibody) can include amino acid substitutions or replacements that alter or tailor function (e.g., effector function). For example, a constant region of human origin (e.g., $\gamma$1 constant region, $\gamma$2 constant region) can be designed to reduce complement activation and/or Fc receptor binding. (See, for example, U.S. Pat. Nos. 5,648,260 (Winter et al.), 5,624,821 (Winter et al.) and 5,834,597 (Tso et al.). Preferably, the amino acid sequence of a constant region of human origin that contains such amino acid substitutions or replacements is at least about 95% identical over the full length to the amino acid sequence of the unaltered constant region of human origin, more preferably at least about 99% identical over the full length to the amino acid sequence of the unaltered constant region of human origin.

In present disclosure, effector functions can also be altered by modulating the glycosylation pattern of the antibody. By altering is meant deleting one or more carbohydrate moieties found in the antibody, and/or adding one or more glycosylation sites that are not present in the antibody. For example, antibodies with enhanced ADCC activities with a mature carbohydrate structure that lacks fucose attached to an Fc region of the antibody are described in U.S. Patent Application Publication No. 2003/0157108 (Presta). See also U.S. Patent Application Publication No. 2004/0093621 (Kyowa Hakko Kogyo Co., Ltd). Glycofi has also developed yeast cell lines capable of producing specific glycoforms of antibodies.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which are engineered to express recombinant antibodies of the disclosure to thereby produce an antibody with altered glycosylation. For example, EP 1,176,195 by Hang et al. describes a cell line with a functionally disrupted FUT8 gene, which encodes a fucosyl transferase, such that antibodies expressed in such a cell line exhibit hypofucosylation. PCT Publication WO 03/035835 by Presta describes a variant CHO cell line, Lec13 cells, with reduced ability to attach fucose to Asn(297)-linked carbohydrates, also resulting in hypofucosylation of antibodies expressed in that host cell (see also Shields, R. L. et al., 2002 J. Biol. Chem. 277:26733-26740). PCT Publication WO 99/54342 by Umana et al. describes cell lines engineered to express glycoprotein-modifying glycosyl transferases (e.g., beta(1,4)-N acetylglucosaminyltransferase III (GnTIII)) such that antibodies expressed in the engineered cell lines exhibit increased bisecting GlcNac structures which results in increased ADCC activity of the antibodies (see also Umana et al., 1999 Nat. Biotech. 17:176-180).

Humanized antibodies can also be made using a CDR-grafted approach. Techniques of generation of such humanized antibodies are known in the art. Generally, humanized antibodies are produced by obtaining nucleic acid sequences that encode the variable heavy and variable light sequences of an antibody that binds to GCC, identifying the complementary determining region or "CDR" in the variable heavy and variable light sequences and grafting the CDR nucleic acid sequences on to human framework nucleic acid sequences. (See, for example, U.S. Pat. Nos. 4,816,567 and 5,225,539). The location of the CDRs and framework residues can be determined (see, Kabat, E. A., et al. (1991) Sequences of Proteins of Immunological Interest, Fifth Edition, U.S. Department of Health and Human Services, NIH Publication No. 91-3242, and Chothia, C. et al. J. Mol. Biol. 196:901-917 (1987)). Anti-GCC antibody molecules described herein have the CDR amino acid sequences and nucleic acid sequences encoding CDRs listed in Tables 5 and 6. In the present disclosure sequences from Tables 5 and 6 can be incorporated into molecules which recognize GCC for use in the therapeutic or diagnostic methods described herein. The human framework that is selected is one that is suitable for *in vivo* administration, meaning that it does not exhibit immunogenicity. For example, such a determination can be made by prior experience with *in vivo* usage of such antibodies and studies of amino acid similarities. A suitable framework region can be selected from an antibody of human origin having at least about 65% amino acid sequence identity, and preferably at least about 70%, 80%, 90% or 95% amino acid sequence identity over the length of the framework region within the amino acid sequence of the equivalent portion (e.g., framework region) of the donor antibody, e.g., an anti-GCC antibody molecule (e.g., 3G1). Amino acid sequence identity can be determined using a suitable amino acid sequence alignment algorithm, such as CLUSTAL W, using the default parameters. (Thompson J. D. et al., Nucleic Acids Res. 22:4673-4680 (1994).)

Once the CDRs and FRs of the cloned antibody that are to be humanized are identified, the amino acid sequences encoding the CDRs are identified and the corresponding nucleic acid sequences grafted on to selected human FRs. This can be done using known primers and linkers, the selection of which are known in the art. All of the CDRs of a particular human antibody may be replaced with at least a portion of a non-human CDR or only some of the CDRs may be replaced with non-human CDRs. It is only necessary to replace the number of CDRs required for binding of the humanized antibody to a predetermined antigen. After the CDRs are grafted onto selected human FRs, the resulting "humanized" variable heavy and variable light sequences are expressed to produce a humanized Fv or humanized antibody that binds to GCC. Preferably, the CDR-grafted (e.g., humanized) antibody binds a GCC protein with an affinity similar to, substantially the same as, or better than that of the donor antibody. Typically, the humanized variable heavy and light sequences are expressed as a fusion protein with human constant domain sequences so an intact antibody that binds to GCC is obtained. However, a humanized Fv antibody can be produced that does not contain the constant sequences.

Also within the scope of the disclosure are humanized antibodies in which specific amino acids have been substituted, deleted or added. In particular, humanized antibodies can have amino acid substitutions in the framework region, such as to improve binding to the antigen. For example, a selected, small number of acceptor framework residues of the humanized immunoglobulin chain can be replaced by the corresponding donor amino acids. Locations of the substitutions include amino acid residues adjacent to the CDR, or which are capable of interacting with a CDR (see e.g., U.S. Pat. No. 5,585,089 or 5,859,205). The acceptor framework can be a mature human antibody framework sequence or a consensus sequence. As used herein, the term "consensus sequence" refers to the sequence found most frequently, or devised from the most common residues at each position in a sequence in a region among related family members. A number of human antibody consensus sequences are available, including consensus sequences for the different subgroups of human variable regions (see, Kabat, E. A., et al., Sequences of Proteins of Immunological Interest, Fifth Edition, U.S. Department of Health and Human Services, U.S. Government Printing Office (1991)). The Kabat database and its applications are freely available on line, e.g. via IgBLAST at the National Center for Biotechnology Information,

Bethesda, Md. (also see, Johnson, G. and Wu, T. T., Nucleic Acids Research 29:205-206 (2001)).

Other techniques for humanizing antibodies are described in Padlan et al. EP 519596 A1, published on Dec. 23, 1992.

The anti-GCC antibody molecule includes other humanized antibodies which may also be modified by specific deletion of human T cell epitopes or "deimmunization" by the methods disclosed in PCT Publication Nos. WO 98/52976 and WO 00/34317. Briefly, the rabbit, or other non-human species, heavy and light chain variable regions of an anti-GCC antibody can be analyzed for peptides that bind to MHC Class II; these peptides represent potential T-cell epitopes. For detection of potential T-cell epitopes, a computer modeling approach termed "peptide threading" can be applied, and in addition a database of human MHC class II binding peptides can be searched for motifs present in the rabbit VH and VL sequences, as described in PCT Publication Nos. WO 98/52976 and WO 00/34317. These motifs bind to any of the 18 major MHC class II DR allotypes, and thus constitute potential T cell epitopes. Potential T-cell epitopes detected can be eliminated by substituting small numbers of amino acid residues in the variable regions, or preferably, by single amino acid substitutions. As far as possible, conservative substitutions are made, often but not exclusively, an amino acid common at this position in human germline antibody sequences may be used. Human germline sequences are disclosed in Tomlinson, I. A. et al., J. Mol. Biol. 227:776-798 (1992); Cook, G. P. et al., Immunol. Today Vol. 16 (5): 237-242 (1995); Chothia, D. et al., J. Mol. Bio. 227:799-817 (1992). The V BASE directory provides a comprehensive directory of human immunoglobulin variable region sequences (compiled by Tomlinson, I. A. et al. MRC Centre for Protein Engineering, Cambridge, UK). After the deimmunized VH and VL of an anti-GCC antibody are constructed by mutagenesis of the rabbit VH and VL genes, the mutagenized variable sequence can, optionally, be fused to a human constant region, e.g., human IgG1 or K (kappa) constant regions.

In the present disclosure, reduction of an immunogenic response by a CDR-grafted antibody can be achieved by changes, e.g., deletions, substitutions, of amino acid residues in CDRs (Kashmiri et al. Methods 36:25-34 (2005), U.S. Pat. No. 6,818,749, Tan et al. J. Immunol. 169:1119-1125 (2006)). For example, residues at positions involved in contact with the antigen preferably would not be changed. Typically, such residues, the specificity determining residues (SDRs), are in positions which display high levels of variability among antibodies. Consensus sequences derived, e.g., by the Clustal method (Higgins D. G. et al., Meth. Enzymol. 266:383-402 (1996)), from anti-GCC antibody molecules, e.g., from antibodies described herein, aid in identifying SDRs. In the anti-GCC antibody molecules described herein, the SDRs are the following, at least the first residue or in the present disclosure, the first four residues of heavy chain CDR1; at least the N-terminal portion, e.g., the first seven, ten or 13 residues of heavy chain CDR2; nearly all of heavy chain CDR3; the C-terminal portion, e.g., after residue six, eight, or nine of light chain CDR1; about the first, middle and/or last residue of light chain CDR2; and most of light chain CDR3, or at least after residue two or three. Accordingly, to maintain binding to GCC protein after humanization or modification of an anti-GCC antibody molecule, such SDR residues in CDRs of the anti-GCC antibody molecules are less amenable to changes, e.g., from rabbit residues to human consensus residues than are residues in other residues of the CDRs or the framework regions. Conversely, it can be beneficial to change residues in non-human, e.g., rabbit CDRs to residues identified as consensus in human CDRs, e.g., CDRs of anti-GCC antibody molecules described in US Published Patent Application No. 20110110936.

Anti-GCC antibodies that are not intact antibodies are also useful in this disclosure. Such antibodies may be derived from any of the antibodies described above. Useful antibody molecules of this type include (i) a Fab fragment, a monovalent fragment consisting of the VL, VH, CL and CH1 domains; (ii) a $F(ab')_2$ fragment, a bivalent fragment comprising two Fab fragments linked by a disulfide bridge at the hinge region; (iii) an Fd fragment consisting of the VH and CH1 domains; (iv) an Fv fragment consisting of the VL and VH domains of a single arm of an antibody, (v) a dAb fragment (Ward et al., Nature 341:544-546 (1989)), which consists of a VH domain; (vii) a single domain functional heavy chain antibody, which consists of a VHH domain (known as a nanobody) see e.g., Cortez-Retamozo, et al., Cancer Res. 64: 2853-2857 (2004), and references cited therein; and (vii) an isolated CDR, e.g., one or more isolated CDRs together with sufficient framework to provide an antigen binding fragment. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. Science 242:423-426 (1988); and Huston et al. Proc. Natl. Acad. Sci. USA 85:5879-5883 (1988). Such single chain antibodies are also intended to be encompassed within the term "antigen-binding fragment" of an antibody. These antibody fragments are obtained using conventional techniques known to those with skill in the art, and the fragments are screened for utility in the same manner as are intact antibodies. Antibody fragments, such as Fv, $F(ab')_2$ and Fab may be prepared by cleavage of the intact protein, e.g. by protease or chemical cleavage.

In the present disclosure, some or all of the CDRs sequences, of one or both the heavy and light chain, can be used in another antibody molecule, e.g., in a CDR-grafted, humanized, or chimeric antibody molecule.

Embodiments include an antibody molecule that comprises sufficient CDRs, e.g., all six CDRs from one of the rabbit hybridoma antibodies described herein to allow binding to cell surface GCC.

In the present disclosure the CDRs, e.g., all of the HCDRs, or all of the LCDRs, or all six, are embedded in

human or human derived framework region(s). Examples of human framework regions include human germline framework sequences, human germline sequences that have been affinity matured (either *in vivo* or *in vitro*), or synthetic human sequences, e.g., consensus sequences. In the present disclosure the heavy chain framework is an IgG1 or IgG2 framework. In the present disclosure the light chain framework is a kappa framework.

In the present disclosure the anti-GCC antibody molecule, e.g., a CDR-grafted or humanized antibody molecule, comprises sufficient CDRs, e.g., all six CDRs from one of the antibodies described herein, e.g., sequences listed in Table 5, to allow binding to GCC. (Exemplary nucleic acid sequences which can encode the CDR amino acid sequences listed in Table 5, are provided, in Table 6 herein). In the present disclosure, an anti-GCC antibody molecule can comprise CDRs from MIL-44-148-2 or MIL-44-67-4.

Antibody fragments for *in vivo* therapeutic or diagnostic use can benefit from modifications which improve their serum half lives. Suitable organic moieties intended to increase the *in vivo* serum half-life of the antibody can include one, two or more linear or branched moiety selected from a hydrophilic polymeric group (e.g., a linear or a branched polymer (e.g., a polyalkane glycol such as polyethylene glycol, monomethoxy-polyethylene glycol and the like), a carbohydrate (e.g., a dextran, a cellulose, a polysaccharide and the like), a polymer of a hydrophilic amino acid (e.g., polylysine, polyaspartate and the like), a polyalkane oxide and polyvinyl pyrrolidone), a fatty acid group (e.g., a monocarboxylic acid or a dicarboxylic acid), a fatty acid ester group, a lipid group (e.g., diacylglycerol group, sphingolipid group (e.g., ceramidyl)) or a phospholipid group (e.g., phosphatidyl ethanolamine group). Preferably, the organic moiety is bound to a predetermined site where the organic moiety does not impair the function (e.g., decrease the antigen binding affinity) of the resulting immunoconjugate compared to the non-conjugated antibody moiety. The organic moiety can have a molecular weight of about 500 Da to about 50,000 Da, preferably about 2000, 5000, 10,000 or 20,000 Da. Examples and methods for modifying polypeptides, e.g., antibodies, with organic moieties can be found, for example, in U.S. Pat. Nos. 4,179,337 and 5,612,460, PCT Publication Nos. WO 95/06058 and WO 00/26256, and U.S. Patent Application Publication No. 20030026805.

An anti-GCC antibody molecule can comprise all, or an antigen binding fragment of the variable region, of one or both, the heavy and light chain, of one of the above-referenced rabbit hybridoma antibodies.

In the present disclosure the light chain amino acid sequence of (a) can differ from one of the reference amino acid sequence(s) referred to in (a)(i-ii) by as many as 1, 2, 3, 4, 5, 10, or 15 residues. In the present disclosure the differences are conservative substitutions. In the present disclosure, the differences are in the framework regions. In the present disclosure the heavy chain amino acid sequence of (b) can differ from one of the reference amino acid sequence(s) referred to in (b)(i-ii) by as many as 1, 2, 3, 4, 5, 10, or 15 residues. In the present disclosure the differences are conservative substitutions. In the present disclosure the differences are in the framework regions.

In the present disclosure the anti-GCC antibody molecule comprises one or both of:

(a) a light chain amino acid sequence of all, or an antigen binding fragment of, either, (i) a light chain variable region amino acid sequence from Table 3, e.g., SEQ ID NO:13, or (ii) a light chain variable region amino acid encoded by a nucleotide sequence from Table 4, e.g., SEQ ID NO:12; and

(b) a heavy chain amino acid sequence of all, or an antigen binding fragment of, either (i) a heavy chain variable region amino acid sequence from Table 3, e.g., SEQ ID NO:11, or (ii) a heavy chain amino acid sequence encoded by a nucleotide sequence from Table 4, e.g., SEQ ID NO:10.

In the present disclosure the anti-GCC antibody molecule comprises one or both of:

a) a light chain variable region, or an antigen binding fragment thereof, having at least 85, 90, 95, 97 or 99% homology with the light chain variable region of an anti-GCC antibody molecule of the disclosure; and

(b) a heavy chain variable region, or an antigen binding fragment thereof, having at least 85, 90, 95, 97 or 99% homology with the heavy chain variable region of an anti-GCC antibody molecule of the disclosure.

Amino acid sequences of the variable regions of the anti-GCC antibodies of the disclosure can be found in Table 3.

In one approach, consensus sequences encoding the heavy and light chain J regions may be used to design oligonucleotides for use as primers to introduce useful restriction sites into the J region for subsequent linkage of V region segments to human C region segments. C region cDNA can be modified by site directed mutagenesis to place a restriction site at the analogous position in the human sequence.

Expression vectors include plasmids, retroviruses, cosmids, YACs, EBV derived episomes, and the like. A convenient vector is one that encodes a functionally complete human CH or CL immunoglobulin sequence, with appropriate restriction sites engineered so that any VH or VL sequence can be easily inserted and expressed. In such vectors,

splicing usually occurs between the splice donor site in the inserted J region and the splice acceptor site preceding the human C region, and also at the splice regions that occur within the human CH exons. Suitable expression vectors can contain a number of components, for example, an origin of replication, a selectable marker gene, one or more expression control elements, such as a transcription control element (e.g., promoter, enhancer, terminator) and/or one or more translation signals, a signal sequence or leader sequence, and the like. Polyadenylation and transcription termination occur at native chromosomal sites downstream of the coding regions. The resulting chimeric antibody may be joined to any strong promoter. Examples of suitable vectors that can be used include those that are suitable for mammalian hosts and based on viral replication systems, such as simian virus 40 (SV40), Rous sarcoma virus (RSV), adenovirus 2, bovine papilloma virus (BPV), papovavirus BK mutant (BKV), or mouse and human cytomegalovirus (CMV), and moloney murine leukemia virus (MMLV), native Ig promoters, etc. A variety of suitable vectors are known in the art, including vectors which are maintained in single copy or multiple copies, or which become integrated into the host cell chromosome, e.g., via LTRs, or via artificial chromosomes engineered with multiple integration sites (Lindenbaum et al. Nucleic Acids Res. 32:e172 (2004), Kennard et al. Biotechnol. Bioeng. Online May 20, 2009). Additional examples of suitable vectors are listed in a later section.

Thus, the disclosure describes an expression vector comprising a nucleic acid encoding an antibody, antigen-binding fragment of an antibody (e.g., a humanized, chimeric antibody or antigen-binding fragment of any of the foregoing), antibody chain (e.g., heavy chain, light chain) or antigen-binding portion of an antibody chain that binds a GCC protein.

Expression in eukaryotic host cells is useful because such cells are more likely than prokaryotic cells to assemble and secrete a properly folded and immunologically active antibody. However, any antibody produced that is inactive due to improper folding may be renaturable according to known methods (Kim and Baldwin, "Specific Intermediates in the Folding Reactions of Small Proteins and the Mechanism of Protein Folding", Ann. Rev. Biochem. 51, pp. 459-89 (1982)). It is possible that the host cells will produce portions of intact antibodies, such as light chain dimers or heavy chain dimers, which also are antibody homologs according to the present disclosure.

Further, as described elsewhere herein, antibodies or antibodies from human or non-human species can be generated through display-type technologies, including, without limitation, phage display, retroviral display, ribosomal display, and other techniques, using techniques well known in the art and the resulting molecules can be subjected to additional maturation, such as affinity maturation, as such techniques are known in the art. Winter and Harris Immunol Today 14:43-46 (1993) and Wright et al. Crit. Reviews in Immunol. 12125-168 (1992), Hanes and Plucthau PNAS USA 94:4937-4942 (1997) (ribosomal display), Parmley and Smith Gene 73:305-318 (1988) (phage display), Scott TIBS 17:241-245 (1992), Cwirla et al. Proc Natl Acad Sci USA 87:6378-6382 (1990), Russel et al. Nucl. Acids Research 21:1081-1085 (1993), Hoganboom et al. Immunol. Reviews 130:43-68 (1992), Chiswell and McCafferty TIBTECH 10:80-84 (1992), and U.S. Pat. No. 5,733,743. If display technologies are utilized to produce antibodies that are not human, such antibodies can be humanized as described above.

It will be appreciated that antibodies that are generated need not initially possess a particular desired isotype but, rather, the antibody as generated can possess any isotype. For example, the antibody produced by the MIL-44-148-2 rabbit hybridoma has an IgG isotype. The isotype of the antibody can be switched thereafter, e.g., to IgG2, or IgG3 to elicit an ADCC response when the antibody binds GCC on a cell, using conventional techniques that are known in the art. Such techniques include the use of direct recombinant techniques (see e.g., U.S. Pat. No. 4,816,397), cell-cell fusion techniques (see e.g., U.S. Pat No 5,916,771), among others. In the cell-cell fusion technique, a myeloma or other cell line is prepared that possesses a heavy chain with any desired isotype and another myeloma or other cell line is prepared that possesses the light chain. Such cells can, thereafter, be fused and a cell line expressing an intact antibody can be isolated.

In the present disclosure, the GCC antibody molecule is a rabbit anti-GCC IgG1 antibody. Since such antibodies possess desired binding to the GCC molecule, any one of such antibodies can be readily isotype-switched to generate another isotype while still possessing the same variable region (which defines the antibody's specificity and affinity, to a certain extent). Accordingly, as antibody candidates are generated that meet desired "structural" attributes as discussed above, they can generally be provided with at least certain additional "functional" attributes that are desired through isotype switching.

In the present disclosure the variable region or antigen binding fragment thereof can be coupled to a constant region (or fragment thereof) other than the constant region it was generated with, e.g., a constant region (or fragment thereof) from another antibody or to a synthetic constant region (or fragment thereof). In the present disclosure the constant region is an IgG1 or IgG2 constant region (or fragment thereof). Sequence changes can be made in the variable or constant regions to modify effector activity of the antibody molecule.

**Design and Generation of Other Therapeutics**

The antibodies that are produced and characterized herein with respect to GCC provide for the design of other therapeutic modalities including other antibodies, other antagonists, or chemical moieties other than antibodies is facilitated. Such modalities include, without limitation, antibodies having similar binding activity or functionality, advanced antibody therapeutics, such as bispecific antibodies, immunoconjugates, and radiolabeled therapeutics, generation of peptide therapeutics, particularly intrabodies, and small molecules. Furthermore, as discussed above, the effector function of the antibodies of the disclosure may be changed by isotype switching to an IgG1, IgG2, IgG3, IgG4, IgD, IgA1, IgA2, IgE, or IgM for various therapeutic uses.

In connection with bispecific antibodies, bispecific antibodies can be generated that comprise (i) two antibodies, one with a specificity to GCC and another to a second molecule that are conjugated together, (ii) a single antibody that has one chain specific to GCC and a second chain specific to a second molecule, or (iii) a single chain antibody that has specificity to GCC and the other molecule. Such bispecific antibodies can be generated using techniques that are known. For example, bispecific antibodies may be produced by crosslinking two or more antibodies (of the same type or of different types). Suitable crosslinkers include those that are heterobifunctional, having two distinctly reactive groups separated by an appropriate spacer (e.g., m-maleimidobenzoyl-N-hydroxysuccinimide ester) or homobifunctional (e.g., disuccinimidyl suberate). Such linkers are available from Pierce Chemical Company, Rockford, Ill. See also, e.g., Fanger et al. Immunomethods 4:72-81 (1994) and Winter and Harris Immunol Today 14:43-46 (1993) and Wright et al. Crit. Reviews in Immunol. 12125-168 (1992) and in connection with (iii) see e.g., Traunecker et al. Int. J. Cancer (Suppl.) 7:51-52 (1992). Songsivilai & Lachmann Clin. Exp. Immunol. 79: 315-321 (1990), Kostelny et al. J. Immunol. 148:1547-1553 (1992).

In addition, "Kappabodies" (Ill. et al. "Design and construction of a hybrid immunoglobulin domain with properties of both heavy and light chain variable regions" Protein Eng 10:949-57 (1997)), "Minibodies" (Martin et al. EMBO J. 13:5303-9 (1994), U.S. Pat. No. 5,837,821), "Diabodies" (Holliger et al. Proc Natl Acad Sci USA 90:6444-6448 (1993)), or "Janusins" (Traunecker et al. EMBO J. 10:3655-3659 (1991) and Traunecker et al. Int J Cancer Suppl 7:51-52 (1992)) may also be prepared.

**Nucleic Acids and Polypeptides**

In the present disclosure, the present disclosure relates to polynucleotide and polypeptide sequences that encode for or represent the antibody molecules described herein. Such polynucleotides encode for both the variable and constant regions of each of the heavy and light chains, although other combinations are also contemplated by the present disclosure in accordance with the compositions described herein. The present disclosure also contemplates oligonucleotide fragments derived from the disclosed polynucleotides and nucleic acid sequences complementary to these polynucleotides.

The polynucleotides can be in the form of RNA or DNA. Polynucleotides in the form of DNA, cDNA, genomic DNA, nucleic acid analogs and synthetic DNA are within the scope of the present disclosure. The DNA may be double-stranded or single-stranded, and if single stranded, may be the coding (sense) strand or non-coding (anti-sense) strand. The coding sequence that encodes the polypeptide may be identical to the coding sequence provided herein or may be a different coding sequence which coding sequence, as a result of the redundancy or degeneracy of the genetic code, encodes the same polypeptide as the DNA provided herein.

In the present disclosure provided, polynucleotides encode at least one heavy chain variable region and at least one light chain variable region of the present disclosure, e.g., as summarized in Table 4.

The present disclosure also includes variant polynucleotides containing modifications such as polynucleotide deletions, substitutions or additions, and any polypeptide modification resulting from the variant polynucleotide sequence. A polynucleotide of the present disclosure may also have a coding sequence that is a variant of the coding sequence provided herein. For example, a variant polynucleotide can have at least 50%, 60%, 70%, 75%, 80%, 85%, 90%, 95% or 97% identity with a polynucleotide listed in Table 4. In the present disclosure, the variant polynucleotide encodes for an anti-GCC antibody molecule.

The present disclosure further relates to polypeptides that represent the antibodies of the present disclosure as well as fragments, analogs and derivatives of such polypeptides. The polypeptides of the present disclosure may be recombinant polypeptides, naturally produced polypeptides or synthetic polypeptides. The fragment, derivative or analogs of the polypeptides of the present disclosure may be one in which one or more of the amino acid residues is substituted with a conserved or non-conserved amino acid residue (preferably a conserved amino acid residue) and such substituted amino acid residue may or may not be one encoded by the genetic code; or it may be one in which one or more of the amino acid residues includes a substituent group; or it may be one in which the polypeptide is fused with another compound, such as a compound to increase the half-life of the polypeptide (for example, polyethylene glycol); or it may be one in which the additional amino acids are fused to the polypeptide, such as a leader or secretory sequence or a sequence that is employed for purification of the polypeptide or a proprotein sequence. Such fragments, derivatives and analogs are within the scope of the present disclosure. In various aspects, the polypeptides of the disclosure may be partially purified, or purified product.

A polypeptide of the present disclosure can have an amino acid sequence that is identical to that of the antibodies

described herein, e.g., summarized in Tables 2 or 3, or that is different by minor variations due to one or more amino acid substitutions. The variation may be a "conservative change" typically in the range of about 1 to 5 amino acids, wherein the substituted amino acid has similar structural or chemical properties, e.g., replacement of leucine with isoleucine or threonine with serine; replacement of lysine with arginine or histidine. In contrast, variations may include nonconservative changes, e.g., replacement of a glycine with a tryptophan. Similar minor variations may also include amino acid deletions or insertions or both. Guidance in determining which and how many amino acid residues may be substituted, inserted, or deleted without changing biological or immunological activity may be found using computer programs known in the art, for example DNASTAR software (DNASTAR, Inc., Madison, Wis.).

In another aspect, the disclosure features, isolated and/or recombinant nucleic acids encoding anti-GCC antibody molecules. In the present disclosure, the nucleic acids encode one or more of an antibody molecule, a heavy chain, a light chain, a light chain variable region, a heavy chain variable region, portions of the heavy chains and light chains of the antibody molecules described herein (e.g., a light chain variable region fragment which when paired with a full length heavy chain variable region is antigen binding, or a heavy chain variable region fragment which when paired with a full length light chain variable region is antigen binding), and CDRs. Embodiments include such nucleic acids disposed in vectors, e.g., expression vectors. Still further, the disclosure encompasses antibody molecules produced by host cells, e.g., expressing the antibody molecules encoded by such plasmids

In the present disclosure, is provided a vector, e.g., an expression vector, comprising one or both of:

sequences encoding a light chain variable region, e.g., a light chain variable region described in Table 3, e.g., a sequence listed in Table 4, an antigen binding fragment thereof, or one, two or three CDRs from a light chain (and optionally a framework region), described herein, e.g., CDRs described in Table 5,e.g., a CDR encoding sequence in Table 6; and

sequences encoding a heavy chain variable region, e.g., a heavy chain variable region described in Table 3, e.g., a sequence listed in Table 4, an antigen binding fragment thereof, or one, two or three CDRs from a heavy chain (and optionally a framework region), described herein, e.g., CDRs described in Table 5, e.g., a CDR encoding sequence in Table 6.

In the present disclosure provided, polynucleotides encode at least one heavy chain variable region or at least one light chain variable region of the antibodies of the present disclosure. In the present disclosure provided, polypeptides can encode at least one heavy chain variable region and one light chain variable region of the antibodies of the present disclosure.

In the present disclosure the anti-GCC antibody molecule comprises one or both of:

(a) a light chain variable region, or an antigen binding fragment thereof, encoded by a nucleic acid that hybridizes under selected stringency conditions with, (i) the complement of an anti-GCC antibody molecule-encoding-nucleic acid sequence described herein, e.g., in Table 4, or (ii) any nucleic acid sequence that encodes a light chain of an anti-GCC antibody molecule of the disclosure, e.g., one of the above-referenced rabbit antibodies summarized in Tables 1 and 2; and

(b) a heavy chain variable region, or an antigen binding fragment thereof, encoded by a nucleic acid that hybridizes under selected stringency conditions with, (i) the complement of an anti-GCC antibody molecule-encoding-nucleic acid sequence described herein, e.g., in Table 4, or (ii) any nucleic acid sequence that encodes a heavy chain of an anti-GCC antibody molecule of the disclosure, e.g., one of the above-referenced rabbit antibodies summarized in Tables 1 and 2.

In the present disclosure selected stringency conditions are high stringency or very high stringency conditions, e.g., as those conditions are described herein.

The present disclosure also provides vectors that include the polynucleotides of the present disclosure, host cells which are genetically engineered with vectors of the present disclosure and the production of the antibodies of the present disclosure by recombinant techniques.

The appropriate DNA sequence may be inserted into the vector by a variety of procedures. In general, the DNA sequence is inserted into appropriate restriction endonuclease sites by procedures known in the art. The polynucleotide sequence in the expression vector is operatively linked to an appropriate expression control sequence (i.e. promoter) to direct mRNA synthesis. Examples of such promoters include, but are not limited to, the Rous sarcoma virus LTR or the early or late SV40 promoter, the *E. coli* lac or trp, the phage lambda $P_L$ promoter and other promoters known to control expression of genes in prokaryotic (e.g., tac, T3, T7 promoters for *E. coli*) or eukaryotic (e.g., cytomegalovirus promoter, adenovirus late promoter, EF-1a promoter) cells or their viruses. The expression vector also contains a

ribosome binding site for translation initiation and a transcription terminator. The vector may also include appropriate sequences for amplifying expression. For example, the vector can contain enhancers, which are transcription-stimulating DNA sequences of viral origin, such as those derived form simian virus such as SV40, polyoma virus, cytomegalovirus, bovine papilloma virus or Moloney sarcoma virus, or genomic, origin. The vector preferably also contains an origin of replication. The vector can be constructed to contain an exogenous origin of replication or, such an origin of replication can be derived from SV40 or another viral source, or by the host cell chromosomal replication mechanism.

In addition, the vectors optionally contain a marker gene for selection of transfected host cells such as dihydrofolate reductase marker genes to permit selection with methotrexate in a variety of hosts, or antibiotics, such as .beta.-lactamase gene (ampicillin resistance), Tet gene (for tetracycline resistance) used in prokaryotic cells or neomycin, GA418 (geneticin, a neomycin-derivative) gpt (mycophenolic acid), ampicillin, or hygromycin resistance genes, or genes which complement a genetic lesion of the host cells such as the absence of thymidine kinase, hypoxanthine phosphoribosyl transferase, dihydrofolate reductase, etc. Genes encoding the gene product of auxotrophic markers of the host (e.g., LEU2, URA3, HIS3) are often used as selectable markers in yeast.

In order to obtain the antibodies of the present disclosure, one or more polynucleotide sequences that encode for the light and heavy chain variable regions and light and heavy chain constant regions of the antibodies of the present disclosure should be incorporated into a vector. Polynucleotide sequences encoding the light and heavy chains of the antibodies of the present disclosure can be incorporated into one or multiple vectors and then incorporated into the host cells.

Suitable expression vectors for expression in mammalian cells include, for example, pCDM8, pcDNA1.1/amp, pcDNA3.1, pRc/RSV, pEF-1 (Invitrogen Life Technologies, Carlsbad, Calif.), pCMV-SCRIPT, pFB, pSG5, pXT1 (Stratagene, La Jolla, Calif.), pCDEF3 (Goldman, L. A., et al., Biotechniques, 21:1013-1015 (1996)), pSVSPORT (GIBCO division of Invitrogen Life Technologies, Carlsbad, Calif.), pEF-Bos (Mizushima, S., et al., Nucleic Acids Res., 18:5322 (1990)), Bicistronic GPEX® Retrovector (Gala Biotech, Middleton, Wis.) and the like. Expression vectors which are suitable for use in various expression hosts, such as prokaryotic cells (*E. coli*), insect cells (*Drosophila* Schnieder S2 cells, Sf9) and yeast (*P. methanolica, P. pastoris, S. cerevisiae*) are also available. Exemplary vectors are pLKTOK58 (wild type IgG1 Fc sequence) and pLKTOK59 (mutated IgG1 Fc sequence) (see U.S. Patent Application publication no. 20060147445).

As will be appreciated, antibodies in accordance with the present disclosure can be expressed in cell lines other than hybridoma cell lines. Sequences encoding the cDNAs or genomic clones for the particular antibodies can be used for a suitable mammalian or nonmammalian host cells. Transformation can be by any known method for introducing polynucleotides into a host cell, including, for example packaging the polynucleotide in a virus (or into a viral vector) and transducing a host cell with the virus (or vector) or by transfection procedures known in the art, for introducing heterologous polynucleotides into mammalian cells, e.g., dextran-mediated transfection, calcium phosphate precipitation, polybrene mediated transfection, protoplast fusion, electroporation, encapsulation of the polynucleotide(s) into liposomes and direct microinjection of the DNA molecule. The transformation procedure used depends upon the host to be transformed. Methods for introduction of heterologous polynucleotides into mammalian cells are known in the art and include, but are not limited to, dextran-mediated transfection, calcium phosphate precipitation, polybrene mediated transfection, protoplast fusion, electroporation, particle bombardment, encapsulation of the polynucleotide(s) in liposomes, peptide conjugates, dendrimers, and direct microinjection of the DNA into nuclei.

In another aspect, the disclosure features, a host cell comprising a nucleic acid described herein. In the present disclosure the cell expresses an antibody molecule, or component thereof, described herein. Still further the present disclosure describes a method of producing an antibody molecule, e.g., an anti-GCC antibody molecule described herein, e.g. a rabbit antibody molecule, or a humanized version thereof, comprising maintaining the host cell under conditions appropriate for expression, whereby immunoglobulin chain(s) are expressed and an antibody molecule is produced. The present disclosure describes a host cell comprising any of the foregoing expression vectors encoding heavy and light chain antibody sequences. The host cell can be a eukaryotic cell, e.g., a mammalian cell, an insect cell, a yeast cell, or a prokaryotic cell, e.g., *E. coli.* For example, the mammalian cell can be a cultured cell or a cell line. Exemplary mammalian cells include lymphocytic cell lines (e.g., NS0), Chinese hamster ovary cells (CHO), COS cells. In the present disclosure, the cultured host cell is a CHO cell comprising nucleic acid sequences encoding a MIL-44-148-2 antibody molecule. In the present disclosure, the host cell is Hybridoma MIL-44-148-2 (PTA-8132). Additionally cells include oocyte cells, and cells from a transgenic animal, e.g., mammary epithelial cell. For example, nucleic acids encoding an antibody molecule described herein can be expressed in a transgenic nonhuman animal.

Mammalian cell lines available as hosts for expression are known in the art and include many immortalized cell lines available from the American Type Culture Collection (ATCC), including but not limited to Chinese hamster ovary (CHO) cells, NSO cells, HeLa cells, baby hamster kidney (BHK) cells, monkey kidney cells (COS), human hepatocellular carcinoma cells (e.g., Hep G2), and a number of other cell lines. Non-mammalian cells including but not limited to bacterial, yeast, insect, and plants can also be used to express recombinant antibodies. Site directed mutagenesis of the antibody CH2 domain to eliminate glycosylation may be preferred in order to prevent changes in either the

immunogenicity, pharmacokinetic, and/or effector functions resulting from non-human glycosylation. The expression methods are selected by determining which system generates the highest expression levels and produce antibodies with constitutive GCC binding properties.

In the present disclosure describes a method of producing an anti-GCC antibody molecule, e.g., a rabbit antibody molecule or a humanized version thereof, comprising maintaining the host cell comprising nucleic acids described herein, e.g., one or more nucleic acid sequence listed in Table 4 or 6, under conditions appropriate for expression of an immunoglobulin, whereby immunoglobulin chains, are expressed and an antibody molecule, e.g., a rabbit antibody molecule, or a humanized version thereof, that binds GCC, or a fragment or variant thereof, is produced. For example, methods of expression of antibody molecules include the use of host cells wherein a first recombinant nucleic acid molecule encoding an antibody molecule, e.g., a rabbit antibody light chain or a humanized version thereof, and a second recombinant nucleic acid molecule encoding an antibody molecule, e.g., a rabbit antibody heavy chain or a humanized version thereof, are comprised in a single expression vector. In the present disclosure, they are in separate vectors. The method can further comprise the step of isolating or recovering the antibody, antigen-binding fragment of an antibody, antibody chain or antigen-binding fragment of an antibody chain, if desired.

For example, a nucleic acid molecule (i.e., one or more nucleic acid molecules) encoding the heavy and light chains of a rabbit (or humanized) antibody that binds a GCC protein, or an expression construct (i.e., one or more constructs) comprising such nucleic acid molecule(s), can be introduced into a suitable host cell to create a recombinant host cell using any method appropriate to the host cell selected (e.g., transformation, transfection, electroporation, infection), such that the nucleic acid molecule(s) are operably linked to one or more expression control elements (e.g., in a vector, in a construct created by processes in the cell, integrated into the host cell genome). The resulting recombinant host cell can be maintained under conditions suitable for expression (e.g., in the presence of an inducer, in a suitable non-human animal, in suitable culture media supplemented with appropriate salts, growth factors, antibiotics, nutritional supplements, etc.), whereby the encoded polypeptide(s) are produced. If desired, the encoded protein can be isolated or recovered (e.g., from the animal, the host cell, medium, milk). This process encompasses expression in a host cell of a transgenic non-human animal (see, e.g., WO 92/03918, GenPharm International) or plant.

Further, expression of antibodies of the disclosure (or other moieties therefrom) from production cell lines can be enhanced using a number of known techniques. For example, the glutamine synthetase and DHFR gene expression systems are common approaches for enhancing expression under certain conditions. High expressing cell clones can be identified using conventional techniques, such as limited dilution cloning, Microdrop technology, or any other methods known in the art. The GS system is discussed in whole or part in connection with European Patent Nos. 0 216 846, 0 256 055, and 0 323 997 and European Patent Application No. 89303964.4.

In an exemplary system for recombinant expression of a modified antibody, or antigen-binding portion thereof, of the disclosure, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to enhancer/promoter regulatory elements (e.g., derived from SV40, CMV, adenovirus and the like, such as a CMV enhancer/AdMLP promoter regulatory element or an SV40 enhancer/AdMLP promoter regulatory element) to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

Antibodies of the disclosure can also be produced transgenically through the generation of a mammal or plant that is transgenic for the immunoglobulin heavy and light chain sequences of interest and production of the antibody in a recoverable form therefrom. In connection with the transgenic production in mammals, antibodies can be produced in, and recovered from, the milk of goats, cows, or other mammals. See, e.g., U.S. Pat. Nos. 5,827,690, 5,756,687, 5,750,172, and 5,741,957.

The antibodies, antigen-binding fragments, antibody chains and antigen-binding portions thereof described herein also can be produced in a suitable *in vitro* expression system, by chemical synthesis or by any other suitable method.

**Fusion Proteins and Immunoconjugates**

The anti-GCC antibodies described herein can be functionally linked by any suitable method (e.g., chemical coupling, genetic fusion, noncovalent association or otherwise) to one or more non-antibody molecular entities.

Fusion proteins can be produced in which an anti-GCC antibody molecule as described herein and a non-antibody moiety are components of a single continuous polypeptide chain. The non-antibody moiety can be located N-terminally, C-terminally, or internally, with respect to the antibody moiety. For example, the present disclosure can be

produced by the insertion of a nucleic acid encoding immunoglobulin sequences into a suitable expression vector, such as a pET vector (e.g., pET-15b, Novagen), a phage vector (e.g., pCNATAB 5 E, Pharmacia), or other vector, e.g., pRIT2T Protein A fusion vector, Pharmacia). The resulting construct can be expressed to produce antibody chains that comprise a non-antibody moiety (e.g., Histidine tag, E tag, or Protein A IgG binding domain). Fusion proteins can be isolated or recovered using any suitable technique, such as chromatography using a suitable affinity matrix (see, e.g., Current Protocols in Molecular Biology (Ausubel, F. M et al., eds., Vol. 2, Suppl. 26, pp. 16.4.1-16.7.8 (1991)).

The disclosure describes anti-GCC antibody molecules which are directed to and, in the present disclosure, are internalized into cells. They are capable of delivering therapeutic agents or detectable agents to or into cells expressing GCC, but not to or into cells where the target is not expressed. Thus, the disclosure also describes anti-GCC immunoconjugates comprising an anti-GCC antibody molecule as described herein, which is conjugated to a therapeutic agent or a detectable agent. In the present disclosure, the affinity for GCC of an anti-GCC immunoconjugate is at least 10, 25, 50, 75, 80, 90, or 95% of that for the unconjugated antibody. This can be determined using cell surface GCC or isolated GCC. In the present disclosure the anti-GCC antibody molecule, e.g., an immunoconjugate, has an LD50, as determined by an assay described herein, of less than 1,000, 500, 250, 100, or 50 pM.

The anti-GCC antibody molecule can be modified to act as an immunoconjugate utilizing techniques that are known in the art. See e.g., Vitetta Immunol Today 14:252 (1993). See also U.S. Pat. No. 5,194,594. The preparation of radiolabeled antibodies can also be readily prepared utilizing techniques that are known in the art. See e.g., Junghans et al. in Cancer Chemotherapy and Biotherapy 655-686 (2d edition, Chafner and Longo, eds., Lippincott Raven (1996)). See also U.S. Pat. Nos. 4,681,581, 4,735,210, 5,101,827, 5,102,990 (U.S. Re. Pat. No. 35,500), 5,648,471, and 5,697,902.

In the present disclosure, the antibody molecule and non-antibody moiety are connected by means of a linker. In the present disclosure, the immunoconjugate is represented by formula (I):

$$Ab\{X\text{-}Z)_m$$

wherein,

Ab is an anti-GCC antibody molecule described herein;

X is a moiety which connects Ab and Z, e.g., the residue of a linker described herein after covalent linkage to one or both of Ab and Z;

Z is a therapeutic agent or label; and

m ranges from about 1 to about 15.

The variable m represents the number of --X--Z moieties per antibody molecule in an immunoconjugate of formula (I). In the present disclosure, m ranges from 1 to 15, 1 to 10, 1 to 9, 1 to 8, 1 to 7, 1 to 6, 1 to 5, 1 to 4, 1 to 3, or 1 to 2. In the present disclosure, m ranges from 2 to 10, 2 to 9, 2 to 8, 2 to 7, 2 to 6, 2 to 5, 2 to 4 or 2 to 3. In other the present disclosure, m is 1, 2, 3, 4, 5 or 6. In compositions comprising a plurality of immunoconjugates of formula (I), m is the average number of --X--Z moieties per Ab, also referred to as the average drug loading. Average drug loading may range from 1 to about 15-X--Z moieties per Ab. In the present disclosure, when m represents the average drug loading, m is about 1, about 2, about 3, about 4, about 5, about 6, about 7, or about 8. In the present disclosure, m is from about 2 to about 8. In the present disclosure, m is about 8. In the present disclosure, m is about 4. In the present disclosure, m is about 2.

The average number of --X--Z moieties per Ab may be characterized by conventional means such as mass spectroscopy, ELISA assay, and HPLC. The quantitative distribution of immunoconjugates in terms of m may also be determined. In some instances, separation, purification, and characterization of homogeneous immunoconjugates where m is a certain value, as distinguished from immunoconjugates with other drug loadings, may be achieved by means such as reverse phase HPLC or electrophoresis.

The immunoconjugates of formula (I) may exist as mixtures, wherein each component of the mixture has a different m value. For example, an immunoconjugate of formula (I) may exist as a mixture of two separate immunoconjugate components: one immunoconjugate component wherein m is 7, and the other immunoconjugate component wherein m is 8.

In the present disclosure, the immunoconjugate of formula (I) exists as a mixture of three separate immunoconjugates wherein m for the three separate immunoconjugates is 1, 2, and 3, respectively; 3, 4, and 5, respectively; 5, 6, and 7, respectively; 7, 8, and 9, respectively; 9, 10, and 11, respectively; 11, 12, and 13, respectively; or 13, 14, and 15, respectively.

A variety of suitable linkers (e.g., heterobifunctional reagents for connecting an antibody molecule to a therapeutic agent or label) and methods for preparing immunoconjugates are known in the art. (See, for example, Chari et al., Cancer Research 52:127-131 (1992).) The linker can be cleavable, e.g., under physiological conditions.,e.g., under intracellular conditions, such that cleavage of the linker releases the drug (i.e., therapeutic agent or label) in the intracellular environment. In the present disclosure, the linker is not cleavable, and the drug is released, for example, by antibody degradation.

The linker can be bonded to a chemically reactive group on the antibody moiety, e.g., to a free amino, imino, hydroxyl, thiol or carboxyl group (e.g., to the N- or C-terminus, to the epsilon amino group of one or more lysine residues, the free carboxylic acid group of one or more glutamic acid or aspartic acid residues, or to the sulfhydryl group of one or more cysteinyl residues). The site to which the linker is bound can be a natural residue in the amino acid sequence of the antibody moiety or it can be introduced into the antibody moiety, e.g., by DNA recombinant technology (e.g., by introducing a cysteine or protease cleavage site in the amino acid sequence) or by protein biochemistry (e.g., reduction, pH adjustment or proteolysis).

One of the most commonly used non-specific methods of covalent attachment is the carbodiimide reaction to link a carboxy (or amino) group of a compound to amino (or carboxy) groups of the antibody molecule. Additionally, bifunctional agents such as dialdehydes or imidoesters have been used to link the amino group of a compound to amino groups of an antibody molecule. Also available for attachment of drug (i.e., therapeutic agent or label) to antibody molecules is the Schiff base reaction. This method involves the periodate oxidation of a drug that contains glycol or hydroxy groups, thus forming an aldehyde which is then reacted with the antibody molecule. Attachment occurs via formation of a Schiff base with amino groups of the antibody molecule. Isothiocyanates can also be used as coupling agents for covalently attaching drugs to antibody molecule. Other techniques are known to the skilled artisan and within the scope of the present disclosure.

In certain the present disclosure, an intermediate, which is the precursor of the linker (X), is reacted with the drug (Z) under appropriate conditions. In certain the present disclosure, reactive groups are used on the drug and/or the intermediate. The product of the reaction between the drug (i.e., therapeutic agent or label) and the intermediate, or the derivatized drug, is subsequently reacted with the antibody molecule under appropriate conditions.

The immunoconjugate can be purified from reactants by employing methodologies well known to those of skill in the art, e.g., column chromatography (e.g., affinity chromatography, ion exchange chromatography, gel filtration, hydrophobic interaction chromatography), dialysis, diafiltration or precipitation. The immunoconjugate can be evaluated by employing methodologies well known to those skilled in the art, e.g., SDS-PAGE, mass spectroscopy, or capillary electrophoresis.

In some the present disclosure, the linker is cleavable by a cleaving agent that is present in the intracellular environment (e.g., within a lysosome or endosome or caveolea). The linker can be, e.g., a peptidyl linker that is cleaved by an intracellular peptidase or protease enzyme, including, but not limited to, a lysosomal or endosomal protease. In the present disclosure, the peptidyl linker is at least two amino acids long or at least three amino acids long. Cleaving agents can include cathepsins B and D and plasmin, all of which are known to hydrolyze dipeptide drug derivatives resulting in the release of active drug (i.e., therapeutic agent or label) inside target cells (see, e.g., Dubowchik and Walker, 1999, Pharm. Therapeutics 83:67-123). Most typical are peptidyl linkers that are cleavable by enzymes that are present in GCC-expressing cells. For example, a peptidyl linker that is cleavable by the thiol-dependent protease cathepsin-B, which is highly expressed in cancerous tissue, can be used (e.g., a Phe-Leu or a Gly-Phe-Leu-Gly linker (SEQ ID NO:319)). Other examples of such linkers are described, e.g., in U.S. Pat. No. 6,214,345. In the present disclosure, the peptidyl linker cleavable by an intracellular protease is a Val-Cit linker or a Phe-Lys linker (see, e.g., U.S. Pat. No. 6,214,345, which describes the synthesis of doxorubicin with the val-cit linker). One advantage of using intracellular proteolytic release of the drug (i.e., therapeutic agent or label) is that the drug is typically attenuated when conjugated and the serum stabilities of the conjugates are typically high.

In the present disclosure, the cleavable linker is pH-sensitive, i.e., sensitive to hydrolysis at certain pH values. Typically, the pH-sensitive linker is hydrolyzable under acidic conditions. For example, an acid-labile linker that is hydrolyzable in the lysosome (e.g., a hydrazone, semicarbazone, thiosemicarbazone, cis-aconitic amide, orthoester, acetal, ketal, or the like) can be used. (See, e.g., U.S. Pat. Nos. 5,122,368; 5,824,805; 5,622,929; Dubowchik and Walker, 1999, Pharm. Therapeutics 83:67-123; Neville et al., 1989, Biol. Chem. 264:14653-14661.) Such linkers are relatively stable under neutral pH conditions, such as those in the blood, but are unstable at below pH 5.5 or 5.0, the approximate pH of the lysosome. In the present disclosure, the hydrolyzable linker is a thioether linker (such as, e.g., a thioether attached to the therapeutic agent via an acylhydrazone bond (see, e.g., U.S. Pat. No. 5,622,929).

In the present disclosure, the linker is cleavable under reducing conditions (e.g., a disulfide linker). A variety of disulfide linkers are known in the art, including, for example, those that can be formed using SATA (N-succinimidyl-5-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)toluene)-, SPDB and SMPT (See, e.g., Thorpe et al., 1987, Cancer Res. 47:5924-5931; Wawrzynczak et al., In Immunoconjugates: Antibody Conjugates

in Radioimagery and Therapy of Cancer (C. W. Vogel ed., Oxford U. Press, 1987. See also U.S. Pat. No. 4,880,935.)

In the present disclosure, the linker is a malonate linker (Johnson et al., 1995, Anticancer Res. 15:1387-93), a maleimidobenzoyl linker (Lau et al., 1995, Bioorg Med. Chem. 3(10):1299-1304), or a 3'-N-amide analog (Lau et al., 1995, Bioorg-Med-Chem. 3(10):1305-12).

In the present disclosure, the linker unit is not cleavable and the drug (i.e., therapeutic agent or label) is released by antibody degradation. (See for example U.S. Publication No. 20050238649).

Typically, the linker is not substantially sensitive to the extracellular environment. As used herein, "not substantially sensitive to the extracellular environment," in the context of a linker, means that no more than about 20%, typically no more than about 15%, more typically no more than about 10%, and even more typically no more than about 5%, no more than about 3%, or no more than about 1% of the linkers, in a sample of immunoconjugate, are cleaved when the immunoconjugate presents in an extracellular environment (e.g., in plasma). Whether a linker is not substantially sensitive to the extracellular environment can be determined, for example, by incubating with plasma the immunoconjugate for a predetermined time period (e.g., 2, 4, 8, 16, or 24 hours) and then quantifying the amount of free drug present in the plasma.

In the present disclosure, the linker promotes cellular internalization. In the present disclosure, the linker promotes cellular internalization when conjugated to the therapeutic agent or label (Z). In the present disclosure, the linker promotes cellular internalization when conjugated to both the Z moiety and the anti-GCC antibody molecule.

A variety of exemplary linkers that can be used with the present compositions and methods are described in WO 2004-010957, U.S. Publication No. 20060074008, U.S. Publication No. 20050238649, and U.S. Publication No. 20060024317.

Examples of linkers capable of being used to couple an antibody molecule to a therapeutic agent or label include, for example, maleimidocaproyl (mc); maleimidocaproyl-p-aminobenzylcarbamate; maleimidocaproyl-peptide-aminobenzylcarbamate linkers, e.g., maleimidocaproyl-L-phenylalanine-L-lysine-p-aminobenzylcarbamate and maleimidocaproyl-L-valine-L-citrulline-p-aminobenzylcarbamate (vc); N-succinimidyl 3-(2-pyridyldithio)proprionate (also known as N-succinimidyl 4-(2-pyridyldithio)pentanoate or SPP); 4-succinimidyl-oxycarbonyl-2-methyl-2-(2-pyridyldithio)-toluene (SMPT); N-succinimidyl 3-(2-pyridyldithio)propionate (SPDP); N-succinimidyl 4-(2-pyridyldithio)butyrate (SPDB); 2-iminothiolane; S-acetylsuccinic anhydride; disulfide benzyl carbamate; carbonate; hydrazone linkers; N-(α-Maleimidoacetoxy) succinimide ester; N-[4-(p-Azidosalicylamido) butyl]-3'-(2'-pyridyldithio)propionamide (AMAS); N-[.beta.-Maleimidopropyloxy]succinimide ester (BMPS); [N-ε-Maleimidocaproyloxy]succinimide ester (EMCS); N-[γ-Maleimidobutyryloxy]succinimide ester (GMBS); Succinimidyl-4-[N-Maleimidomethyl]cyclohexane-1-carboxy-[6-amidocaproate] (LC-SMCC); Succinimidyl 6-(3-[2-pyridyldithio]-propionamido)hexanoate (LC-SPDP); m-Maleimidobenzoyl-N-hydroxysuccinimide ester (MBS); N-Succinimidyl[4-iodoacetyl]aminobenzoate (SIAB); Succinimidyl 4-[N-maleimidomethyl]cyclohexane-1-carboxylate (SMCC); N-Succinimidyl 3-[2-pyridyldithio]-propionamido (SPDP); [N-ε-Maleimidocaproyloxy]sulfosuccinimide ester (Sulfo-EMCS); N-[γ-Maleimidobutyryloxy]sulfosuccinimide ester (Sulfo-GMBS); 4-Sulfosuccinimidyl-6-methyl-α-(2-pyridyldithio)toluamido]hexanoate) (Sulfo-LC-SMPT); Sulfosuccinimidyl 6-(3'-[2-pyridyldithio]-propionamido)hexanoate (Sulfo-LC-SPDP); m-Maleimidobenzoyl-N-hydroxysulfosuccinimide ester (Sulfo-MBS); N-Sulfosuccinimidyl[4-iodoacetyl]aminobenzoate (Sulfo-SIAB); Sulfosuccinimidyl 4-[N-maleimidomethyl]cyclohexane-1-carboxylate (Sulfo-SMCC); Sulfosuccinimidyl 4-[p-maleimidophenyl]butyrate (Sulfo-SMPB); ethylene glycol-bis(succinic acidN-hydroxysuccinimide ester) (EGS); disuccinimidyl tartrate (DST); 1,4,7,10-tetraazacyclododecane-1,4,7,10-tetraacetic acid (DOTA); diethylenetriamine-pentaacetic acid (DTPA); and thiourea linkers.

In the present disclosure, the therapeutic agent is a cytostatic or cytotoxic agent. Examples include, without limitation, antimetabolites (e.g., azathioprine, 6-mercaptopurine, 6-thioguanine, fludarabine, pentostatin, cladribine, 5-fluorouracil (5FU), floxuridine (FUDR), cytosine arabinoside (cytarabine), methotrexate, trimethoprim, pyrimethamine, pemetrexed); alkylating agents (e.g., cyclophosphamide, mechlorethamine, uramustine, melphalan, chlorambucil, thiotepa/chlorambucil, ifosfamide, carmustine, lomustine, streptozocin, busulfan, dibromomannitol, cisplatin, carboplatin, nedaplatin, oxaliplatin, satraplatin, triplatin tetranitrate, procarbazine, altretamine, dacarbazine, mitozolomide, temozolomide); anthracyclines (e.g., daunorubicin, doxorubicin, epirubicin, idarubicin, valrubicin); antibiotics (e.g., dactinomycin, bleomycin, mithramycin, anthramycin, streptozotocin, gramicidin D, mitomycins (e.g., mitomycin C), duocarmycins (e.g., CC-1065), calicheamicins); antimitotic agents (including, e.g., maytansinoids, auristatins, dolastatins, cryptophycins, vinca alkaloids (e.g., vincristine, vinblastine, vindesine, vinorelbine), taxanes (e.g., paclitaxel, docetaxel, or a novel taxane (see, e.g., International Patent Publication No. WO 01/38318, published May 31, 2001)), and colchicines; topoisomerase inhibitors (e.g., irinotecan, topotecan, amsacrine, etoposide, teniposide, mitoxantrone); and proteasome inhibitors (e.g., peptidyl boronic acids).

In the present disclosure, the therapeutic agent is a maytansinoid. Maytansinoid compounds and methods for their conjugation to antibodies are described, for example, in Chari et al., Cancer Res., 52: 127-131 (1992); Widdison et al., J. Med. Chem. 49: 4392-4408 (2006); and U.S. Pat. Nos. 5,208,020 and 6,333,410. Examples of maytansinoids include maytansine analogues having a modified aromatic ring (e.g., C-19-dechloro, C-20-demethoxy, C-20-acyloxy) and those having modifications at other positions (e.g., C-9-CH, C-14-alkoxymethyl, C-14-hydroxymethyl or acyloxymethyl, C-15-hydroxy/acyloxy, C-15-methoxy, C-18-N-demethyl, 4,5-deoxy). In the present disclosure, the maytansinoid is N.sup.2'-deacetyl-N.sup.2'-(4-mercapto-1-oxopentyl)maytansine (DM3), N.sup.2'-deacetyl-N.sup.2'-(3-mercapto-1-oxopropyl)-maytansine (DM1), or N.sup.2'-deacetyl-N.sup.2'-(4-mercapto-4-methyl-1-oxopentyl)maytansine (DM4).

Maytansinoid compounds that comprise a sulfhydryl group can be coupled to antibodies using a heterobifunctional linker that is connected to the maytansinoid compound by way of a thioether or disulfide linkage. In the present disclosure, the linker is coupled to an amino group on the antibody (e.g., a terminal amino group or the epsilon amino group of a lysine residue. In the present disclosure, the heterobifunctional linker that is used to couple a maytansinoid compounds to an antibody is N-succinimidyl 3-(2-pyridyldithio)proprionate (also known as N-succinimidyl 4-(2-pyridyldithio)pentanoate, or SPP), 4-succinimidyl-oxycarbonyl-2-methyl-2-(2-pyridyldithio)-toluene (SMPT), N-succinimidyl 4[N-maleimidomethyl]cyclohexane-1-carboxylate (SMCC), N-succinimidyl 3-(2-pyridyldithio)propionate (SPDP); N-succinimidyl 4-(2-pyridyldithio)butyrate (SPDB), 2-iminothiolane, or S-acetylsuccinic anhydride.

In the present disclosure the therapeutic agent is a dolastatin. In some the present disclosure, the therapeutic agent is an auristatin, such as auristatin E (also known in the art as a derivative of dolastatin-10) or a derivative thereof. Auristatin compounds and methods for their conjugation to antibodies are described, for example, in Doronina et al., Nature Biotech., 21: 778-784 (2003); Hamblett et al, Clin. Cancer Res., 10: 7063-7070 (2004); Carter and Senter, Cancer J., 14 154-169 (2008); U.S. Pat. Nos. 7,498,298, 7,091,186, 6,884,869; 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414; U.S. Patent Publication Nos. 20090010945, 20060074008, 20080300192, 20050009751, 20050238649, and 20030083236; and International Patent Publication Nos. WO 04/010957 and WO 02/088172.

The auristatin can be, for example, an ester formed between auristatin E and a keto acid. For example, auristatin E can be reacted with paraacetyl benzoic acid or benzoylvaleric acid to produce AEB and AEVB, respectively. Other typical auristatins include auristatin phenylalanine phenylenediamine (AFP), monomethyl auristatin E (MMAE), and monomethyl auristatin F (MMAF).

In the present disclosure, the therapeutic agent is a radionuclide. Examples of radionuclides useful as toxins in radiation therapy include: $^{47}$Sc, $^{67}$Cu, $^{90}$Y, $^{109}$Pd, $^{123}$I, $^{125}$I, $^{131}$I, $^{186}$Re, $^{188}$Re, $^{199}$Au, $^{211}$At, $^{212}$Pb and $^{212}$B. Other radionuclides which have been used by those having ordinary skill in the art include: $^{32}$P and $^{33}$P, $^{71}$Ge, $^{77}$As, $^{103}$Pb, $^{105}$Rh, $^{111}$Ag, $^{119}$Sb, $^{121}$Sn, $^{131}$Cs, $^{143}$Pr, $^{161}$Tb, $^{177}$Lu, $^{191}$Os, $^{193}$MPt, $^{197}$Hg, all beta negative and/or auger emitters. Some preferred radionuclides include: $^{90}$Y, $^{131}$I, $^{211}$At and $^{212}$Pb/$^{212}$ Bi.

One having ordinary skill in the art may conjugate an anti-GCC antibody molecule to a radionuclide using well-known techniques. For example, Magerstadt, M. (1991) Antibody Conjugates And Malignant Disease, CRC Press, Boca Raton, Fla.,; and Barchel, S. W. and Rhodes, B. H., (1983) Radioimaging and Radiotherapy, Elsevier, NY, N.Y., teach the conjugation of various therapeutic and diagnostic radionuclides to amino acids of antibodies. Such reactions may be applied to conjugate radionuclides to anti-GCC antibody molecules of the disclosure with an appropriate chelating agent and/or linker.

### Anti-GCC Antibody Sequences

Rabbit monoclonal anti-GCC antibodies were generated by several methods, as is discussed in more detail in the Examples. Briefly, rabbit monoclonal antibodies MIL-44-148-2 and MIL-44-67-4 were generated by traditional immunization technology in rabbits. True rabbit-rabbit hybridomas were generated at Epitomics (Burlingame, CA) by fusing isolated B-cells from an immunized rabbit with Epitomics' proprietary fusion partner cell line (see U.S. Patents 7,402,409; 7,429,487; 7,462,697; 7,575,896; 7,732,168; and 8,062,867). Specificity of the antibodies against GCC was tested by ELISA and flow cytometry (FCM).

Table 1 below summarizes the rabbit monoclonal anti-GCC antibodies of the disclosure generated using the hGCC(ECD)/mIgG2a FcR-mutII immunogen.

The sequences of the light and heavy chain variable regions were determined Table 2 below is a summary of the SEQ ID NOs for the variable regions of several antibodies. The amino acid and nucleic acid sequences for the variable regions of each of the heavy and light chains for rabbit anti-GCC antibodies are shown in Tables 3 and 4, respectively.

The amino acid and nucleic acid sequences for each of the CDRs of the heavy and light chains for anti-GCC antibodies are shown in Tables 5 and 6, respectively.

Sequencing of the CDRs allowed determination of the abundance of residues that might serve as toxin conjugation sites. For example, an unpaired free cysteine in the antigen binding region could be a site for auristatin conjugation and a lysine could be a site for maytansine conjugation. Toxin conjugation to an amino acid of the CDR would raise the concern of altering the binding affinity of the antibody to GCC. Thus, in the present disclosure the CDRs lack an amino acid which can be conjugated to a therapeutic agent.

**Table 1: Summary of SEQ ID NOs for heavy and light chains of anti-GCC rabbit mAbs**

| mAb | IgG Chain | Nucleic Acid SEQ ID NO | Amino Acid SEQ ID NO |
|---|---|---|---|
| MIL-44-148-2 H2 | Heavy | 4 | 42 |
| MIL-44-148-2 L5 | Light | 5 | 43 |
| MIL-44-67-4 H2 | Heavy | 6 | 44 |
| MIL-44-67-4 L4 | Light | 7 | 45 |

**MIL-44-148-2 H2 Nucleic Acid (SEQ ID NO: 4)**

ATGGAGACTGGGCTGCGCTGGCTTCTCCTGGTCGCTGTGCTCAAAGGTGTCCAGTGTCAGTCAGTGAAGGAGTCCGG
GGGAGGCCTCTTCAAGCCAACGGATACCCTGACACTCACCTGCACCGTCTCTGGATTCTCCCTCAGTAGTCATAGAA
TGAACTGGGTCCGCCAGACTCCAGGGAAGGGGCTGGAATGGATCGCAATCATTACTCATAATAGTATCACATACTAC
GCGAGCTGGGCGAAAAGCCGATCCACCATCACCAGAAACACCAGCGAGAACACGGTGACTCTGAAAATGACCAGTCT
GACAGCCGCGGACACGGCCACTTATTTCTGTGCCAGAGAGGATAGTATGGGGTATTATTTTGACTTGTGGGGCCCAG
GCACCCTGGTCACCATCTCCTCA
GGGCAACCTAAGGCTCCATCAGTCTTCCCACTGGCCCCCTGCTGCGGGGACACACCCAGCTCCACGGTGACCCTGGG
CTGCCTGGTCAAAGGGTACCTCCCGGAGCCAGTGACCGTGACCTGGAACTCGGGCACCCTCACCAATGGGGTACGCA
CCTTCCCGTCCGTCCGGCAGTCCTCAGGCCTCTACTCGCTGAGCAGCGTGGTGAGCGTGACCTCAAGCAGCCAGCCC
GTCACCTGCAACGTGGCCCACCCAGCCACCAACACCAAAGTGGACAAGACCGTTGCGCCCTCGACATGCAGCAAGCC
CACGTGCCCACCCCCTGAACTCCTGGGGGGACCGTCTGTCTTCATCTTCCCCCCAAAACCCAAGGACACCCTCATGA
TCTCACGCACCCCCGAGGTCACATGCGTGGTGGTGGACGTGAGCCAGGATGACCCCGAGGTGCAGTTCACATGGTAC
ATAAACAACGAGCAGGTGCGCACCGCCCGGCCGCCGCTACGGGAGCAGCAGTTCAACAGCACGATCCGCGTGGTCAG
CACCCTCCCCATCGCGCACCAGGACTGGCTGAGGGGCAAGGAGTTCAAGTGCAAAGTCCACAACAAGGCACTCCCGG
CCCCCATCGAGAAAACCATCTCCAAAGCCAGAGGGCAGCCCCTGGAGCCGAAGGTCTACACCATGGGCCCTCCCCGG
GAGGAGCTGAGCAGCAGGTCGGTCAGCCTGACCTGCATGATCAACGGCTTCTACCCTTCCGACATCTCGGTGGAGTG
GGAGAAGAACGGGAAGGCAGAGGACAACTACAAGACCACGCCGGCCGTGCTGGACAGCGACGGCTCCTACTTCCTCT
ACAGCAAGCTCTCAGTGCCCACGAGTGAGTGGCAGCGGGGCGACGTCTTCACCTGCTCCGTGATGCACGAGGCCTTG
CACAACCACTACACGCAGAAGTCCATCTCCCGCTCTCCGGGTAAATGA

**MIL-44-148-2 H2 Amino Acid (SEQ ID NO: 42)**

METGLRWLLLVAVLKGVQCQSVKESGGGLFKPTDTLTLTCTVSGFSLSSHRMNWVRQTPGKGLEWIAIITHNSITYY
ASWAKSRSTITRNTSENTVTLKMTSLTAADTATYFCAREDSMGYYFDLWGPGTLVTISSGQPKAPSVFPLAPCCGDT
PSSTVTLGCLVKGYLPEPVTVTWNSGTLTNGVRTFPSVRQSSGLYSLSSVVSVTSSSQPVTCNVAHPATNTKVDKTV
APSTCSKPTCPPPELLGGPSVFIFPPKPKDTLMISRTPEVTCVVVDVSQDDPEVQFTWYINNEQVRTARPPLREQQF
NSTIRVVSTLPIAHQDWLRGKEFKCKVHNKALPAPIEKTISKARGQPLEPKVYTMGPPREELSSRSVSLTCMINGFY
PSDISVEWEKNGKAEDNYKTTPAVLDSDGSYFLYSKLSVPTSEWQRGDVFTCSVMHEALHNHYTQKSISRSPGK

**MIL-44-148-2 L5 Nucleic Acid (SEQ ID NO: 5)**

ATGGACACGAGGGCCCCCACTCAGCTGCTGGGGCTCCTGCTGCTCTGGCTCCCAGGTGCCAGATGTGCCTATGATAT GACCCAGACTCCAGCCTCTGTGGAGGTAGCTGTGGGAGGCACAGTCACCATCAAGTGCCAGGCCAGTCAGAGCATTA GTAACTGGTTAGCCTGGTATCAGCAGAAACCAGGGCAGTCTCCCAAGCCCCTGATCTACAGGGCATCCACTCTGGCA TCTGGGGTCTCATCGCGGTTCAGAGGCAGTGGATCTGGGACACAGTTCACTCTCACCATCAGTGGCGTGGAGTGTGC CGATGCTGCCACTTACTACTGTCAGCAGACTTATACTAATAATCATCTTGATAATGGTTTCGGCGGAGGGACCGAGG TGGTGGTCAAA

GGTGATCCAGTTGCACCTACTGTCCTCATCTTCCCACCAGCTGCTGATCAGGTGGCAACTGGAACAGTCACCATCGT GTGTGTGGCGAATAAATACTTTCCCGATGTCACCGTCACCTGGGAGGTGGATGGCACCACCCAAACAACTGGCATCG AGAACAGTAAAACACCGCAGAATTCTGCAGATTGTACCTACAACCTCAGCAGCACTCTGACACTGACCAGCACACAG TACAACAGCCACAAAGAGTACACCTGCAGGGTGACCCAGGGCACGACCTCAGTCGTCCAGAGCTTCAATAGGGGTGA CTGTTAG

**MIL-44-148-2 L5 Amino Acid (SEQ ID NO: 43)**

MDTRAPTQLLGLLLLWLPGARCAYDMTQTPASVEVAVGGTVTIKCQASQSISNWLAWYQQKPGQSPKPLIYRASTLA SGVSSRFRGSGSGTQFTLTISGVECADAATYYCQQTYTNNHLDNGFGGGTEVVVKGDPVAPTVLIFPPAADQVATGT VTIVCVANKYFPDVTVTWEVDGTTQTTGIENSKTPQNSADCTYNLSSTLTLTSTQYNSHKEYTCRVTQGTTSVVQSF NRGDC

**MIL-44-67-4 H2 Nucleic Acid (SEQ ID NO: 6)**

ATGGAGACTGGGCTGCGCTGGCTTCTCCTGGTCGCTGTGCTCAAAGGTGTCCAGTGTCAGTCGGTGGAGGAGTCCGG GGGTCGCCTGGTCACGCCTGGGACACCCCTGACACTCACCTGCACAGCCTCTGGATCCGACATCAGTAACTATGCAA TATCCTGGGTCCGCCAGGCTCCAGGGAAGGGGCTGGAATTCATCGGATATATTAGTTATGGTAAAAGTATATACTAC GCGAGCTGGGCGAAAGGCCGGTTCGCCATCTCCAAAACCTCGTCGACCACGGTGGATCTGGAAATCACCAGTCCGAC AACCGAGGACACGGCCACCTATTTTTGTGCCAGAGAGGATAGTGCTACTTATAGTCCTAACTTGTGGGGCCCAGGCA CCCTGGTCACCGTCTCCTCA

GGGCAACCTAAGGCTCCATCAGTCTTCCCACTGGCCCCCTGCTGCGGGGACACACCCAGCTCCACGGTGACCCTGGG CTGCCTGGTCAAAGGGTACCTCCCGGAGCCAGTGACCGTGACCTGGAACTCGGGCACCCTCACCAATGGGGTACGCA CCTTCCCGTCCGTCCGGCAGTCCTCAGGCCTCTACTCGCTGAGCAGCGTGGTGAGCGTGACCTCAAGCAGCCAGCCC GTCACCTGCAACGTGGCCCACCCAGCCACCAACACCAAAGTGGACAAGACCGTTGCGCCCTCGACATGCAGCAAGCC CACGTGCCCACCCCCTGAACTCCTGGGGGGACCGTCTGTCTTCATCTTCCCCCCAAAACCCAAGGACACCCTCATGA TCTCACGCACCCCCGAGGTCACATGCGTGGTGGTGGACGTGAGCCAGGATGACCCCGAGGTGCAGTTCACATGGTAC ATAAACAACGAGCAGGTGCGCACCGCCCGGCCGCCGCTACGGGAGCAGCAGTTCAACAGCACGATCCGCGTGGTCAG CACCCTCCCCATCGCGCACCAGGACTGGCTGAGGGGCAAGGAGTTCAAGTGCAAAGTCCACAACAAGGCACTCCCGG CCCCCATCGAGAAAACCATCTCCAAAGCCAGAGGGCAGCCCCTGGAGCCGAAGGTCTACACCATGGGCCCTCCCCGG GAGGAGCTGAGCAGCAGGTCGGTCAGCCTGACCTGCATGATCAACGGCTTCTACCCTTCCGACATCTCGGTGGAGTG GGAGAAGAACGGGAAGGCAGAGGACAACTACAAGACCACGCCGGCCGTGCTGGACAGCGACGGCTCCTACTTCCTCT ACAGCAAGCTCTCAGTGCCCACGAGTGAGTGGCAGCGGGGCGACGTCTTCACCTGCTCCGTGATGCACGAGGCCTTG CACAACCACTACACGCAGAAGTCCATCTCCCGCTCTCCGGGTAAATGA

**MIL-44-67-4 H2 Amino Acid (SEQ ID NO: 44)**

METGLRWLLLVAVLKGVQCQSVEESGGRLVTPGTPLTLTCTASGSDISNYAISWVRQAPGKGLEFIGYISYGKSIYY
ASWAKGRFAISKTSSTTVDLEITSPTTEDTATYFCAREDSATYSPNLWGPGTLVTVSSGQPKAPSVFPLAPCCGDTP
SSTVTLGCLVKGYLPEPVTVTWNSGTLTNGVRTFPSVRQSSGLYSLSSVVSVTSSSQPVTCNVAHPATNTKVDKTVA
PSTCSKPTCPPPELLGGPSVFIFPPKPKDTLMISRTPEVTCVVVDVSQDDPEVQFTWYINNEQVRTARPPLREQQFN
STIRVVSTLPIAHQDWLRGKEFKCKVHNKALPAPIEKTISKARGQPLEPKVYTMGPPREELSSRSVSLTCMINGFYP
SDISVEWEKNGKAEDNYKTTPAVLDSDGSYFLYSKLSVPTSEWQRGDVFTCSVMHEALHNHYTQKSISRSPGK

**MIL-44-67-4 L4 Nucleic acid (SEQ ID NO: 7)**

ATGGACACGAGGGCCCCCACTCAGCTGCTGGGGCTCCTGCTGCTCTGGCTCCCAGGTGCCAGATGTGCCTATGATAT
GACCCAGACTCCAGCCTCTGTGGAGGTAGCTGTGGGAGGCACAGTCACCATCAAGTGCCAGGCCAGTCAGAGTATTA
ACACCTACTTAGCCTGGTATCAGCAGAAACCAGGGCAGCGTCCCAAGCTCCTGATCTACAGGGCATCCACTCTGGCA
TCTGGGGTCTCATCGCGGTTCAAAGGCAGTGGATCTGGGACAGAGTTCACTCTCACCATCAGCGGCGTGGAGTGTGC
CGATGCTGCCACTTACTACTGTCAACAGGGTTATAGTTATAATAATCTTGATCGTGCTTTCGGCGGAGGGACCGAGG
TGGTGGTCACA

GGTGATCCAGTTGCACCTACTGTCCTCATCTTCCCACCAGCTGCTGATCAGGTGGCAACTGGAACAGTCACCATCGT
GTGTGTGGCGAATAAATACTTTCCCGATGTCACCGTCACCTGGGAGGTGGATGGCACCACCCAAACAACTGGCATCG
AGAACAGTAAAACACCGCAGAATTCTGCAGATTGTACCTACAACCTCAGCAGCACTCTGACACTGACCAGCACACAG
TACAACAGCCACAAAGAGTACACCTGCAAGGTGACCCAGGGCACGACCTCAGTCGTCCAGAGCTTCAATAGGGGTGA
CTGTTAG

**MIL-44-67-4 L4 Amino acid (SEQ ID NO: 45)**

MDTRAPTQLLGLLLLWLPGARCAYDMTQTPASVEVAVGGTVTIKCQASQSINTYLAWYQQKPGQRPKLLIYRASTLA
SGVSSRFKGSGSGTEFTLTISGVECADAATYYCQQGYSYNNLDRAFGGGTEVVVTGDPVAPTVLIFPPAADQVATGT
VTIVCVANKYFPDVTVTWEVDGTTQTTGIENSKTPQNSADCTYNLSSTLTLTSTQYNSHKEYTCKVTQGTTSVVQSF
NRGDC

**Table 2: Summary of SEQ ID NOs for variable regions of anti-GCC rabbit mAbs**

| mAb | IgG Chain | Nucleic Acid SEQ ID NO | Amino Acid SEQ ID NO |
|---|---|---|---|
| MIL-44-148-2 H2 | Heavy | 10 | 11 |
| MIL-44-148-2 L5 | Light | 12 | 13 |
| MIL-44-67-4 H2 | Heavy | 14 | 15 |
| MIL-44-67-4 L4 | Light | 16 | 17 |

**Table 3: Amino Acid Sequences of mAb variable regions of anti-GCC rabbit mAbs**

| mAb | IgG Chain | SEQ ID NO: | Amino Acid Sequence |
|---|---|---|---|
| MIL-44-148-2 | Heavy | 11 | QSVKESGGGLFKPTDTLTLTCTVSGFSLSSHRMNWVRQTPGKGLEWIA IITHNSITYYASWAKSRSTITRNTSENTVTLKMTSLTAADTATYFCAR EDSMGYYFDLWGPGTLVTISS |

(continued)

| mAb | IgG Chain | SEQ ID NO: | Amino Acid Sequence |
|---|---|---|---|
| MIL-44-148-2 | Light | 13 | AYDMTQTPASVEVAVGGTVTIKCQASQSISNWLAWYQQ KPGQSPKPLIYRASTLASGVSSRFRGSGSGTQFTLTISGVECADAATYYC QQTYTNNHLDNGFGGGTEVVVK |
| MIL-44-67-4 | Heavy | 15 | QSVEESGGRLVTPGTPLTLTCTASGSDISNYAISWVRQAPG KGLEFIGYISYGKSIYYASWAKGRFAISKTSSTTVDLEITSPTTEDTATYFCAR EDSATYSPNLWGPGTLVTVSS |
| MIL-44-67-4 | Light | 17 | AYDMTQTPASVEVAVGGTVTIKCQASQSINTYLAWYQQ KPGQRPKLLIYRASTLASGVSSRFKGSGSGTEFTLTISGVECADAATYYC QQGYSYNNLDRAFGGGTEVVVT |

**Table 4: Nucleic Acid Sequences of mAb variable regions of anti-GCC rabbit mAbs**

| mAb | IgG Chain | SEQ ID NO: | Nucleic Acid Sequence |
|---|---|---|---|
| MIL-44-148-2 | Heavy | 10 | CAGTCAGTGAAGGAGTCCGGGGGAGGCCTCTTCAAGCCAACGGATACCCTGACACT<br>CACCTGCACCGTCTCTGGATTCTCCCTCAGTAGTCATAGAATGAACTGGGTCCGCC<br>AGACTCCAGGGAAGGGGCTGGAATGGATCGCAATCATTACTCATAATAGTATCACA<br>TACTACGCGAGCTGGGCGAAAAGCCGATCCACCATCACCAGAAACACCAGCGAGAA<br>CACGGTGACTCTGAAAATGACCAGTCTGACAGCCGCGGACACGGCCACTTATTTCT<br>GTGCCAGAGAGGATAGTATGGGGTATTATTTTGACTTGTGGGGCCCAGGCACCCTG<br>GTCACCATCTCCTCA |
| MIL-44-148-2 | Light | 12 | GCCTATGATATGACCCAGACTCCAGCCTCTGTGGAGGTAGCTGTGGGAGGCACAGT<br>CACCATCAAGTGCCAGGCCAGTCAGAGCATTAGTAACTGGTTAGCCTGGTATCAGC<br>AGAAACCAGGGCAGTCTCCCAAGCCCCTGATCTACAGGGCATCCACTCTGGCATCT<br>GGGGTCTCATCGCGGTTCAGAGGCAGTGGATCTGGGACACAGTTCACTCTCACCAT<br>CAGTGGCGTGGAGTGTGCCGATGCTGCCACTTACTACTGTCAGCAGACTTATACTA<br>ATAATCATCTTGATAATGGTTTCGGCGGAGGGACCGAGGTGGTGGTCAAA |
| MIL-44-67-4 | Heavy | 14 | CAGTCGGTGGAGGAGTCCGGGGGTCGCCTGGTCACGCCTGGGACACCCCTGACACT<br>CACCTGCACAGCCTCTGGATCCGACATCAGTAACTATGCAATATCCTGGGTCCGCC<br>AGGCTCCAGGGAAGGGGCTGGAATTCATCGGATATATTAGTTATGGTAAAAGTATA<br>TACTACGCGAGCTGGGCGAAAGGCCGGTTCGCCATCTCCAAAACCTCGTCGACCAC<br>GGTGGATCTGGAAATCACCAGTCCGACAACCGAGGACACGGCCACCTATTTTTGTG<br>CCAGAGAGGATAGTGCTACTTATAGTCCTAACTTGTGGGGCCCAGGCACCCTGGTC<br>ACCGTCTCCTCA |
| MIL-44-67-4 | Light | 16 | GCCTATGATATGACCCAGACTCCAGCCTCTGTGGAGGTAGCTGTGGGAGGCACAGT<br>CACCATCAAGTGCCAGGCCAGTCAGAGTATTAACACCTACTTAGCCTGGTATCAGC<br>AGAAACCAGGGCAGCGTCCCAAGCTCCTGATCTACAGGGCATCCACTCTGGCATCT<br>GGGGTCTCATCGCGGTTCAAAGGCAGTGGATCTGGGACAGAGTTCACTCTCACCAT<br>CAGCGGCGTGGAGTGTGCCGATGCTGCCACTTACTACTGTCAACAGGGTTATAGTT<br>ATAATAATCTTGATCGTGCTTTCGGCGGAGGGACCGAGGTGGTGGTCACA |

**Table 5: Amino Acid Sequences of CDRs of anti-GCC rabbit mAbs**

| mAb | IgG | SEQ ID NO: | Amino Acid Sequence |
|---|---|---|---|
| MIL-44-148-2-H2 | VH CDR1 | 21 | SHRMN |
| MIL-44-148-2-H2 | VH CDR2 | 22 | IITHNSITYYASWAKS |
| MIL-44-148-2-H2 | VH CDR3 | 23 | EDSMGYYFDL |
| MIL-44-148-2-L5 | VK CDR1 | 27 | QASQSISNWLA |
| MIL-44-148-2-L5 | VK CDR2 | 28 | RASTLAS |
| MIL-44-148-2-L5 | VK CDR3 | 29 | QQTYTNNHLDNG |
| MIL-44-67-4 H2 | VH CDR1 | 33 | NYAIS |
| MIL-44-67-4 H2 | VH CDR2 | 34 | YISYGKSIYYASWAKG |
| MIL-44-67-4 H2 | VH CDR3 | 35 | EDSATYSPNL |
| MIL-44-67-4 L4 | VK CDR1 | 39 | QASQSINTYLA |
| MIL-44-67-4 L4 | VK CDR2 | 40 | RASTLAS |
| MIL-44-67-4 L4 | VK CDR3 | 41 | QQGYSYNNLDRA |

**Table 6: Nucleic Acid Sequences of CDRs of anti-GCC rabbit mAbs**

| mAb | IgG | SEQ ID NO: | Nucleic Acid Sequence |
|---|---|---|---|
| MIL-44-148-2-H2 | VH CDR1 | 18 | AGTCATAGAATGAAC |
| MIL-44-148-2-H2 | VH CDR2 | 19 | ATCATTACTCATAATAGTATCACATACTACGCGAGCTGGGCGAAAAGC |
| MIL-44-148-2-H2 | VH CDR3 | 20 | GAGGATAGTATGGGGTATTATTTTGACTTG |
| MIL-44-148-2-L5 | VK CDR1 | 24 | CAGGCCAGTCAGAGCATTAGTAACTGGTTAGCC |
| MIL-44-148-2-L5 | VK CDR2 | 25 | AGGGCATCCACTCTGGCATCT |
| MIL-44-148-2-L5 | VK CDR3 | 26 | CAGCAGACTTATACTAATAATCATCTTGATAATGGT |
| MIL-44-67-4 H2 | VH CDR1 | 30 | AACTATGCAATATCC |
| MIL-44-67-4 H2 | VH CDR2 | 31 | TATATTAGTTATGGTAAAAGTATATACTACGCGAGCTGGGCGAAAGGC |
| MIL-44-67-4 H2 | VH CDR3 | 32 | AGTCCTAACTTG |
| MIL-44-67-4 L4 | VK CDR1 | 36 | CAGGCCAGTCAGAGTATTAACACCTACTTAGCC |
| MIL-44-67-4 L4 | VK CDR2 | 37 | AGGGCATCCACTCTGGCATCT |
| MIL-44-67-4 L4 | VK CDR3 | 38 | CAACAGGGTTATAGTTATAATAATCTTGATCGTGCT |

**Therapeutic Uses**

The rabbit monoclonal anti-GCC antibody molecules described herein have *in vitro* and *in vivo* utilities. For example, these antibody molecules can be administered to cells in culture, e.g. *in vitro* or *ex vivo,* or administered in a subject, e.g., *in vivo,* to treat and/or prevent, a variety of disorders. In certain embodiments of therapeutic applications of the disclosure, the rabbit monoclonal anti-GCC antibody molecules of the disclosure are humanized, using one or more techniques described above herein.

The antibody molecules, immunoconjugates, and fusion proteins described herein can be used to modulate an activity or function of a GCC protein, such as ligand binding (e.g., binding of ST or guanylin), GCC-mediated signal transduction, maintenance of intestinal fluid, electrolyte homeostasis, intracellular calcium release (calcium flux), cell differentiation, cell proliferation, or cell activation.

In one aspect, the disclosure features a method of killing, inhibiting or modulating the growth of, or interfering with the metabolism of, a GCC-expressing cell. In the present disclosure, the disclosure describes a method of inhibiting GCC-mediated cell signaling or a method of killing a cell. The method may be used with any cell or tissue which expresses GCC, such as a cancerous cell. Examples of cancerous cells which express GCC include, but are not limited to, a cell from a cancer of gastrointestinal origin (e.g., colorectal cancer, stomach cancer, small intestine cancer, or esophageal cancer), pancreatic cancer, lung cancer (e.g., squamous cell carcinoma, adenosquamous carcinoma, adenocarcinoma), soft-tissue sarcomas such as leiosacroma or rhabdomyosarcoma, gastrointestinal or bronchopulmonary neuroendocrine tumors, or neuroectodermal tumors, or any metastatic lesions thereof. Nonlimiting examples of GCC-expressing cells include T84 human colonic adenocarcinoma cells, fresh or frozen colonic tumor cells, and cells comprising a recombinant nucleic acid encoding GCC or a portion thereof.

Methods of the disclosure include the steps of contacting the cell with an anti-GCC antibody molecule or immunoconjugate thereof, as described herein, in an effective amount, i.e., amount sufficient to inhibit GCC-mediated cell signaling or an amount sufficient to kill the cell. The method can be used on cells in culture, e.g. *in vitro, in vivo, ex vivo,* or *in situ.* For example, cells that express GCC (e.g., cells collected by biopsy of a tumor or metastatic lesion; cells from an established cancer cell line; or recombinant cells), can be cultured *in vitro* in culture medium and the contacting step can be effected by adding the anti-GCC antibody molecule or immunoconjugate to the culture medium. In methods of killing a cell, the method comprises using a naked anti-GCC antibody molecule, or an immunoconjugate comprising an anti-GCC antibody molecule and a cytotoxic agent. The method will result in killing of cells expressing GCC, including in particular tumor cells expressing GCC (e.g., colonic tumor cells).

The rabbit monoclonal antibodies of the disclosure, or humanized versions thereof, can be tested for cellular internalization after binding to GCC using immunofluorescence microscopy techniquies well known to those skilled in the art. Such antibodies that are confirmed to internalize would be useful when linked to a cytotoxic moiety for therapeutic purposes, or to a moiety for cell imaging. Antibodies which do not internalize can still be used for diagnostic purposes or for therapeutic methods using naked antibody designed to elicit an antibody-dependent cell-mediated cytotoxic response, or perhaps for liposome delivery methods.

Anti-GCC antibody molecules of the present disclosure bind to extracellular domains of GCC or portions thereof in cells expressing the antigen. As a result, when practicing the methods of the present disclosure to kill, suppress, or detect cancerous cells, the antibodies or antigen binding fragments, bind to all such cells, not only to cells which are fixed or cells whose intracellular antigenic domains are otherwise exposed to the extracellular environment. Consequently, binding of the antibodies or antigen binding fragments, is concentrated in areas where there are cells expressing GCC, irrespective of whether these cells are fixed or unfixed, viable or necrotic. Additionally or alternatively, the anti-GCC antibody molecules, bind to and are internalized with GCC upon binding cells expressing the antigen.

The method also can be performed on cells present in a subject, as part of an *in vivo* protocol. In the present disclosure, the subject is a human subject. Alternatively, the subject can be a mammal expressing a GCC antigen with which an anti-GCC antibody molecule disclosed herein cross-reacts. An anti-GCC antibody molecule or immunoconjugate thereof can be administered to a human subject for therapeutic purposes. An anti-GCC antibody molecule or immunoconjugate also can be administered to a non-human mammal expressing the GCC-like antigen with which the antibody cross-reacts (e.g., a primate, pig or mouse) for veterinary purposes or as an animal model of human disease. Animal models may be useful for evaluating the therapeutic efficacy of antibodies of the disclosure (e.g., testing of dosages and time courses of administration). For *in vivo* the present disclosure, the contacting step is effected in a subject and includes administering an anti-GCC antibody molecule or immunoconjugate thereof to the subject under conditions effective to permit both binding of the antibody molecule to the extracellular domain of GCC expressed on the cell, and the treating of the cell.

In the present disclosure, the disclosure describes a method of treating cancer by administering an anti-GCC antibody molecule or an immunoconjugate comprising an anti-GCC antibody molecule and a cytotoxic agent to a patient in need of such treatment. The method can be used for the treatment of any cancerous disorder which includes at least some cells that express the GCC antigen. As used herein, the term "cancer" is meant to include all types of cancerous

growths or oncogenic processes, metastatic tissues or malignantly transformed cells, tissues, or organs, irrespective of histopathologic type or stage of invasiveness. The terms "cancer" and "tumor" may be used interchangeably (e.g., when used in the context of treatment methods, "treatment of a cancer" and "treatment of a tumor" have the same meaning).

In the present disclosure, the treatment is sufficient to reduce or inhibit the growth of the subject's tumor, reduce the number or size of metastatic lesions, reduce tumor load, reduce primary tumor load, reduce invasiveness, prolong survival time, or maintain or improve the quality of life.

Examples of cancerous disorders include, but are not limited to, solid tumors, soft tissue tumors, and metastatic lesions. Examples of solid tumors include malignancies, e.g., sarcomas, adenocarcinomas, and carcinomas, of the various organ systems, such as those affecting colon. Adenocarcinomas include malignancies such as non-small cell carcinoma of the lung. Metastatic lesions of the aforementioned cancers can also be treated or prevented using the methods and compositions of the disclosure.

In the present disclosure, the GCC-expressing cancer to be treated is a primary or metastatic cancer of gastrointestinal origin, such as colorectal cancer, stomach cancer, small intestine cancer, or esophageal cancer. In the present disclosure, the GCC_expressing cancer to be treated is primary or metastatic pancreatic cancer. In the present disclosure, the GCC_expressing cancer to be treated is primary or metastatic lung cancer, such as squamous cell carcinoma, adenosquamous carcinoma, or adenocarcinoma. In the present disclosure, the GCC-expressing cancer to be treated is a sarcoma, such as leiomyosarcoma or rhabdomyosarcoma. In the present disclosure, the GCC-expressing cancer to be treated is a primary or metastasized neuroectodermal tumor, such as aphaechromotcytoma or a paraganglioma. In the present disclosure, the GCC-expressing cancer is a primary or a metastatized bronchopulmonary or a gastrointestinal neuroendocrine tumor.

The method can be useful in treating a relevant disorder at any stage or subclassification. For example, method can be used to treat early or late stage colon cancer, or colon cancer of any of stages 0, I, IIA, IIB, IIIA, IIIB, IIIC, and IV.

In the present disclosure, the method for treating GCC-expressing cancer (e.g., colorectal cancer, stomach cancer, small intestine cancer, esophageal cancer, pancreatic cancer, lung cancer, leiomyosarcoma, rhabdomyosarcoma, neuroendocrine tumor, neuroectodermal tumor etc.) comprises administering to a patient in need of such treatment a naked anti-GCC antibody molecule described herein. In the present disclosure, the method comprises administering an immunoconjugate comprising an anti-GCC antibody molecule described herein and a cytotoxic agent such as a maytansanoid or an auristatin, or derivatives thereof. Methods of administering antibody molecules and immunoconjugates are described above. Suitable dosages of the molecules used will depend on the age and weight of the subject and the particular compound used.

In the present disclosure, the anti-GCC antibody molecule or immunoconjugate is administered in treatment cycles. A "treatment cycle" consists of a treatment period, during which the anti-GCC antibody molecule or immunoconjugate is administered as described above, followed by a rest period, during which no anti-GCC antibody molecule or immunoconjugate is administered. The treatment cycle can be repeated as necessary to achieve the desired effect.

The anti-GCC antibodies described herein (e.g., naked anti-GCC antibody molecules or immunoconjugates comprising an anti-GCC antibody molecule and a therapeutic agent) may be used in combination with other therapies. For example, the combination therapy can include a composition of the present disclosure co-formulated with, and/or co-administered with, one or more additional therapeutic agents, e.g., one or more anti-cancer agents, e.g., cytotoxic or cytostatic agents, hormone treatment, vaccines, and/or other immunotherapies. In the present disclosure, the anti-GCC antibodies are administered in combination with other therapeutic treatment modalities, including surgery, radiation, cryosurgery, and/or thermotherapy. Such combination therapies may advantageously utilize lower dosages of the administered therapeutic agents, thus avoiding possible toxicities or complications associated with the various monotherapies.

Administered "in combination," as used herein, means that two (or more) different treatments are delivered to the subject during the course of the subject's affliction with the disorder, e.g., the two or more treatments are delivered after the subject has been diagnosed with the disorder and before the disorder has been cured or eliminated. In the present disclosure, the delivery of one treatment is still occurring when the delivery of the second begins, so that there is overlap. This is sometimes referred to herein as "simultaneous" or "concurrent delivery." In the present disclosure, the delivery of one treatment ends before the delivery of the other treatment begins. In the present disclosure of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In the present disclosure, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

In the present disclosure, the anti-GCC antibody molecule or immunoconjugate thereof is used in combination

with a chemotherapeutic agent. Non-limiting examples of DNA damaging chemotherapeutic agents include topoisomerase I inhibitors (e.g., irinotecan, topotecan, camptothecin and analogs or metabolites thereof, and doxorubicin); topoisomerase II inhibitors (e.g., etoposide, teniposide, and daunorubicin); alkylating agents (e.g., melphalan, chlorambucil, busulfan, thiotepa, ifosfamide, carmustine, lomustine, semustine, streptozocin, decarbazine, methotrexate, mitomycin C, and cyclophosphamide); DNA intercalators (e.g., cisplatin, oxaliplatin, and carboplatin); DNA intercalators and free radical generators such as bleomycin; and nucleoside mimetics (e.g., 5-fluorouracil, capecitibine, gemcitabine, fludarabine, cytarabine, mercaptopurine, thioguanine, pentostatin, and hydroxyurea).

Chemotherapeutic agents that disrupt cell replication include: paclitaxel, docetaxel, and related analogs; vincristine, vinblastin, and related analogs; thalidomide, lenalidomide, and related analogs (e.g., CC-5013 and CC-4047); protein tyrosine kinase inhibitors (e.g., imatinib mesylate and gefitinib); proteasome inhibitors (e.g., bortezomib); NF-κB inhibitors, including inhibitors of IκB kinase; antibodies which bind to proteins overexpressed in cancers and thereby downregulate cell replication (e.g., trastuzumab, rituximab, cetuximab, and bevacizumab); and other inhibitors of proteins or enzymes known to be upregulated, over-expressed or activated in cancers, the inhibition of which downregulates cell replication.

The selection of therapeutic agent(s) or treatment modality to be combined with an anti-GCC antibody molecule or immunoconjugate of the disclosure will depend on the disorder to be treated. The additional agent(s) or treatment modality may include, for example, standard approved therapies for the indication being treated. For example, when the anti-GCC antibody molecule or immunoconjugate thereof is used to treat colon cancer, it may be used in combination with, e.g., surgery; radiation therapy; 5-fluorouricil (5-FU), capecitibine, leucovorin, irinotecan, oxaliplatin, bevacizumab, cetuximab, panitumum, or combinations thereof (e.g., oxaliplatin/capecitibine (XELOX), 5-fluorouricil/leucovorin/-oxaliplatin (FOLFOX), 5-fluorouricil/leucovorin/irinotecan (FOLFIRI), FOLFOX plus bevacizumab, or FOLFIRI plus bevacizumab).

In another aspect, the disclosure features the use of an anti-GCC antibody molecule or immunoconjugate as described herein in the manufacture of a medicament. In the present disclosure, the medicament is for treating cancer, e.g., a gastrointestinal cancer. In the present disclosure, the medicament comprises an anti-GCC antibody molecule having features summarized in Tables 1-6. In the present disclosure, the medicament comprises a MIL-44-148-2 or a MIL-44-67-4 antibody molecule, or humanized versions thereof.

**Antibody Labeling and Detection**

Anti-GCC antibody molecules used in methods described herein, e.g., in the *in vitro* and *in vivo* detection, e.g., diagnostic, staging, or imaging methods, can be directly or indirectly labeled with a detectable substance to facilitate detection of the bound or unbound binding agent. Suitable detectable substances include various biologically active enzymes, ligands, prosthetic groups, fluorescent materials, luminescent materials, chemiluminescent materials, bioluminescent materials, chromophoric materials, electron dense materials, paramagnetic (e.g., nuclear magnetic resonance active) materials, and radioactive materials. In the present disclosure, the anti-GCC antibody molecule is coupled to a radioactive ion, e.g., indium ($^{111}In$), iodine ($^{131}I$ or $^{125}I$), yttrium ($^{90}Y$), lutetium ($^{177}Lu$), actinium ($^{225}Ac$), bismuth ($^{212}Bi$ or $^{213}Bi$), sulfur ($^{35}S$), carbon ($^{14}C$), tritium ($^{3}H$), rhodium ($^{188}Rh$), technetium ($^{99}mTc$), praseodymium, or phosphorous ($^{32}P$); or a positron-emitting radionuclide, e.g., carbon-11 ($^{11}C$) potassium-40 ($^{40}K$), nitrogen-13 ($^{13}N$), oxygen-15 ($^{15}O$), fluorine-18 ($^{18}F$), gallium ($^{68}Ga$), and iodine-121 ($^{121}I$). Additional radioactive agents that can be conjugated to the antibodies of the disclosure for use in in *in vitro* or *in vivo* diagnostic/detection methods are described below.

Exemplary labels include fluorophores such as rare earth chelates or fluorescein and its derivatives, rhodamine and its derivatives, dansyl, umbelliferone, luceriferases, e.g., firefly luciferase and bacterial luciferase (U.S. Pat. No. 4,737,456), luciferin, and 2,3-dihydrophthalazinediones. Other exemplary labels include horseradish peroxidase (HRP), alkaline phosphatase, galactosidase, glucoamylase, lysozyme, saccharide oxidases, e.g., glucose oxidase, galactose oxidase, and glucose 6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

Fluorophore and chromophore labeled antibody molecules can be prepared from standard moieties known in the art. Since antibodies and other proteins absorb light having wavelengths up to about 310 nm, the fluorescent moieties should be selected to have substantial absorption at wavelengths above 310 nm and preferably above 400 nm. A variety of suitable fluorescent compounds and chromophores are described by Stryer Science, 162:526 (1968) and Brand, L. et al. Annual Review of Biochemistry, 41:843-868 (1972). The antibodies can be labeled with fluorescent chromophore groups by conventional procedures such as those disclosed in U.S. Pat. Nos. 3,940,475, 4,289,747, and 4,376,110.

One group of fluorescers having a number of the desirable properties described above is the xanthene dyes, which include the fluoresceins derived from 3,6-dihydroxy-9-henylxanthhydrol and resamines and rhodamines derived from 3,6-diamino-9-phenylxanthydrol and lissanime rhodamine B. The rhodamine and fluorescein derivatives of 9-o-carboxyphenylxanthhydrol have a 9-o-carboxyphenyl group. Fluorescein compounds having reactive coupling groups

such as amino and isothiocyanate groups such as fluorescein isothiocyanate and fluorescamine are readily available. Another group of fluorescent compounds are the naphthylamines, having an amino group in the α or β position.

Labeled antibody molecules can be used, for example, diagnostically and/or experimentally in a number of contexts, including (i) to isolate a predetermined antigen by standard techniques, such as affinity chromatography or immunoprecipitation; (ii) to detect a predetermined antigen (e.g., in a cellular lysate or cell supernatant) in order to evaluate the abundance and pattern of expression of the protein; (iii) to monitor protein levels in tissue as part of a clinical testing procedure, e.g., to determine the efficacy of a given treatment regimen.

In Vitro Diagnostics

The anti-GCC antibodies and immunoconjugates described herein can be used to detect the presence or absence of GCC, e.g., to detect the presence or absence of GCC in an *ex vivo* biological sample obtained from a subject (i.e., in vitro detection), or to detect the presence or distribution or absence of GCC in a subject (i.e., *in vivo* detection). Such detection methods are useful to detect or diagnose a variety of disorders, or to guide therapeutic decisions. The term "detecting" as used herein encompasses quantitative or qualitative detection. Detecting GCC or GCC protein, as used herein, means detecting intact GCC protein or detecting a portion of the GCC protein that comprises the epitope to which the anti-GCC antibody molecule binds.

Accordingly, in another aspect, the disclosure features, a method of detecting GCC expression in a biological sample such as a cell or tissue, e.g., a tumor cell, or a tumor having one or more cells that express GCC. The method comprises: contacting a biological sample, with an anti-GCC antibody molecule described herein (e.g., MIL-44-148-2 or MIL-44-67-4), under conditions which allow formation of a complex between the anti-GCC antibody molecule and GCC protein; and detecting formation of a complex between the anti-GCC antibody molecule and GCC protein, to thereby detect the presence of GCC protein, e.g., to detect a GCC expressing cell or tumor.

In the present disclosure, the anti-GCC antibody molecule is an immunoconjugate comprising a detectable label. The detectable label can be a radioactive agent. Alternatively, the detectable label is a non-radioactive agent (e.g. a fluorophore or a chromophore as described above).

In the present disclosure, the biological sample include normal and/or cancerous cells or tissues that express GCC. Examples of normal cells or tissues that express GCC include but are not limited cells or tissue or gastrointestinal origin, particularly normal colorectal cells or tissue. Examples of cancerous cells or tissues that express GCC include but are not limited to: cancer of gastrointestinal origin, such as colorectal cancer, stomach cancer, small intestine cancer and esophageal cancer; pancreatic cancer; lung cancer such as squamous cell carcinoma, adenosquamous carcinoma and adenocarcinoma; soft-tissue sarcomas such as leiomyosarcoma and rhabdomyosarcoma; gastrointential and bronchopulmonary neuroendocrine tumors; and neuroectodermal tumors. In the present disclosure, the normal and/or cancerous cells tissues may express GCC at higher levels relative to other tissues, for example other tissue such as B cells and/or B cell associated tissues.

Methods of detection described herein, whether *in vitro* or *in vivo,* can be used to evaluate a disorder in a subject. In the present disclosure, the disorder is a cell proliferative disorder, such as a cancer or a tumor, e.g., colorectal cancer, stomach cancer or pancreatic cancer.

In one aspect, the disclosure describes, a method for detecting the presence or absence of GCC protein in a biological sample *in vitro* (e.g., e.g., in a cell or tissue biopsy obtainedfrom a subject) . The method comprises: (i) contacting a biological sample obtained from a subject with an anti-GCC antibody molecule or immunoconjugate thereofand (ii) detecting formation of a complex between the anti-GCC antibody molecule and GCC protein. Complex formation is indicative of the presence or level of GCC protein in the biological sample, whereas no complex formation is indicative of the absence of GCC protein in the biological sample.

Exemplary biological samples for methods described herein comprise a cell, cells, tissue or body fluid, such as an inflammatory exudate, blood, serum, bowel fluid, stool sample. In the present disclosure, the biological sample comprises a cancerous cell(s) or tissue. For example, the sample can be a tumor biopsy, e.g., biopsy of a colorectal tumor, a gastric tumor, an esophageal tumor, a small intestine tumor, a lung tumor, a soft-tissue sarcoma, a neuroendocrine tumor, a neuroectodermal tumor, or from a tissue sample from any metastatic site thereof. In the present disclosure, the biological sample can be blood or another fluid, where the fluid comprises a cancer cell. A biological sample can be obtained using any of a number of methods in the art. Further, a biological sample can be treated with a fixative such as formaldehyde and embedded in paraffin and sectioned for use. Alternatively, fresh or frozen tissue can be employed. In the present disclosure, fine-needle aspirates may be used.

In the present disclosure, a test cell or tissue is obtained from an individual suspected of having a disorder associated with GCC expression. In the present disclosure, a test cell or tissue is obtained from an individual suspected of having a disorder associated with GCC expression in a location other than the apical surface of intestinal epithelial cells (e.g., cytoplasmic GCC expression in intestinal epithelial cells), or a disorder associated with GCC expression in non-intestinal cells or tissue, such as pancreatic, lung, soft-tissue, or tissue of neuroendocrine or neuroectodermal origin.

In the present disclosure, a test cell or tissue is obtained from an individual suspected of having a disorder associated with increased expression of GCC.

In the present disclosure the level of GCC, in a sample from the subject, or in the subject, is compared with a reference level, e.g., the level of GCC in a control material, e.g., a normal cell of the same tissue origin as the subject's cell or a cell having GCC at levels comparable to such a normal cell. The method can comprise, e.g., responsive to the detected level of GCC, providing a diagnosis, a prognosis, an evaluation of the efficacy of treatment, or the staging of a disorder. A higher level of GCC in the sample or subject, as compared to the control material, indicates the presence of a disorder associated with increased expression of GCC. A higher level of GCC in the sample or subject, as compared to the control material, can also indicate, the relative lack of efficacy of a treatment, a relatively poorer prognosis, or a later stage of disease. The level of GCC can also be used to evaluate or select future treatment, e.g., the need for more or less aggressive treatment, or the need to switch from one treatment regimen to another. In the present disclosure, the methods further comprise selecting a GCC-targetd therapy, e.g., a GCC-targeted therapy described herein, based, at least in part, on the determined GCC levels, and optionally administering the selected GCC-targeted therapy to the subject.

Complex formation between the anti-GCC antibody molecule and GCC can be detected by measuring or visualizing either the antibody (or antibody fragment) bound to the GCC antigen or unbound antibody molecule. One having ordinary skill in the art can readily appreciate the multitude of ways to to detect binding of anti-GCC antibodies to GCC. Such methods include, but are not limited to, antigen-binding assays that are known in the art, such as western blots, radioimmunoassays, ELISA (enzyme linked immunosorbent assay), "sandwich" Immunoassays, immunoprecipitation assays, fluorescent immunoassays, protein A immunoassays, and immunohistochemistry (IHC).

In the present disclosure, GCC is detected or measured by immunohistochemistry using an anti-GCC antibody of the disclosure. Immunohistochemistry techniques may be used to identify and essentially stain cells that express GCC. Such "staining" allows for analysis of metastatic migration. Anti-GCC antibodies such as those described herein are contacted with fixed cells and the GCC present in the cells reacts with the antibodies. The antibodies are detectably labeled or detected using labeled second antibody or protein A to stain the cells. In the present disclosure, the MIL-44-148-2 antibody is used in an IHC assay to detect or measure GCC expression in a biological sample.

Other conventional detection assays can be used, e.g., western blots, radioimmunoassays, ELISA (enzyme linked immunosorbent assay), "sandwich" immunoassays, immunoprecipitation assays, fluorescent immunoassays, protein A immunoassays, and immunohistochemistry (IHC) or radioimmunoassay (RIA).

Alternative to labeling the anti-GCC antibody molecule, the presence of GCC can be assayed in a sample by a competition immunoassay utilizing standards labeled with a detectable substance and an unlabeled anti-GCC antibody molecule. In this assay, the biological sample, the labeled standards and the GCC binding agent are combined and the amount of labeled standard bound to the unlabeled antibody is determined. The amount of GCC in the sample is inversely proportional to the amount of labeled standard bound to the GCC binding agent.

It is also possible to directly detect GCC to anti-GCC antibody molecule complex formation without further manipulation or labeling of either component (GCC or antibody molecule), for example by utilizing the technique of fluorescence energy transfer (FET, see, for example, Lakowicz et al., U.S. Pat. No. 5,631,169; Stavrianopoulos, et al., U.S. Pat. No. 4,868,103). A fluorophore label on the first, "donor" molecule is selected such that, upon excitation with incident light of appropriate wavelength, its emitted fluorescent energy will be absorbed by a fluorescent label on a second "acceptor" molecule, which in turn is able to fluoresce due to the absorbed energy. Alternately, the "donor" protein molecule may simply utilize the natural fluorescent energy of tryptophan residues. Labels are chosen that emit different wavelengths of light, such that the "acceptor" molecule label may be differentiated from that of the "donor". Since the efficiency of energy transfer between the labels is related to the distance separating the molecules, spatial relationships between the molecules can be assessed. In a situation in which binding occurs between the molecules, the fluorescent emission of the "acceptor" molecule label in the assay should be maximal. An FET binding event can be conveniently measured through standard fluorometric detection means well known in the art (e.g., using a fluorimeter).

In another example, determination of the ability of an antibody molecule to recognize GCC can be accomplished without labeling either assay component (GCC or antibody molecule) by utilizing a technology such as real-time Biomolecular Interaction Analysis (BIA) (see, e.g., Sjolander, S, and Urbaniczky, C., 1991, Anal. Chem. 63:2338-2345 and Szabo et al., 1995, Curr. Opin. Struct. Biol. 5:699-705). As used herein, "BIA" or "surface plasmon resonance" is a technology for studying biospecific interactions in real time, without labeling any of the interactants (e.g., BIACORE™). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In some aspects, the disclosure features a reaction mixture that includes an antibody molecule described herein (e.g., an immunoconjugate that includes an antibody molecule described herein and, e.g., a label) and a biological sample, e.g., a biological sample described herein. In the present disclosure, the reaction mixture can include an antibody molecule described herein (e.g., an immunoconjugate that includes an antibody molecule described herein and, e.g., a

label) and GCC obtained from a biological sample, e.g., a biological sample described herein.

In the present disclosure, a method, such as those described above, comprises detecting binding of an anti-GCC antibody to GCC expressed on the surface of a cell or in a membrane preparation obtained from a cell expressing GCC on its surface. In the present disclosure, the method comprises contacting a cell with an anti-GCC antibody under conditions permissive for binding of the anti-GCC antibody to GCC, and detecting whether a complex is formed between the anti-GCC antibody and GCC on the cell surface. An exemplary assay for detecting binding of an anti-GCC antibody to GCC expressed on the surface of a cell is a "FACS" assay.

In Vivo Diagnostics

In the present disclosure, the disclosure describes a method for detecting the presence or absence of GCC-expressing cells or tissues *in vivo.* The method includes (i) administering to a subject (e.g., a patient having a cancer) an anti-GCC antibody molecule of the disclosure (i.e., MIL-44-148-2 or MIL-44-67-4), or antigen binding fragment thereof, preferably an antibody or antigen binding fragment thereof conjugated to a detectable label or marker; (ii) exposing the subject to a means for detecting said detectable label or marker to the GCC-expressing tissues or cells. Such *in vivo* methods can be used for evaluation, diagnosis, staging and/or prognosis of a patient suffering from a disorder such as cancer. The method comprises: (i) administering to a subject, an anti-GCC antibody molecule or immunoconjugate thereof; and (ii) detecting formation of a complex between the anti-GCC antibody molecule and GCC protein. Complex formation is indicative of the presence or level of GCC in the subject whereas no complex formation is indicative of the absence of GCC in the subject.

Such individuals may be diagnosed as suffering from metastasized GCC-expressing cancer and the metastasized GCC-expressing cancer cells may be detected by administering to the individual, preferably by intravenous administration, a pharmaceutical composition that comprises a pharmaceutically acceptable carrier or diluent and a conjugated compound that comprises an anti-GCC antibody molecule and an active moiety wherein the active moiety is a radioactive agent, and detecting the presence of a localized accumulation or aggregation of radioactivity, indicating the presence of cells expressing GCC. In the present disclosure of the present disclosure, the pharmaceutical composition comprises a pharmaceutically acceptable carrier or diluent and a conjugated compound that comprises an anti-GCC antibody molecule and an active moiety wherein the active moiety is a radioactive agent and the anti-GCC antibody molecule is the MIL-44-148-2 antibody described herein, or fragments or derivatives thereof.

In the present disclosure, radionuclides may be conjugated to an anti-GCC antibody molecule of the disclosure for use as an imaging agent in *in vivo* imaging procedures. Imaging agents are useful diagnostic procedures as well as the procedures used to identify the location of metastasized cells. For example, individuals may be diagnosed as suffering from metastasized colorectal cancer and the metastasized colorectal cancer cells may be detected by administering to the individual, preferably by intravenous administration, a pharmaceutical composition that comprises a pharmaceutically acceptable carrier or diluent and a conjugated compound that comprises an anti-GCC antibody molecule of the disclosure and an active moiety wherein the active moiety is a radionuclide and detecting the presence of a localized accumulation or aggregation of radioactivity, indicating the presence of cells that express GCC.

Imaging can be performed by many procedures well-known to those having ordinary skill in the art and the appropriate imaging agent useful in such procedures may be conjugated to an anti-GCC antibody molecule of the disclosure by well-known means. Examples of labels useful for diagnostic imaging in accordance with the present disclosure are radiolabels such as $^{32}P$, $^{3}H$, $^{14}C$, $^{188}Rh$, $^{43}K$, $^{52}Fe$, $^{57}Co$, $^{67}Cu$, $^{67}Ga$, $^{68}Ga$, $^{77}Br$, $^{81}Rb$/ $^{81M}Kr$, $^{87M}Sr$, $^{99}Tc$, $^{111}In$, $^{113M}In$, $^{123}I$, $^{125}I$, $^{127}Cs$, $^{129}Cs$, $^{131}I$, $^{132}I$, $^{197}Hg$, $^{203}Pb$ and $^{206}Bi$, and $^{213}Bi$; fluorescent labels such as fluorescein and rhodamine; nuclear magnetic resonance active labels; positron emitting isotopes of oxygen, nitrogen, iron, carbon, or gallium (e.g., $^{68}Ga$, $^{18}F$) detectable by a single photon emission computed tomography ("SPECT") detector or positron emission tomography ("PET") scanner; chemiluminescers such as luciferin; and enzymatic markers such as peroxidase or phosphatase. Short-range radiation emitters, such as isotopes detectable by short-range detector probes, such as a transrectal probe, can also be employed. Imaging can also be performed, for example, by radioscintigraphy, nuclear magnetic resonance imaging (MRI) or computed tomography (CT scan). Imaging by CT scan may employ a heavy metal such as iron chelates. MRI scanning may employ chelates of gadolinium or manganese.

The antibody can be labeled with such reagents using techniques known in the art. For example, Magerstadt, M. (1991) Antibody Conjugates And Malignant Disease, CRC Press, Boca Raton, Fla.; and Barchel, S. W. and Rhodes, B. H., (1983) Radioimaging and Radiotherapy, Elsevier, NY, N.Y., teach the conjugation of various therapeutic and diagnostic radionuclides to amino acids of antibodies. Such reactions may be applied to conjugate radionuclides to anti-GCC antibody molecules of the disclosure with an appropriate chelating agent and/or linker. See also Wensel and Meares (1983) Radioimmunoimaging and Radioimmunotherapy, Elsevier, N.Y., for techniques relating to the radiolabeling of antibodies. See also, D. Colcher et al. Meth. Enzymol. 121: 802-816 (1986).

In the case of a radiolabeled antibody, the antibody is administered to the patient, is localized to the tumor bearing the antigen with which the antibody reacts, and is detected or "imaged" *in vivo* using known techniques such

as radionuclear scanning using e.g., a gamma camera or emission tomography or computed tomography. See e.g., A. R. Bradwell et al., "Developments in Antibody Imaging", Monoclonal Antibodies for Cancer Detection and Therapy, R. W. Baldwin et al., (eds.), pp 65-85 (Academic Press 1985). Alternatively, a positron emission transaxial tomography scanner, such as designated Pet VI located at Brookhaven National Laboratory, can be used where the radiolabel emits positrons (e.g., $^{11}C$, $^{18}F$, $^{15}O$, and $^{13}N$, $^{68}Ga$).

In the present disclosure, the disclosure describes methods for determining the dose, e.g., radiation dose, that different tissues are exposed to when a subject, e.g., a human subject, is administered an anti-GCC antibody molecule that is conjugated to a radioactive isotope. The method includes: (i) administering an anti-GCC antibody molecule as described herein, e.g., a anti-GCC antibody molecule, that is labeled with a radioactive isotope to a subject; (ii) measuring the amount of radioactive isotope located in different tissues, e.g., tumor, or blood, at various time points until some or all of the radioactive isotope has been eliminated from the body of the subject; and (iii) calculating the total dose of radiation received by each tissue analyzed. The measurements can be taken at scheduled time points, e.g., day 1, 2, 3, 5, 7, and 12, following administration (at day 0) of the radioactively labeled anti-GCC antibody molecule to the subject. The concentration of radioisotope present in a given tissue, integrated over time, and multiplied by the specific activity of the radioisotope can be used to calculate the dose that a given tissue receives. Pharmacological information generated using anti-GCC antibody molecules labeled with one radioactive isotope, e.g., a gamma-emitter, e.g., $^{111}In$ can be used to calculate the expected dose that the same tissue would receive from a different radioactive isotope which cannot be easily measured, e.g., a beta-emitter, e.g., $^{90}Y$.

## Companion Diagnostic for GCC-Targeted Therapy

The *in vitro* and *in vivo* diagnostic methods described herein are useful to inform whether a patient suffering from a profliferative disease such as cancer, or a gastrointestinal disorder such as inflammatory bowel syndrome, Crohn's Disease or constipation, or should be treated or not with a GCC-targeted therapy, based on the presence or absence, respectively, of GCC expression on the surface of or within the patient's cells or tissue. A patient having one more cells that express GCC on the cell surface or within the cell is a candidate for treatment with a GCC-targeted therapy.

In certain aspects, the disclosure describes a method of determining sensitivity of a patient suspected of suffering from a GCC-expressing disease or disorder to a GCC-targeted therapy, comprising the steps of: (i) contacting a biological sample obtained from a subject with an anti-GCC antibody molecule of the disclosure; (ii) detecting formation of a complex between the anti-GCC antibody molecule and GCC protein; wherein complex formation is indicative of the presence or level of GCC protein in the biological sample, whereas no complex formation is indicative of the absence of GCC protein in the biological sample, thereby determining the sensitivity of the patient to a GCC-targeted therapy. In the present disclosure, complex formation between the anti-GCC antibody molecule and GCC protein in the biological sample is detected via immunohistochemistry using an antibody molecule described herein, e.g., the MIL-44-1482 antibody described herein.

Exemplary biological samples can comprise a cell, cells, tissue or body fluid, such as an inflammatory exudate, blood, serum, bowel fluid, stool sample. In the present disclosure, the biological sample comprises a cancerous cell(s) or tissue. For example, the sample can be a tumor biopsy, e.g., biopsy of a colorectal tumor, a gastric tumor, an esophageal tumor, a small intestine tumor, a lung tumor, a soft-tissue sarcoma, a neuroendocrine tumor, a neuroectodermal tumor, or from a tissue sample from any metastatic site thereof. In the present disclosure, the biological sample can be blood or another fluid, where the fluid comprises a cancer cell. A biological sample can be obtained using any of a number of methods in the art. Further, a biological sample can be treated with a fixative such as formaldehyde and embedded in paraffin and sectioned for use. Alternatively, fresh or frozen tissue can be employed. In the present disclosure, fine-needle aspirates may be used.

Exemplary diseases/disorders that may be evaluated (e.g., diagnosed) and treated using the companion diagnostic methods described herein include , but not limited to proliferative disorders including but not limited to colorectal cancer, stomach cancer, small intestine cancer, esophageal cancer, pancreatic cancer, lung cancer (e.g., squamous cell carcinoma, adenosquamous carcinoma, adenocarcinoma), soft tissue sarcoma such as leiomyosarcoma and rhabdomyosarcoma, gastrointestinal and bronchopulmonary neuroendocrine tumors, and neuroectodermal tumors, gastrointestinal disorders such as inflammatory bowel syndrome, Crohn's Disease, and constipation, and neurological disorders such as Parkinson's Disease.

The methods of the disclosure guide physician's decisions in determining whether to treat a patient with a GCC-targeted therapy. The methods provided herein also allow for the generation of a personalized treatment report, e.g., a personalized cancer treatment report, e.g., with a GCC-targeted therapy described herein.

A GCC-targeted therapy is a therapeutic agent that treats or prevents a GCC-expressing disease or a GCC-mediated disease, e.g., a GCC-expressing disease or GCC-mediated disease described herein. In certain aspects of the disclosure, the GCC-targeted therapy is a GCC-ligand such as an anti-GCC antibody molecule or a peptide ligand (e.g., an ST peptide) conjugated to an agent, such as a therapeutic agent. Exemplary GCC-ligands conjugated to

therapeutic agents (i.e., immunoconjugates) are described, e.g., U.S. Published Patent Application No. 20110110936. In the present disclosure, the GCC-targeted therapeutic agent is anti-GCC human IgG1 monoclonal antibody conjugated to a cytotoxic agent, wherein the mAb includes a light chain variable region (VL) having the three light chain complementarity determining regions (CDR1, CDR2, and CDR3) and a heavy chain variable region (VH) having the three heavy chain complementarity determining regions (CDR1, CDR2, and CDR3) listed in Tables 7 (amino acid sequences) and 8 (corresponding nucleic acid sequences) below, and a heavy chain variable region and light chain variable region listed in Tables 9 (amino acid sequences) and 10 (corresponding nucleic acid sequence) below.

**Table 7: Amino acid sequence of VL CDRs and VH CDRs**

| | | |
|---|---|---|
| **VH CDR1** | SEQ ID NO: 67 | GYYWS |
| **VH CDR2** | SEQ ID NO: 68 | EINHRGNTNDNPSLKS |
| **VH CDR3** | SEQ ID NO: 69 | ERGYTYGNFDH |
| **VL CDR1** | SEQ ID NO: 70 | RASQSVSRNLA |
| **VL CDR2** | SEQ ID NO: 71 | GASTRAT |
| **VL CDR3** | SEQ ID NO: 72 | QQYKTWPRT |

**Table 8: Nucleic acid sequence of VL CDRs and VH CDRs**

| | | |
|---|---|---|
| **VH CDR1** | SEQ ID NO: 73 | GGTTACTACTGGAGC |
| **VH CDR2** | SEQ ID NO: 74 | GAAATCAATCATCGTGGAAACACCAACGAC AACCCGTCCCTCAAG |
| **VH CDR3** | SEQ ID NO: 75 | GAACGTGGATACACCTATGGTAACTTTGACC AC |
| **VL CDR1** | SEQ ID NO: 76 | AGGGCCAGTCAGAGTGTTAGCAGAAACTTA GCC |
| **VL CDR2** | SEQ ID NO: 77 | GGTGCATCCACCAGGGCCACT |
| **VL CDR3** | SEQ ID NO: 78 | CAGCAGTATAAAACCTGGCCTCGGACG |

**Table 9: Amino acid sequence of mAb variable region**

| | | |
|---|---|---|
| **Heavy chain** | SEQ ID NO: 79 | QVQLQQWGAGLLKPSETLSLTCAVFGGSFSGYYWSWI RQPPGKGLEWIGEINHRGNTNDNPSLKSRVTISVDTSK NQFALKLSSVTAADTAVYYCARERGYTYGNFDHWGQ GTLVTVSS |
| **Light chain** | SEQ ID NO: 80 | EIVMTQSPATLSVSPGERATLSCRASQSVSRNLAWYQ QKPGQAPRLLIYGASTRATGIPARFSGSGSGTEFTLTIG SLQSEDFAVYYCQQYKTWPRTFGQGTNVEIK |

**Table 10: Nucleic acid sequence of mAb variable region**

| | | |
|---|---|---|
| **Heavy chain** | SEQ ID NO: 81 | CAGGTGCAGCTACAGCAGTGGGGCGCAGGACTGTTGAAGCCTTCGGAGACCCTGTCCCTCACCTGCGCTGTCTTTGGTGGGTCCTTCAGTGGTTACTACTGGAGCTGGATCCGCCAGCCCCCAGGGAAGGGGCTGGAGTGGATTGGGGAAATCAATCATCGTGGAAACACCAACGACAACCCGTCCCTCAAGAGTCGAGTCACCATATCAGTAGACACGTCCAAGAACCAGTTCGCCCTGAAGCTGAGTTCTGTGACCGCCGCGGACACGGCTGTTTATTACTGTGCGAGAGAACGTGGATACACCTATGGTAACTTTGACCACTGGGGCCAGGGAACCCTGGTCACCGTCTCCTCA |
| **Light chain** | SEQ ID NO: 82 | GAAATAGTGATGACGCAGTCTCCAGCCACCCTGTCTGTGTCTCCAGGGGAAAGAGCCACCCTCTCCTGCAGGGCCAGTCAGAGTGTTAGCAGAAACTTAGCCTGGTATCAGCAGAAACCTGGCCAGGCTCCCAGGCTCCTCATCTATGGTGCATCCACCAGGGCCACTGGAATCCCAGCCAGGTTCAGTGGCAGTGGGTCTGGGACAGAGTTCACTCTCACCATCGGCAGCCTGCAGTCTGAAGATTTTGCAGTTTATTACTGTCAGCAGTATAAAACCTGGCCTCGGACGTTCGGCCAAGGGACCAACGTGGAAATCAAA |

The amino acid and corresponding nucleic acid sequences for the full hIgG1 heavy chain and hKappa light chain sequences containing the VL CDRs and VH CDRs shown in Tables 7 and 8, and variable heavy and light chain regions shown in Tables 9 and 10 are listed below:

The hIgG1 heavy chain nucleotide sequence is:

GAATTCCTCACCATGGGATGGAGCTGTATCATCCTCTTCTTGGTAGCAACAGCTACA
GGTGTCCACTCCCAGGTGCAGCTACAGCAGTGGGGCGCAGGACTGTTGAAGCCTTC
GGAGACCCTGTCCCTCACCTGCGCTGTCTTTGGTGGGTCTTTCAGTGGTTACTACTGG
AGCTGGATCCGCCAGCCCCCAGGGAAGGGGCTGGAGTGGATTGGGGAAATCAATCA
TCGTGGAAACACCAACGACAACCCGTCCCTCAAGAGTCGAGTCACCATATCAGTAG
ACACGTCCAAGAACCAGTTCGCCCTGAAGCTGAGTTCTGTGACCGCCGCGGACACG
GCTGTTTATTACTGTGCGAGAGAACGTGGATACACCTATGGTAACTTTGACCACTGG
GGCCAGGGAACCCTGGTCACCGTCAGCTCAGCCTCCACCAAGGGCCCATCGGTCTTC
CCCCTGGCACCCTCCTCCAAGAGCACCTCTGGGGGCACAGCGGCCCTGGGCTGCCTG
GTCAAGGACTACTTCCCCGAACCGGTGACGGTGTCGTGGAACTCAGGCGCCCTGAC
CAGCGGCGTGCACACCTTCCCGGCTGTCCTACAGTCCTCAGGACTCTACTCCCTCAG
CAGCGTGGTGACCGTGCCCTCCAGCAGCTTGGGCACCCAGACCTACATCTGCAACGT
GAATCACAAGCCCAGCAACACCAAGGTGGACAAGAAAGTTGAGCCCAAATCTTGTG
ACAAAACTCACACATGCCCACCGTGCCCAGCACCTGAACTCCTGGGGGGACCGTCA
GTCTTCCTCTTCCCCCCAAAACCCAAGGACACCCTCATGATCTCCCGGACCCCTGAG

GTCACATGCGTGGTGGTGGACGTGAGCCACGAAGACCCTGAGGTCAAGTTCAACTG
GTACGTGGACGGCGTGGAGGTGCATAATGCCAAGACAAAGCCGCGGGAGGAGCAG
TACAACAGCACGTACCGTGTGGTCAGCGTCCTCACCGTCCTGCACCAGGACTGGCTG
AATGGCAAGGAGTACAAGTGCAAGGTCTCCAACAAAGCCCTCCCAGCCCCCATCGA
GAAAACCATCTCCAAAGCCAAAGGGCAGCCCCGAGAACCACAGGTGTACACCCTGC
CCCCATCCCGGGATGAGCTGACCAAGAACCAGGTCAGCCTGACCTGCCTGGTCAAA
GGCTTCTATCCCAGCGACATCGCCGTGGAGTGGGAGAGCAATGGGCAGCCGGAGAA
CAACTACAAGACCACGCCTCCCGTGCTGGACTCCGACGGCTCCTTCTTCCTCTACAG
CAAGCTCACCGTGGACAAGAGCAGGTGGCAGCAGGGGAACGTCTTCTCATGCTCCG
TGATGCATGAGGCTCTGCACAACCACTACACGCAGAAGAGCCTCTCCCTGTCTCCGG
GTAAATAATAGGGATAACAGGGTAATACTAGAG (SEQ ID NO: 83)

The hIgG1 heavy chain protein sequence is;

MGWSCIILFLVATATGVHSQVQLQQWGAGLLKPSETLSLTCAVFGGSFSGYYWSWIRQPPGKGLEWIGEINHRGNTNDNPSLKSRVTISVDTSKNQFALKLSSVTAADTAVYYCARERGYTYGNFDHWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

(SEQ ID NO: 84)

The Kappa light chain nucleotide sequence is:

GCGGCCGCCTCACCATGGGATGGAGCTGTATCATCCTCTTCTTGGTAGCAACAGCTACAGGTGTCCACTCCGAAATAGTGATGACGCAGTCTCCAGCCACCCTGTCTGTGTCTCCAGGGGAAAGAGCCACCCTCTCCTGCAGGGCCAGTCAGAGTGTTAGCAGAAACTTAGCCTGGTATCAGCAGAAACCTGGCCAGGCTCCCAGGCTCCTCATCTATGGTGCATCC

ACCAGGGCCACTGGAATCCCAGCCAGGTTCAGTGGCAGTGGGTCTGGGACAGAGTTCACTCTCACCATCGGCAGCCTGCAGTCTGAAGATTTTGCAGTTTATTACTGTCAGCAGTATAAAACCTGGCCTCGGACGTTCGGCCAAGGGACCAACGTGGAAATCAAACGTACGGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACCCTGAGCAAAGCAGACTACGAGAAACACAAAGTCTACGCCTGCGAAGTCACCCATCAGGGCCTGAGCTCGCCCGTCACAAAGAGCTTCAACAGGGGAGAGTGTTAGTCTAGA (SEQ ID NO: 85)

The hKappa light chain protein sequence is:

MGWSCIILFLVATATGVHSEIVMTQSPATLSVSPGERATLSCRASQSVSRNLAWYQQKPGQAPRLLIYGASTRATGIPARFSGSGSGTEFTLTIGSLQSEDFAVYYCQQYKTWPRTFGQGTNVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 86)

In one particular aspect of the disclosure, the GCC-targeted therapy is an immunoconjugate having an anti-GCC antibody molecule conjugated to an auristatin molecule. In the present disclosure the immunoconjugate comprises

an anti-GCC antibody molecule that includes three heavy chain (VH) CDRs according to SEQ ID NOs: 67, 68 and 69, and three light chain (VL) CDR regions according to SEQ ID NOs: 70, 71 and 72, conjugated to an auristatin molecule. In the present disclosure, the immunoconjugate comprises an anti-GCC antibody molecule that includes a heavy chain variable region according to SEQ ID NO: 79, and a light chain variable region according to SEQ ID NO: 80, conjugated to an auristatin molecule. In the present disclosure, the immunoconjugate comprises an anti-GCC antibody molecule that includes the heavy and light chain variable regions according to SEQ ID NOs 79 and 80, respectively, conjugated to an auristatin molecule.

In the present disclosure, the auristatin molecule is linked to a cysteine moiety on the anti-GCC antibody molecule by way of a linker containing a maleimide moiety, e.g., a maleimidocaproyl moiety.

In the present disclosure, the auristatin molecule is coupled to an anti-GCC antibody molecule using a heterobifunctional linker that is connected to a hydroxyl group on the auristatin molecule. In the present disclosure, the linker comprises a hydrazone. In the present disclosure, the linker is a hydrazone compound formed by reaction of maleimidocaproylhydrazide and a ketocarboxylic acid, e.g., 5-benzoylvaleric acid. In the present disclosure, the linker is (Z)-6-(2-(6-(2,5-dioxo-2,5-dihydro-1H-pyrrol-1-yl)hexanoyl)hydrazono)-6-phenylhexanoic acid.

In the present disclosure the auristatin molecule is coupled to the anti-GCC antibody molecule using a heterobifunctional linker that is connected to a monomethyl amino group on the auristatin molecule. In the present disclosure, the linker comprises a cleavable moiety, e.g., a peptide moiety, and a self-immolative *p*-aminobenzylcarbamate spacer. Exemplary linkers include maleimidocaproyl (mc), maleimidocaproyl-L-phenylalanine-L-lysine-*p*-aminobenzylcarbamate, and maleimidocaproyl-L-valine-L-citrulline-*p*-aminobenzylcarbamate (vc).

In the present disclosure, the GCC-targeted therapy is an immunoconjugate characterized by the formula Ab-(vc-MMAF)$_m$ (formula (***I-4***)); Ab-(vc-MMAE)$_m$ (formula (***I-5***)); Ab-(mc-MMAE)$_m$ (formula (***I-6***)); or Ab-(mc-MMAF)$_m$, (formula (***I-7***)), wherein Ab is an anti-GCC antibody molecule that includes features such as the features described in any one of Tables 7, 8, 9 or 10, S is a sulfur atom of the antibody, and *m* ranges from about 1 to about 15. In the present disclosure, *m* is an integer from 1 to about 5.

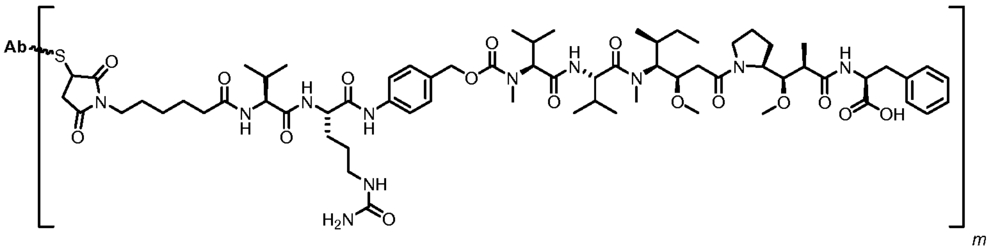

(***I-4***)

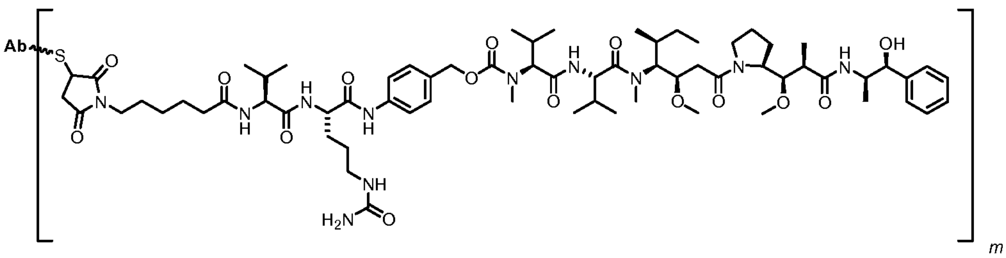

(***I-5***)

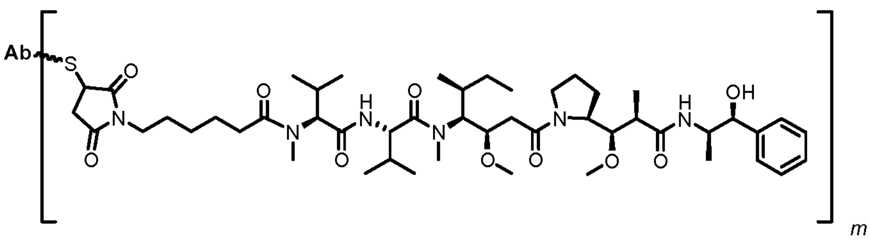

(***I-6***)

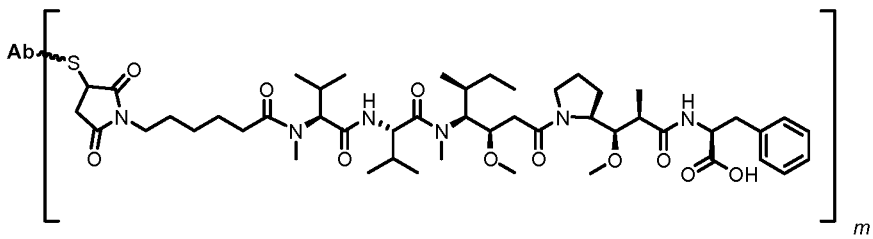

*(I-7)*

In the present disclosure, the variable *m* in formula (***I-4***), (***I-5***), (***I-6***), or (***I-7***) ranges from about 2 to about 10, from about 6 to about 8, from about 4 to about 6, from about 3 to about 5, or from about 1 to about 3 .

In the present disclosure, the targeted GCC therapy is an immunoconjugate of formula (***I-4***), (***I-5***), (***I-6***), or (***I-7***), wherein Ab is a an antibody molecule that includes three heavy chain (VH) CDRs according to SEQ ID NOs: 67, 68 and 69, and three light chain (VL) CDR regions according to SEQ ID NOs: 70, 71 and 72, and *m* is about 3 to about 5 (e.g., about 4). In certain aspects, the Ab that includes the heavy chain CDRs according to SEQ ID NOs: 67, 68 and 69, and the three light chain CDRs according to SEQ ID NOs: 70, 71 and 72, respectively, is a human monoclonal antibody, preferably human IgG1 antibody.

In the present disclosure, the GCC-targeted therapy is an immunoconjugate of formula (***I-4***), (***I-5***), (***I-6***), or (***I-7***), wherein Ab is a monoclonal antibody molecule that includes a heavy chain variable region according to SEQ ID NO: 79, and a light chain variable region according to SEQ ID NO: 80, and *m* is about 3 to about 5 (e.g., about 4). In certain aspects, the Ab that includes the heavy and light chain variable regions according to SEQ ID NOs 79 and 80, respectively, is a human monoclonal antibody, preferably human IgG1 antibody.

In the present disclosure, the GCC-targeted therapy is an immunoconjugate of formula (***I-4***), (***I-5***), (***I-6***), or (***I-7***), wherein Ab is a human IgG monoclonal antibody molecule that includes a heavy chain IgG1 sequence according to SEQ ID NO: 84, and a Kappa light chain sequence according to SEQ ID NO: 86, and *m* is about 3 to about 5 (e.g., about 4).

In the present disclosure, the GCC-targeted therapy is a GCC-ligand conjugate capable of crossing the blood-brain barrier. For example, in certain aspects the disclosure relates to a GCC-ligand conjugated to a neuroprotective agent such as L-dopa, which is capable of crossing the blood-brain barrier. Examples of such GCC-ligand conjugates are described in published PCT application WO2013/016662. In embodiments of the invention, patients suffering from or suspected of having a neurological disorder (e.g., Parkinson's Disease) whose neurons express GCC would be considered good candidates for treatment with a GCC-ligand conjugated to a neuroprotective agent.

In some aspects of the invention, the GCC-targeted therapy is a GCC antagonist. In one emodiment, the GCC-targeted therapy is a peptide antagonist (e.g., an ST peptide) or a a small molecule inhibitor of GCC.

In some aspects, the GCC-targeted therapy is a GCC agonist. In the present disclosure, the GCC agonist is an ST peptide. In the present disclosure, the GCC agonist is an ST peptide comprising the amino acid sequence: H-$Cys^1$-$cys^2$-$Glu^3$-$Tyr^4$-$Cys^5$-$Cys^6$-$Asn^7$-$Pro^8$-$Ala^9$-$Cys^{10}$-$Thr^{11}$-$Gly^{12}$-$Cys^{13}$-$Tyr^{14}$-OH (SEQ ID NO: 87), wherein there are three disulfide bonds: Between $Cys^1$ and $Cys^6$, between $Cys^2$ and $Cys^{10}$, and between $Cys^5$ and $Cys^{13}$. In the present disclosure the GCC agonist is a peptide agonist that binds GCC such as Linaclotide (Ironwood Pharmaceuticals).

In the present disclosure of the disclosure, patients whose tumor cells express GCC on their surfaces would be considered good candidates for treatment with toxin-conjugated anti-GCC antibody molecules, such as an immunoconjugate as described herein, or the toxin-conjugated antibodies as described in U.S. Published Patent Application No. 20110110936. Without intending to be bound by any theory, patients whose tumor cells express low amounts of GCC on their surfaces may not be as good candidates for this or might be candidates for combining the GCC-targeted therapy with an additional treatment method, or be candidates for naked antibody therapy. In another example, the dose of theGCC-targeted therapy could be adjusted to reflect the number of GCC molecules expressed on the surfaces of tumor cells. For example, patients with high numbers of GCC molecules on their tumor cell surfaces might be treated with lower doses of a GCC-targeted therapy than patients with low numbers of GCC molecules expressed on the tumor cell surface. Detecting the presence of GCC-expressing tumor cells *in vivo* can allow identification of tissues into the primary GCC-expressing tumor has metastasized. Knowledge of which tissues have metastases can lead to targeted application of tumor therapy.

As discussed above, the antibody molecules described herein permit assessment of the presence of a GCC protein in normal versus neoplastic tissues, through which the presence or severity of disease, disease progress and/or the efficacy of therapy can be assessed. For example, therapy can be monitored and efficacy assessed. In one example, a GCC protein can be detected and/or measured in a first sample obtained from a subject having a proliferative disease and therapy can be initiated. Later, a second sample can be obtained from the subject and GCC protein in the sample can be detected and/or measured. A decrease in the quantity of GCC protein detected or measured in the second sample can be indicative of therapeutic efficacy.

Without intending to be bound by any theory, vascularization may be required for a GCC-targeted therapeutic to access a GCC expressing tumor, particularly in instances where the GCC-targeted therapeutic is administered intravenously. Thus, in certain embodiments of the methods of the invention, it may be useful to evaluate or characterize tumor vasculature in addition to or in conjunction with the detection of GCC protein. For example, a tissue sample can be stained with an agent that identifies a vascular endothelial cell, such as an anti-CD-31 antibody or an anti-von Willebrand Factor antibody molecule, and an anti-GCC antibody of the invention to simultaneously or contemporaneously characterize GCC expression and tissue vascularization. In certain aspects of the invention, such simultaneously or contemporaneous characterization of GCC expression and vasculature is useful as a patient selection tool for a targeted -targetedtherapeutic.

In another aspect, cell surface expression of GCC may be required for a GCC-targeted therapeutic to affect the killing of a GCC expressing tumor cell. For example, some tumor cells may be expressing GCC, but not on the cell surface. If a therapeutic depends on cell surface GCC expression, such a therapeutic may not be able to kill a cell where GCC is primarily intracellular. Thus, in some embodiments of the invention, the diagnostic or prognostic assay can further include analysis and/or quantification of cellular location of GCC, e.g., a method which can distinguish and/or quantify cell surface expression from intracellular expression. Without intending to be boundy by any theory, a patient whose tumor primarily has intracellular GCC expression may not be a good candidate for an anti-GCC antibody molecule which binds to the extracellular domain of GCC. Alternatively, such an analytical result may prompt initial treatment with an agent which induces cell surface expression of GCC (see, e.g., PCT publication No. WO04/071436). Following, or in conjunction with GCC cell surface induction, an anti-GCC antibody molecule which has access to or binds only to extracellularly expressed GCC can be administered.

**Kits**

Also within the scope of the invention are kits comprising an anti-GCC antibody molecule or immunoconjugate as described herein. Further included are kits comprising liposome compositions comprising an anti-GCC antibody molecule or immunoconjugate. The kit can include one or more other elements including: instructions for use; other reagents, e.g., a label, a therapeutic agent, or an agent useful for chelating, or otherwise coupling, an antibody to a label or therapeutic agent, or a radioprotective composition; devices or other materials for preparing the antibody for administration; pharmaceutically acceptable carriers; and devices or other materials for administration to a subject. Instructions for use can include instructions for diagnostic applications of the anti-GCC antibody molecule or immunoconjugate to detect GCC, *in vitro,* e.g., in a sample, e.g., a biopsy or cells from a patient having a cancer, or *in vivo.* The instructions can include guidance for therapeutic application including suggested dosages and/or modes of administration, e.g., in a patient with a cancer (e.g., a cancer of gastrointestinal origin, such as, for example, colon cancer, stomach cancer, esophageal cancer). Other instructions can include instructions on coupling of the antibody to a chelator, a label or a therapeutic agent, or for purification of a conjugated antibody, e.g., from unreacted conjugation components. As discussed above, the kit can include a label, e.g., any of the labels described herein. As discussed above, the kit can include a therapeutic agent, e.g., a therapeutic agent described herein. In some applications the antibody will be reacted with other components, e.g., a chelator or a label or therapeutic agent, e.g., a radioisotope, e.g., yttrium or lutetium. In such cases the kit can include one or more of a reaction vessel to carry out the reaction or a separation device, e.g., a chromatographic column, for use in separating the finished product from starting materials or reaction intermediates.

The kit can further contain at least one additional reagent, such as a diagnostic or therapeutic agent, e.g., a diagnostic or therapeutic agent as described herein, and/or one or more additional anti-GCC antibody molecules or immunoconjugates, formulated as appropriate, in one or more separate pharmaceutical preparations.

The kit can further contain a radioprotectant. The radiolytic nature of isotopes, e.g., $^{90}$Yttrium ($^{90}$Y) is known. In order to overcome this radiolysis, radioprotectants may be included, e.g., in the reaction buffer, as long as such radioprotectants are benign, meaning that they do not inhibit or otherwise adversely affect the labeling reaction, e.g., of an isotope, such as of $^{90}$Y, to the antibody. The formulation buffer of the present disclosure may include a radioprotectant such as human serum albumin (HSA) or ascorbate, which minimize radiolysis due to yttrium or other strong radionuclides. Other radioprotectants are known in the art and can also be used in the formulation buffer of the present disclosure, i.e., free radical scavengers (phenol, sulfites, glutathione, cysteine, gentisic acid, nicotinic acid, ascorbyl palmitate, $HOP(:O)H_2I$ glycerol, sodium formaldehyde sulfoxylate, $Na_2S_2O$, $Na_2S_2O_3$, and $SO_2$, etc.).

A provided kit is one useful for radiolabeling a chelator-conjugated protein or peptide with a therapeutic radioisotope for administration to a patient. The kit includes (i) a vial containing chelator-conjugated antibody, (ii) a vial containing formulation buffer for stabilizing and administering the radiolabeled antibody to a patient, and (iii) instructions for performing the radiolabeling procedure. The kit provides for exposing a chelator-conjugated antibody to the radioisotope or a salt thereof for a sufficient amount of time under amiable conditions, e.g., as recommended in the instructions. A radiolabeled antibody having sufficient purity, specific activity and binding specificity is produced. The radiolabeled antibody may be diluted to an appropriate concentration, e.g., in formulation buffer, and administered directly to the

patient with or without further purification. The chelator-conjugated antibody may be supplied in lyophilized form.

The following examples are illustrative but are not meant to be limiting of the present invention.

## EXAMPLES

**Example 1:** Generation of a human GCC extracellular domain-mouse Fc (hGCC-ECD-mFc) fusion protein

The generation of a secreted human (h) guanylyl cyclase (GCC) (hGCC) extracellular domain (ECD)/mouse immunoglobulin (Ig)G2a heavy chain constant (Fc) (with receptor binding region mutation (FcRbr-mutII) fusion protein (i.e., hGCC(ECD)-mIgG2a RcRbr-mutII fusion protein, also referred to herein as pLKTOK108 and MIL-44) for immunization and screening was performed as follows. GCC antigen was prepared by subcloning a portion of the GCC gene encoding a sequenced comprising the following GCC sequence (signal sequence and extracellular domain) into the pLKTOK4 expression vector:

MKTLLLDLALWSLLFQPGWLSFSSQVSQNCHNGSYEISVLMMGNSAFAEP
LKNLEDAVNEGLEIVRGRLQNAGLNVTVNATFMYSDGLIHNSGDCRSSTC
EGLDLLRKISNAQRMGCVLIGPSCTYSTFQMYLDTELSYPMISAGSFGLS
CDYKETLTRLMSPARKLMYFLVNFWKTNDLPFKTYSWSTSYVYKNGTETE
DCFWYLNALEASVSYFSHELGFKVVLRQDKEFQDILMDHNRKSNVIIMCG
GPEFLYKLKGDRAVAEDIVIILVDLFNDQYFEDNVTAPDYMKNVLVLTLS
PGNSLLNSSFSRNLSPTKRDFALAYLNGILLFGHMLKIFLENGENITTPK
FAHAFRNLTFEGYDGPVTLDDWGDVDSTMVLLYTSVDTKKYKVLLTYDTH
VNKTYPVDMSPTFTWKNSKL (SEQ ID NO: 46)

The amino acid sequence GLy-Arg-Gly-Pro-Gln (SEQ ID NO: 66), at positions 427 to 430, was selected to terminate the extracellular GCC fragment. In GCC, this sequence is immediately followed by a Pro that aligns well with a Pro at the position homologous to the Pro that is historically used to initiate human IgG1 Fc fusion proteins.

The mouse IgG2a Fc region of pLKTOK108 was designed to start with the amino acid sequence that functionally is the end of the CH1 domain [Pro-Arg-Valine (Val)-Pro-Isoleucine (Ile)-Threonine (Thr)-Glu-Asparagine (Asn)] (SEQ ID NO: 58). Two regions were mutated in the mouse IgG2a constant region. In addition to the leucine (Leu)-Leu-Gly-Gly (SEQ ID NO: 59) to Leu-alanine (Ala)-Gly-Ala (SEQ ID NO: 60) mutations (positions 234 to 237 Lysine [Lys]-Lys-Gly-Gly (SEQ ID NO: 61) to Lys-Ala-Gly-Ala (SEQ ID NO: 62), the second Fc receptor region at positions 318 to 322 was also mutated as follows: glutamic acid (Glu)-Phenylalanine (Phe)-Lys-Cysteine (Cys)-Lys (SEQ ID NO: 63) to Ala-Phe-Lys-Cys-Lys (SEQ ID NO: 64) and then to Phe-Lys-Cys-Lys (SEQ ID NO: 65).

Once the complete fusion protein sequence was designed, flanking restriction enzyme sequences for BamHI and XbaI, as well as the Kozak sequence (CTCACC) and a terminal stop codon were added to complete the fusion protein cDNA. The nucleotide and amino acid sequences of the fusion protein pLKTOK108 (hGCC/mIgG2a FcRmutII) is provided below (the BamHI and XbaI restriction sites are shown in lower case letters in SEQ ID NO: 47):

**Human GCC-ECD/mouse IgG2a Fc nucleotide sequence** (SEQ ID NO: 47)

cgcggatccctcaccATGAAGACGTTGCTGTTGGACTTGGCTTTGTGGTCACTGCTCTTCCAG
CCCGGGTGGCTGTCCTTTAGTTCCCAGGTGAGTCAGAACTGCCACAATGGCAGCTAT
GAAATCAGCGTCCTGATGATGGGCAACTCAGCCTTTGCAGAGCCCCTGAAAAACTTG
GAAGATGCGGTGAATGAGGGGCTGGAAATAGTGAGAGGACGTCTGCAAAATGCTGG
CCTAAATGTGACTGTGAACGCTACTTTCATGTATTCGGATGGTCTGATTCATAACTCA
GGCGACTGCCGGAGTAGCACCTGTGAAGGCCTCGACCTACTCAGGAAAATTTCAAA
TGCACAACGGATGGGCTGTGTCCTCATAGGGCCCTCATGTACATACTCCACCTTCCA
GATGTACCTTGACACAGAATTGAGCTACCCCATGATCTCAGCTGGAAGTTTTGGATT
GTCATGTGACTATAAAGAAACCTTAACCAGGCTGATGTCTCCAGCTAGAAAGTTGAT
GTACTTCTTGGTTAACTTTTGGAAAACCAACGATCTGCCCTTCAAAACTTATTCCTGG
AGCACTTCGTATGTTTACAAGAATGGTACAGAAACTGAGGACTGTTTCTGGTACCTT
AATGCTCTGGAGGCTAGCGTTTCCTATTTCTCCCACGAACTCGGCTTTAAGGTGGTGT
TAAGACAAGATAAGGAGTTTCAGGATATCTTAATGGACCACAACAGGAAAAGCAAT
GTGATTATTATGTGTGGTGGTCCAGAGTTCCTCTACAAGCTGAAGGGTGACCGAGCA
GTGGCTGAAGACATTGTCATTATTCTAGTGGATCTTTTCAATGACCAGTACTTGGAG
GACAATGTCACAGCCCCTGACTATATGAAAAATGTCCTTGTTCTGACGCTGTCTCCT
GGGAATTCCCTTCTAAATAGCTCTTTCTCCAGGAATCTATCACCAACAAAACGAGAC

TTTGCTCTTGCCTATTTGAATGGAATCCTGCTCTTTGGACATATGCTGAAGATATTTC
TTGAAAATGGAGAAAATATTACCACCCCCAAATTTGCTCATGCTTTCAGGAATCTCA
CTTTTGAAGGGTATGACGGTCCAGTGACCTTGGATGACTGGGGGGATGTTGACAGTA
CCATGGTGCTTCTGTATACCTCTGTGGACACCAAGAAATACAAGGTTCTTTTGACCT
ATGATACCCACGTAAATAAGACCTATCCTGTGGATATGAGCCCCACATTCACTTGGA
AGAACTCTAAACTTCCTAATGATATTACAGGCCGGGGCCCTCAGCCCAGAGTGCCCA
TAACACAGAACCCCTGTCCTCCACTCAAAGAGTGTCCCCCATGCGCAGCTCCAGACC
TCGCAGGTGCACCATCCGTCTTCATCTTCCCTCCAAAGATCAAGGATGTACTCATGA
TCTCCCTGAGCCCCATGGTCACATGTGTGGTGGTGGATGTGAGCGAGGATGACCCAG
ACGTCCAGATCAGCTGGTTTGTGAACAACGTGGAAGTACACACAGCTCAGACACAA
ACCCATAGAGAGGATTACAACAGTACTCTCCGGGTGGTCAGTGCCCTCCCCATCCAG
CACCAGGACTGGATGAGTGGCAAGGCATTCAAATGCAAGGTCAACAACAGAGCCCT
CCCATCCCCCATCGAGAAAACCATCTCAAAACCCAGAGGGCCAGTAAGAGCTCCAC
AGGTATATGTCTTGCCTCCACCAGCAGAAGAGATGACTAAGAAAGAGTTCAGTCTG
ACCTGCATGATCACAGGCTTCTTACCTGCCGAAATTGCTGTGGACTGGACCAGCAAT
GGGCGTACAGAGCAAAACTACAAGAACACCGCAACAGTCCTGGACTCTGATGGTTC
TTACTTCATGTACAGCAAGCTCAGAGTACAAAAGAGCACTTGGGAAAGAGGAAGTC
TTTTCGCCTGCTCAGTGGTCCACGAGGGTCTGCACAATCACCTTACGACTAAGACCA
TCTCCCGGTCTCTGGGTAAATAAtctagagca

**Human GCC-ECD/mouse IgG2a Fc amino acid sequence (SEQ ID NO: 48):**

MKTLLLDLALWSLLFQPGWLSFSSQVSQNCHNGSYEISVLMMGNSAFAEPLKNLEDAV
NEGLEIVRGRLQNAGLNVTVNATFMYSDGLIHNSGDCRSSTCEGLDLLRKISNAQRMGC
VLIGPSCTYSTFQMYLDTELSYPMISAGSFGLSCDYKETLTRLMSPARKLMYFLVNFWK
TNDLPFKTYSWSTSYVVYKNGTETEDCFWYLNALEASVSYFSHELGFKVVLRQDKEFQDI
LMDHNRKSNVIIMCGGPEFLYKLKGDRAVAEDIVIILVDLFNDQYLEDNVTAPDYMKN
VLVLTLSPGNSLLNSSFSRNLSPTKRDFALAYLNGILLFGHMLKIFLENGENITTPKFAHA
FRNLTFEGYDGPVTLDDWGDVDSTMVLLYTSVDTKKYKVLLTYDTHVNKTYPVDMSP
TFTWKNSKLPNDITGRGPQPRVPITQNPCPPLKECPPCAAPDLAGAPSVFIFPPKIKDVLMI

SLSPMVTCVVVDVSEDDPDVQISWFVNNVEVHTAQTQTHREDYNSTLRVVSALPIQHQ
DWMSGKAFKCKVNNRALPSPIEKTISKPRGPVRAPQVYVLPPPAEEMTKKEFSLTCMIT
GFLPAEIAVDWTSNGRTEQNYKNTATVLDSDGSYFMYSKLRVQKSTWERGSLFACSVV
HEGLHNHLTTKTISRSLGK

As stated above, the recombinant protein pLKTOK108 combines the extracellular region of human GCC fused to a mouse IgG2a Fc region in which the two mutated Fc receptor binding regions (FcRs) were mutated to prevent Fc receptor binding (mIgG2a FcRmutII). The recombinant DNA insert for pLKTOK108 was created by a three-step PCR process as follows:
The first step created the adapted extracellular human GCC and the adapted mouse IgG2a FcRmutII DNA fragments containing 35 nucleotides of overlapping sequences. These PCR reactions used the templates and primers described in Table 11 and Table 12 with the protocol described in Table 13 to create the two fragments. These DNA fragments were isolated from a 1% agarose gel using a Qiagen Gel Purification kit (Valencia, CA). The human GCC template was provided by a protein expression vector containing the sequence for human GCC (Clontech Laboratories, Inc., Mountain View, CA, USA. The template for the Fc domain was obtained from an expression construct for human 1228 fused to mouse IgG2aFc with two mutated Fc receptor binding regions (FcRmutII), referred to as pLKTOK84, that itself were created using the vector pLKTOK61 (described in U.S. Patent 7,053,202) as a template.

**Table 11: Templates Used in First Step PCR Assembly Reactions to Create Recombinant DNA for pLKTOK108**

| Number | Product | Template | Primer 1 | Primer 2 | Size |
|---|---|---|---|---|---|
| 1A | Extracellular GCC | Human GCC-Vect | pGCCFC5 | pGCCFCMuA | 1300 bp |
| 1B | Mouse IgG2a-FcRmutII | pLKTOK84 | pGCCFCMuB | pMICOS-4 | 700 bp |

**Table 12: Primers Used in All PCR Reactions to Create pLKTOK108**

| Primer Name | Sequence | SEQ ID NO: |
|---|---|---|
| pGCCFC5 | 5'-CGCGGATCCCTCACCATGAAGACGTTGCTGTTGGACTTGGC-3' | 49 |
| pGCCFCMuA | 5'- TGGGCACTCTGGGCTGAGGGCCCCGGCCTGTAATATCATTAG -3' | 50 |
| pGCCFCMuB | 5'-CAGGCCGGGGCCCTCAGCCCAGAGTGCCCATAACACAGAACCCCTGTCC -3' | 51 |
| pMICOS-4 | 5'-TGCTCTAGATTATTTACCCAGAGACCGGGAGATGGTCTTA | 52 |
| pSMUCH2 | 5'-ACCTGTGGAGCTCTTACTGG-3' | 53 |
| EF5S | 5'-CATTTCAGGTGTCGTGAGGA-3' | 54 |
| SP6 | 5'-ATTTAGGTGACACTATAG-3' | 55 |
| M13f | 5'-GTTTTCCCAGTCACGAC-3' | 56 |
| M13r | 5'-AACAGCTATGACCATG-3' | 57 |

**Table 13: Reaction Protocol Used in First Step PCR Assembly Reactions to Create Recombinant DNA for pLKTOK108**

| Reaction Mixture | Machine settings |
|---|---|
| 1 uL DNA (1:100 miniprep of template) | 94°C- 2 minutes |
| 0.2 uL 200mM Primer 1 | |
| 0.2 uL 200mM Primer 2 | 30 cycles |
| 10 uL 10x PCR buffer | 94°C- 1 minutes |
| 3 uL 50mM $MgCl_2$ | 55°C- 30 seconds |
| 2 uL 10mM dNTP mix | 72°C- 2 minutes |
| 83.5 uL $H_2O$ | |

(continued)

| Reaction Mixture | Machine settings |
|---|---|
| 0.5 uL Taq polymerase | 72°C- 10 minutes |

The second PCR reaction combined the templates in the concentrations listed in Table 14. The reaction protocol listed in Table 15 created a single recombinant fusion protein gene. The product of this reaction was used directly as the template in the third PCR reaction.

**Table 14: Templates Used in the Second Step PCR Assembly Reactions to Create Recombinant DNA for pLKTOK108**

| Number | Template 1 | Template 2 | Concentration |
|---|---|---|---|
| 2A | Extracellular GCC | Mouse IgG2a-FcRmutII | 10 uM (2.5 ul each) |
| 2B | Extracellular GCC | Mouse IgG2a-FcRmutII | 30 uM (7.5 ul each) |

**Table 15: Reaction Protocol Used in the Second Step PCR Assembly Reactions to Create Recombinant DNA for pLKTOK108**

| Reaction Mixture | Machine Settings |
|---|---|
| 2.5 or 7.5 uL each DNA | 8 cycles |
| 10 uL 10x PCR buffer | 94°C- 1 minute |
| 3 uL $MgCl_2$ | 30 sec ramp |
| 2 uL dNTP | 72°C- 2 minutes |
| 79.5 or 69.5 uL $H_2O$ | 30 sec ramp |
| 0.5 uL Taq | |

The third PCR reaction used the templates and primers described in Table 16 and Table 12 with the protocol described in Table 17 below to create the complete fragments. These DNA fragments were isolated from a 0.7% agarose gel using a Qiagen Gel Purification kit (Appendix F), and a thiamine adenosine (TA) overhang TOPO® TA Cloning Kit (Appendix F). Unique clones were isolated and DNA purified using Qiagen's DNA miniprep kit (Appendix F). The DNA was sequenced with the primers M13f, M13r and pSMUCH2 to identify those with the desired sequence. The intermediate TOPO clone TOK108-15 contained the desired recombinant DNA sequence.

**Table 16: Templates Used in the Third Step PCR Assembly Reactions to Create Recombinant DNA for pLKTOK108**

| Number | Product | Template | Primer 1 | Primer 2 | Size |
|---|---|---|---|---|---|
| 3A | TOK108 Insert | Reaction 2A (5 ul) | pGCCFC5 | pMICOS-4 | 2028 bp |
| 3B | TOK108 Insert | Reaction 2B (5 ul) | pGCCFC5 | pMICOS-4 | 2028 bp |

**Table 17: Reaction Protocol Used in the Third Step PCR Assembly Reaction to Create Recombinant DNA for pLKTOK108**

| Reaction Mixture | Machine settings |
|---|---|
| 5 uL PCR Reaction 2A or 2B | 94°C- 2 minutes |
| 0.2 uL 200mM Primer 1 | |
| 0.2 uL 200mM Primer 2 | 30 cycles |

(continued)

| Reaction Mixture | Machine settings |
|---|---|
| 10 uL 10x PCR buffer | 94°C- 1 minute |
| 3 uL 50mM MgCl2 | 55°C- 30 seconds |
| 2 uL 10mM dNTP mix | 72°C- 2 minutes |
| 79.5 uL H2O | |
| 0.5 uL Taq polymerase | 72°C- 10 minutes |

To create the expression vector pLKTOK4, pcDNA3.1™ was used as a backbone vector. It contains the neomycin (NEO) gene for resistance to G-418 (Geneticin®) to allow for easy selection under research conditions. The SpeI restriction site was eliminated from pcDNA ™3.1 by site-directed mutagenesis. The EF-1α promoter from plasmid pcDEF3 (originally pEF-BOS[4]) was inserted into pcDNA ™3.1, thus eliminating the CMV promoter. A circular map for the pLKTOK4 expression vector is depicted in Figure 1.

Cloning was performed on the final PCR products using a TOPO® TA Cloning kit. After digestion with BamHI and XbaI restriction enzymes, the desired fragment from the TOPO clone was ligated to the expression vector pLKTOK4 that was also digested with BamHI and XbaI. The ligation reaction was used to transform K12 chemically competent *E.coli* cells and then selected on Luria broth (LB)/ampicillin agar plates. Plasmids from individual *E. Coli* clones were isolated using QIAGEN's DNA miniprep kit and sequenced with the primers SP6 (SEQ ID NO: 55), EF5S (SEQ ID NO: 54) and pSMUCH2 (SEQ ID NO: 53).

A clone determined to contain the desired recombinant DNA by DNA sequencing and used to make a large quantity of pure plasmid DNA using a QIAGEN Maxiprep kit. This maxiprep DNA was used for transfection into dihydrofolate reductase-deficient Chinese hamster ovary (CHO-DG44) cells.

A serum-free, suspension adapted CHO-DG44 cell line, called S1-CHO-DG44, was used for developing pLKTOK108 production cell lines. Briefly, transfections were done using a Nucleofector® device from Amaxa Biosystems and Nucleofection® kit V using either non-linearized, circular DNA or linearized plasmid DNA treated with Pvu I restriction enzyme. Transfected cells were maintained in IS-CHO-V-GS growth media for 48 hours before exchanging into G-418 selection media. The live, transfected cells were fed with fresh G-418 selection media and maintained in culture until confluence (~10 to 14 days). The pLKTOK108 productivity of each transfection pool was assessed using a mouse IgG2a ELISA assay and the cells expanded for making frozen cell banks. The transfection pool with the highest productivity by mouse IgG2a ELISA was identified for limited dilution cloning where cells were plated into 5 × 96-well tissue culture plates in G-418 Selection Medium (approximately 1 cell in every other well). The 96-well plates were incubated in a 37°C incubator with 5% $CO_2$ for 2 weeks without feeding. Fifty μL of supernatant from each well that had a single colony was transferred directly into a 96-well assay plate to perform the mouse IgG2a ELISA assay. Twenty three clones with high productivity were identified and expanded sequentially through 24-well cell culture plates and then 6-well cell culture plates. The antibody titer of the supernatant from these clones was measured at 3 different dilutions in the mouse IgG2a ELISA assay.

The best 6 clones based on the mouse IgG2a titer were expanded in G-418 Selection Medium for making frozen cell banks and were adapted to serum-free, suspension Sigma #21 medium. The cell density and viability were determined using the Cedex Automated Cell Culture analyzer, and the protein concentration in the supernatant was measured using the mouse IgG2a ELISA assay. Once the cells reached logarithmic growth phase, they were harvested and frozen at -80°C overnight and then transferred to a liquid nitrogen cryochamber for storage.

To produce the fusion protein, cells were thawed and plated, and subsequently serially expanded into larger T-flasks and then into shaker flasks at starting densities of $3.0 \times 10^5$ cells/mL and incubated in a humidified incubator set at 37°C, with 5% $CO_2$ in an orbital shaker set at 105 rpm. The final cultures were fed with 10% volume of the Sigma #21 Special Feed Medium on Days 4 and 7, and 5% volume on Day 10. Sigma #21 Feed Medium consists of Sigma #21 Medium supplemented with 40 g/L glucose, 10 g/L L-glutamine, 10 g/L yeast extract, and 10 g/L soy peptone. The shake culture was harvested by centrifugation. The supernatant containing secreted pLKTOK108 protein was filtered through a 0.2-μm low protein binding polyethersulfone (PES) membrane filter unit, with the crude pLKTOK108-containing filtrate ready for purification or stored at -80°C for future purification.

Initial purification involved circulating filtered supernatants containing pLKTOK108 over Protein A Sepharose column at approximately 4°C. The resin was then washed with PBS pH7.4, and the protein eluted with 0.1M glycine in PBS at pH 3.0 and neutralized with 1M sodium phosphate at pH 6.5. The neutralized eluate was concentrated using a Vivaspin concentrator with a molecular weight cut-off (MWCO) of 30kDa and loaded onto a Superdex 200 size-exclusion chromatography (SEC) column (Appendix G) that was pre-equilibrated with PBS pH 7.4 buffer in order to separate out

aggregates of this protein. Purified pLKTOK108 protein elutes as a single peak, with purity confirmed on SDS-PAGE and Coomassie staining. Fractions containing the hGCC(ECD)/mIgG2a Fc homodimers were pooled. After the concentration of the pooled material was determined by UV absorbance at 280 nm on a NanoDrop™ ND1000 spectrophotometer, the purified pLKTOK108 protein was aliquoted and stored at - 80°C.

**Example 2**: Generation of rabbit mAbs by protein immunization

Rabbit monoclonal antibodies against the hGCC(ECD)-mIgG2a RcRbr-mutII fusion protein (pLKTOK108) were generated using the RabMAb® service provided by Epitomics (Burlingame, CA). For the purposes of MAb generation, the hGCC(ECD)-mIgG2a RcRbr-mutII fusion protein (pLKTOK108) fusion protein is referred to herein as MIL-44.

Three rabbits (ML1009, ML1010 and ML1011) were immunized with MIL-44 using conventional immunization techniques. The serum titer against MIL-44 and a non-GCC counterscreen antigen (hMadCAM-mFc) was evaluated using test bleeds. Booster immunizations were given subsequent to the initial immunizations. The rabbit with the highest serum titer, rabbit ML1010, was chosen as a candidate for splenectomy and monoclonal fusion using Epitomics' proprietary fusion partner cell line and methods.

On two separate days (Day 1 and Day 2), two hundred million lymphocyte cells were fused with 100 million fusion partner cells and plated on 20X 96-well plates, respectively. The plates were kept in tissue culture incubators under standard conditions. Cell growth was examined 2-3 weeks after fusion and fusion efficiency computed using the number of wells with growth divided by the total number of wells examined. The fusion efficiency for the fusion on Day 1 was measured at 72% fusion efficiency, whereas the fusion efficiency on Day 2 was 79%. A minimum of two plates were examined for each fusion as follows:

All 40 plates were screened using standard ELISA methods with plates coated with 50 ng of MIL-44/well. A bleed of ML1010 at 1:10K dilution was used as a positive control. 151 clones having an O.D. greater than 0.5 were considered putatively positive and were further expanded into a 24-well plate.

A subsequent confirmatory screen was performed by ELISA using plates coated with 50 ng of MIL-44 or 50 ng of hMadCAM-mFc/well. 143 clones were confirmed positive against MIL-44 and among them 72 were identified as MIL-44 specific, i.e., they were negative against hMadCAM-mFc protein.

Following the multiclone supernatant evaluation, several of the MIL-44 specific multiclones were sub-cloned: including multiclone #148 and #67. Subcloning was done using limited cell dilution method. Several subclone supernatants were screened by ELISA. The hybridoma cells for subclones #148-2 and #67-4 were selected for freezing/banking and for further screening and analysis as a GCC detection reagent in an immunohistochemistry (IHC) assay, as described in Example 3.

The MIL-44-148-2 and MIL-44-67-4 antibodies were also cloned into pcDNA3.1+ neo (Invitrogen) for production by transient transfection in mammalian cells and for sequencing. The nucleic acid and amino acid sequences for the heavy and light chains for MIL-44-148-2 and MIL-44-67-4 antibodies are provided below. The signal sequence in each IgG chain is shown italicized; the variable region in each IgG chain is shown in bold font; the CDR's are shown underlined.

**MIL-44-148-2 H2 Nucleic Acid Sequence (SEQ ID NO: 4)**

*ATGGAGACTGGGCTGCGCTGGCTTCTCCTGGTCGCTGTGCTCAAAGGTGTCCAGTGT*
**CAGTCAGTGAAGGAGTCCGGGGGAGGCCTCTTCAAGCCAACGGATACCCTGACACTCACCTGCA**
**CCGTCTCTGGATTCTCCCTCAGTAGTCATAGAATGAACTGGGTCCGCCAGACTCCAGGGAAGGG**
**GCTGGAATGGATCGCAATCATTACTCATAATAGTATCACATACTACGCGAGCTGGGCGAAAAGC**
**CGATCCACCATCACCAGAAACACCAGCGAGAACACGGTGACTCTGAAAATGACCAGTCTGACAG**
**CCGCGGACACGGCCACTTATTTCTGTGCCAGAGAGGATAGTATGGGGTATTATTTTGACTTGTG**
**GGGCCCAGGCACCCTGGTCACCATCTCCTCA**
GGGCAACCTAAGGCTCCATCAGTCTTCCCACTGGCCCCCTGCTGCGGGGACACACCCAGCTCCA
CGGTGACCCTGGGCTGCCTGGTCAAAGGGTACCTCCCGGAGCCAGTGACCGTGACCTGGAACTC
GGGCACCCTCACCAATGGGGTACGCACCTTCCCGTCCGTCCGGCAGTCCTCAGGCCTCTACTCG
CTGAGCAGCGTGGTGAGCGTGACCTCAAGCAGCCAGCCCGTCACCTGCAACGTGGCCCACCCAG
CCACCAACACCAAAGTGGACAAGACCGTTGCGCCCTCGACATGCAGCAAGCCCACGTGCCCACC
CCCTGAACTCCTGGGGGGACCGTCTGTCTTCATCTTCCCCCCAAAACCCAAGGACACCCTCATG
ATCTCACGCACCCCCGAGGTCACATGCGTGGTGGTGGACGTGAGCCAGGATGACCCCGAGGTGC
AGTTCACATGGTACATAAACAACGAGCAGGTGCGCACCGCCCGGCCGCCGCTACGGGAGCAGCA
GTTCAACAGCACGATCCGCGTGGTCAGCACCCTCCCCATCGCGCACCAGGACTGGCTGAGGGGC
AAGGAGTTCAAGTGCAAAGTCCACAACAAGGCACTCCCGGCCCCCATCGAGAAAACCATCTCCA
AAGCCAGAGGGCAGCCCCTGGAGCCGAAGGTCTACACCATGGGCCCTCCCCGGGAGGAGCTGAG
CAGCAGGTCGGTCAGCCTGACCTGCATGATCAACGGCTTCTACCCTTCCGACATCTCGGTGGAG
TGGGAGAAGAACGGGAAGGCAGAGGACAACTACAAGACCACGCCGGCCGTGCTGGACAGCGACG
GCTCCTACTTCCTCTACAGCAAGCTCTCAGTGCCCACGAGTGAGTGGCAGCGGGGCGACGTCTTCACCTGCTCC
GTGATGCACGAGGCCTTGCACAACCACTACACGCAGAAGTCCATCTCCCGCTCTCCGGGTAAATGA

**MIL-44-148-2 H2 Amino Acid Sequence (SEQ ID NO: 42)**

*METGLRWLLLVAVLKGVQC***QSVKESGGGLFKPTDTLTLTCTVSGFSLSSHRMNWVRQTPGKGLE**
**WIAIIITHNSITYYASWAKSRSTITRNTSENTVTLKMTSLTAADTATYFCAREDSMGYYFDLWGP**
**GTLVTISS**
GQPKAPSVFPLAPCCGDTPSSTVTLGCLVKGYLPEPVTVTWNSGTLTNGVRTFPSVRQSSGLYS
LSSVVSVTSSSQPVTCNVAHPATNTKVDKTVAPSTCSKPTCPPPELLGGPSVFIFPPKPKDTLM
ISRTPEVTCVVVDVSQDDPEVQFTWYINNEQVRTARPPLREQQFNSTIRVVSTLPIAHQDWLRG
KEFKCKVHNKALPAPIEKTISKARGQPLEPKVYTMGPPREELSSRSVSLTCMINGFYPSDISVE
WEKNGKAEDNYKTTPAVLDSDGSYFLYSKLSVPTSEWQRGDVFTCSVMHEALHNHYTQKSISRS
PGK

**MIL-44-148-2 L5 Nucleic Acid Sequence (SEQ ID NO: 5)**

*ATGGACACGAGGGCCCCCACTCAGCTGCTGGGGCTCCTGCTGCTCTGGCTCCCAGGTGCCAGATGT*GCCTATGATATGACCCAGACTCCAGCCTCTGTGGAGGTAGCTGTGGGAGGCACAGTCACCATCAAGTGCCAGGCCAGTCAGAGCATTAGTAACTGGTTAGCCTGGTATCAGCAGAAACCAGGGCAGTCTCCCAAGCCCCTGATCTACAGGGCATCCACTCTGGCATCTGGGGTCTCATCGCGGTTCAGAGGCAGTGGATCTGGGACACAGTTCACTCTCACCATCAGTGGCGTGGAGTGTGCCGATGCTGCCACTTACTACTGTCAGCAGACTTATACTAATAATCATCTTGATAATGGTTTCGGCGGAGGGACCGAGGTGGTGGTCAAA

GGTGATCCAGTTGCACCTACTGTCCTCATCTTCCCACCAGCTGCTGATCAGGTGGCAACTGGAACAGTCACCATCGTGTGTGTGGCGAATAAATACTTTCCCGATGTCACCGTCACCTGGGAGGTGGATGGCACCACCCAAACAACTGGCATCGAGAACAGTAAAACACCGCAGAATTCTGCAGATTGTACCTACAACCTCAGCAGCACTCTGACACTGACCAGCACACAGTACAACAGCCACAAAGAGTACACCTGCAGGGTGACCCAGGGCACGACCTCAGTCGTCCAGAGCTTCAATAGGGGTGACTGTTAG

**MIL-44-148-2 L5 Amino Acid Sequence (SEQ ID NO: 43)**

*MDTRAPTQLLGLLLLWLPGARC*AYDMTQTPASVEVAVGGTVTIKCQASQSISNWLAWYQQKPGQSPKPLIYRASTLASGVSSRFRGSGSGTQFTLTISGVECADAATYYCQQTYTNNHLDNGFGGGTEVVVK

GDPVAPTVLIFPPAADQVATGTVTIVCVANKYFPDVTVTWEVDGTTQTTGIENSKTPQNSADCTYNLSSTLTLTSTQYNSHKEYTCRVTQGTTSVVQSFNRGDC

**MIL-44-67-4 H2 Nucleic Acid Sequence (SEQ ID NO: 6)**

*ATGGAGACTGGGCTGCGCTGGCTTCTCCTGGTCGCTGTGCTCAAAGGTGTCCAGTGT***CAGTCGG**
**TGGAGGAGTCCGGGGGTCGCCTGGTCACGCCTGGGACACCCCTGACACTCACCTGCACAGCCTC**
**TGGATCCGACATCAGTAACTATGCAATATCCTGGGTCCGCCAGGCTCCAGGGAAGGGGCTGGAA**
**TTCATCGGATATATTAGTTATGGTAAAAGTATATACTACGCGAGCTGGGCGAAAGGCCGGTTCG**
**CCATCTCCAAAACCTCGTCGACCACGGTGGATCTGGAAATCACCAGTCCGACAACCGAGGACAC**
**GGCCACCTATTTTTGTGCCAGAGAGGATAGTGCTACTTATAGTCCTAACTTGTGGGGCCCAGGC**
**ACCCTGGTCACCGTCTCCTCA**
GGGCAACCTAAGGCTCCATCAGTCTTCCCACTGGCCCCCTGCTGCGGGGACACACCCAGCTCCA
CGGTGACCCTGGGCTGCCTGGTCAAAGGGTACCTCCCGGAGCCAGTGACCGTGACCTGGAACTC
GGGCACCCTCACCAATGGGGTACGCACCTTCCCGTCCGTCCGGCAGTCCTCAGGCCTCTACTCG
CTGAGCAGCGTGGTGAGCGTGACCTCAAGCAGCCAGCCCGTCACCTGCAACGTGGCCCACCCAG
CCACCAACACCAAAGTGGACAAGACCGTTGCGCCCTCGACATGCAGCAAGCCCACGTGCCCACC
CCCTGAACTCCTGGGGGGACCGTCTGTCTTCATCTTCCCCCCAAAACCCAAGGACACCCTCATG
ATCTCACGCACCCCCGAGGTCACATGCGTGGTGGTGGACGTGAGCCAGGATGACCCCGAGGTGC
AGTTCACATGGTACATAAACAACGAGCAGGTGCGCACCGCCCGGCCGCCGCTACGGGAGCAGCA
GTTCAACAGCACGATCCGCGTGGTCAGCACCCTCCCCATCGCGCACCAGGACTGGCTGAGGGGC
AAGGAGTTCAAGTGCAAAGTCCACAACAAGGCACTCCCGGCCCCCATCGAGAAAACCATCTCCA
AAGCCAGAGGGCAGCCCCTGGAGCCGAAGGTCTACACCATGGGCCCTCCCCGGGAGGAGCTGAG
CAGCAGGTCGGTCAGCCTGACCTGCATGATCAACGGCTTCTACCCTTCCGACATCTCGGTGGAG
TGGGAGAAGAACGGGAAGGCAGAGGACAACTACAAGACCACGCCGGCCGTGCTGGACAGCGACG

GCTCCTACTTCCTCTACAGCAAGCTCTCAGTGCCCACGAGTGAGTGGCAGCGGGGCGACGTCTT
CACCTGCTCCGTGATGCACGAGGCCTTGCACAACCACTACACGCAGAAGTCCATCTCCCGCTCT
CCGGGTAAATGA

**MIL-44-67-4 H2 Amino Acid Sequence (SEQ ID NO: 44)**

*METGLRWLLLVAVLKGVQC***QSVEESGGRLVTPGTPLTLTCTASGSDISNYAISWVRQAPGKGLE**
**FIGYISYGKSIYYASWAKGRFAISKTSSTTVDLEITSPTTEDTATYFCAREDSATYSPNLWGPG**
**TLVTVSS**
GQPKAPSVFPLAPCCGDTPSSTVTLGCLVKGYLPEPVTVTWNSGTLTNGVRTFPSVRQSSGLYS
LSSVVSVTSSSQPVTCNVAHPATNTKVDKTVAPSTCSKPTCPPPELLGGPSVFIFPPKPKDTLM
ISRTPEVTCVVVDVSQDDPEVQFTWYINNEQVRTARPPLREQQFNSTIRVVSTLPIAHQDWLRG
KEFKCKVHNKALPAPIEKTISKARGQPLEPKVYTMGPPREELSSRSVSLTCMINGFYPSDISVE
WEKNGKAEDNYKTTPAVLDSDGSYFLYSKLSVPTSEWQRGDVFTCSVMHEALHNHYTQKSISRS
PGK

**MIL-44-67-4 L4 Nucleic Acid Sequence (SEQ ID NO: 7)**

*ATGGACACGAGGGCCCCCACTCAGCTGCTGGGGCTCCTGCTGCTCTGGCTCCCAGGTGCCAGAT*
*GT***GCCTATGATATGACCCAGACTCCAGCCTCTGTGGAGGTAGCTGTGGGAGGCACAGTCACCAT**
**CAAGTGCCAGGCCAGTCAGAGTATTAACACCTACTTAGCCTGGTATCAGCAGAAACCAGGGCAG**
**CGTCCCAAGCTCCTGATCTACAGGGCATCCACTCTGGCATCTGGGGTCTCATCGCGGTTCAAAG**
**GCAGTGGATCTGGGACAGAGTTCACTCTCACCATCAGCGGCGTGGAGTGTGCCGATGCTGCCAC**
**TTACTACTGTCAACAGGGTTATAGTTATAATAATCTTGATCGTGCTTTCGGCGGAGGGACCGAG**
**GTGGTGGTCACA**
GGTGATCCAGTTGCACCTACTGTCCTCATCTTCCCACCAGCTGCTGATCAGGTGGCAACTGGAA
CAGTCACCATCGTGTGTGTGGCGAATAAATACTTTCCCGATGTCACCGTCACCTGGGAGGTGGA
TGGCACCACCCAAACAACTGGCATCGAGAACAGTAAAACACCGCAGAATTCTGCAGATTGTACC
TACAACCTCAGCAGCACTCTGACACTGACCAGCACACAGTACAACAGCCACAAAGAGTACACCT
GCAAGGTGACCCAGGGCACGACCTCAGTCGTCCAGAGCTTCAATAGGGGTGACTGTTAG

**MIL-44-67-4 L4 Amino Acid Sequence (SEQ ID NO: 45)**

*MDTRAPTQLLGLLLLWLPGARC***AYDMTQTPASVEVAVGGTVTIKCQASQSINTYLAWYQQKPGQ**
**RPKLLIYRASTLASGVSSRFKGSGSGTEFTLTISGVECADAATYYCQQGYSYNNLDRAFGGGTE**
**VVVT**
GDPVAPTVLIFPPAADQVATGTVTIVCVANKYFPDVTVTWEVDGTTQTTGIENSKTPQNSADCT
YNLSSTLTLTSTQYNSHKEYTCKVTQGTTSVVQSFNRGDC

**Example 3**: Immunohistochemistry using anti-GCC antibodies

*Detection of GCC expression in human tumor xenograft models of mCRC*

An IHC assay using the MIL-44-148-2 antibody was developed to evaluate GCC expression in HEK293-GCC xenograft tumors and several primary human tumor xenografts (PHTX) derived from metastatic colorectal cancer (mCRC) patient samples in female SCID mice.

GCC protein levels in Formalin-Fixed, Paraffin-Embedded (FFPE) tissues were assessed on 5 μm thick sections and incubated with MIL-44-148-2 antibody (3.5 μg/mL) for 1 hour on the Ventana Medical Systems (Tucson, AZ) Discovery XT® automated stainer. Antibodies were biotinylated with a rabbit anti-goat secondary antibody (Vector Laboratories) and developed with the 3,3'-diaminobexidine (DAB) substrate map system (Ventana Medical Systems). Slides were counterstained with hematoxylin and imaged using the Aperio whole slide scanning system.

GCC levels differed significantly among these tumors with H-scores (scoring system described below) ranging from 4+ in HEK293-GCC tumor xenografts, and from 1+, 1-2+, 2+, 2-3+, and 4+ in various PHTX tumor xenografts. In general, in tumors with moderate/well differentiated tumor cells that maintained a polarized epithelial structure, GCC was concentrated on the luminal side of the tumor tissue.

*Detection of GCC expression in human colon samples and tumor microarrays*

The MIL-44-148-2 and MIL-44-67-4 antibodies described herein were also screened as a GCC detection reagent in an IHC protocol described above using the above-reference primary human tumor xenografts (PHTX), HT29 and HEK293 GCC transfected cell pellets, in addition to malignant and benign human colon samples (FFPE and tumor microarrays (TMAs)).

HT-29 and HEK293 GCC transfected cell pellets stained as expected. The PHTXs demonstrated a wide range

of staining intensities with the MIL-44-148-2 and MIL-44-67-4 clones. Both MIL-44-148-2 and MIL-44-67-4 stained positive in well or moderately differentiated colon carcinoma *in situ* or metastasis. Poorly differentiated tumors stained less intensely. Normal colon tissue demonstrated positive apical staining using antibodies produced by both the MIL-44-148-2 and MIL-44-67-4 subclones.

Antibodies from both the MIL-44-148-2 and MIL-44-67-4 subclones provided readily apparent, intense and specific staining in the GCC transfected HEK293 cells and HT29 cells without any non-specific staining in the HEK293 and HT29 parental cell lines. Both clones also stained positive in well or moderately differentiated colon carcinoma in situ or metastasis. Less staining was seen for both subclones in poorly differentiated carcinomas. Normal colon demonstrated positive apical staining for both clones, as expected. MIL-44-148-2 demonstrated an overall higher sensitivity and specificity than MIL-44-67-4 in IHC. While MIL-44-67-4 demonstrated a better dynamic range than MIL-44-148-2 in cell pellets, MIL-44-148-2 demonstrated a superior dynamic range over that of MIL-44-67-4 in TMAs. Overall, the MIL-44-148-2 subclone showed superiority over the MIL-67-4, demonstrating higher sensitivity and specificity in IHC, and a full dynamic range in TMA's, and an intense staining (+2 IHC score) in normal colon at a low concentration.

Based on the results of the initial IHC experiments described above, the MIL-44-148-2 subclone was selected for the development and validation of an automated protocol equivalent to the IHC protocol described above using a Tek-Mate automated stainer. The automated IHC assay is a useful tool for screening cancer patients for GCC expressing tumors as a clinical trial enrollment criteria for a GCC-targeted cancer therapeutic, and generally as a screening tool for selecting patients (e.g., cancer patients) who should receive a GCC-targeted therapy.

The IHC protocol shown in Table 18 was developed for detection of GCC in FFPE human cells and tissues, and approximately 53 colorectal tumors and 20 normal colon tissues, as well as 2 colon cancer TMAs (purchased from US Biomax) were screened for GCC expression. These tumors covered a range of tumor grades as well as colon cancer metastatic tissues.

Four-micron sections were prepared from the various tissue samples. Tissue sections were dewaxed through 4, 5-minute changes of xylene followed by a graded alcohol series to distilled water. Steam heat induced epitope recovery (SHIER) was used with SHIER2 solution for 20 minutes in the capillary gap in the upper chamber of a Black and Decker Steamer.

**Table 18A: IHC Procedure**

| TechMate Steps | UltraVision Detection (UV) |
|---|---|
| 1. | UltraVision Block-15 minutes |
| 2. | Primary Antibody Incubation - Overnight |
| 3. | Primary Antibody Enhancer-25 minutes |
| 4. | Hydrogen peroxide block- 3 x 2.5 minutes each |
| 5. | Polymer Detection-25 minutes |
| 6. | DAB Chromogen- 3 x 5.0 minutes each |
| 7. | Hematoxylin Counter Stain - 1 minute |

**Table 18B: Antibody Reactivity Spec Sheet**

| **Antibody** | **GCC** |
|---|---|
| **Supplier** | MLNM Takeda in-house antibody |
| **Catalog No**. | N/A |
| **Source/Isotype** | RbIgG |
| **Supplier Lot #** | Not determined |
| **QualTek Lot #** | R3512 |
| **Clone** | 148-2 |
| **Concentration** | 0.475μg/ml |
| **Suggested Dilution** | 1.0μg/ml |
| **Incubation Time** | Overnight |

(continued)

| Antibody | GCC |
|---|---|
| Pretreatments | SHIER2, no enzyme |
| TechMate Protocol | MIP |
| Detection system | Ultra Vision Detection System |
| Sub-Cellular Localization | Cytoplasmic and/or apical |

One skilled in the art would recognize that the primary antibody enhancer can be an anti-rabbit secondary antibody raised in a species other than rabbit (e.g., human, rat, goat, mouse, etc.) having the same isotype as the MIL-44-148-2 or MIL-44-67 rabbit mAbs (rabbit IgG) or a similar reagent that is suitable to amplify the MIL-44 signal.

The above protocol used an overnight antibody incubation of MIL-44-148-2 at 1.0μg/ml with a non-biotin based peroxidase detection (Ultravision kit from Thermo/Lab Vision) and DAB as chromogen. This procedure was completely automated using the TechMate 500 or TechMate 1000 (Roche Diagnostics). After staining, slides were dehydrated through an alcohol series to absolute ethanol followed by xylene rinses. Slides were permanently coverslipped with glass coverslips and CytoSeal. Slides were examined under a microscope to assess staining. Positive staining is indicated by the presence of a brown (DAB-HRP) reaction product. Hematoxylin counterstain provides a blue nuclear stain to assess cell and tissue morphology.

Upon evaluating the GCC staining, it was determined that an H-score approach would be the best approach for quantifying GCC expression. The H-score approach provides optimal data resolution for determining variation in intensity and tumor percentage of staining within and among tumor types. It also provides a good tool for determining thresholds for positive staining. In this method, the percentage of cells (0-100) within a tumor with staining intensities ranging from 0-3+ are provided. With the instant method, scores with intensities of 0, 0.5, 1, 2 and 3 were provided. Depending on the marker, 0.5 staining can be scored as positive or negative, and reflects light but perceptible staining for the marker. To obtain an H-score, the percentage of tumor cells are multiplied by each intensity and added together: H score = (% tumor*1) + (% tumor*2) + (% tumor*3). For example, if a tumor is 20% negative (0), 30% +1, 10% +2, 40% +3, this would give an H score of 170.

The maximum H-score is 300 (100% * +3), per sub-cellular localization (i.e., apical or cytoplasmic), if 100% of tumor cells label with 3+ intensity. Initially, as a control, the total H-score alone was not be used to compare samples, but evaluated in addition to a review of the break-down of the percentage of cells at each intensity. For example, a score of 90 could represent 90% of tumor cells staining with 1+ intensity or 30% of cells with 3+ intensity. These samples have the same H-score but very different GCC expression. The percentage of cells to be scored at each intensity can vary, but are normally scored in increments of 10%; however, a small percentage of scoring of a single component can be estimated at 1% and 5% as well in order to demonstrate that some level of staining is present. For GCC, apical staining may be considered for evaluating at low level increments, such as 1 and 5%.

Two different sub-cellular localizations were scored for GCC using the H-score approach. These include cytoplasmic staining and apical associated staining. The cytoplasmic staining pattern was generally observed as diffuse throughout the cytoplasm of tumor cells. However, in some cases there were variations of the cytoplasmic staining, which included intense globular staining or punctate, coarse granular staining. Intense globular staining was scored as 3+ cytoplasmic staining. The punctate staining was associated with apical staining and was not given a separate score for this type of cytoplasmic staining (n=4 samples for punctate staining). GCC apical staining was observed when lumen were present. Other GCC staining patterns observed included membrane-like, non-lumen staining (one case) and extra-cellular staining present in tumor lumen. In normal colon tissues, staining was generally apical along with diffuse cytoplasmic staining.

Since H scores were obtained for both cytoplasmic and apical GCC expression, and since it is not known whether one type of localization is more critical over another for efficacy of a GCC-targeted therapy, all data was captured and an some instances, an aggregate H score was generated by using the sum of both apical and cytoplasmic GCC expression. In such instances, the maximum H score became 600 for the aggregate score (300 apical + 300 cytoplasmic).

Overall, staining in a normal colon samples illustrated that GCC is anatomically privileged, being expressed on the apical surface. GCC was expressed on more than 95% of tumor samples and, in contrast to normal tissue, demonstrated diffuse cytoplasmic staining in some cases. Strong focal GCC staining in human CRC liver metastasis samples was also seen.

Tables 19A shows cytoplasmic and apical H-score staining results for normal and tumor tissues that were screened. Data shown in Table 19A is broken-out according to sample origin (in-house (denoted as MLNM), TMAs (denoted as BIOMAX), and CRO (denoted as QualTek)) and tumor grade. Summary data of positivity is provided when using thresholds of 0.5 and 1.0+ staining intensity. A total of 173 tumor samples were scored. When using a 0.5+ cut-off for positive staining intensity for either cytoplasmic or apical staining, 95% of tumors are considered positive. When using a 1.0+ cut-off, 92% of samples are considered positive.

The source of tissues shows variation in the percentage of positive tumors cells as well as the H-scores. For 1+ staining positivity threshold, the range is from 84% (CRO tumor MTB samples) to 100% (in-house samples or CRO single tissue samples - note the smaller number of samples in these groups). The in-house tumor tissues showed a very high apical H-score of 253 (n=9 samples). There were also differences in the scoring results of the 2 TMAs. US Biomax TMA C0992 stained stronger than C0701. Without intending to be bound by any theory, the difference in the TMAs may be due to a difference with fixation with the source of tissues or one block could have been cut more recently than the other.

The stability of the antigen in a cut section and the freshness of the cut samples were considered. Samples tested the instant study included samples that were cut and stored and samples that were cut fresh, indicating a need to further research the stability of the tissue samples over time.

Some differences were observed in GCC positivity and tumor grade (see Table 19B), with greater positive staining associated with well differentiated tumors vs. poorly differentiated tumors (six tumors from US Biomax did not include a grade). Grade 1 tumors (n=20) showed 100% positivity; Grade 2 tumors (n=95) labeled with 98% of positive cases; and grade 3 tumors (n=44) labeled at a positivity rate of 88%. Poorly differentiated tumors generally lack lumen, which may account for some of this decrease in staining due to a lack of apical staining. This percent positivity was based on a 0.5+ staining intensity threshold. Seven of 7 distant mets were positive (from in-house and CRO tissues). Metastatic tumors from the US Biomax TMA were listed as mets to lymph nodes.

Overall, GCC stains a very high percentage of colon tumors and normal colon tissues regardless of the source of the tissue or the tumor grade.

**Table 19A: Summary of colon cancer staining by sample source**

| Colon CA Samples | Total Colon CA Samples | No. Sample Positive | | | | Mean H-Score | |
|---|---|---|---|---|---|---|---|
| | | 0.5+ & Greater | | 1.0+ & Greater | | 0-300 | |
| | | No. | % | No. | % | Cyto | Apical |
| MLNM Samples | 9 | 9 | 100% | 9 | 100% | 83 | 253 |
| QualTek Single Samples | 4 | 4 | 100% | 4 | 100% | 69 | 98 |
| QualTek Colon CA MTBS | 43 | 39 | 91% | 36 | 84% | 66 | 118 |
| BIOMAX C0992 Array | 65 | 63 | 97% | 63 | 97% | 144 | 164 |
| BIOMAX C0701 Array | 52 | 49 | 94% | 48 | 92% | 98 | 118 |
| Total | 173 | 164 | 95% | 160 | 92% | 102 | 138 |

**Table 19B: Summary of colon cancer staining by tumor grade**

| Colon Samples | No. Positive | Total Samples | % |
|---|---|---|---|
| Normal | 57 | 58 | 98% |
| Grade 1 | 20 | 20 | 100% |
| Grade 2 | 95 | 97 | 98% |
| Grade 3 | 44 | 50 | 88% |
| Total | 216 | 225 | 96% |

Intra-assay precision of the GCC IHC assay was evaluated within one run utilizing 5 replicates each from three cell pellets and 14 different colon carcinoma tissues. Cell pellets were prepared on separate slides. Colon tumor samples were included in two different multi-tumor blocks. These tissues were scored for GCC IHC reactivity.

Precision staining of cell pellets: Near identical staining was observed in all of the 5 intra-run replicates of the 3 cell pellets.

Precision staining of colon tumor samples: Very similar to near identical staining was observed among the 5 intra-run replicates of the 14 colon tumor samples. Samples were scored by a certified pathologist using the H-score approach as described previously above. The standard deviation demonstrated that in all cases the variance was minimal, thus demonstrating good precision of staining within the same run. Overall, there was very consistent intra-run GCC

IHC staining of the cell pellet and colon carcinoma samples tested.

Between-run assay variability and variability due to different operators was evaluated in 5 separate GCC IHC staining runs. Four runs were performed on different days by one operator and a second operator performed the fifth run. Staining included testing of the same tissues in the precision testing described above.

Reproducibility staining of cell pellets: Near identical staining as observed in all of the 5 inter-run replicates of the 3 cell pellets.

Reproducibility staining of colon tumor samples: Very similar to near identical staining was observed among the 5 inter-run replicates of the 14 colon tumor samples. Samples were scored by a certified pathologist using the H-score approach as described previously. The standard deviation demonstrated that in all cases the variance as minimal, thus demonstrating good reproducibility of staining from day to day and with a different operator. Overall, there was very consistent inter-run and inter-operator GCC IHC staining of the cell pellet and colon carcinoma samples tested.

Specificity of the GCC IHC assay was evaluated by testing a panel of normal human tissues. These normal human tissues included 30 different tissue types: adrenal, bladder, bone marrow, breast, cerebral cortex, cervix, fallopian tube, heart, kidney, liver, lung, lymph node, nerve, ovary, pancreas, parotid (salivary gland), pituitary, placenta, prostate, skeletal muscle, skin, spinal cord, spleen, stomach, testis, thymus, thyroid, tonsil, ureter, and uterus. For each tissue type, at least 3 unique specimens were stained and evaluated for GCC immunoreactivity.

Overall, the GCC IHC assay using the MIL-44-148-2 antibody was shown by the IHC assay described herein to be very specific for colon tumor samples compared to normal tissue staining, particularly for apical staining. Apical staining was only detected in 2 of the stomach samples; however, this staining was also observed in the negative control. Cytoplasmic staining, generally light, was observed in several tissue types, including ovarian follicle (1 of 3 samples), skin (follicle and dermis, 2 of 3 samples), stomach parietal cells (2 of 3 samples), prostate glandular epithelium (light in 3 of 3 cases), pituitary (2 of 3 cases), uterus epithelium (3 of 3 cases), fallopian tube epithelium (2 of 3 cases), placenta trophoblast (light in 2 of 3 cases) and lung (endothelium in 3 of 3 cases and bronchiole epithelium in 1 of 3 cases). The strongest cytoplasmic staining (2+) was present in one case of fallopian tube and one case of pituitary. In both cases there was lighter staining in the same compartments in the negative control. Plasma cells were positive in a number of tissues, including spleen, tonsil and lymph node. Histiocytes were positive in spleen, lung and lymph node. Stromal staining was present in testis (2 of 3 cases), uterus (3 of 3 cases) and ovary (1 of 3 cases). Extracellular staining of blood vessels was widely observed and appears to be non-specific binding of serum.

The GCC assay, using the rabbit monoclonal antibody, MIL-44-148-2, on the TechMate staining platform shows consistent inter and intra-run staining of tumors and control cell pellets. The GCC assay appears to be highly sensitive in colon carcinoma as it stains the vast majority of colon tumor samples tested. The GCC assay also appears to be much more specific for colon tumors compared to normal tissues. GCC expression observed in many colon tumors is far stronger than any staining observed in a 30 tissue normal panel with at least 3 replicates of each tissue type. No specific apical GCC staining was detected in any of the normal tissues, whereas apical staining is common in the majority of GCC samples. Only cytoplasmic staining is observed in some normal tissue types and this staining is generally light. The MIL-44-148-2 antibody appears to be a reproducible, sensitive and relatively specific IHC marker for staining formalin-fixed, paraffin-embedded (FFPE) colon tumors.

**Example 4:** Additional Immunohistochemistry in Non-Colorectal PHTX Models and Tumor MicroArrays and Colorectal and Non-Colorectal Human Clinical Samples

The automated IHC assay described in Example 3 was used to evaluate GCC expression (i.e., apical, cytoplasmic and/or aggregate GCC expression) in a variety of non-colorectal samples from different sources, including primary human tumor xenografts (PHTX) derived from gastric cancer and pancreatic cancer patient samples in female SCID mice, and various tumor microarrays (TMAs) purchased from US BioMax, Pantomics and other commercial sources) specific for pancreatic cancer, gastric cancer, esophageal cancer, lung cancer and leiomyosarcomas/rhabdomyosarcomas. GCC expression was also assessed via the automated IHC assay described in Example 3 in a variety human clinical samples, including human gastric, pancreatic and esophageal tumor samples obtained from the tissue database of a specialty CRO (QualTek) engaged to run the automated IHC assay, and colorectal cancer, gastric cancer, pancreatic cancer, esophageal cancer, and small intestine cancer derived from cancer patients tested for GCC expression prior to enrollment in an open-label, multicenter, dose escalation, first-in-human study of a GCC-targeted antibody-drug conjugate, designated as MLN0264, in adult patients with advanced gastrointestinal malignancies expressing Guanylyl Cyclase C (Study C26001, ClinicalTrials.gov identifier NCT01577758).

The results of the IHC staining in various normal and cancerous colorectal tissue samples and non-colorectal tissues (gastric, pancreatic, small intestine, esophageal, lung, rhabdomyosarcoma, leiomyosarcoma) from various sources (PHTX, human clinical samples and tumormicroarrays) are shown in Tables 20-32 below. Tables 20-32 include the IHC scores (0, 0.5+, 1+, 2+ and 3+) and the corresponding H scores calculated based on the IHC scores for both Apical ("A") and cytoplasmic ("C" or "Cyto") GCC staining in the various tissues tested. In addition, Table 32 shows a comparison

of GCC IHC expression data in human clinical samples of primary and metastatic cancer, in each case obtained from the same patient. As shown in the first lefthand column, the "A" samples (i.e., 1A, 2A, 3A, 4A, 5A, 6A, 7A, 8A, 9A 10A, etc.), refer to primary tumor samples, whereas the "B" samples (i.e., 1B, 2B, 3B, 4B, 5B, 6B, 7B, 8B, 9B, 10B, etc.) refer to metastatic tumor samples obtained from the same patient from which the corresponding primary tumor sample (the corresponding"A" sample) was obtained. In otherwords, the designation "1A" and "1B" refer to primary and metastatic tumors, respectively, obtained from the same patient; the designation "2A" and "2B" refer to primary and metastatic tumors, respectively, obtained from another patient, and so forth. A majority of the tumor samples shown in Table 32 were obtained from patients having primary and metastatic colorectal cancer, except where indicated otherwise as reflected in the column labeled "Comments", in which case the particular type of non-colorectal cancer is specified. As can be seen in the "Comments" column in Table 32, some samples of neuroendocrine tumors, renal cell carcinomas, gastric tumors, gastric GIST, pancreatic tumors, and uterine leiomyosarcoma were also for GCC expression by the IHC assay.

**Table 20: IHC Assay in PHTX Model of Human Gastric Cancer**

| Sample No. | Sample Type | GCC Apical & Cytoplasmic Staining (Percent) | | | | | | | | | | H-Score | H-Score |
|---|---|---|---|---|---|---|---|---|---|---|---|---|---|
| | | 0C | 0A | 0.5+ C | 0.5+ A | 1+ C | 1+ A | 2+ C | 2+ A | 3+ C | 3+A | Cyto | Apical |
| GAF-023 | Gastric CA Xenograft | | | 100 | | | | | | | 100 | 50 | 300 |
| GAF-025 | Gastric CA Xenograft | | 100 | | | 90 | | 5 | | 5 | | 115 | 0 |
| GAF-055 | Gastric CA Xenograft | | | | | 70 | | 30 | | | 100 | 130 | 300 |
| GAF-074 | Gastric CA Xenograft | 100 | 90 | | | | | | 10 | | | 0 | 20 |
| GAF-075 | Gastric CA Xenograft | 90 | 100 | 10 | | | | | | | | 5 | 0 |
| GAF-087 | Gastric CA Xenograft | 100 | 100 | | | | | | | | | 0 | 0 |
| GAF-114 | Gastric CA Xenograft | 100 | 100 | | | | | | | | | 0 | 0 |
| GAF-152 | Gastric CA Xenograft | 100 | 100 | | | | | | | | | 0 | 0 |
| GAF-318 | Gastric CA Xenograft | | | | | 60 | | 40 | 20 | | 80 | 140 | 280 |
| GAF-019 | Gastric CA Xenograft | | | | | 60 | | 20 | | 20 | 100 | 160 | 300 |
| GAF-151 | Gastric CA Xenograft | 50 | 50 | 50 | | | | | 40 | | 10 | 25 | 110 |

(continued)

| Sample No. | Sample Type | GCC Apical & Cytoplasmic Staining (Percent) | | | | | | | | | | H-Score | H-Score |
|---|---|---|---|---|---|---|---|---|---|---|---|---|---|
| | | 0C | 0A | 0.5+ C | 0.5+ A | 1+ C | 1+ A | 2+ C | 2+ A | 3+ C | 3+A | Cyto | Apical |
| GAM-006 | Gastric CA Xenograft | | 100 | 50 | | 40 | | 10 | | | | 85 | 0 |
| GAM-016 | Gastric CA Xenograft | 100 | 100 | | | | | | | | | 0 | 0 |
| GAM-022 | Gastric CA Xenograft | | 100 | 100 | | | | | | | | 50 | 0 |
| GAM-031 | Gastric CA Xenograft | 90 | 100 | 10 | | | | | | | | 5 | 0 |
| GAM-033 | Gastric CA Xenograft | 70 | 30 | 20 | | 10 | | | 40 | | 30 | 20 | 170 |
| GAM-037 | Gastric CA Xenograft | 100 | 100 | | | | | | | | | 0 | 0 |
| GAM-042 | Gastric CA Xenograft | 100 | 100 | | | | | | | | | 0 | 0 |
| GAM-044 | Gastric CA Xenograft | | | | | 50 | | 50 | 50 | | 50 | 150 | 250 |
| GAM-046 | Gastric CA Xenograft | 50 | 100 | 50 | | | | | | | | 25 | 0 |
| GAM-060 | Gastric CA Xenograft | | | | | 20 | | 30 | | 50 | 100 | 230 | 300 |
| GAM-080 | Gastric CA Xenograft | 80 | 100 | 20 | | | | | | | | 10 | 0 |
| GAM-093 | Gastric CA Xenograft | 10 | 20 | 10 | | 20 | 10 | 30 | 40 | 30 | 30 | 175 | 180 |
| GAM-095 | Gastric CA Xenograft | 80 | 100 | 10 | | 10 | | | | | | 15 | 0 |
| GAM-098 | Gastric CA Xenograft | | 10 | 70 | | 30 | | | 40 | | 50 | 65 | 230 |

(continued)

| Sample No. | Sample Type | GCC Apical & Cytoplasmic Staining (Percent) | | | | | | | | | | H-Score | H-Score |
|---|---|---|---|---|---|---|---|---|---|---|---|---|---|
| | | 0C | 0A | 0.5+ C | 0.5+ A | 1+ C | 1+ A | 2+ C | 2+ A | 3+ C | 3+A | Cyto | Apical |
| GAM-110 | Gastric CA Xenograft | 50 | 100 | 50 | | | | | | | | 25 | 0 |
| GAM-119 | Gastric CA Xenograft | | 100 | | | | | 40 | | 60 | | 260 | 0 |
| GAM-138 | Gastric CA Xenograft | 100 | 100 | | | | | | | | | 0 | 0 |
| GAM-139 | Gastric CA Xenograft | 90 | 90 | 10 | | | 10 | | | | | 5 | 10 |

**Table 21: PHTX Model of Human Pancreatic Cancer**

| Sample # | Tumor Type | Apical & Cytoplasmic Staining (Percent) | | | | | | | | POS /NEG | % POS | % POS | H | H |
|---|---|---|---|---|---|---|---|---|---|---|---|---|---|---|
| | | 0C | 0A | 1+ C | 1+ A | 2+ C | 2+ A | 3+ C | 3+ A | | Cyto | Apical | Cyto | Apical |
| 117 | Gastric | | 40 | 100 | 20 | | 20 | | 20 | **POS** | 100 | 60 | 100 | 120 |
| 118 | Pancreatic | | 100 | 50 | | 40 | | 10 | | **POS** | 100 | 0 | 160 | 0 |
| 119 | Pancreatic | | 50 | | 50 | 60 | | 40 | | **POS** | 100 | 50 | 240 | 50 |
| 120 | Gastric | 30 | 70 | 70 | 30 | | | | | **POS** | 70 | 30 | 70 | 30 |
| 121 | Pancreatic | 20 | 20 | 80 | 70 | | 10 | | | **POS** | 80 | 80 | 80 | 90 |
| 122 | Pancreatic | | | | 50 | 50 | 50 | 50 | | **POS** | 100 | 100 | 250 | 150 |
| 123 | Gastric | | 100 | | | 50 | | 50 | | **POS** | 100 | 0 | 250 | 0 |
| 124 | Pancreatic | 30 | 80 | 50 | 10 | 20 | 10 | | | **POS** | 70 | 20 | 90 | 30 |
| 125 | Pancreatic | | | | 20 | 50 | 50 | 50 | 30 | **POS** | 100 | 100 | 250 | 210 |
| 126 | Pancreatic | | 50 | 80 | 20 | 20 | 20 | | 10 | **POS** | 100 | 50 | 120 | 90 |
| 127 | Pancreatic | 50 | | 50 | | | 20 | | 80 | **POS** | 50 | 100 | 50 | 280 |
| 128 | Pancreatic | | | 20 | | 40 | | 40 | 100 | **POS** | 100 | 100 | 220 | 300 |
| 129 | Pancreatic | 50 | 100 | 50 | | | | | | **POS** | 50 | 0 | 50 | 0 |
| 130 | Pancreatic | | 80 | 80 | 20 | 20 | | | | **POS** | 100 | 20 | 120 | 20 |
| 131 | Pancreatic | | | | | 50 | | 50 | 100 | **POS** | 100 | 100 | 250 | 300 |
| 132 | Pancreatic | | 30 | 20 | 30 | 40 | 30 | 40 | 10 | **POS** | 100 | 70 | 220 | 120 |
| 133 | Pancreatic | 100 | 100 | | | | | | | **NEG** | 0 | 0 | 0 | 0 |
| 134 | Pancreatic | | | 40 | 40 | 40 | 40 | 20 | 20 | **POS** | 100 | 100 | 180 | 180 |
| 135 | Pancreatic | | 70 | 70 | 20 | 30 | 10 | | | **POS** | 100 | 30 | 130 | 40 |
| 136 | Pancreatic | | 30 | | 30 | 100 | 30 | | 10 | **POS** | 100 | 70 | 200 | 120 |
| 137 | Pancreatic | | | | | 70 | 50 | 30 | 50 | **POS** | 100 | 100 | 230 | 250 |
| 138 | Pancreatic | | 0 | 100 | | | | | | **POS** | 100 | 0 | 100 | 0 |
| 139 | Pancreatic | | | 50 | 40 | 50 | 30 | | 30 | **POS** | 100 | 100 | 150 | 190 |
| 140 | Pancreatic | | 50 | 100 | 50 | | | | | **POS** | 100 | 50 | 100 | 50 |

(continued)

| Sample # | Tumor Type | Apical & Cytoplasmic Staining (Percent) | | | | | | | | POS /NEG | % POS | % POS | H | H |
|---|---|---|---|---|---|---|---|---|---|---|---|---|---|---|
| | | 0C | 0A | 1+ C | 1+ A | 2+ C | 2+ A | 3+ C | 3+ A | | Cyto | Apical | Cyto | Apical |
| 141 | Pancreatic | 30 | 40 | 40 | 30 | 30 | 30 | | | **POS** | 70 | 60 | 100 | 90 |
| 142 | Pancreatic | | 100 | 30 | | 70 | | | | **POS** | 100 | 0 | 170 | 0 |
| 143 | Pancreatic | | 30 | | 30 | 50 | 20 | 50 | 20 | **POS** | 100 | 70 | 250 | 130 |
| 144 | Pancreatic | | 20 | 50 | 20 | 50 | 20 | | 40 | **POS** | 100 | 80 | 150 | 180 |
| 145 | Pancreatic | | | 40 | | 30 | | 30 | 100 | **POS** | 100 | 100 | 190 | 300 |

**Table 22A: GCC IHC Staining in US Biomax P1921 Pancreatic Tumor MicroArray (TMA)**

| USBiomax PA1921 Pancreate TMA | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+C | 1+A | 2+ C | 2+ A | 3+ C | 3+A | NEG | Cyto | Anical | Cyto | Apical |
| A1 | 48 | F | Duct adenocarcinoma (chronic inflammation of pancreas tissue) | - | 100 | 100 | | | | | | | NEG | 0 | 0 | 0 | 0 |
| A2 | 41 | F | Duct adenocarcinoma | 1 | | | | | | | | | | | | | |
| A3 | 57 | M | Duct adenocarcinoma | 1 | 100 | | | | | | | 100 | POS | 0 | 100 | 0 | 300 |
| A4 | 42 | F | Duct adenocarcinoma | 1 | 100 | 100 | | | | | | | NEG | 0 | 0 | 0 | 0 |
| A5 | 47 | F | Duct adenocarcinoma | 1 | | | | | | | | | | | | | |
| A6 | 54 | F | Duct adenocarcinoma | 1 | | | | | | | | | | | | | |
| A7 | 40 | F | Duct adenocarcinoma | 2 | | | 30 | | 70 | | | 100 | POS | 100 | 100 | 170 | 300 |
| A8 | 54 | F | Duct adenocarcinoma | 2 | 100 | 100 | | | | | | | NEG | 0 | 0 | 0 | 0 |
| A9 | 48 | F | Duct adenocarcinoma | 1 | 100 | 100 | | | | | | | NEG | 0 | 0 | 0 | 0 |
| A10 | 41 | F | Duct adenocarcinoma (sparse) | 1 | 100 | 100 | | | | | | | NEG | 0 | 0 | 0 | 0 |
| A11 | 57 | M | Duct adenocarcinoma | 1 | 100 | | | | | 50 | | 50 | POS | 0 | 100 | 0 | 250 |
| A12 | 42 | F | Duct adenocarcinoma | 1 | 100 | 100 | | | | | | | NEG | 0 | 0 | 0 | 0 |
| A13 | 47 | F | Duct adenocarcinoma (sparse) | 1 | | | | | | | | | | | | | |
| A14 | 54 | F | Duct adenocarcinoma | 1 | 50 | 10 | 50 | 40 | | 40 | | 10 | POS | 50 | 90 | 50 | 150 |
| A15 | 40 | F | Duct adenocarcinoma | 1 | | | 30 | | 70 | | | 100 | POS | 100 | 100 | 170 | 300 |
| A16 | 54 | F | Duct adenocarcinoma | 2 | 100 | 100 | | | | | | | NEG | 0 | 0 | 0 | 0 |
| B1 | 51 | F | Duct adenocarcinoma | 2 | 100 | 100 | | | | | | | NEG | 0 | 0 | 0 | 0 |
| B2 | 54 | M | Duct adenocarcinoma | 2 | 80 | 80 | 20 | 20 | | | | | POS | 20 | 20 | 20 | 20 |
| B3 | 60 | M | Duct adenocarcinoma | 1 | 90 | 90 | 10 | 10 | | | | | POS | 10 | 10 | 10 | 10 |

(continued)

| USBiomax PA1921 Pancreate TMA | | | | | | | | | | | | | | POS/ | % POS | % POS | H | H |
|---|---|---|---|---|---|---|---|---|---|---|---|---|---|---|---|---|---|---|
| | | | | | Apical & Cytoplasmic Staining (Percent) | | | | | | | | | | | | | |
| Array Position | Age | Sex | Pathology | Grade | 0C | 0A | 1+C | 1+A | 2+ C | 2+ A | 3+ C | 3+A | NEG | Cyto | Anical | Cyto | Apical |
| B4 | 47 | M | Duct adenocarcinoma | 1 | 100 | 100 | | | | | | | NEG | 0 | 0 | 0 | 0 |
| B5 | 39 | M | Duct adenocarcinoma | 2 | 100 | 100 | | | | | | | NEG | 0 | 0 | 0 | 0 |
| B6 | 54 | M | Duct adenocarcinoma | 2 | 100 | 100 | | | | | | | NEG | 0 | 0 | 0 | 0 |
| B7 | 62 | F | Duct adenocarcinoma | 1 | 100 | 80 | | 10 | | 10 | | | POS | 0 | 20 | 0 | 30 |
| B8 | 64 | F | Duct adenocarcinoma | 2 | 100 | 100 | | | | | | | NEG | 0 | 0 | 0 | 0 |
| B9 | 51 | F | Duct adenocarcinoma | 2 | 100 | 90 | | 10 | | | | | POS | 0 | 10 | 0 | 10 |
| B10 | 54 | M | Duct adenocarcinoma | 2 | 50 | 70 | 50 | 30 | | | | | POS | 50 | 30 | 50 | 30 |
| B11 | 60 | M | Duct adenocarcinoma | 1 | 50 | 80 | 50 | 20 | | | | | POS | 50 | 20 | 50 | 20 |
| B12 | 47 | M | Duct adenocarcinoma | 1 | 100 | 100 | | | | | | | NEG | 0 | 0 | 0 | 0 |
| B13 | 39 | M | Duct adenocarcinoma | 2 | 50 | 100 | 50 | | | | | | POS | 50 | 0 | 50 | 0 |
| B14 | 54 | M | Duct adenocarcinoma | 2 | 100 | 100 | | | | | | | NEG | 0 | 0 | 0 | 0 |
| B15 | 62 | F | Duct adenocarcinoma | 1 | 100 | 80 | | 20 | | | | | POS | 0 | 20 | 0 | 20 |
| B16 | 64 | F | Duct adenocarcinoma (sparse) | - | 100 | 100 | | | | | | | NEG | 0 | 0 | 0 | 0 |
| C1 | 67 | F | Duct adenocarcinoma | 2 | 100 | 90 | | 10 | | | | | POS | 0 | 10 | 0 | 10 |
| C2 | 65 | M | Duct adenocarcinoma | 2 | 50 | 80 | 50 | 20 | | | | | POS | 50 | 20 | 50 | 20 |
| C3 | 57 | M | Duct adenocarcinoma | 2 | 100 | 50 | | 50 | | | | | POS | 0 | 50 | 0 | 50 |
| C4 | 48 | M | Duct adenocarcinoma | 2 | | 50 | 100 | 50 | | | | | POS | 100 | 50 | 100 | 50 |
| C5 | 76 | M | Duct adenocarcinoma | 2 | | 80 | 100 | 20 | | | | | POS | 100 | 20 | 100 | 20 |
| C6 | 43 | F | Duct adenocarcinoma (fibro fatty tissue) | - | 100 | 100 | | | | | | | NEG | 0 | 0 | 0 | 0 |
| C7 | 57 | M | Duct adenocarcinoma | 2 | 100 | 70 | | | | 30 | | | POS | 0 | 30 | 0 | 60 |

(continued)

| USBiomax PA1921 Pancreate TMA | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+C | 1+A | 2+ C | 2+ A | 3+ C | 3+A | NEG | Cyto | Anical | Cyto | Apical |
| C8 | 49 | M | Duct adenocarcinoma | 1 | 100 | 80 | | 20 | | | | | POS | 0 | 20 | 0 | 20 |
| C9 | 67 | F | Duct adenocarcinoma | 2 | 100 | 80 | | 20 | | | | | POS | 0 | 20 | 0 | 20 |
| C10 | 65 | M | Duct adenocarcinoma | 2 | 80 | 50 | 20 | 50 | | | | | POS | 20 | 50 | 20 | 50 |
| C11 | 57 | M | Duct adenocarcinoma | 2 | 50 | 80 | 50 | 20 | | | | | POS | 50 | 20 | 50 | 20 |
| C12 | 48 | M | Duct adenocarcinoma | 2 | 50 | 80 | 50 | 20 | | | | | POS | 50 | 20 | 50 | 20 |
| C13 | 76 | M | Duct adenocarcinoma | 2 | 50 | 40 | 50 | 50 | | 10 | | | POS | 50 | 60 | 50 | 70 |
| C14 | 43 | F | Duct adenocarcinoma (chronic inflammation of fibrofatty tissue) | - | 90 | 90 | 10 | 10 | | | | | POS | 10 | 10 | 10 | 10 |
| C15 | 57 | M | Duct adenocarcinoma | 2 | | | | | | | | | | | | | |
| C16 | 49 | M | Duct adenocarcinoma | 2 | 100 | 100 | | | | | | | NEG | 0 | 0 | 0 | 0 |
| D1 | 52 | M | Duct adenocarcinoma | 2 | | 20 | 80 | 20 | | 20 | 20 | 40 | POS | 100 | 80 | 140 | 180 |
| D2 | 72 | F | Duct adenocarcinoma | 2 | 100 | 100 | | | | | | | NEG | 0 | 0 | 0 | 0 |
| D3 | 53 | M | Duct adenocarcinoma | 2 | 100 | 100 | | | | | | | NEG | 0 | 0 | 0 | 0 |
| D4 | 55 | M | Duct adenocarcinoma | 2 | 100 | 100 | | | | | | | NEG | 0 | 0 | 0 | 0 |
| D5 | 51 | M | Duct adenocarcinoma | 2 | 100 | 90 | | 10 | | | | | POS | 0 | 10 | 0 | 10 |
| D6 | 57 | M | Duct adenocarcinoma (fibrofatty tissue) | - | | 100 | 100 | | | | | | POS | 100 | 0 | 100 | 0 |
| D7 | 49 | M | Duct adenocarcinoma | 2 | 100 | 100 | | | | | | | NEG | 0 | 0 | 0 | 0 |
| D8 | 64 | M | Duct adenocarcinoma | 2 | | 100 | 100 | | | | | | POS | 100 | 0 | 100 | 0 |
| D9 | 52 | M | Duct adenocarcinoma | 2 | | 10 | 100 | 30 | | 30 | | 30 | POS | 100 | 90 | 100 | 180 |
| D10 | 72 | F | Duct adenocarcinoma | 2 | 100 | 100 | | | | | | | NEG | 0 | 0 | 0 | 0 |
| D11 | 53 | M | Duct adenocarcinoma | 2 | 100 | 90 | | 10 | | | | | POS | 0 | 10 | 0 | 10 |

(continued)

| USBiomax PA1921 Pancreate TMA | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+C | 1+A | 2+ C | 2+ A | 3+ C | 3+A | NEG | Cyto | Anical | Cyto | Apical |
| D12 | 55 | M | Duct adenocarcinoma | 2 | 100 | 90 | | 10 | | | | | POS | 0 | 10 | 0 | 10 |
| D13 | 51 | M | Duct adenocarcinoma | 2 | 100 | 100 | | | | | | | NEG | 0 | 0 | 0 | 0 |
| D14 | 57 | M | Duct adenocarcinoma (chronic inflammation of fibrofatty tissue) | - | 100 | 100 | | | | | | | NEG | 0 | 0 | 0 | 0 |
| D15 | 49 | M | Duct adenocarcinoma | 2 | 100 | 100 | | | | | | | NEG | 0 | 0 | 0 | 0 |
| D16 | 64 | M | Duct adenocarcinoma | 2 | | 90 | 100 | 10 | | | | | POS | 100 | 10 | 100 | 10 |
| E1 | 57 | M | Duct adenocarcinoma | 2 | 50 | 50 | 50 | 50 | | | | | POS | 50 | 50 | 50 | 50 |
| E2 | 72 | M | Duct adenocarcinoma (sparse) | - | 50 | 90 | 50 | 10 | | | | | POS | 50 | 10 | 50 | 10 |
| E3 | 42 | M | Duct adenocarcinoma | 2 | 50 | 40 | 50 | 30 | | 30 | | | POS | 50 | 60 | 50 | 90 |
| E4 | 55 | M | Duct adenocarcinoma | 2 | 50 | 100 | 50 | | | | | | POS | 50 | 0 | 50 | 0 |
| E5 | 47 | M | Duct adenocarcinoma (pancreas duct tissue) | - | | | 100 | 100 | | | | | POS | 100 | 100 | 100 | 100 |
| E6 | 44 | M | Duct adenocarcinoma | 2 | | | | | | | | | | | | | |
| E7 | 59 | M | Duct adenocarcinoma | 2 | 100 | 100 | | | | | | | NEG | 0 | 0 | 0 | 0 |
| E8 | 34 | M | Duct adenocarcinoma | 1 | | 50 | 100 | 30 | | 20 | | | POS | 100 | 50 | 100 | 70 |
| E9 | 57 | M | Duct adenocarcinoma | 2 | 50 | 80 | 50 | 20 | | | | | POS | 50 | 20 | 50 | 20 |
| E10 | 72 | M | Duct adenocarcinoma | 2 | 50 | 70 | 50 | 30 | | | | | POS | 50 | 30 | 50 | 30 |
| E11 | 42 | M | Duct adenocarcinoma | 2 | | | | | | | | | | | | | |
| E12 | 55 | M | Duct adenocarcinoma | 2 | 100 | 100 | | | | | | | NEG | 0 | 0 | 0 | 0 |
| E13 | 47 | M | Duct adenocarcinoma | 2 | 50 | 50 | 50 | 50 | | | | | POS | 50 | 50 | 50 | 50 |
| E14 | 44 | M | Duct adenocarcinoma | 2 | | | | | | | | | | | | | |

(continued)

| USBiomax PA1921 Pancreate TMA | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+C | 1+A | 2+ C | 2+ A | 3+ C | 3+A | NEG | Cyto | Anical | Cyto | Apical |
| E15 | 59 | M | Duct adenocarcinoma (sparse) | 2 | 100 | 100 | | | | | | | **NEG** | 0 | 0 | 0 | 0 |
| E16 | 34 | M | Duct adenocarcinoma | 1 | | | | | | | | | | | | | |
| F1 | 61 | M | Duct adenocarcinoma | 2 | 100 | 100 | | | | | | | **NEG** | 0 | 0 | 0 | 0 |
| F2 | 39 | F | Duct adenocarcinoma | 2 | 90 | 100 | 10 | | | | | | **POS** | 10 | 0 | 10 | 0 |
| F3 | 44 | M | Duct adenocarcinoma | 2 | | 90 | 100 | 10 | | | | | **POS** | 100 | 10 | 100 | 10 |
| F4 | 59 | M | Duct adenocarcinoma | 1 | 80 | | 20 | 70 | | 20 | | 10 | **POS** | 20 | 100 | 20 | 140 |
| F5 | 67 | F | Duct adenocarcinoma | 2 | 100 | 100 | | | | | | | **NEG** | 0 | 0 | 0 | 0 |
| F6 | 72 | F | Duct adenocarcinoma | 2 | 50 | 90 | 50 | 10 | | | | | **POS** | 50 | 10 | 50 | 10 |
| F7 | 41 | F | Duct adenocarcinoma | 2 | 50 | 100 | 50 | | | | | | **POS** | 50 | 0 | 50 | 0 |
| F8 | 51 | M | Duct adenocarcinoma | 2 | 80 | | 20 | 70 | | 30 | | | **POS** | 20 | 100 | 20 | 130 |
| F9 | 61 | M | Duct adenocarcinoma | 2 | 50 | 80 | 50 | 20 | | | | | **POS** | 50 | 20 | 50 | 20 |
| F10 | 39 | F | Duct adenocarcinoma | 2 | 100 | 100 | | | | | | | **NEG** | 0 | 0 | 0 | 0 |
| F11 | 44 | M | Duct adenocarcinoma | 2 | | 70 | 80 | | 20 | 30 | | | **POS** | 100 | 30 | 120 | 60 |
| F12 | 59 | M | Duct adenocarcinoma | 2 | | | 80 | 30 | 20 | 30 | | 40 | **POS** | 100 | 100 | 120 | 210 |
| F13 | 67 | F | Duct adenocarcinoma | 2 | 100 | 100 | | | | | | | **NEG** | 0 | 0 | 0 | 0 |
| F14 | 72 | F | Duct adenocarcinoma | 2 | 50 | 90 | 50 | 10 | | | | | **POS** | 50 | 10 | 50 | 10 |
| F15 | 41 | F | Duct adenocarcinoma | 2 | 50 | 100 | 50 | | | | | | **POS** | 50 | 0 | 50 | 0 |
| F16 | 51 | M | Duct adenocarcinoma | 2 | 50 | 50 | 50 | 40 | | 10 | | | **POS** | 50 | 50 | 50 | 60 |
| G1 | 41 | M | Duct adenocarcinoma | 2 | 100 | 100 | | | | | | | **NEG** | 0 | 0 | 0 | 0 |
| G2 | 58 | F | Duct adenocarcinoma | 2 | 100 | 100 | | | | | | | **NEG** | 0 | 0 | 0 | 0 |
| G3 | 60 | M | Duct adenocarcinoma | 2 | 50 | 50 | 50 | 50 | | | | | **POS** | 50 | 50 | 50 | 50 |

(continued)

| USBiomax PA1921 Pancreate TMA | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+C | 1+A | 2+ C | 2+ A | 3+ C | 3+A | NEG | Cyto | Anical | Cyto | Apical |
| G4 | 41 | M | Duct adenocarcinoma | 2 | 50 | 90 | 50 | 10 | | | | | POS | 50 | 10 | 50 | 10 |
| G5 | 68 | F | Duct adenocarcinoma | 2 | 50 | 100 | 50 | | | | | | POS | 50 | 0 | 50 | 0 |
| G6 | 52 | M | Duct adenocarcinoma (sparse) with necrosis | - | 50 | 100 | 50 | | | | | | POS | 50 | 0 | 50 | 0 |
| G7 | 51 | F | Duct adenocarcinoma | 2 | | 90 | 100 | 10 | | | | | POS | 100 | 10 | 100 | 10 |
| G8 | 76 | F | Duct adenocarcinoma | 2 | | 20 | 80 | 40 | 20 | 40 | | | POS | 100 | 80 | 120 | 120 |
| G9 | 41 | M | Duct adenocarcinoma | 2 | 100 | 100 | | | | | | | NEG | 0 | 0 | 0 | 0 |
| G10 | 58 | F | Duct adenocarcinoma | 2 | 50 | 100 | 50 | | | | | | POS | 50 | 0 | 50 | 0 |
| G11 | 60 | M | Duct adenocarcinoma | 2 | 50 | 50 | 50 | 50 | | | | | POS | 50 | 50 | 50 | 50 |
| G12 | 41 | M | Duct adenocarcinoma | 2 | | 50 | 100 | 30 | | 20 | | | POS | 100 | 50 | 100 | 70 |
| G13 | 68 | F | Duct adenocarcinoma | 2 | | 80 | 100 | 20 | | | | | POS | 100 | 20 | 100 | 20 |
| G14 | 52 | M | Duct adenocarcinoma (sparse) with necrosis | - | 50 | 100 | 50 | | | | | | POS | 50 | 0 | 50 | 0 |
| G15 | 51 | F | Duct adenocarcinoma | 2 | 50 | 50 | 50 | 50 | | | | | POS | 50 | 50 | 50 | 50 |
| G16 | 76 | F | Duct adenocarcinoma | 2 | | | | | | | | | | | | | |
| H1 | 62 | F | Adenocarcinoma | 3 | 100 | 100 | | | | | | | NEG | 0 | 0 | 0 | 0 |
| H2 | 51 | M | Adenocarcinoma | 3 | 50 | 100 | 50 | | | | | | POS | 50 | 0 | 50 | 0 |
| H3 | 60 | F | Duct adenocarcinoma | 2 | | 100 | 100 | | | | | | POS | 100 | 0 | 100 | 0 |
| H4 | 76 | M | Duct adenocarcinoma | 2 | 60 | 100 | 30 | | 10 | | | | POS | 40 | 0 | 50 | 0 |
| H5 | 78 | M | Duct adenocarcinoma | 2 | 50 | 80 | 50 | 20 | | | | | POS | 50 | 20 | 50 | 20 |
| H6 | 53 | F | Duct adenocarcinoma | 2 | 100 | 100 | | | | | | | NEG | 0 | 0 | 0 | 0 |
| H7 | 48 | F | Duct adenocarcinoma | 2 | 50 | 100 | 50 | | | | | | POS | 50 | 0 | 50 | 0 |

(continued)

| USBiomax PA1921 Pancreate TMA | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+C | 1+A | 2+ C | 2+ A | 3+ C | 3+A | NEG | Cyto | Anical | Cyto | Apical |
| H8 | 55 | M | Duct adenocarcinoma | 3 | 50 | 80 | 50 | 20 | | | | | POS | 50 | 20 | 50 | 20 |
| H9 | 62 | F | Adenocarcinoma | 3 | 100 | 100 | | | | | | | NEG | 0 | 0 | 0 | 0 |
| H10 | 51 | M | Adenocarcinoma | 3 | 50 | 100 | 50 | | | | | | POS | 50 | 0 | 50 | 0 |
| H11 | 60 | F | Duct adenocarcinoma | 2 | | 100 | 100 | | | | | | POS | 100 | 0 | 100 | 0 |
| H12 | 76 | M | Duct adenocarcinoma (fibrous tissue and blood vessel) | - | 100 | 100 | | | | | | | NEG | 0 | 0 | 0 | 0 |
| H13 | 78 | M | Duct adenocarcinoma | 2 | 50 | 100 | 50 | | | | | | POS | 50 | 0 | 50 | 0 |
| H14 | 53 | F | Duct adenocarcinoma | 2 | | 90 | 100 | 10 | | | | | POS | 100 | 10 | 100 | 10 |
| H15 | 48 | F | Duct adenocarcinoma | 2 | 100 | 100 | | | | | | | NEG | 0 | 0 | 0 | 0 |
| H16 | 55 | M | Duct adenocarcinoma | 2 | | 100 | 90 | | 10 | | | | POS | 100 | 0 | 110 | 0 |
| I1 | 59 | M | Duct adenocarcinoma | 2--3 | 50 | 90 | 50 | 10 | | | | | POS | 50 | 10 | 50 | 10 |
| I2 | 62 | M | Duct adenocarcinoma | 3 | 100 | 100 | | | | | | | NEG | 0 | 0 | 0 | 0 |
| I3 | 67 | M | Adenocarcinoma (sparse) | - | | 100 | 50 | | 50 | | | | POS | 100 | 0 | 150 | 0 |
| I4 | 66 | F | Duct adenocarcinoma | 3 | 50 | 100 | 50 | | | | | | POS | 50 | 0 | 50 | 0 |
| I5 | 49 | M | Adenosquamous carcinoma (fibrous tissue and blood vessel) | - | | | | | | | | | | | | | |
| I6 | 50 | M | Squamous cell carcinoma | 3 | 50 | 100 | 50 | | | | | | POS | 50 | 0 | 50 | 0 |
| I7 | 73 | M | Undifferentiated carcinoma | - | 100 | 100 | | | | | | | NEG | 0 | 0 | 0 | 0 |

(continued)

| USBiomax PA1921 Pancreate TMA | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+C | 1+A | 2+ C | 2+ A | 3+ C | 3+A | NEG | Cyto | Anical | Cyto | Apical |
| I8 | 65 | M | Undifferentiated carcinoma | - | 50 | 100 | 50 | | | | | | POS | 50 | 0 | 50 | 0 |
| I9 | 59 | M | Duct adenocarcinoma | 2 | 50 | 80 | 50 | 20 | | | | | POS | 50 | 20 | 50 | 20 |
| I10 | 62 | M | Duct adenocarcinoma (sparse) | 2 | 100 | 100 | | | | | | | NEG | 0 | 0 | 0 | 0 |
| I11 | 67 | M | Adenocarcinoma | 3 | | 100 | 50 | | 50 | | | | POS | 100 | 0 | 150 | 0 |
| I12 | 66 | F | Duct adenocarcinoma | 3 | 50 | 100 | 50 | | | | | | POS | 50 | 0 | 50 | 0 |
| I13 | 49 | M | Adenosquamous carcinoma | - | | 100 | 100 | | | | | | POS | 100 | 0 | 100 | 0 |
| I14 | 50 | M | Squamous cell carcinoma | 3 | | 80 | 100 | 20 | | | | | POS | 100 | 20 | 100 | 20 |
| I15 | 73 | M | Undifferentiated carcinoma | - | | 100 | 100 | | | | | | POS | 100 | 0 | 100 | 0 |
| I16 | 65 | M | Undifferentiated carcinoma | - | | | | | | | | | | | | | |
| J1 | 56 | F | Undifferentiated carcinoma | - | 100 | 100 | | | | | | | NEG | 0 | 0 | 0 | 0 |
| J2 | 52 | F | Carcinoid | - | 100 | 100 | | | | | | | NEG | 0 | 0 | 0 | 0 |
| J3 | 51 | M | Atypical carcinoid | - | 100 | 100 | | | | | | | NEG | 0 | 0 | 0 | 0 |
| J4 | 42 | M | Neuroendocrine carcinoma | - | 100 | 100 | | | | | | | NEG | 0 | 0 | 0 | 0 |
| J5 | 52 | F | Adenocarcinoma | 1 | 50 | 90 | 50 | 10 | | | | | POS | 50 | 10 | 50 | 10 |
| J6 | 45 | F | Adenocarcinoma | 2 | 100 | 90 | | 10 | | | | | POS | 0 | 10 | 0 | 10 |
| J7 | 49 | M | Adenocarcinoma | 2 | | | | | | | | | | | | | |

(continued)

| USBiomax PA1921 Pancreate TMA | | | | | | | | | | | | | | POS/ | % POS | % POS | H | H |
|---|---|---|---|---|---|---|---|---|---|---|---|---|---|---|---|---|---|---|
| | | | | | Apical & Cytoplasmic Staining (Percent) | | | | | | | | | | | | | |
| Array Position | Age | Sex | Pathology | Grade | 0C | 0A | 1+C | 1+A | 2+ C | 2+ A | 3+ C | 3+A | NEG | Cyto | Anical | Cyto | Apical |
| J8 | 65 | M | Adenocarcinoma | 2 | | | | | | | | | | | | | |
| J9 | 56 | F | Undifferentiated carcinoma | - | 90 | 100 | 10 | | | | | | POS | 10 | 0 | 10 | 0 |
| J10 | 52 | F | Carcinoid | - | 100 | 100 | | | | | | | NEG | 0 | 0 | 0 | 0 |
| J11 | 51 | M | Atypical carcinoid | - | 100 | 100 | | | | | | | NEG | 0 | 0 | 0 | 0 |
| J12 | 42 | M | Neuroendocrine carcinoma | - | 100 | 100 | | | | | | | NEG | 0 | 0 | 0 | 0 |
| J13 | 52 | F | Adenocarcinoma (chronic inflammation of pancreas tissue) | - | | 100 | 100 | | | | | | POS | 100 | 0 | 100 | 0 |
| J14 | 45 | F | Adenocarcinoma | 2 | 50 | 80 | 50 | | | 20 | | | POS | 50 | 20 | 50 | 40 |
| J15 | 49 | M | Adenocarcinoma | 2 | | | | | | | | | | | | | |
| J16 | 65 | M | Adenocarcinoma | 2 | | | | | | | | | | | | | |
| K1 | 52 | M | Adenocarcinoma | 2 | | 80 | 100 | 20 | | | | | POS | 100 | 20 | 100 | 20 |
| K2 | 56 | F | Adenocarcinoma | 2 | | 90 | 100 | 10 | | | | | POS | 100 | 10 | 100 | 10 |
| K3 | 76 | F | Adenocarcinoma | 2 | 100 | 100 | | | | | | | NEG | 0 | 0 | 0 | 0 |
| K4 | 45 | M | Adenocarcinoma | 3 | 100 | 100 | | | | | | | NEG | 0 | 0 | 0 | 0 |
| K5 | 41 | M | Adenocarcinoma | 3 | | | 50 | | 50 | 50 | | 50 | POS | 100 | 100 | 150 | 250 |
| K6 | 62 | M | Adenocarcinoma | 3 | | | | | | | | | | | | | |
| K7 | 50 | M | Adenocarcinoma | 3 | 100 | 100 | | | | | | | NEG | 0 | 0 | 0 | 0 |
| K8 | 60 | M | Adenocarcinoma | 3 | | | | | | | | | | | | | |
| K9 | 52 | M | Adenocarcinoma | 2 | 100 | 90 | | 10 | | | | | POS | 0 | 10 | 0 | 10 |
| K10 | 56 | F | Adenocarcinoma | 2 | 50 | 80 | 50 | 20 | | | | | POS | 50 | 20 | 50 | 20 |

(continued)

| USBiomax PA1921 Pancreate TMA | | | | | | | | | | | | | | POS/ | % POS | % POS | H | H |
|---|---|---|---|---|---|---|---|---|---|---|---|---|---|---|---|---|---|---|
| | | | | | Apical & Cytoplasmic Staining (Percent) | | | | | | | | | | | | | |
| Array Position | Age | Sex | Pathology | Grade | 0C | 0A | 1+C | 1+A | 2+ C | 2+ A | 3+ C | 3+A | NEG | Cyto | Anical | Cyto | Apical |
| K11 | 76 | F | Adenocarcinoma (sparse) of liver | 3 | 100 | 100 | | | | | | | NEG | 0 | 0 | 0 | 0 |
| K12 | 45 | M | Adenocarcinoma | 3 | 100 | 100 | | | | | | | NEG | 0 | 0 | 0 | 0 |
| K13 | 41 | M | Adenocarcinoma | 3 | | | | | | | | | | | | | |
| K14 | 62 | M | Adenocarcinoma | 3 | 100 | 100 | | | | | | | NEG | 0 | 0 | 0 | 0 |
| K15 | 50 | M | Adenocarcinoma | 3 | 100 | 100 | | | | | | | NEG | 0 | 0 | 0 | 0 |
| K16 | 60 | M | Adenocarcinoma | 3 | 100 | 100 | | | | | | | NEG | 0 | 0 | 0 | 0 |
| L1 | 56 | M | Duct adenocarcinoma | 3 | | | | | | | | | | | | | |
| L2 | 49 | F | Adenosquamous carcinoma | - | 100 | 100 | | | | | | | NEG | 0 | 0 | 0 | 0 |
| L3 | 25 | M | Adenocarcinoma | 2 | 100 | 90 | | 10 | | | | | POS | 0 | 10 | 0 | 10 |
| L4 | 38 | M | Adenocarcinoma | 2 | 50 | 90 | 50 | 10 | | | | | POS | 50 | 10 | 50 | 10 |
| L5 | 42 | M | Duct adenocarcinoma | 2 | | | | | | | | | | | | | |
| L6 | 59 | M | Adenocarcinoma (pancreas tissue) | - | | 100 | | | 50 | | 50 | | POS | 100 | 0 | 250 | 0 |
| L7 | 60 | F | Duct adenocarcinoma | 3 | 100 | 100 | | | | | | | NEG | 0 | 0 | 0 | 0 |
| L8 | 13 | F | Solid pseudopapillary carcinoma | - | 100 | 100 | | | | | | | NEG | 0 | 0 | 0 | 0 |
| L9 | 56 | M | Duct adenocarcinoma | 3 | 100 | 100 | | | | | | | NEG | 0 | 0 | 0 | 0 |
| L10 | 49 | F | Adenosquamous carcinoma | - | 90 | 100 | 10 | | | | | | POS | 10 | 0 | 10 | 0 |
| L11 | 25 | M | Adenocarcinoma (sparse) | 2 | 100 | 100 | | | | | | | NEG | 0 | 0 | 0 | 0 |

(continued)

| USBiomax PA1921 Pancreate TMA | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+C | 1+A | 2+ C | 2+ A | 3+ C | 3+A | NEG | Cyto | Anical | Cyto | Apical |
| L12 | 38 | M | Adenocarcinoma | 2 | 100 | 90 | | 10 | | | | | POS | 0 | 10 | 0 | 10 |
| L13 | 42 | M | Duct adenocarcinoma | 2 | 100 | 40 | | 40 | | 20 | | | POS | 0 | 60 | 0 | 80 |
| L14 | 59 | M | Adenocarcinoma | 3 | | 100 | | | 50 | | 50 | | POS | 100 | 0 | 250 | 0 |
| L15 | 60 | F | Duct adenocarcinoma | 3 | 100 | 100 | | | | | | | NEG | 0 | 0 | 0 | 0 |
| L16 | 13 | F | Solid pseudopapillary carcinoma | - | 100 | 100 | | | | | | | NEG | 0 | 0 | 0 | 0 |

**Table 22B: Summary GCC Staining in US Biomax P1921 Pancreatic Tumor MicroArray (TMA)**

| AVG % POS Cyto | AVG % POS Apical | AVG H Cyto | AVG H Apical | Total Pos | Total N | Pct Pos |
|---|---|---|---|---|---|---|
| 33 | 16 | 38 | 25 | 106 | 171 | 62% |

**Table 23A: GCC IHC Staining in US Biomax PA1002 Pancreatic Tumor MicroArray (TMA)**

| USBiomax PA1002 Pancreate TMA | | | | | | | | | | | | | | POS / | % POS | % POS | H | H |
|---|---|---|---|---|---|---|---|---|---|---|---|---|---|---|---|---|---|---|
| | | | | | Apical & Cytoplasmic Staining (Percent) | | | | | | | | | | | | | |
| Array Position | Age | Sex | Pathology | Grade | 0C | 0A | 1+C | 1+ A | 2+ C | 2+A | 3+ C | 3+ A | NEG | Cyto | Apical | Cyto | Apical |
| A1 | 65 | M | Adenocarcinoma | 2 | | | | | | | | | | | | | |
| A2 | 52 | M | Adenocarcinoma | 1 | | | | | | | | | | | | | |
| A3 | 45 | F | Adenocarcinoma | 2 | | 80 | 100 | | | 10 | | 10 | POS | 100 | 20 | 100 | 50 |
| A4 | 44 | M | Adenocarcinoma | 2 | | | | 10 0 | | 100 | | | POS | 0 | 200 | 0 | 300 |
| A5 | 47 | M | Adenocarcinoma (sparse) | 1 | | 90 | 100 | | | 10 | | | POS | 100 | 10 | 100 | 20 |
| B1 | 55 | M | Adenocarcinoma | 2 | 100 | 100 | | | | | | | NEG | 0 | 0 | 0 | 0 |
| B2 | 57 | M | Adenocarcinoma (sparse) with necrosis | 2 | 100 | 100 | | | | | | | NEG | 0 | 0 | 0 | 0 |
| B3 | 34 | M | Adenocarcinoma | 1 | 80 | 80 | | | 20 | 20 | | | POS | 20 | 20 | 40 | 40 |
| B4 | 56 | F | Adenocarcinoma | 1 | 100 | 80 | | | | 20 | | | POS | 0 | 20 | 0 | 40 |
| B5 | 42 | M | Adenocarcinoma | 1 | 100 | 100 | | | | | | | NEG | 0 | 0 | 0 | 0 |
| C1 | 39 | F | Adenocarcinoma | 2 | 100 | 100 | | | | | | | NEG | 0 | 0 | 0 | 0 |
| C2 | 44 | M | Adenocarcinoma | 2 | | 100 | 100 | | | | | | POS | 100 | 0 | 100 | 0 |
| C3 | 59 | M | Adenocarcinoma | 2 | 70 | 80 | 20 | 10 | | | | | POS | 20 | 10 | 20 | 10 |
| C4 | 65 | M | Adenocarcinoma (fibrofatty tissue) | - | | | | | | | | | | | | | |
| C5 | 67 | F | Adenocarcinoma (chronic | - | 80 | 100 | 20 | | | | | | POS | 20 | 0 | 20 | 0 |
| | | | inflammation of pancreas tissue) | | | | | | | | | | | | | | |
| D1 | 53 | M | Adenocarcinoma | 1 | 100 | 100 | | | | | | | NEG | 0 | 0 | 0 | 0 |
| D2 | 52 | M | Adenocarcinoma | 2 | 80 | 60 | 20 | 20 | | 20 | | | POS | 20 | 40 | 20 | 60 |

(continued)

| USBiomax PA1002 Pancreate TMA | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+C | 1+ A | 2+ C | 2+A | 3+ C | 3+ A | NEG | Cyto | Apical | Cyto | Apical |
| D3 | 72 | F | Adenocarcinoma | 1 | 50 | 90 | 50 | 10 | | | | | POS | 50 | 10 | 50 | 10 |
| D4 | 58 | F | Adenocarcinoma | 2 | 30 | 100 | 70 | | | | | | POS | 70 | 0 | 70 | 0 |
| D5 | 41 | M | Adenocarcinoma | 2 | 100 | 100 | | | | | | | NEG | 0 | 0 | 0 | 0 |
| E1 | 51 | M | Adenocarcinoma | 2 | 90 | 80 | 10 | 20 | | | | | POS | 10 | 20 | 10 | 20 |
| E2 | 41 | M | Adenocarcinoma (sparse) | - | | | | | | | | | | | | | |
| E3 | 68 | F | Adenocarcinoma | 2 | 90 | 100 | 10 | | | | | | POS | 10 | 0 | 10 | 0 |
| E4 | 41 | F | Adenocarcinoma | 2 | | 100 | 100 | | | | | | POS | 100 | 0 | 100 | 0 |
| E5 | 72 | F | Adenocarcinoma | 2 | 100 | 100 | | | | | | | NEG | 0 | 0 | 0 | 0 |
| F1 | 76 | F | Adenocarcinoma with necrosis | 2 | 100 | 100 | | | | | | | NEG | 0 | 0 | 0 | 0 |
| F2 | 52 | M | Adenocarcinoma | 2 | 30 | 100 | 70 | | | | | | POS | 70 | 0 | 70 | 0 |
| F3 | 60 | F | Adenocarcinoma | 2 | 70 | 100 | 30 | | | | | | POS | 30 | 0 | 30 | 0 |
| F4 | 76 | M | Adenocarcinoma (sparse) | 2 | 80 | 90 | 20 | 10 | | | | | POS | 20 | 10 | 20 | 10 |
| F5 | 78 | M | Adenocarcinoma | 2 | 100 | 100 | | | | | | | NEG | 0 | 0 | 0 | 0 |
| G1 | 41 | M | Adenocarcinoma | 2 | 70 | 50 | 30 | | | 20 | | 30 | POS | 30 | 50 | 30 | 130 |
| G2 | 62 | F | Adenocarcinoma | 3 | 100 | 100 | | | | | | | NEG | 0 | 0 | 0 | 0 |
| G3 | 51 | M | Adenocarcinoma (sparse) | - | | 100 | 100 | | | | | | POS | 100 | 0 | 100 | 0 |
| G4 | 50 | M | Adenocarcinoma | 3 | | 100 | 100 | | | | | | POS | 100 | 0 | 100 | 0 |
| G5 | 60 | M | Adenocarcinoma | 2 | | | | | | | | | | | | | |
| H1 | 53 | F | Adenocarcinoma | 2 | 100 | 100 | | | | | | | NEG | 0 | 0 | 0 | 0 |

(continued)

| USBiomax PA1002 Pancreate TMA | | | | | | | | | | | | | | POS / | % POS | % POS | H | H |
|---|---|---|---|---|---|---|---|---|---|---|---|---|---|---|---|---|---|---|
| | | | | | Apical & Cytoplasmic Staining (Percent) | | | | | | | | | | | | | |
| Array Position | Age | Sex | Pathology | Grade | 0C | 0A | 1+C | 1+ A | 2+ C | 2+A | 3+ C | 3+ A | NEG | Cyto | Apical | Cyto | Apical |
| H2 | 59 | M | Adenocarcinoma | 2 | | 100 | 70 | | 30 | | | | POS | 100 | 0 | 130 | 0 |
| H3 | 56 | M | Adenocarcinoma | 3 | | 100 | 50 | | 50 | | | | POS | 100 | 0 | 150 | 0 |
| H4 | 60 | F | Adenocarcinoma | 3 | | | | | | | | | | | | | |
| H5 | 66 | F | Adenocarcinoma (fibrous tissue and blood vessel) | - | | | | | | | | | | | | | |
| I1 | 40 | M | Normal pancreas tissue | - | 100 | 100 | | | | | | | NEG | 0 | 0 | 0 | 0 |
| I2 | 47 | M | Normal pancreas tissue | - | | | | | | | | | | 0 | 0 | 0 | 0 |
| I3 | 25 | M | Normal pancreas tissue | - | | 70 | 100 | | | 30 | | | POS | 100 | 30 | 100 | 60 |
| I4 | 35 | F | Normal pancreas tissue | - | | | | | | | | | | 0 | 0 | 0 | 0 |
| I5 | 30 | M | Normal pancreas tissue | - | | 70 | 100 | 30 | | | | | POS | 100 | 30 | 100 | 30 |
| J1 | 50 | M | Normal pancreas tissue | - | | 100 | 100 | | | | | | NEG | 100 | 0 | 100 | 0 |
| J2 | 30 | M | Normal pancreas tissue | - | | 100 | 100 | | | | | | NEG | 100 | 0 | 100 | 0 |
| J3 | 40 | M | Normal pancreas tissue | - | | | | | | | | | | 0 | 0 | 0 | 0 |
| J4 | 35 | M | Normal pancreas tissue | - | | | | | | | | | | 0 | | | 0 |
| J5 | 21 | F | Normal pancreas tissue | - | | | | | | | | | | 0 | 0 | 0 | 0 |

**Table 23B: Summary of GCC Staining in US Biomax PA1002 Pancreatic Tumor MicroArray (TMA)**

| % POS Cyto | % POS Apical | H Cyto | H Apical | Total Pos | Total N | Pet Pos |
|---|---|---|---|---|---|---|
| 35 | 12 | 38 | 21 | 22 | 33 | 67% |

**Table 24A: GCC IHC Staining in Pantomics PAC481 Pancreatic Tumor MicroArray-Part 1**

| Pantomics PAC4S1 Pancreatic TMA-Part 1 | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| A01 | 46 | M | Normal | - | | | | | | | | | | 0 | 0 | 0 | 0 |
| A02 | 48 | M | Norma | - | | | | | | | | | | 0 | 0 | 0 | 0 |
| A03 | 58 | F | Normal | - | 100 | 100 | | | | | | | NEG | 0 | 0 | 0 | 0 |
| A04 | 28 | F | Islet cell tumor | - | 100 | 100 | | | | | | | NEG | 0 | 0 | 0 | 0 |
| A05 | 44 | F | Adenocarcinoma | I | 50 | 100 | 50 | | | | | | POS | 50 | 0 | 50 | 0 |
| A06 | 64 | F | Adenocarcinoma | I | 100 | 100 | | | | | | | NEG | 0 | 0 | 0 | 0 |
| A07 | 39 | F | Adenocarcinoma | I | | | 50 | | 50 | | | 100 | POS | 100 | 100 | 150 | 300 |
| A08 | 49 | F | Adenocarcinoma | I | 90 | 100 | | | 10 | | | | POS | 10 | 0 | 20 | 0 |
| C01 | 56 | F | Adenocarcinoma | I | | | | | | | | | | | | | |
| C02 | 49 | F | Adenocarcinoma | II | 100 | 100 | | | | | | | NEG | 0 | 0 | 0 | 0 |
| C03 | 52 | F | Adenocarcinoma | II | 100 | 100 | | | | | | | NEG | 0 | 0 | 0 | 0 |
| C04 | 42 | F | Adenocarcinoma | II | 90 | 100 | 10 | | | | | | POS | 10 | 0 | 10 | 0 |
| C05 | 54 | M | Adenocarcinoma | II | 100 | 100 | | | | | | | NEG | 0 | 0 | 0 | 0 |
| C06 | 59 | M | Adenocarcinoma | II | 100 | 100 | 30 | | | | | | POS | 30 | 0 | 30 | 0 |
| C07 | 34 | F | Adenocarcinoma | II | | 100 | 100 | 20 | | | | | POS | 100 | 20 | 100 | 20 |
| C08 | 69 | M | Adenocarcinoma | II | | 100 | 100 | | | | | | POS | 100 | 0 | 100 | 0 |
| E01 | 40 | F | Adenocarcinoma | II | 100 | 100 | | | | | | | NEG | 0 | 0 | | |
| E02 | 79 | F | Adenocarcinoma | III | 100 | 100 | | | | | | | NEG | 0 | 0 | 0 | 0 |
| E03 | 76 | F | Adenocarcinoma | III | 100 | 100 | | | | | | | NEG | 0 | 0 | 0 | 0 |
| E04 | 52 | M | Adenocarcinoma | III | 80 | 100 | 20 | | | | | | POS | 20 | 0 | 20 | 0 |
| E05 | 51 | F | Adenocarcinoma | III | 50 | 100 | 50 | | | | | | POS | 50 | 0 | 50 | 0 |
| E06 | 42 | F | Adenocarcinoma | III | | | | | | | | | | | | | |
| E07 | 40 | M | Adenocarcinoma | III | 50 | 100 | 50 | | | | | | POS | 50 | 0 | 50 | 0 |

(continued)

| Pantomics PAC4S1 Pancreatic TMA-Part 1 | | | | | | | | | | | | | | POS/ | % POS | % POS | H | H |
|---|---|---|---|---|---|---|---|---|---|---|---|---|---|---|---|---|---|---|
| | | | | | Apical & Cytoplasmic Staining (Percent) | | | | | | | | | POS/ | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| E08 | 64 | M | Metastatic Carcinoma | II | | | | | | | | | | | | | |

**Table 24B: Summary of GCC Staining in Pantomics PAC481 Pancreatic TMA-Part 1**

| % POS Cyto | % POS Apical | H Cyto | H Apical | Total Pos | Total N | Pet Pos |
|---|---|---|---|---|---|---|
| 29 | 7 | 34 | 19 | 10 | 18 | 56% |

**Table 24C: GCC IHC Staining in Pantomics PAC481 Pancreatic Tumor MicroArray-Part 2**

| Pantomics PAC481 Pancreatic TMA-Part 2 | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS/ NEG | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| B01 | 46 | M | Normal | - | | | | | | | | | | | | | |
| B02 | 48 | M | Norma | - | | | | | | | | | | | | | |
| B03 | 58 | F | Norma | - | | | | | | | | | | | | | |
| B04 | 28 | F | Islet cell tumor | - | 100 | 100 | | | | | | | NEG | 0 | 0 | 0 | 0 |
| B05 | 44 | F | Adenocarcinoma | I | 100 | 100 | | | | | | | NEG | 0 | 0 | 0 | 0 |
| B06 | 64 | F | Adenocarcinoma | I | 70 | 70 | 30 | 30 | | | | | POS | 30 | 30 | 30 | 30 |
| B07 | 39 | F | Adenocarcinoma | I | | | 100 | | | | | 100 | POS | 100 | 100 | 100 | 300 |
| B08 | 49 | F | Adenocarcinoma | I | 90 | 100 | 10 | | | | | | POS | 10 | 0 | 10 | 0 |
| D01 | 56 | F | Adenocarcinoma | I | 100 | 100 | | | | | | | NEG | 0 | 0 | 0 | 0 |
| D02 | 49 | F | Adenocarcinoma | II | 100 | 100 | | | | | | | NEG | 0 | 0 | 0 | 0 |
| D03 | 52 | F | Adenocarcinoma | II | 100 | 100 | | | | | | | NEG | 0 | 0 | 0 | 0 |
| D04 | 42 | F | Adenocarcinoma | II | 90 | 100 | 10 | | | | | | POS | 10 | 0 | 10 | 0 |
| D05 | 54 | M | Adenocarcinoma | II | 100 | 100 | | | | | | | NEG | 0 | 0 | 0 | 0 |
| D06 | 59 | M | Adenocarcinoma | II | 80 | 100 | 20 | | | | | | POS | 20 | 0 | 20 | 0 |
| D07 | 34 | F | Adenocarcinoma | II | | 100 | 100 | | | | | | POS | 100 | 0 | 100 | 0 |
| D08 | 69 | M | Adenocarcinoma | II | 100 | 100 | | | | | | | NEG | 0 | 0 | 0 | 0 |
| F01 | 40 | F | Adenocarcinoma | II | 100 | 100 | | | | | | | NEG | 0 | 0 | 0 | 0 |
| F02 | 79 | F | Adenocarcinoma | III | 100 | 100 | | | | | | | NEG | 0 | 0 | 0 | 0 |
| F03 | 76 | F | Adenocarcinoma | III | 100 | 100 | | | | | | | NEG | 0 | 0 | 0 | 0 |
| F04 | 52 | M | Adenocarcinoma | III | 100 | 100 | | | | | | | NEG | 0 | 0 | 0 | 0 |
| F05 | 51 | F | Adenocarcinoma | III | 60 | 100 | 40 | | | | | | POS | 0 | 0 | 40 | 0 |
| F06 | 42 | F | Adenocarcinoma | III | 50 | 100 | 50 | | | | | | POS | 50 | 0 | 50 | 0 |
| F07 | 40 | M | Adenocarcinoma | III | 50 | 100 | 50 | | | | | | POS | 50 | 0 | 50 | 0 |

(continued)

| Pantomics PAC481 Pancreatic TMA-Part 2 | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS/ NEG | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| F08 | 64 | M | Metastatic Carcinoma | II | 100 | 70 | | | 30 | | | | POS | 30 | 0 | 60 | 0 |

**Table 24D: Summary of GCC Staining in Pantomics PAC481 Pancreatic TMA-Part 2**

| % POS Cyto | % POS Apical | H Cyto | H Apical | Total Pos | Total N | Pct Pos |
|---|---|---|---|---|---|---|
| 21 | 7 | 21 | 17 | 10 | 21 | 48% |

**Table 25: GCC IHC Staining in US Biomax STC1501 Gastric Tumor MicroArray-Part 1**

| USBiomax STC1501 Gastric TMA - Part 1 | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| A1 | 35 | M | Normal | - | | | | | | | | | | | | | |
| A2 | 83 | M | Normal | - | | 100 | 100 | | | | | | POS | 100 | 0 | 100 | 0 |
| A3 | 53 | M | Chronic gastritis | - | | 70 | 100 | | | | | 30 | POS | 100 | 30 | 100 | 90 |
| A4 | 54 | M | Chronic gastritis | - | | | 50 | | 20 | 50 | 30 | 50 | POS | 100 | 100 | 180 | 250 |
| A5 | 59 | M | Gastric stromal tumor | - | 100 | 100 | | | | | | | NEG | 0 | 0 | 0 | 0 |
| A6 | 68 | F | Signet-ring cell adenocarcinoma | - | | 100 | | | 100 | | | | POS | 100 | 0 | 200 | 0 |
| A7 | 42 | M | Adenocarcinoma | III | | 100 | 100 | | | | | | POS | 100 | 0 | 100 | 0 |
| A8 | 44 | F | Adenocarcinoma | II~III | | 20 | 50 | | 50 | 30 | | 50 | POS | 100 | 80 | 150 | 210 |
| A9 | 54 | F | Adenocarcinoma | I | | 50 | | | 100 | 20 | | 30 | POS | 100 | 50 | 200 | 130 |
| A10 | 51 | M | Adenocarcinoma | II~III | | 100 | 100 | | | | | | POS | 100 | 0 | 100 | 0 |
| A1 | 61 | M | Adenocarcinoma | II~III | | | | | 100 | 50 | | 50 | POS | 100 | 100 | 200 | 250 |
| A12 | 50 | M | Adenocarcinoma | II | | 70 | | | 100 | | | 30 | POS | 100 | 30 | 200 | 90 |
| A13 | 53 | F | Adenocarcinoma | III | | 100 | 70 | | 30 | | | | POS | 100 | 0 | 130 | 0 |
| A14 | 55 | M | Adenocarcinoma | III | | 100 | | | 70 | | 30 | | POS | 100 | 0 | 230 | 0 |
| A15 | 73 | F | Adenocarcinoma | II | | | | | | | | | | | | | |
| C1 | 47 | F | Adenocarcinoma | II~III | 100 | 100 | | | | | | | NEG | 0 | 0 | 0 | 0 |
| C2 | 70 | M | Adenocarcinoma | II~III | 50 | 50 | 50 | | | | | 50 | POS | 50 | 50 | 50 | 150 |
| C3 | 36 | M | Adenocarcinoma | I~II | 100 | 50 | | | | | | 50 | POS | 0 | 50 | 0 | 150 |
| C4 | 65 | M | Adenocarcinoma | III | 60 | 70 | | | 20 | | 20 | 30 | POS | 40 | 30 | 100 | 90 |
| C5 | 62 | M | Adenocarcinoma | III | 100 | 100 | | | | | | | NEG | 0 | 0 | 0 | 0 |
| C6 | 77 | M | Adenocarcinoma | II~III | 50 | 100 | 50 | | | | | | POS | 50 | 0 | 50 | 0 |
| C7 | 45 | F | Adenocarcinoma | I~II | 100 | 30 | | | | | | 70 | POS | 0 | 70 | 0 | 210 |
| C8 | 67 | M | Adenocarcinoma | II~III | 70 | 90 | 30 | | | 10 | | | POS | 30 | 10 | 30 | 20 |

(continued)

| USBiomax STC1501 Gastric TMA - Part 1 | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| C9 | 62 | M | Adenocarcinoma | | 100 | 100 | | | | | | | NEG | 0 | 0 | 0 | 0 |
| C10 | 55 | F | Adenocarcinoma | II~III | 50 | 100 | 50 | | | | | | POS | 50 | 0 | 50 | 0 |
| C11 | 75 | M | Adenocarcinoma | III | | 100 | 100 | | | | | | POS | 100 | 0 | 100 | 0 |
| C12 | 73 | M | Undifferentiated carcinoma | - | 100 | 100 | | | | | | | NEG | 0 | 0 | 0 | 0 |
| C13 | 41 | M | Adenocarcinoma | II~III | 50 | 80 | 50 | 10 | | 10 | | | POS | 50 | 20 | 50 | 30 |
| C14 | 58 | M | Adenocarcinoma | II~III | 70 | 80 | 30 | 20 | | | | | POS | 30 | 20 | 30 | 20 |
| C15 | 52 | F | Adenocarcinoma | I~II | | 40 | | | 100 | 30 | | 30 | POS | 100 | 60 | 200 | 150 |
| E1 | 63 | M | Gastric stromal tumor | - | 60 | 100 | 20 | | 20 | | | | POS | 40 | 0 | 60 | 0 |
| E2 | 72 | M | Adenocarcinoma | II | | 100 | 20 | | 30 | | 50 | | POS | 100 | 0 | 230 | 0 |
| E3 | 57 | M | Signet-ring cell adenocarcinoma | - | 100 | 100 | | | | | | | NEG | 0 | 0 | 0 | 0 |
| E4 | 60 | M | Adenocarcinoma | III | 100 | 100 | | | | | | | NEG | 0 | 0 | 0 | 0 |
| E5 | 66 | F | Adenocarcinoma | III | | | | | | | | | | | | | |
| E6 | 62 | M | Adenocarcinoma | I~II | | | 10 | | 60 | | 30 | 100 | POS | 100 | 100 | 220 | 300 |
| E7 | 48 | F | Adenocarcinoma | III | 50 | 100 | 20 | | 30 | | | | POS | 50 | 0 | 80 | 0 |
| E8 | 60 | M | Adenocarcinoma | II~III | | 100 | 20 | | 30 | | 50 | | POS | 100 | 0 | 230 | 0 |
| E9 | 56 | M | Adenocarcinoma | III | | 100 | 50 | | | | 50 | | POS | 100 | 0 | 200 | 0 |
| E10 | 54 | M | Adenocarcinoma | III | | 100 | 100 | | | | | | POS | 100 | 0 | 100 | 0 |
| E11 | 64 | F | Adenocarcinoma | III | | 100 | 30 | | | | 70 | | POS | 100 | 0 | 240 | 0 |
| E12 | 59 | M | Adenocarcinoma | III | 80 | 100 | | | 20 | | | | POS | 20 | 0 | 40 | 0 |
| E13 | 75 | M | Undifferentiated carcinoma | - | 30 | 100 | | | | | 70 | | POS | 70 | 0 | 210 | 0 |
| E14 | 65 | M | Signet-ring cell adenocarcinoma | - | | 100 | | | | | 100 | | POS | 100 | 0 | 300 | 0 |
| E15 | 35 | F | Adenocarcinoma | III | | 100 | | | 70 | | 30 | | POS | 100 | 0 | 230 | 0 |

(continued)

| USBiomax STC1501 Gastric TMA - Part 1 | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| G1 | 71 | M | Adenocarcinoma | III | 90 | 100 | 10 | | | | | | POS | 10 | 0 | 10 | 0 |
| G2 | 38 | M | Signet-ring cell adenocarcinoma | - | 50 | 100 | | | 50 | | | | POS | 50 | 0 | 100 | 0 |
| G3 | 68 | M | Gastric stromal tumor | - | 100 | 100 | | | | | | | NEG | 0 | 0 | 0 | 0 |
| G4 | 56 | F | Adenocarcinoma | I~II | | | 50 | | 30 | | 20 | 100 | POS | 100 | 100 | 170 | 300 |
| G5 | 45 | M | Adenocarcinoma | II | | 100 | 100 | | | | | | POS | 100 | 0 | 100 | 0 |
| G6 | 45 | M | Adenocarcinoma | III | 100 | 100 | | | | | | | NEG | 0 | 0 | 0 | 0 |
| G7 | 74 | M | Adenocarcinoma | III | 100 | 100 | | | | | | | NEG | 0 | 0 | 0 | 0 |
| G8 | 24 | F | Adenocarcinoma | II | 30 | 50 | 50 | | 20 | 50 | | | POS | 70 | 50 | 90 | 100 |
| G9 | 51 | M | Lymphoma? | - | | 100 | 100 | | | | | | POS | 100 | 0 | 100 | 0 |
| G10 | 53 | M | Signet-ring cell adenocarcinoma | - | 50 | 100 | 50 | | | | | | POS | 50 | 0 | 50 | 0 |
| G11 | 58 | F | Lymphoma | - | | | | | | | | | | | | | |
| G12 | 58 | F | Lymphoma | - | | | | | | | | | | | | | |
| G13 | 81 | M | Adenocarcinoma | I~II | 20 | 70 | 50 | 30 | 30 | | | | POS | 80 | 30 | 110 | 30 |
| G14 | 56 | M | Signet-ring cell adenocarcinoma | - | | | | | | | | | | | | | |
| G15 | 35 | M | Adenocarcinoma | I~II | | | 100 | | | 100 | | | POS | 100 | 100 | 100 | 200 |
| I1 | 61 | M | Adenocarcinoma | II | 100 | 100 | | | | | | | NEG | 0 | 0 | 0 | 0 |
| I2 | 42 | M | Undifferentiated carcinoma | - | 100 | 100 | | | | | | | NEG | 0 | 0 | 0 | 0 |
| I3 | 78 | M | Adenocarcinoma | III | 100 | 100 | | | | | | | NEG | 0 | 0 | 0 | 0 |
| I4 | 65 | F | Adenocarcinoma | III | 40 | 80 | 20 | | 40 | | | 20 | POS | 60 | 20 | 100 | 60 |
| I5 | 68 | M | Adenocarcinoma | II~III | 100 | 100 | | | | | | | NEG | 0 | 0 | 0 | 0 |
| I6 | 60 | M | Adenocarcinoma | II~III | | | | | | | | | | | | | |

(continued)

| USBiomax STC1501 Gastric TMA - Part 1 | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS/ | % POS | % POS | H | H |
| **Array Position** | **Age** | **Sex** | **Pathology** | **Grade** | **0C** | **0A** | **1+ C** | **1+ A** | **2+ C** | **2+ A** | **3+ C** | **3+ A** | **NEG** | **Cyto** | **Apical** | **Cyto** | **Apical** |
| **I7** | **53** | **M** | **Adenocarcinoma** | **II** | 100 | 50 | | | | | | 50 | **POS** | 0 | 50 | 0 | 150 |
| **I8** | **76** | **F** | **Mucinous adenocarcinoma** | **II** | | 100 | | | 20 | | 80 | | **POS** | 100 | 0 | 280 | 0 |
| **I9** | **50** | **M** | **Adenocarcinoma** | **II** | | | | | | | | | | | | | |
| **I10** | **74** | **M** | **Adenocarcinoma** | **III** | 100 | 100 | | | | | | | **NEG** | 0 | 0 | 0 | 0 |
| **I11** | **75** | **F** | **Signet-ring cell adenocarcinoma** | **-** | | 100 | | | | | 10 0 | | **POS** | 100 | 0 | 300 | 0 |
| **I12** | **68** | **F** | **Adenocarcinoma** | **II~III** | | 70 | 100 | | | | | 30 | **POS** | 100 | 30 | 100 | 90 |
| **I13** | **56** | **F** | **Adenocarcinoma** | **II~III** | | | | | | | | | | | | | |
| **I14** | **53** | **F** | **Lymphoma** | **-** | | | | | | | | | | | | | |
| **I15** | **80** | **M** | **Adenocarcinoma** | **II** | | 100 | | | 100 | | | | **POS** | 100 | 0 | 200 | 0 |

**Table 25B: Summary of GCC Staining in US Biomax STC1501 Gastric TMA-Part 1**

| % POS Cyto | % POS Apical | H Cyto | H Apical | Total Pos | Total N | Pct Pos |
|---|---|---|---|---|---|---|
| 57 | 17 | 100 | 45 | 47 | 61 | 77% |

**Table 25C: GCC IHC Staining in US Biomax STC1501 Gastric TumorMicroArray-Part 2**

| | | | | | Apical & Cytoplasmic Staining (Percent) | | | | | | | | POS / | % POS | % POS | H | H |
|---|---|---|---|---|---|---|---|---|---|---|---|---|---|---|---|---|---|
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| B1 | M | 35 | Normal | - | 100 | 100 | | | | | | | | 0 | 0 | 0 | 0 |
| B2 | M | 83 | Normal | - | | 90 | 100 | | | | | 10 | | 100 | 10 | 100 | 30 |
| B3 | M | 53 | Chronic gastritis | - | | 100 | 100 | | | | | | | 100 | 0 | 100 | 0 |
| B4 | M | 54 | Chronic gastritis | - | | | | | 50 | | 50 | 100 | | 100 | 100 | 250 | 300 |
| B5 | M | 59 | Gastric stromal tumor | - | 100 | 100 | | | | | | | | 0 | 0 | 0 | 0 |
| B6 | F | 68 | Signet-ring cell adenocarcinoma | - | 50 | 100 | | | 50 | | | | | 50 | 0 | 100 | 0 |
| B7 | M | 42 | Adenocarcinoma | III | | 100 | 50 | | 50 | | | | | 100 | 0 | 150 | 0 |
| B8 | F | 44 | Adenocarcinoma | II~III | | 100 | | | 50 | | 50 | | | 100 | 0 | 250 | 0 |
| B9 | F | 54 | Adenocarcinoma | I | | | | | 100 | 30 | | 70 | | 100 | 100 | 200 | 270 |
| B10 | M | 51 | Adenocarcinoma | II~III | | 90 | 100 | | | 10 | | | | 100 | 10 | 100 | 20 |
| B11 | M | 61 | Adenocarcinoma | II~III | | | | | 100 | | | 100 | | 100 | 100 | 200 | 300 |
| B12 | M | 50 | Adenocarcinoma | II | | | | | | | | | | | | | |
| B13 | F | 53 | Adenocarcinoma | III | | 100 | 90 | | | | 10 | | | 100 | 0 | 120 | 0 |
| B14 | M | 55 | Adenocarcinoma | III | | 100 | 40 | | 30 | | 30 | | | 100 | 0 | 190 | 0 |
| B15 | F | 73 | Adenocarcinoma | II | | 100 | 100 | | | | | | | 100 | 0 | 100 | 0 |
| D1 | F | 47 | Adenocarcinoma | II~III | 100 | 100 | | | | | | | | 0 | 0 | 0 | 0 |
| D2 | M | 70 | Adenocarcinoma | II~III | | 50 | 100 | | | | | 50 | | 100 | 50 | 100 | 150 |
| D3 | M | 36 | Adenocarcinoma | I~II | 100 | 50 | | | | | | 50 | | 0 | 50 | 0 | 150 |
| D4 | M | 65 | Adenocarcinoma | III | | **100** | **20** | | **30** | | **50** | | | 100 | 0 | 230 | 0 |
| D5 | M | 62 | Adenocarcinoma | III | 90 | 90 | 10 | 10 | | | | | | 10 | 10 | 10 | 10 |
| D6 | M | 77 | Adenocarcinoma | II~III | 40 | 100 | 30 | | 30 | | | | | 60 | 0 | 90 | 0 |
| D7 | F | 45 | Adenocarcinoma | I~II | 100 | 50 | | | | | | 50 | | 0 | 50 | 0 | 150 |
| D8 | M | 67 | Adenocarcinoma | II~III | 80 | 90 | 10 | | 10 | 10 | | | | 20 | 10 | 30 | 20 |
| D9 | M | 62 | Adenocarcinoma | II~III | 100 | 100 | | | | | | | | 0 | 0 | 0 | 0 |

(continued)

| | | | | | Apical & Cytoplasmic Staining (Percent) | | | | | | | | POS / | % POS | % POS | H | H |
|---|---|---|---|---|---|---|---|---|---|---|---|---|---|---|---|---|---|
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| D10 | F | 55 | Adenocarcinoma | II~III | 100 | 100 | | | | | | | | 0 | 0 | 0 | 0 |
| D11 | M | 75 | Adenocarcinoma | III | 50 | 100 | | | 50 | | | | | 50 | 0 | 100 | 0 |
| D12 | M | 73 | Undifferentiated carcinoma | - | 100 | 100 | | | | | | | | 0 | 0 | 0 | 0 |
| D13 | M | 41 | Adenocarcinoma | II~III | | | | | 100 | | | 100 | | 100 | 100 | 200 | 300 |
| D14 | M | 58 | Adenocarcinoma | II~III | 100 | 90 | | | | 10 | | | | 0 | 10 | 0 | 20 |
| D15 | F | 52 | Adenocarcinoma | I~II | | 80 | | | 100 | | | 20 | | 100 | 20 | 200 | 60 |
| F1 | M | 63 | Gastric stromal tumor | - | 80 | 100 | 20 | | | | | | | 20 | 0 | 20 | 0 |
| F2 | M | 72 | Adenocarcinoma | II | | 100 | 20 | | 30 | | 50 | | | 100 | 0 | 230 | 0 |
| F3 | M | 57 | Signet-ring cell adenocarcinoma | - | 100 | 100 | | | | | | | | 0 | 0 | 0 | 0 |
| F4 | M | 60 | Adenocarcinoma | III | | | | | | | | | | | | | |
| F5 | F | 66 | Adenocarcinoma | III | | 100 | 30 | | 50 | | 20 | | | 100 | 0 | 190 | 0 |
| F6 | M | 62 | Adenocarcinoma | I~II | | | | | 100 | | | 100 | | 100 | 100 | 200 | 300 |
| F7 | F | 48 | Adenocarcinoma | III | 50 | 100 | 50 | | | | | | | 50 | 0 | 50 | 0 |
| F8 | M | 60 | Adenocarcinoma | II~III | 40 | 50 | | | 50 | 10 | 10 | 50 | | 60 | 60 | 130 | 170 |
| F9 | M | 56 | Adenocarcinoma | III | | 100 | | | 100 | | | | | 100 | 0 | 200 | 0 |
| F10 | M | 54 | Adenocarcinoma | III | 80 | 100 | 20 | | | | | | | 20 | 0 | 20 | 0 |
| F11 | F | 64 | Adenocarcinoma | III | | 100 | 50 | | 20 | | 30 | | | 100 | 0 | 180 | 0 |
| F12 | M | 59 | Adenocarcinoma | III | 80 | 100 | | | 20 | | | | | 20 | 0 | 40 | 0 |
| F13 | M | 75 | Undifferentiated carcinoma | - | 100 | 100 | | | | | | | | 0 | 0 | 0 | 0 |
| F14 | M | 65 | Signet-ring cell adenocarcinoma | - | | 100 | | | | | 100 | | | 100 | 0 | 300 | 0 |
| F15 | F | 35 | Adenocarcinoma | III | | 100 | 30 | | 50 | | 20 | | | 100 | 0 | 190 | 0 |
| H1 | M | 71 | Adenocarcinoma | III | 90 | 100 | 10 | | | | | | | 10 | 0 | 10 | 0 |

(continued)

| | | | | | Apical & Cytoplasmic Staining (Percent) | | | | | | | | POS / | % POS | % POS | H | H |
|---|---|---|---|---|---|---|---|---|---|---|---|---|---|---|---|---|---|
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| H2 | M | 38 | Signet-ring cell adenocarcinoma | - | | | | | | | | | | | | | |
| H3 | M | 68 | Gastric stromal tumor | - | 100 | 100 | | | | | | | | 0 | 0 | 0 | 0 |
| H4 | F | 56 | Adenocarcinoma | I~II | | | | | 100 | | | 100 | | 100 | 100 | 200 | 300 |
| H5 | M | 45 | Adenocarcinoma | II | | 100 | 100 | | | | | | | 100 | 0 | 100 | 0 |
| H6 | M | 45 | Adenocarcinoma | III | | 100 | 100 | | | | | | | 100 | 0 | 100 | 0 |
| H7 | M | 74 | Adenocarcinoma | III | 100 | 100 | | | | | | | | 0 | 0 | 0 | 0 |
| H8 | F | 24 | Adenocarcinoma | II | 80 | 80 | | | 20 | | | 20 | | 20 | 20 | 40 | 60 |
| H9 | M | 51 | Lymphoma? | - | | | | | | | | | | | | | |
| H10 | M | 53 | Signet-ring cell adenocarcinoma | - | 70 | 100 | 30 | | | | | | | 30 | 0 | 30 | 0 |
| H11 | F | 58 | Lymphoma | - | | | | | | | | | | | | | |
| H12 | F | 58 | Lymphoma | - | | | | | | | | | | | | | |
| H13 | M | 81 | Adenocarcinoma | I~II | | 90 | 100 | 10 | | | | | | 100 | 10 | 100 | 10 |
| H14 | M | 56 | Signet-ring cell adenocarcinoma | - | | | | | | | | | | | | | |
| H15 | M | 35 | Adenocarcinoma | I~II | | 70 | 80 | | 20 | | | 30 | | 100 | 30 | 120 | 90 |
| J1 | M | 61 | Adenocarcinoma | II | 80 | 100 | 20 | | | | | | | 20 | 0 | 20 | 0 |
| J2 | M | 42 | Undifferentiated carcinoma | - | | | | | | | | | | | | | |
| J3 | M | 78 | Adenocarcinoma | III | 100 | 100 | | | | | | | | 0 | 0 | 0 | 0 |
| J4 | F | 65 | Adenocarcinoma | III | | 50 | 50 | | 50 | | | 50 | | 100 | 50 | 150 | 150 |
| J5 | M | 68 | Adenocarcinoma | II~III | 100 | 80 | | | | 20 | | | | 0 | 20 | 0 | 40 |
| J6 | M | 60 | Adenocarcinoma | II~III | | 100 | | | 50 | | 50 | | | 100 | 0 | 250 | 0 |
| J7 | M | 53 | Adenocarcinoma | II | 50 | 80 | 50 | | | 10 | | 10 | | 50 | 20 | 50 | 50 |
| J8 | F | 76 | Mucinous adenocarcinoma | II | | | | | | | | | | | | | |

(continued)

| | | | | | Apical & Cytoplasmic Staining (Percent) | | | | | | | | POS / | % POS | % POS | H | H |
|---|---|---|---|---|---|---|---|---|---|---|---|---|---|---|---|---|---|
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| J9 | M | 50 | Adenocarcinoma | II | | 40 | | | 100 | 30 | | 30 | | 100 | 60 | 200 | 150 |
| J10 | M | 74 | Adenocarcinoma | III | 100 | 100 | | | | | | | | 0 | 0 | 0 | 0 |
| J11 | F | 75 | Signet-ring cell adenocarcinoma | - | | 100 | | | | | 100 | | | 100 | 0 | 300 | 0 |
| J12 | F | 68 | Adenocarcinoma | II~III | 90 | 70 | 10 | | | 30 | | | | 10 | 30 | 10 | 60 |
| J13 | F | 56 | Adenocarcinoma | II~III | | | | | | | | | | | | | |
| J14 | F | 53 | Lymphoma | - | | | | | | | | | | | | | |
| J15 | M | 80 | Adenocarcinoma | II | | 100 | | | 100 | | | | | 100 | 0 | 200 | 0 |

**Table 25D: Summary of GCC Staining in US Biomax STC1501 Gastric TMA-Part 2**

| % POS Cyto | % POS Apical | H Cyto | H Apical | Total Pos | Total N | Pct Pos |
|---|---|---|---|---|---|---|
| 58 | 17 | 102 | 48 | 49 | 59 | 83% |

**Table 26A: GCC IHC Staining in Pantomics ESC1021 Esophageal Tumor Microarray**

| Pantomics ESC1021 Esophageal TMA | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| A01 | 56 | M | Normal | | 100 | 100 | | | | | | | NEG | 0 | 0 | 0 | 0 |
| A02 | 53 | M | Normal | | 100 | 100 | | | | | | | NEG | 0 | 0 | 0 | 0 |
| A03 | 53 | M | Normal | | 100 | 100 | | | | | | | NEG | 0 | 0 | 0 | 0 |
| A04 | 62 | M | Esophagitis | | 100 | 100 | | | | | | | NEG | 0 | 0 | 0 | 0 |
| A05 | 66 | M | Esophagitis | | 100 | 100 | | | | | | | NEG | 0 | 0 | 0 | 0 |
| A06 | 58 | M | Squamous cell carcinoma | I | 30 | 100 | | | 20 | | 50 | | POS | 70 | 0 | 190 | 0 |
| A07 | 36 | M | Squamous cell carcinoma | I~II | 100 | 100 | | | | | | | NEG | 0 | 0 | 0 | 0 |
| A08 | 72 | F | Squamous cell carcinoma | I | 50 | 100 | | | 50 | | | | POS | 50 | 0 | 100 | 0 |
| A09 | 62 | M | Squamous cell carcinoma | I~II | 100 | 100 | | | | | | | NEG | 0 | 0 | 0 | 0 |
| A10 | 56 | M | Squamous cell carcinoma | I | 70 | 100 | | | 30 | | | | POS | 30 | 0 | 60 | 0 |
| A11 | 68 | F | Squamous cell carcinoma | I | 100 | 100 | | | | | | | NEG | 0 | 0 | 0 | 0 |
| A12 | 62 | M | Squamous cell carcinoma | I | 80 | 100 | 20 | | | | | | POS | 20 | 0 | 20 | 0 |
| A13 | 56 | M | Squamous cell carcinoma | I | 70 | 100 | 30 | | | | | | POS | 30 | 0 | 30 | 0 |
| B01 | 60 | M | Squamous cell carcinoma | I | 100 | 100 | | | | | | | NEG | 0 | 0 | 0 | 0 |
| B02 | 50 | M | Squamous cell carcinoma | I | 90 | 100 | | | | | 10 | | POS | 10 | 0 | 30 | 0 |
| B03 | 44 | M | Squamous cell carcinoma | I | 70 | 100 | | | 30 | | | | POS | 30 | 0 | 60 | 0 |
| B04 | 60 | M | Squamous cell carcinoma | I | 100 | 100 | | | | | | | NEG | 0 | 0 | 0 | 0 |
| B05 | 64 | F | Squamous cell carcinoma | I | 70 | 100 | 30 | | | | | | POS | 30 | 0 | 30 | 0 |
| B06 | 55 | M | Squamous cell carcinoma | I | 70 | 100 | 30 | | | | | | POS | 30 | 0 | 30 | 0 |
| B07 | 56 | M | Squamous cell carcinoma | I | 60 | 100 | | | 20 | | 20 | | POS | 40 | 0 | 100 | 0 |
| B08 | 48 | M | Squamous cell carcinoma | I | 80 | 100 | | | 20 | | | | POS | 20 | 0 | 40 | 0 |
| B09 | 51 | M | Squamous cell carcinoma | I~II | 100 | 100 | | | | | | | NEG | 0 | 0 | 0 | 0 |
| B10 | 56 | M | Squamous cell carcinoma | I~II | 100 | 100 | | | | | | | NEG | 0 | 0 | 0 | 0 |

(continued)

| Pantomics ESC1021 Esophageal TMA | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| B11 | 71 | M | Squamous cell carcinoma | I~II | 100 | 100 | | | | | | | NEG | 0 | 0 | 0 | 0 |
| B12 | 55 | M | Squamous cell carcinoma | I~II | 80 | 100 | | | 10 | | 10 | | POS | 20 | 0 | 50 | 0 |
| B13 | 58 | M | Squamous cell carcinoma | I~II | 70 | 100 | 30 | | | | | | POS | 30 | 0 | 30 | 0 |
| C01 | 26 | F | Squamous cell carcinoma | I~II | 30 | 100 | | | 70 | | | | POS | 70 | 0 | 140 | 0 |
| C02 | 56 | F | Squamous cell carcinoma | I~II | 100 | 100 | | | | | | | NEG | 0 | 0 | 0 | 0 |
| C03 | 65 | M | Squamous cell carcinoma | I~II | 30 | 100 | | | 70 | | | | POS | 70 | 0 | 140 | 0 |
| C04 | 43 | M | Squamous cell carcinoma | I~II | 30 | 100 | 60 | | 10 | | | | POS | 70 | 0 | 80 | 0 |
| C05 | 62 | M | Squamous cell carcinoma | I~II | 90 | 100 | 10 | | | | | | POS | 10 | 0 | 10 | 0 |
| C06 | 55 | M | Squamous cell carcinoma | I~II | 50 | 100 | 50 | | | | | | POS | 50 | 0 | 50 | 0 |
| C07 | 59 | M | Squamous cell carcinoma | I~II | 70 | 100 | | | 30 | | | | POS | 30 | 0 | 60 | 0 |
| C08 | 42 | M | Squamous cell carcinoma | I~II | 90 | 100 | 10 | | | | | | POS | 10 | 0 | 10 | 0 |
| C09 | 61 | M | Squamous cell carcinoma | I~II | 20 | 100 | 80 | | | | | | POS | 80 | 0 | 80 | 0 |
| C10 | 57 | M | Squamous cell carcinoma | I~II | 80 | 100 | 20 | | | | | | POS | 20 | 0 | 20 | 0 |
| C11 | 62 | M | Squamous cell carcinoma | I~II | | 100 | 100 | | | | | | POS | 100 | 0 | 100 | 0 |
| C12 | 62 | M | Squamous cell carcinoma | I~II | 80 | 100 | | | 10 | | 10 | | POS | 20 | 0 | 50 | 0 |
| C13 | 55 | M | Squamous cell carcinoma | I~II | 50 | 100 | 50 | | | | | | POS | 50 | 0 | 50 | 0 |
| D01 | 70 | M | Squamous cell carcinoma | I~II | 100 | 100 | | | | | | | NEG | 0 | 0 | 0 | 0 |
| D02 | 68 | M | Squamous cell carcinoma | I~II | 90 | 100 | 10 | | | | | | POS | 10 | 0 | 10 | 0 |
| D03 | 55 | M | Squamous cell carcinoma | I~II | 90 | 100 | | | 10 | | | | POS | 10 | 0 | 20 | 0 |
| D04 | 65 | F | Squamous cell carcinoma | I~II | 80 | 100 | 20 | | | | | | POS | 20 | 0 | 20 | 0 |
| D05 | 60 | M | Squamous cell carcinoma | I~II | | 100 | 100 | | | | | | POS | 100 | 0 | 100 | 0 |
| D06 | 66 | M | Squamous cell carcinoma | I~II | 80 | 100 | 20 | | | | | | POS | 20 | 0 | 20 | 0 |
| D07 | 56 | M | Squamous cell carcinoma | I~II | 100 | 100 | | | | | | | NEG | 0 | 0 | 0 | 0 |

(continued)

| Pantomics ESC1021 Esophageal TMA | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| D08 | 68 | F | Squamous cell carcinoma | I~II | 100 | 100 | | | | | | | NEG | 0 | 0 | 0 | 0 |
| D09 | 55 | M | Squamous cell carcinoma | I~II | 50 | 100 | 50 | | | | | | POS | 50 | 0 | 50 | 0 |
| D10 | 60 | M | Squamous cell carcinoma | I~II | 80 | 100 | 20 | | | | | | POS | 20 | 0 | 20 | 0 |
| D11 | 61 | F | Squamous cell carcinoma | I~II | 100 | 100 | | | | | | | NEG | 0 | 0 | 0 | 0 |
| D12 | 56 | M | Squamous cell carcinoma | I~II | 100 | 100 | | | | | | | NEG | 0 | 0 | 0 | 0 |
| D13 | 71 | F | Squamous cell carcinoma | I~II | | 100 | | | 100 | | | | POS | 100 | 0 | 200 | 0 |
| E01 | 57 | M | Squamous cell carcinoma | I~II | 90 | 100 | 10 | | | | | | POS | 10 | 0 | 10 | 0 |
| E02 | 64 | M | Squamous cell carcinoma | I~II | 50 | 100 | 20 | | 30 | | | | POS | 50 | 0 | 80 | 0 |
| E03 | 72 | M | Squamous cell carcinoma | I~II | 90 | 100 | 10 | | | | | | POS | 10 | 0 | 10 | 0 |
| E04 | 74 | M | Squamous cell carcinoma | I~II | 100 | 100 | | | | | | | NEG | 0 | 0 | 0 | 0 |
| E05 | 51 | M | Squamous cell carcinoma | I~II | 80 | 100 | | | 20 | | | | POS | 20 | 0 | 40 | 0 |
| E06 | 61 | M | Squamous cell carcinoma | II | 100 | 100 | | | | | | | NEG | 0 | 0 | 0 | 0 |
| E07 | 62 | M | Squamous cell carcinoma | II | 100 | 100 | | | | | | | NEG | 0 | 0 | 0 | 0 |
| E08 | 63 | M | Squamous cell carcinoma | II | 100 | 100 | | | | | | | NEG | 0 | 0 | 0 | 0 |
| E09 | 71 | M | Squamous cell carcinoma | I~II | 100 | 100 | | | | | | | NEG | 0 | 0 | 0 | 0 |
| E10 | 51 | M | Squamous cell carcinoma | II | | | | | | | | | | | | | |
| E11 | 54 | M | Squamous cell carcinoma | II | 70 | 100 | 30 | | | | | | POS | 30 | 0 | 30 | 0 |
| E12 | 77 | M | Adenocarcinoma | II | 80 | 70 | 20 | | | | | 30 | POS | 20 | 30 | 20 | 90 |
| E13 | 64 | M | Squamous cell carcinoma | II | 100 | 100 | | | | | | | NEG | 0 | 0 | 0 | 0 |
| F01 | 45 | M | Squamous cell carcinoma | II | 100 | 100 | | | | | | | NEG | 0 | 0 | 0 | 0 |
| F02 | 52 | M | Squamous cell carcinoma | II | 100 | 100 | | | | | | | NEG | 0 | 0 | 0 | 0 |
| F03 | 59 | M | Squamous cell carcinoma | II | | 100 | 50 | | | | | | POS | 50 | 0 | 50 | 0 |
| F04 | 68 | M | Squamous cell carcinoma | II | | 100 | 100 | | | | | | POS | 100 | 0 | 100 | 0 |

(continued)

| Pantomics ESC1021 Esophageal TMA | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| F05 | 53 | M | Squamous cell carcinoma | II | 100 | 100 | | | | | | | NEG | 0 | 0 | 0 | 0 |
| F06 | 62 | M | Squamous cell carcinoma | II | | 100 | | | 50 | | 50 | | POS | 100 | 0 | 250 | 0 |
| F07 | 67 | M | Squamous cell carcinoma | II | 100 | 100 | | | | | | | NEG | 0 | 0 | 0 | 0 |
| F08 | 54 | M | Squamous cell carcinoma | II | 70 | 100 | 30 | | | | | | POS | 30 | 0 | 30 | 0 |
| F09 | 67 | M | Squamous cell carcinoma | II | 100 | 100 | | | | | | | NEG | 0 | 0 | 0 | 0 |
| F10 | 56 | F | Squamous cell carcinoma | II | 80 | 100 | 20 | | | | | | POS | 20 | 0 | 20 | 0 |
| F11 | 56 | M | Adenocarcinoma | II | 20 | | | | 80 | | | 100 | POS | 80 | 100 | 160 | 300 |
| F12 | 57 | F | Squamous cell carcinoma | II~III | 80 | 100 | | | 20 | | | | POS | 20 | 0 | 40 | 0 |
| F13 | 53 | M | Squamous cell carcinoma | II~III | 70 | 100 | | | 30 | | | | POS | 30 | 0 | 60 | 0 |
| G01 | 57 | M | Squamous cell carcinoma | II~III | 100 | 100 | | | | | | | NEG | 0 | 0 | 0 | 0 |
| G02 | 47 | M | Squamous cell carcinoma | II~III | 100 | 100 | | | | | | | NEG | 0 | 0 | 0 | 0 |
| G03 | 66 | M | Squamous cell carcinoma | II~III | 100 | 100 | | | | | | | NEG | 0 | 0 | 0 | 0 |
| G04 | 47 | F | Squamous cell carcinoma | II~III | 70 | 100 | 30 | | | | | | POS | 30 | 0 | 30 | 0 |
| G05 | 53 | M | Squamous cell carcinoma | II~III | | 100 | 100 | | | | | | POS | 100 | 0 | 100 | 0 |
| G06 | 57 | M | Squamous cell carcinoma | II~III | 90 | 100 | 10 | | | | | | POS | 10 | 0 | 10 | 0 |
| G07 | 59 | M | Squamous cell carcinoma | II~III | 100 | 100 | | | | | | | NEG | 0 | 0 | 0 | 0 |
| G08 | 42 | M | Squamous cell carcinoma | II~III | | 100 | 100 | | | | | | POS | 100 | 0 | 100 | 0 |
| G09 | 49 | M | Squamous cell carcinoma | III | 100 | 100 | | | | | | | NEG | 0 | 0 | 0 | 0 |
| G10 | 48 | M | Squamous cell carcinoma | III | 100 | 100 | | | | | | | NEG | 0 | 0 | 0 | 0 |
| G11 | 67 | M | Squamous cell carcinoma | III | | 100 | 100 | | | | | | POS | 100 | 0 | 100 | 0 |
| G12 | 58 | M | Squamous cell carcinoma | III | 100 | 100 | | | | | | | NEG | 0 | 0 | 0 | 0 |
| H01 | 58 | M | Adenocarcinoma ? | III | | 100 | 100 | | | | | | POS | 100 | 0 | 100 | 0 |
| H02 | 56 | M | Squamous cell carcinoma | III | 100 | 100 | | | | | | | NEG | 0 | 0 | 0 | 0 |

(continued)

| Pantomics ESC1021 Esophageal TMA | | | | | | | | | | | | | | POS / | % POS | % POS | H | H |
|---|---|---|---|---|---|---|---|---|---|---|---|---|---|---|---|---|---|---|
| | | | | | Apical & Cytoplasmic Staining (Percent) | | | | | | | | | POS / | % POS | % POS | H | H |
| Array Position | Age | Sex | Pathology | Grade | 0C | 0A | 1+ C | 1+ A | 2+ C | 2+ A | 3+ C | 3+ A | NEG | Cyto | Apical | Cyto | Apical |
| H03 | 61 | F | Squamous cell carcinoma | III | 90 | 100 | 10 | | | | | | POS | 10 | 0 | 10 | 0 |
| H04 | 72 | F | Squamous cell carcinoma | III | 100 | 100 | | | | | | | NEG | 0 | 0 | 0 | 0 |
| H05 | 60 | M | Squamous cell carcinoma | III | | 100 | 100 | | | | | | POS | 100 | 0 | 100 | 0 |
| H06 | 49 | M | Squamous cell carcinoma | III | | 100 | 50 | | 50 | | | | POS | 100 | 0 | 150 | 0 |
| H07 | 56 | M | Squamous cell carcinoma | III | 100 | 100 | | | | | | | NEG | 0 | 0 | 0 | 0 |
| H08 | 53 | M | Squamous cell carcinoma | III | | 100 | 100 | | | | | | POS | 100 | 0 | 100 | 0 |
| H09 | 66 | F | Squamous cell carcinoma | III | 100 | 100 | | | | | | | NEG | 0 | 0 | 0 | 0 |
| H10 | 65 | M | Carcinoid | | 100 | 100 | | | | | | | NEG | 0 | 0 | 0 | 0 |
| H11 | 55 | M | Adenosquamous carcinoma | | 100 | 100 | | | | | | | NEG | 0 | 0 | 0 | 0 |
| H12 | 60 | M | Undifferentiated carcinoma | | 100 | 100 | | | | | | | NEG | 0 | 0 | 0 | 0 |

**Table 26B: Summary of GCC Staining in Pantomics ESC1021 Esophageal Tumor MicroArray**

| % POS Cyto | % POS Apical | H Cyto | H Apical | Total Pos | Total N | Pet Pos |
|---|---|---|---|---|---|---|
| 28 | 1 | 39 | 4 | 57 | 96 | 59% |

**Table 27A: GCC IHC Staining in US BioMax ES8010 Esophageal Tumor MicroArray-Part 1**

| USBioMax ES8010 Esophageal TMA-PART 1 | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+C | 1+ A | 2+ C | 2+ A | 3+ C | 3+ A | NEG | Cyto | Apical | Cyto | Apical |
| A1 | 69 | M | Squamous cell carcinoma | 1 | 50 | 100 | 50 | | | | | | POS | 50 | 0 | 50 | 0 |
| A3 | 56 | M | Squamous cell carcinoma | 1 | 50 | 100 | | | | | 50 | | POS | 50 | 0 | 150 | 0 |
| A5 | 62 | M | Squamous cell carcinoma | 2 | 80 | 100 | | | 20 | | | | POS | 20 | 0 | 40 | 0 |
| A7 | 58 | M | Squamous cell carcinoma | 1 | | 100 | | | 70 | | 30 | | POS | 100 | 0 | 230 | 0 |
| A9 | 46 | M | Squamous cell carcinoma | 1 | 50 | 100 | | | 50 | | | | POS | 50 | 0 | 100 | 0 |
| B1 | 43 | M | Squamous cell carcinoma | 1 | 40 | 100 | 30 | | | | 30 | | POS | 60 | 0 | 120 | 0 |
| B3 | 61 | F | Squamous cell carcinoma | 1 | 100 | 100 | | | | | | | NEG | 0 | 0 | 0 | 0 |
| B5 | 62 | M | Squamous cell carcinoma | 2 | 90 | 100 | | | | | 10 | | POS | 10 | 0 | 30 | 0 |
| B7 | 50 | M | Squamous cell carcinoma | 1 | 40 | 100 | 30 | | 30 | | | | POS | 60 | 0 | 90 | 0 |
| B9 | 68 | M | Squamous cell carcinoma | 2 | 70 | 100 | 30 | | | | | | POS | 30 | 0 | 30 | 0 |
| C1 | 65 | M | Squamous cell carcinoma | 1 | 100 | 100 | | | | | | | NEG | 0 | 0 | 0 | 0 |
| C3 | 50 | M | Squamous cell carcinoma | 2 | 90 | 100 | 10 | | | | | | POS | 10 | 0 | 10 | 0 |
| C5 | 60 | M | Squamous cell carcinoma | 2 | 70 | 100 | | | 30 | | | | POS | 30 | 0 | 60 | 0 |

(continued)

| USBioMax ES8010 Esophageal TMA-PART 1 | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+C | 1+ A | 2+ C | 2+ A | 3+ C | 3+ A | NEG | Cyto | Apical | Cyto | Apical |
| C7 | 49 | F | Squamous cell carcinoma | 2 | 80 | 100 | | | 20 | | | | POS | 20 | 0 | 40 | 0 |
| C9 | 59 | F | Squamous cell carcinoma | 2 | 80 | 100 | 20 | | | | | | POS | 20 | 0 | 20 | 0 |
| D1 | 43 | M | Squamous cell carcinoma | 2 | 80 | 100 | | | 20 | | | | POS | 20 | 0 | 40 | 0 |
| D3 | 62 | M | Squamous cell carcinoma | 2 | 60 | 100 | 40 | | | | | | POS | 40 | 0 | 40 | 0 |
| D5 | 62 | M | Squamous cell carcinoma | 3 | 90 | 100 | | | 10 | | | | POS | 10 | 0 | 20 | 0 |
| D7 | 60 | F | Squamous cell carcinoma | 2 | | 100 | 70 | | 30 | | | | POS | 100 | 0 | 130 | 0 |
| D9 | 54 | F | Squamous cell carcinoma | 2 | 80 | 100 | 20 | | | | | | POS | 20 | 0 | 20 | 0 |
| E1 | 48 | M | Squamous cell carcinoma | 2 | 80 | 100 | 20 | | | | | | POS | 20 | 0 | 20 | 0 |
| E3 | 57 | M | Squamous cell carcinoma | 3 | 100 | 100 | | | | | | | NEG | 0 | 0 | 0 | 0 |
| E5 | 58 | M | Squamous cell carcinoma | 2 | 80 | 100 | 20 | | | | | | POS | 20 | 0 | 20 | 0 |
| E7 | 53 | F | Squamous cell carcinoma | 3 | 100 | 100 | | | | | | | NEG | 0 | 0 | 0 | 0 |
| E9 | 56 | F | Squamous cell carcinoma | 2 | 30 | 100 | 70 | | | | | | POS | 70 | 0 | 70 | 0 |
| F1 | 76 | M | Squamous cell carcinoma | 2 | 100 | 100 | | | | | | | NEG | 0 | 0 | 0 | 0 |

(continued)

| USBioMax ES8010 Esophageal TMA-PART 1 | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+C | 1+ A | 2+ C | 2+ A | 3+ C | 3+ A | NEG | Cyto | Apical | Cyto | Apical |
| F3 | 60 | M | Squamous cell carcinoma (sparse esophagus tissue) | - | 20 | 100 | 80 | | | | | | POS | 80 | 0 | 80 | 0 |
| F5 | 53 | M | Squamous cell carcinoma | 2 | 100 | 100 | | | | | | | NEG | 0 | 0 | 0 | 0 |
| F7 | 49 | F | Carcinoma in situ | - | | 100 | 100 | | | | | | POS | 100 | 0 | 100 | 0 |
| F9 | 62 | F | Squamous cell carcinoma | 2 | 100 | 100 | | | | | | | NEG | 0 | 0 | 0 | 0 |
| G1 | 48 | F | Squamous cell carcinoma | 2 | 100 | 100 | | | | | | | NEG | 0 | 0 | 0 | 0 |
| G3 | 57 | M | Squamous cell carcinoma | 3 | 100 | 100 | | | | | | | NEG | 0 | 0 | 0 | 0 |
| G5 | 45 | M | Squamous cell carcinoma | 3 | 100 | 100 | | | | | | | NEG | 0 | 0 | 0 | 0 |
| G7 | 55 | F | Squamous cell carcinoma | 3 | 100 | 100 | | | | | | | NEG | 0 | 0 | 0 | 0 |
| G9 | 53 | F | Squamous cell carcinoma | 3 | 100 | 100 | | | | | | | NEG | 0 | 0 | 0 | 0 |
| H1 | 68 | M | Squamous cell carcinoma | 3 | 100 | 100 | | | | | | | NEG | 0 | 0 | 0 | 0 |
| H3 | 64 | M | Squamous cell carcinoma | 2 | 90 | 100 | | | 10 | | | | POS | 10 | 0 | 20 | 0 |
| H5 | 55 | M | Squamous cell carcinoma | 3 | 100 | 100 | | | | | | | NEG | 0 | 0 | 0 | 0 |
| H7 | 63 | F | Squamous cell carcinoma | 3 | 100 | 100 | | | | | | | NEG | 0 | 0 | 0 | 0 |

(continued)

| USBioMax ES8010 Esophageal TMA-PART 1 | | | | | | | | | | | | | | | | | |
|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+C | 1+ A | 2+ C | 2+ A | 3+ C | 3+ A | NEG | Cyto | Apical | Cyto | Apical |
| H9 | 54 | F | Squamous cell carcinoma | 3 | 100 | 100 | | | | | | | NEG | 0 | 0 | 0 | 0 |

**Table 27B: Summary of GCC Staining in US BioMax ES8010 Esophageal TMA-Part 1**

| % POS Cyto | % POS Apical | H Cyto | H Apical | Total Pos | Total N | Pct Pos |
|---|---|---|---|---|---|---|
| 25 | 0 | 38 | 0 | 24 | 40 | 60% |

Table 27C: GCC IHC Staining in US BioMax ES8010 Esophageal Tumor MicroArray-Part 2

| USBioMax ES8010 Esophageal TMA (80 Cores) | | | | | | | | | | | | | | POS / | % POS | % POS | H | H |
|---|---|---|---|---|---|---|---|---|---|---|---|---|---|---|---|---|---|---|
| | | | | | Apical & Cytoplasmic Staining (Percent) | | | | | | | | | | | | | |
| Array Position | Age | Sex | Pathology | Grade | 0C | 0A | 1+C | 1+ A | 2+ C | 2+ A | 3+ C | 3+ A | NEG | Cyto | Apical | Cyto | Apical |
| A2 | 69 | M | Cancer adjacent normal esophageal tissue (chronic inflammation of mucosa) | - | 100 | 100 | | | | | | | NEG | 0 | 0 | 0 | 0 |
| A4 | 56 | M | Cancer adjacent normal esophageal tissue | - | 50 | 100 | 50 | | | | | | POS | 50 | 0 | 50 | 0 |
| A6 | 62 | M | Cancer adjacent normal esophageal tissue | - | | 100 | 100 | | | | | | POS | 100 | 0 | 100 | 0 |
| A8 | 58 | M | Cancer adjacent normal esophageal tissue (chronic inflammation of mucosa) | - | | | | | | | | | | | | | |
| A10 | 46 | M | Cancer adjacent normal esophageal tissue | - | 20 | 100 | 80 | | | | | | POS | 80 | 0 | 80 | 0 |
| B2 | 43 | M | Cancer adjacent normal esophageal tissue | - | 70 | 100 | 30 | | | | | | POS | 30 | 0 | 30 | 0 |
| B4 | 61 | F | Cancer adjacent normal esophageal tissue | - | 100 | 100 | | | | | | | NEG | 0 | 0 | 0 | 0 |
| B6 | 62 | M | Cancer adjacent normal esophageal tissue | - | 70 | 100 | | | | | 30 | | POS | 30 | 0 | 90 | 0 |
| B8 | 50 | M | Cancer adjacent normal esophageal tissue | - | 20 | 100 | 80 | | | | | | POS | 80 | 0 | 80 | 0 |
| B10 | 68 | M | Cancer adjacent normal esophageal tissue | - | | 100 | 100 | | | | | | POS | 100 | 0 | 100 | 0 |
| C2 | 65 | M | Cancer adjacent normal esophageal tissue | - | | 100 | 70 | | 30 | | | | POS | 100 | 0 | 130 | 0 |

(continued)

| USBioMax ES8010 Esophageal TMA (80 Cores) | | | | | | | | | | | | | | POS / | % POS | % POS | H | H |
|---|---|---|---|---|---|---|---|---|---|---|---|---|---|---|---|---|---|---|
| | | | | | Apical & Cytoplasmic Staining (Percent) | | | | | | | | | | | | | |
| Array Position | Age | Sex | Pathology | Grade | 0C | 0A | 1+C | 1+ A | 2+ C | 2+ A | 3+ C | 3+ A | NEG | Cyto | Apical | Cyto | Apical |
| C4 | 50 | M | Cancer adjacent normal esophageal tissue | - | | 100 | 100 | | | | | | POS | 100 | 0 | 100 | 0 |
| C6 | 60 | M | Cancer adjacent normal esophageal tissue | - | | 100 | 100 | | | | | | POS | 100 | 0 | 100 | 0 |
| C8 | 49 | F | Cancer adjacent normal esophageal tissue | - | | 100 | 100 | | | | | | POS | 100 | 0 | 100 | 0 |
| C10 | 59 | F | Cancer adjacent normal esophageal tissue | - | 30 | 100 | 70 | | | | | | POS | 70 | 0 | 70 | 0 |
| D2 | 43 | M | Cancer adjacent normal esophageal tissue (chronic inflammation of mucosa) | - | 50 | 100 | 50 | | | | | | POS | 50 | 0 | 50 | 0 |
| D4 | 62 | M | Cancer adjacent normal esophageal tissue (sparse mucosa) | - | | 100 | 100 | | | | | | POS | 100 | 0 | 100 | 0 |
| D6 | 62 | M | Cancer adjacent normal esophageal tissue | - | | 100 | 100 | | | | | | POS | 100 | 0 | 100 | 0 |
| D8 | 60 | F | Cancer adjacent normal esophageal tissue (chronic inflammation of mucosa) | - | 100 | 100 | | | | | | | NEG | 0 | 0 | 0 | 0 |
| D10 | 54 | F | Cancer adjacent normal esophageal tissue | - | | 100 | 100 | | | | | | POS | 100 | 0 | 100 | 0 |
| E2 | 48 | M | Cancer adjacent normal esophageal tissue | - | | 100 | 100 | | | | | | POS | 100 | 0 | 100 | 0 |
| E4 | 57 | M | Cancer adjacent normal esophageal tissue | - | | 100 | 100 | | | | | | POS | 100 | 0 | 100 | 0 |

(continued)

| USBioMax ES8010 Esophageal TMA (80 Cores) | | | | | | | | | | | | | | | | | | |
|---|---|---|---|---|---|---|---|---|---|---|---|---|---|---|---|---|---|---|
| | | | | | Apical & Cytoplasmic Staining (Percent) | | | | | | | | POS / | % POS | % POS | H | H |
| Array Position | Age | Sex | Pathology | Grade | 0C | 0A | 1+C | 1+ A | 2+ C | 2+ A | 3+ C | 3+ A | NEG | Cyto | Apical | Cyto | Apical |
| E6 | 58 | M | Cancer adjacent normal esophageal tissue | - | | 100 | 100 | | | | | | POS | 100 | 0 | 100 | 0 |
| E8 | 53 | F | Cancer adjacent normal esophageal tissue (chronic inflammation of mucosa) | - | | 100 | 100 | | | | | | POS | 100 | 0 | 100 | 0 |
| E10 | 56 | F | Cancer adjacent normal esophageal tissue | - | | 100 | 100 | | | | | | POS | 100 | 0 | 100 | 0 |
| F2 | 76 | M | Cancer adjacent normal esophageal tissue (chronic inflammation of mucosa) | - | 70 | 100 | 30 | | | | | | POS | 30 | 0 | 30 | 0 |
| F4 | 60 | M | Cancer adjacent normal esophageal tissue | - | 20 | 100 | 80 | | | | | | POS | 80 | 0 | 80 | 0 |
| F6 | 53 | M | Cancer adjacent normal esophageal tissue | - | | 100 | 100 | | | | | | POS | 100 | 0 | 100 | 0 |
| F8 | 49 | F | Cancer adjacent normal esophageal tissue (chronic inflammation of mucosa) | - | | 100 | 100 | | | | | | POS | 100 | 0 | 100 | 0 |
| F10 | 62 | F | Cancer adjacent normal esophageal tissue | - | | 100 | 100 | | | | | | POS | 100 | 0 | 100 | 0 |
| G2 | 48 | F | Cancer adjacent normal esophageal tissue (smooth muscle and mucous gland tissue) | - | 20 | 100 | 80 | | | | | | POS | 80 | 0 | 80 | 0 |

(continued)

| USBioMax ES8010 Esophageal TMA (80 Cores) | | | | | | | | | | | | | | POS / | % POS | % POS | H | H |
|---|---|---|---|---|---|---|---|---|---|---|---|---|---|---|---|---|---|---|
| | | | | | Apical & Cytoplasmic Staining (Percent) | | | | | | | | | POS / | % POS | % POS | H | H |
| Array Position | Age | Sex | Pathology | Grade | 0C | 0A | 1+C | 1+ A | 2+ C | 2+ A | 3+ C | 3+ A | NEG | Cyto | Apical | Cyto | Apical |
| G4 | 57 | M | Cancer adjacent normal esophageal tissue (fibrous tissue, blood vessel and smooth muscle tissue) | - | 20 | 100 | 80 | | | | | | POS | 80 | 0 | 80 | 0 |
| G6 | 45 | M | Cancer adjacent normal esophageal tissue (smooth muscle tissue) | - | | | | | | | | | | | | | |
| G8 | 55 | F | Cancer adjacent normal esophageal tissue | - | | 100 | 100 | | | | | | POS | 100 | 0 | 100 | 0 |
| G10 | 53 | F | Cancer adjacent normal esophageal tissue | - | | 100 | 100 | | | | | | POS | 100 | 0 | 100 | 0 |
| H2 | 68 | M | Cancer adjacent normal esophageal tissue (chronic inflammation of mucosa) | - | 70 | 100 | 30 | | | | | | POS | 30 | 0 | 30 | 0 |
| H4 | 64 | M | Cancer adjacent normal esophageal tissue | - | 100 | 100 | | | | | | | NEG | 0 | 0 | 0 | 0 |
| H6 | 55 | M | Cancer adjacent normal esophageal tissue | - | | 100 | 100 | | | | | | POS | 100 | 0 | 100 | 0 |
| H8 | 63 | F | Cancer adjacent normal esophageal tissue (chronic inflammation of mucosa) | - | | 100 | 100 | | | | | | POS | 100 | 0 | 100 | 0 |
| H10 | 54 | F | Cancer adjacent normal esophageal tissue | - | | 100 | 100 | | | | | | POS | 100 | 0 | 100 | 0 |

**Table 27D: Summary of GCC Staining in US BioMax ES8010 Esophageal TMA -Part 2**

| % POS | % POS | H | H | Total | Total | Pct |
|---|---|---|---|---|---|---|
| Cyto | Apical | Cyto | Apical | Pos | N | Pos |
| 76 | 0 | 78 | 0 | 34 | 38 | 89% |

**Table 28A: GCC IHC Staining in US BioMax LC20813 Lung Tumor MicroArray**

| USBiomax LC20813 Lung TMA | | | | | | | | | | | | | | | | | | | |
|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| A1 | 72 | M | Lung | Squamous cell carcinoma | 1 | 100 | 100 | | | | | | | **NEG** | 0 | 0 | 0 | 0 |
| A2 | 63 | M | Lung | Squamous cell carcinoma | 2 | 100 | 100 | | | | | | | **NEG** | 0 | 0 | 0 | 0 |
| A3 | 55 | M | Lung | Squamous cell carcinoma | 2 | 100 | 100 | | | | | | | **NEG** | 0 | 0 | 0 | 0 |
| A4 | 50 | F | Lung | Squamous cell carcinoma | 2 | 100 | 100 | | | | | | | **NEG** | 0 | 0 | 0 | 0 |
| A5 | 61 | F | Lung | Squamous cell carcinoma | 2 | 100 | 100 | | | | | | | **NEG** | 0 | 0 | 0 | 0 |
| A6 | 63 | M | Lung | Squamous cell carcinoma (sparse) | - | 100 | 100 | | | | | | | **NEG** | 0 | 0 | 0 | 0 |
| A7 | 73 | M | Lung | Squamous cell carcinoma | 2 | 100 | 100 | | | | | | | **NEG** | 0 | 0 | 0 | 0 |
| A8 | 53 | M | Lung | Squamous cell carcinoma | 2 | 100 | 100 | | | | | | | **NEG** | 0 | 0 | 0 | 0 |
| A9 | 69 | M | Lung | Squamous cell carcinoma | 2 | 100 | 100 | | | | | | | **NEG** | 0 | 0 | 0 | 0 |
| A10 | 66 | M | Lung | Squamous cell carcinoma | 2 | 100 | 100 | | | | | | | **NEG** | 0 | 0 | 0 | 0 |
| A11 | 61 | M | Lung | Squamous cell carcinoma | 2 | 100 | 100 | | | | | | | **NEG** | 0 | 0 | 0 | 0 |
| A12 | 64 | M | Lung | Squamous cell carcinoma | 2 | 100 | 100 | | | | | | | **NEG** | 0 | 0 | 0 | 0 |
| A13 | 48 | M | Lung | Squamous cell carcinoma | 2 | 100 | 100 | | | | | | | **NEG** | 0 | 0 | 0 | 0 |

(continued)

| USBiomax LC20813 Lung TMA | | | | | | | | | | | | | | | | | | | |
|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| A14 | 59 | M | Lung | Squamous cell carcinoma | 2 | 100 | 100 | | | | | | | NEG | 0 | 0 | 0 | 0 |
| A15 | 51 | M | Lung | Squamous cell carcinoma | 2 | 100 | 100 | | | | | | | NEG | 0 | 0 | 0 | 0 |
| A16 | 70 | F | Lung | Squamous cell carcinoma | 2 | 100 | 100 | | | | | | | NEG | 0 | 0 | 0 | 0 |
| B1 | 63 | M | Lung | Squamous cell carcinoma | 2 | 100 | 100 | | | | | | | NEG | 0 | 0 | 0 | 0 |
| B2 | 55 | M | Lung | Squamous cell carcinoma | 2 | 100 | 100 | | | | | | | NEG | 0 | 0 | 0 | 0 |
| B3 | 76 | M | Lung | Squamous cell carcinoma | 2 | 100 | 100 | | | | | | | NEG | 0 | 0 | 0 | 0 |
| B4 | 68 | M | Lung | Squamous cell carcinoma | 2 | 50 | 100 | 50 | | | | | | POS | 50 | 0 | 50 | 0 |
| B5 | 45 | M | Lung | Squamous cell carcinoma | 2 | 100 | 100 | | | | | | | NEG | 0 | 0 | 0 | 0 |
| B6 | 54 | M | Lung | Squamous cell carcinoma | 2 | 100 | 100 | | | | | | | NEG | 0 | 0 | 0 | 0 |
| B7 | 50 | M | Lung | Squamous cell carcinoma | 2 | 100 | 100 | | | | | | | NEG | 0 | 0 | 0 | 0 |
| B8 | 57 | M | Lung | Squamous cell carcinoma | 2 | 100 | 100 | | | | | | | NEG | 0 | 0 | 0 | 0 |
| B9 | 43 | F | Lung | Squamous cell carcinoma | 2 | 100 | 100 | | | | | | | NEG | 0 | 0 | 0 | 0 |
| B10 | 46 | M | Lung | Squamous cell carcinoma | 2 | 100 | 100 | | | | | | | NEG | 0 | 0 | 0 | 0 |

(continued)

| USBiomax LC20813 Lung TMA | | | | | | | | | | | | | | | | | | | |
|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| B11 | 72 | M | Lung | Squamous cell carcinoma | 2 | 100 | 100 | | | | | | | **NEG** | 0 | 0 | 0 | 0 |
| B12 | 62 | M | Lung | Squamous cell carcinoma | 2 | 100 | 100 | | | | | | | **NEG** | 0 | 0 | 0 | 0 |
| B13 | 64 | M | Lung | Squamous cell carcinoma with necrosis | 2 | 100 | 100 | | | | | | | **NEG** | 0 | 0 | 0 | 0 |
| B14 | 61 | M | Lung | Squamous cell carcinoma with necrosis | 3 | 90 | 100 | 10 | | | | | | **POS** | 10 | 0 | 10 | 0 |
| B15 | 54 | M | Lung | Squamous cell carcinoma | 2 | 100 | 100 | | | | | | | **NEG** | 0 | 0 | 0 | 0 |
| B16 | 61 | M | Lung | Squamous cell carcinoma | 2 | 100 | 100 | | | | | | | **NEG** | 0 | 0 | 0 | 0 |
| C1 | 35 | M | Lung | Squamous cell carcinoma | 2 | 100 | 100 | | | | | | | **NEG** | 0 | 0 | 0 | 0 |
| C2 | 53 | M | Lung | Squamous cell carcinoma | 2 | 100 | 100 | | | | | | | **NEG** | 0 | 0 | 0 | 0 |
| C3 | 76 | M | Lung | Squamous cell carcinoma (sparse) with necrosis | 2 | 100 | 100 | | | | | | | **NEG** | 0 | 0 | 0 | 0 |
| C4 | 53 | M | Lung | Squamous cell carcinoma | 2 | 100 | 100 | | | | | | | **NEG** | 0 | 0 | 0 | 0 |
| C5 | 66 | M | Lung | Squamous cell carcinoma | 2 | 100 | 100 | | | | | | | **NEG** | 0 | 0 | 0 | 0 |

(continued)

| USBiomax LC20813 Lung TMA | | | | | | | | | | | | | | | | | | | | |
|---|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| C6 | 76 | M | Lung | Squamous cell carcinoma (lung tissue) | - | 100 | 100 | | | | | | | **NEG** | 0 | 0 | 0 | 0 |
| C7 | 70 | M | Lung | Squamous cell carcinoma (tumoral necrosis) | - | | | | | | | | | | | | | |
| C8 | 64 | M | Lung | Squamous cell carcinoma | 2 | 100 | 100 | | | | | | | **NEG** | 0 | 0 | 0 | 0 |
| C9 | 68 | M | Lung | Squamous cell carcinoma (bronchus, fibrous tissue and blood vessel) | - | 100 | 100 | | | | | | | **NEG** | 0 | 0 | 0 | 0 |
| C10 | 25 | M | Lung | Squamous cell carcinoma | 2 | | | | | | | | | | | | | |
| C11 | 66 | M | Lung | Squamous cell carcinoma | 2 | 90 | 100 | 10 | | | | | | **POS** | 10 | 0 | 10 | 0 |
| C12 | 66 | M | Lung | Squamous cell carcinoma | 2 | 90 | 100 | 10 | | | | | | **POS** | 10 | 0 | 10 | 0 |
| C13 | 53 | M | Lung | Squamous cell carcinoma | 2 | | 100 | 100 | | | | | | **POS** | 100 | 0 | 100 | 0 |
| C14 | 39 | M | Lung | Squamous cell carcinoma | 2 | 60 | 100 | 40 | | | | | | **POS** | 40 | 0 | 40 | 0 |
| C15 | 60 | M | Lung | Squamous cell carcinoma | 2 | 70 | 100 | 30 | | | | | | **POS** | 30 | 0 | 30 | 0 |

(continued)

| USBiomax LC20813 Lung TMA | | | | | | | | | | | | | | | | | | | |
|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| C16 | 59 | M | Lung | Squamous cell carcinoma (cartilage, chronic inflammation of fibrous tissue and blood vessel) | - | | | | | | | | | | | | | |
| D1 | 62 | M | Lung | Squamous cell carcinoma | 2 | 100 | 100 | | | | | | | NEG | 0 | 0 | 0 | 0 |
| D2 | 54 | M | Lung | Squamous cell carcinoma | - | 100 | 100 | | | | | | | NEG | 0 | 0 | 0 | 0 |
| D3 | 67 | M | Lung | Squamous cell carcinoma | 2 | 100 | 100 | | | | | | | NEG | 0 | 0 | 0 | 0 |
| D4 | 54 | M | Lung | Squamous cell carcinoma | 2 | 100 | 100 | | | | | | | NEG | 0 | 0 | 0 | 0 |
| D5 | 49 | M | Lung | Squamous cell carcinoma | 2 | 100 | 100 | | | | | | | NEG | 0 | 0 | 0 | 0 |
| D6 | 46 | M | Lung | Squamous cell carcinoma | 2 | 100 | 100 | | | | | | | NEG | 0 | 0 | 0 | 0 |
| D7 | 56 | M | Lung | Squamous cell carcinoma | 3 | 90 | 100 | | | 10 | | | | POS | 10 | 0 | 20 | 0 |
| D8 | 55 | F | Lung | Squamous cell carcinoma | 2 | 100 | 100 | | | | | | | NEG | 0 | 0 | 0 | 0 |
| D9 | 45 | M | Lung | Squamous cell carcinoma | 2 | 80 | 100 | 20 | | | | | | POS | 20 | 0 | 20 | 0 |
| D10 | 47 | M | Lung | Squamous cell carcinoma | 2 | 100 | 100 | | | | | | | NEG | 0 | 0 | 0 | 0 |
| D11 | 72 | M | Lung | Squamous cell carcinoma | 2 | 100 | 100 | | | | | | | NEG | 0 | 0 | 0 | 0 |

(continued)

| USBiomax LC20813 Lung TMA | | | | | | | | | | | | | | | | | | | | |
|---|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| D12 | 66 | M | Lung | Squamous cell carcinoma | 2 | 50 | 100 | 50 | | | | | | **POS** | 50 | 0 | 50 | 0 |
| D13 | 62 | M | Lung | Squamous cell carcinoma | 2 | 100 | 100 | | | | | | | **NEG** | 0 | 0 | 0 | 0 |
| D14 | 48 | M | Lung | Squamous cell carcinoma | 2 | 100 | 100 | | | | | | | **NEG** | 0 | 0 | 0 | 0 |
| D15 | 57 | M | Lung | Squamous cell carcinoma | 2 | 100 | 100 | | | | | | | **NEG** | 0 | 0 | 0 | 0 |
| D16 | 70 | M | Lung | Squamous cell carcinoma | 2 | 100 | 100 | | | | | | | **NEG** | 0 | 0 | 0 | 0 |
| E1 | 55 | M | Lung | Squamous cell carcinoma | 2 | 100 | 100 | | | | | | | **NEG** | 0 | 0 | 0 | 0 |
| E2 | 64 | M | Lung | Squamous cell carcinoma | 2 | 50 | 100 | 50 | | | | | | **POS** | 50 | 0 | 50 | 0 |
| E3 | 67 | M | Lung | Squamous cell carcinoma | 2 | 100 | 100 | | | | | | | **NEG** | 0 | 0 | 0 | 0 |
| E4 | 75 | M | Lung | Squamous cell carcinoma | - | 100 | 100 | | | | | | | **NEG** | 0 | 0 | 0 | 0 |
| E5 | 56 | F | Lung | Squamous cell carcinoma | 2 | 100 | 100 | | | | | | | **NEG** | 0 | 0 | 0 | 0 |
| E6 | 77 | M | Lung | Squamous cell carcinoma | 2 | 100 | 100 | | | | | | | **NEG** | 0 | 0 | 0 | 0 |
| E7 | 51 | F | Lung | Squamous cell carcinoma | 2 | 100 | 100 | | | | | | | **NEG** | 0 | 0 | 0 | 0 |
| E8 | 64 | M | Lung | Squamous cell carcinoma (lung tissue) | - | | | | | | | | | | | | | |

(continued)

| USBiomax LC20813 Lung TMA | | | | | | | | | | | | | | | POS / | % POS | % POS | H | H |
|---|---|---|---|---|---|---|---|---|---|---|---|---|---|---|---|---|---|---|---|
| | | | | | | Apical & Cytoplasmic Staining (Percent) | | | | | | | | | | | | | |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| E9 | 65 | M | Lung | Squamous cell carcinoma with necrosis | 2 | 100 | 100 | | | | | | | NEG | 0 | 0 | 0 | 0 |
| E10 | 63 | M | Lung | Squamous cell carcinoma | 2 | 100 | 100 | | | | | | | NEG | 0 | 0 | 0 | 0 |
| E11 | 52 | M | Lung | Squamous cell carcinoma | 2 | | 100 | 100 | | | | | | POS | 100 | 0 | 100 | 0 |
| E12 | 55 | F | Lung | Squamous cell carcinoma | 2 | | 100 | 100 | | | | | | POS | 100 | 0 | 100 | 0 |
| E13 | 71 | M | Lung | Squamous cell carcinoma | 2 | | 100 | 50 | | 30 | | 20 | | POS | 100 | 0 | 170 | 0 |
| E14 | 52 | M | Lung | Squamous cell carcinoma | 2 | | 100 | 100 | | | | | | POS | 100 | 0 | 100 | 0 |
| E15 | 63 | F | Lung | Adenosquamous carcinoma | - | 50 | 100 | 50 | | | | | | POS | 50 | 0 | 50 | 0 |
| E16 | 67 | F | Lung | Adenosquamous carcinoma | - | | 100 | 50 | | 40 | | 10 | | POS | 100 | 0 | 160 | 0 |
| F1 | 46 | F | Lung | Adenosquamous carcinoma | - | 50 | 100 | 50 | | | | | | POS | 50 | 0 | 50 | 0 |
| F2 | 61 | M | Lung | Adenosquamous carcinoma | - | 100 | 100 | | | | | | | NEG | 0 | 0 | 0 | 0 |
| F3 | 54 | M | Lung | Adenosquamous carcinoma | - | 100 | 100 | | | | | | | NEG | 0 | 0 | 0 | 0 |
| F4 | 55 | F | Lung | Adenosquamous carcinoma | - | 50 | 100 | 50 | | | | | | POS | 50 | 0 | 50 | 0 |
| F5 | 68 | M | Lung | Adenosquamous carcinoma | - | | 100 | 50 | | 50 | | | | POS | 100 | 0 | 150 | 0 |

(continued)

| USBiomax LC20813 Lung TMA | | | | | | | | | | | | | | | | | | | |
|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| F6 | 54 | F | Lung | Papillary adenocarcinoma | 2 | 100 | 100 | | | | | | | NEG | 0 | 0 | 0 | 0 |
| F7 | 61 | M | Lung | Adenocarcinoma | 2 | | | 80 | | 20 | 50 | | 50 | POS | 100 | 100 | 120 | 250 |
| F8 | 60 | F | Lung | Adenocarcinoma | 2 | 100 | 100 | | | | | | | NEG | 0 | 0 | 0 | 0 |
| F9 | 62 | F | Lung | Adenocarcinoma | 2 | 100 | 100 | | | | | | | NEG | 0 | 0 | 0 | 0 |
| F10 | 38 | M | Lung | Adenocarcinoma | 2 | 100 | 100 | | | | | | | NEG | 0 | 0 | 0 | 0 |
| F11 | 42 | F | Lung | Adenocarcinoma | 2 | 50 | 80 | 50 | 20 | | | | | POS | 50 | 20 | 50 | 20 |
| F12 | 56 | F | Lung | Adenocarcinoma | 2 | 100 | 100 | | | | | | | NEG | 0 | 0 | 0 | 0 |
| F13 | 59 | M | Lung | Adenocarcinoma | 3 | 100 | 100 | | | | | | | NEG | 0 | 0 | 0 | 0 |
| F14 | 33 | M | Lung | Adenosquamous carcinoma | - | 80 | 100 | 20 | | | | | | POS | 20 | 0 | 20 | 0 |
| F15 | 68 | F | Lung | Adenocarcinoma | 3 | 100 | 100 | | | | | | | NEG | 0 | 0 | 0 | 0 |
| F16 | 49 | F | Lung | Adenocarcinoma with necrosis | 2 | 100 | 100 | | | | | | | NEG | 0 | 0 | 0 | 0 |
| G1 | 56 | F | Lung | Adenocarcinoma (chronic inflammation of fibrous tissue and blood vessel) | - | | | | | | | | | | | | | |
| G3 | 39 | M | Lung | Papillary adenocarcinoma | 2 | 80 | 100 | 20 | | | | | | POS | 20 | 0 | 20 | 0 |
| G4 | 58 | M | Lung | Papillary adenocarcinoma | 2 | | | | | | | | | | | | | |
| G5 | 56 | F | Lung | Papillary adenocarcinoma | 2 | 100 | 100 | | | | | | | NEG | 0 | 0 | 0 | 0 |

(continued)

| USBiomax LC20813 Lung TMA | | | | | | | | | | | | | | | | | | |
|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| G6 | 55 | M | Lung | Papillary adenocarcinoma | 2 | 100 | 100 | | | | | | | **NEG** | 0 | 0 | 0 | 0 |
| G7 | 62 | M | Lung | Papillary adenocarcinoma | 2 | 100 | 100 | | | | | | | **NEG** | 0 | 0 | 0 | 0 |
| G8 | 64 | F | Lung | Papillary adenocarcinoma | 2 | 100 | 100 | | | | | | | **NEG** | 0 | 0 | 0 | 0 |
| G9 | 72 | M | Lung | Adenocarcinoma | 3 | 50 | 100 | 50 | | | | | | **POS** | 50 | 0 | 50 | 0 |
| G10 | 53 | F | Lung | Adenocarcinoma | 2 | 100 | 100 | | | | | | | **NEG** | 0 | 0 | 0 | 0 |
| G11 | 65 | M | Lung | Papillary adenocarcinoma | 2 | 90 | 100 | | | 10 | | | | **POS** | 10 | 0 | 20 | 0 |
| G12 | 52 | F | Lung | Papillary adenocarcinoma | 2 | | 100 | 100 | | | | | | **POS** | 100 | 0 | 100 | 0 |
| G13 | 47 | F | Lung | Papillary adenocarcinoma | 2 | 70 | 100 | 20 | | 10 | | | | **POS** | 30 | 0 | 40 | 0 |
| G14 | 71 | F | Lung | Papillary adenocarcinoma | 2 | 100 | 100 | | | | | | | **NEG** | 0 | 0 | 0 | 0 |
| G15 | 49 | M | Lung | Adenocarcinoma | 2 | | 100 | 100 | | | | | | **POS** | 100 | 0 | 100 | 0 |
| G16 | 58 | M | Lung | Adenocarcinoma | 2 | | 100 | 100 | | | | | | **POS** | 100 | 0 | 100 | 0 |
| H1 | 62 | M | Lung | Adenocarcinoma | 2 | 100 | 90 | | 10 | | | | | **POS** | 0 | 10 | 0 | 10 |
| H2 | 54 | F | Lung | Adenocarcinoma | 3 | 100 | 100 | | | | | | | **NEG** | 0 | 0 | 0 | 0 |
| H3 | 38 | M | Lung | Adenocarcinoma with necrosis | 2 | 100 | 100 | | | | | | | **NEG** | 0 | 0 | 0 | 0 |
| H4 | 68 | M | Lung | Adenocarcinoma | 2 | 50 | 100 | 50 | | | | | | **POS** | 50 | 0 | 50 | 0 |
| H5 | 64 | F | Lung | Adenocarcinoma | 2 | 100 | 100 | | | | | | | **NEG** | 0 | 0 | 0 | 0 |
| H6 | 41 | F | Lung | Adenocarcinoma | - | 90 | 100 | 10 | | | | | | **POS** | 10 | 0 | 10 | 0 |

(continued)

| USBiomax LC20813 Lung TMA | | | | | | | | | | | | | | | | | | |
|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| H7 | 40 | M | Lung | Adenocarcinoma | 3 | 100 | 100 | | | | | | | **NEG** | 0 | 0 | 0 | 0 |
| H8 | 58 | F | Lung | Adenocarcinoma with necrosis | 3 | 100 | 100 | | | | | | | **NEG** | 0 | 0 | 0 | 0 |
| H9 | 64 | M | Lung | Adenocarcinoma with necrosis | 3 | 100 | 100 | | | | | | | **NEG** | 0 | 0 | 0 | 0 |
| H10 | 56 | F | Lung | Papillary adenocarcinoma | 2 | | 90 | 100 | 10 | | | | | **POS** | 100 | 10 | 100 | 10 |
| H11 | 57 | F | Lung | Papillary adenocarcinoma | 2 | 80 | 100 | 20 | | | | | | **POS** | 20 | 0 | 20 | 0 |
| H12 | 62 | F | Lung | Adenocarcinoma with necrosis | 2 | | 100 | 80 | | 20 | | | | **POS** | 100 | 0 | 120 | 0 |
| H13 | 60 | M | Lung | Adenocarcinoma | 2 | 100 | 100 | | | | | | | **NEG** | 0 | 0 | 0 | 0 |
| H14 | 64 | F | Lung | Adenocarcinoma | 2 | 100 | 100 | | | | | | | **NEG** | 0 | 0 | 0 | 0 |
| H15 | 82 | M | Lung | Adenocarcinoma | 3 | 100 | 90 | | 10 | | | | | **POS** | 0 | 10 | 0 | 10 |
| H16 | 60 | M | Lung | Adenocarcinoma | 3 | 100 | 100 | | | | | | | **NEG** | 0 | 0 | 0 | 0 |
| I1 | 50 | F | Lung | Adenocarcinoma | 3 | 50 | 100 | 50 | | | | | | **POS** | 50 | 0 | 50 | 0 |
| I2 | 46 | M | Lung | Adenocarcinoma | 3 | 100 | 100 | | | | | | | **NEG** | 0 | 0 | 0 | 0 |
| I3 | 69 | M | Lung | Adenocarcinoma (sparse) | 3 | | | | | | | | | | | | | |
| I4 | 51 | M | Lung | Adenocarcinoma | 3 | 100 | 100 | | | | | | | **NEG** | 0 | 0 | 0 | 0 |
| I5 | 70 | M | Lung | Adenocarcinoma | 3 | 100 | 100 | | | | | | | **NEG** | 0 | 0 | 0 | 0 |
| I6 | 67 | M | Lung | Adenocarcinoma | 3 | | 10 | 60 | | 20 | 30 | 20 | 60 | **POS** | 100 | 90 | 160 | 240 |
| I7 | 36 | M | Lung | Adenocarcinoma | 3 | 100 | 100 | | | | | | | **NEG** | 0 | 0 | 0 | 0 |
| I8 | 49 | M | Lung | Adenocarcinoma | 3 | 100 | 100 | | | | | | | **NEG** | 0 | 0 | 0 | 0 |

(continued)

| USBiomax LC20813 Lung TMA | | | | | | | | | | | | | | | | | | | |
|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| I9 | 60 | M | Lung | Adenocarcinoma | 3 | 100 | 100 | | | | | | | **NEG** | 0 | 0 | 0 | 0 |
| I10 | 39 | F | Lung | Adenocarcinoma | 3 | 100 | 100 | | | | | | | **NEG** | 0 | 0 | 0 | 0 |
| I11 | 65 | M | Lung | Adenocarcinoma | 3 | | 100 | 100 | | | | | | **POS** | 100 | 0 | 100 | 0 |
| I12 | 69 | F | Lung | Adenocarcinoma | 3 | | 100 | 70 | | 30 | | | | **POS** | 100 | 0 | 130 | 0 |
| I13 | 75 | M | Lung | Adenocarcinoma | 3 | 100 | 100 | | | | | | | **NEG** | 0 | 0 | 0 | 0 |
| I14 | 75 | M | Lung | Adenocarcinoma | 3 | 100 | 100 | | | | | | | **NEG** | 0 | 0 | 0 | 0 |
| I15 | 61 | F | Lung | Adenocarcinoma | 3 | | 100 | 100 | | | | | | **POS** | 100 | 0 | 100 | 0 |
| I16 | 44 | M | Lung | Adenocarcinoma | 3 | 100 | 90 | | 10 | | | | | **POS** | 0 | 10 | 0 | 10 |
| J1 | 59 | M | Lung | Adenocarcinoma | 3 | 100 | 100 | | | | | | | **NEG** | 0 | 0 | 0 | 0 |
| J2 | 68 | F | Lung | Adenocarcinoma | 3 | 80 | 100 | 20 | | | | | | **POS** | 20 | 0 | 20 | 0 |
| J3 | 65 | M | Lung | Adenocarcinoma with necrosis | 3 | 100 | 100 | | | | | | | **NEG** | 0 | 0 | 0 | 0 |
| J4 | 65 | M | Lung | Adenosquamous carcinoma | - | 70 | 100 | 30 | | | | | | **POS** | 30 | 0 | 30 | 0 |
| J5 | 39 | M | Lung | Adenocarcinoma | 3 | 80 | 100 | 20 | | | | | | **POS** | 20 | 0 | 20 | 0 |
| J6 | 74 | M | Lung | Adenocarcinoma | 3 | 100 | 100 | | | | | | | **NEG** | 0 | 0 | 0 | 0 |
| J7 | 50 | M | Lung | Adenocarcinoma | - | 100 | 100 | | | | | | | **NEG** | 0 | 0 | 0 | 0 |
| J8 | 36 | F | Lung | Adenocarcinoma | 3 | 100 | 100 | | | | | | | **NEG** | 0 | 0 | 0 | 0 |
| J9 | 46 | M | Lung | Adenocarcinoma | 3 | 90 | 100 | 10 | | | | | | **POS** | 10 | 0 | 10 | 0 |
| J10 | 69 | M | Lung | Adenocarcinoma | 3 | 50 | 100 | 50 | | | | | | **POS** | 50 | 0 | 50 | 0 |
| J11 | 30 | M | Lung | Adenocarcinoma | 3 | 100 | 100 | | | | | | | **NEG** | 0 | 0 | 0 | 0 |
| J12 | 65 | M | Lung | Adenocarcinoma | 3 | 100 | 100 | | | | | | | **NEG** | 0 | 0 | 0 | 0 |
| J13 | 52 | M | Lung | Adenocarcinoma | 3 | 100 | 100 | | | | | | | **NEG** | 0 | 0 | 0 | 0 |

(continued)

| USBiomax LC20813 Lung TMA | | | | | | | | | | | | | | | | | | | |
|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| J14 | 66 | M | Lung | Adenocarcinoma | 3 | 100 | 100 | | | | | | | **NEG** | 0 | 0 | 0 | 0 |
| J15 | 47 | M | Lung | Adenocarcinoma | 3 | 100 | 100 | | | | | | | **NEG** | 0 | 0 | 0 | 0 |
| J16 | 52 | F | Lung | Adenocarcinoma | 3 | 90 | 100 | 10 | | | | | | **POS** | 10 | 0 | 10 | 0 |
| K1 | 58 | F | Lung | Adenocarcinoma | 3 | 100 | 100 | | | | | | | **NEG** | 0 | 0 | 0 | 0 |
| K2 | 46 | F | Lung | Adenocarcinoma | 3 | 50 | 100 | 50 | | | | | | **POS** | 50 | 0 | 50 | 0 |
| K3 | 69 | M | Lung | Adenocarcinoma | 3 | 100 | 100 | | | | | | | **NEG** | 0 | 0 | 0 | 0 |
| K4 | 58 | M | Lung | Small cell carcinoma | - | 100 | 100 | | | | | | | **NEG** | 0 | 0 | 0 | 0 |
| K5 | 51 | M | Lung | Small cell carcinoma | - | 100 | 100 | | | | | | | **NEG** | 0 | 0 | 0 | 0 |
| K6 | 63 | F | Lung | Small cell carcinoma | - | 100 | 100 | | | | | | | **NEG** | 0 | 0 | 0 | 0 |
| K7 | 63 | M | Lung | Small cell carcinoma | - | 100 | 100 | | | | | | | **NEG** | 0 | 0 | 0 | 0 |
| K8 | 60 | M | Lung | Small cell carcinoma | - | 100 | 100 | | | | | | | **NEG** | 0 | 0 | 0 | 0 |
| K9 | 63 | M | Lung | Small cell carcinoma | - | 100 | 100 | | | | | | | **NEG** | 0 | 0 | 0 | 0 |
| K10 | 66 | M | Lung | Small cell carcinoma | - | 100 | 100 | | | | | | | **NEG** | 0 | 0 | 0 | 0 |
| K11 | 71 | M | Lung | Small cell carcinoma | - | 100 | 100 | | | | | | | **NEG** | 0 | 0 | 0 | 0 |
| K12 | 60 | M | Lung | Small cell carcinoma | - | 100 | 100 | | | | | | | **NEG** | 0 | 0 | 0 | 0 |
| K13 | 31 | F | Lung | Small cell carcinoma | - | 100 | 100 | | | | | | | **NEG** | 0 | 0 | 0 | 0 |
| K14 | 61 | M | Lung | Small cell carcinoma | - | 100 | 100 | | | | | | | **NEG** | 0 | 0 | 0 | 0 |
| K15 | 52 | F | Lung | Small cell carcinoma | - | 100 | 100 | | | | | | | **NEG** | 0 | 0 | 0 | 0 |
| K16 | 69 | F | Lung | Small cell carcinoma | - | 100 | 100 | | | | | | | **NEG** | 0 | 0 | 0 | 0 |
| L1 | 43 | F | Lung | Small cell carcinoma | - | 100 | 100 | | | | | | | **NEG** | 0 | 0 | 0 | 0 |
| L2 | 56 | M | Lung | Small cell carcinoma | - | 100 | 100 | | | | | | | **NEG** | 0 | 0 | 0 | 0 |
| L3 | 62 | M | Lung | Small cell carcinoma | - | 100 | 100 | | | | | | | **NEG** | 0 | 0 | 0 | 0 |

(continued)

| USBiomax LC20813 Lung TMA | | | | | | | | | | | | | | | | | | | |
|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| L4 | 42 | F | Lung | Small cell carcinoma | - | 100 | 100 | | | | | | | **NEG** | 0 | 0 | 0 | 0 |
| L5 | 59 | F | Lung | Small cell carcinoma (fibrous tissue and blood vessel) | - | 100 | 100 | | | | | | | **NEG** | 0 | 0 | 0 | 0 |
| L6 | 55 | F | Lung | Small cell carcinoma | - | 100 | 100 | | | | | | | **NEG** | 0 | 0 | 0 | 0 |
| L7 | 28 | M | Lung | Small cell carcinoma | - | 100 | 100 | | | | | | | **NEG** | 0 | 0 | 0 | 0 |
| L8 | 39 | M | Lung | Small cell carcinoma | - | 100 | 100 | | | | | | | **NEG** | 0 | 0 | 0 | 0 |
| L9 | 61 | M | Lung | Large cell carcinoma | - | 100 | 100 | | | | | | | **NEG** | 0 | 0 | 0 | 0 |
| L10 | 72 | F | Lung | Large cell carcinoma | - | 90 | 100 | 10 | | | | | | **POS** | 10 | 0 | 10 | 0 |
| L11 | 46 | M | Lung | Large cell carcinoma | - | 70 | 100 | 30 | | | | | | **POS** | 30 | 0 | 30 | 0 |
| L12 | 64 | F | Lung | Large cell carcinoma | - | 100 | 100 | | | | | | | **NEG** | 0 | 0 | 0 | 0 |
| L13 | 55 | F | Lung | Bronchioloalve olar carcinoma | - | 100 | 100 | | | | | | | **NEG** | 0 | 0 | 0 | 0 |
| L14 | 52 | M | Lung | Bronchioloalve olar carcinoma | - | 90 | 100 | 10 | | | | | | **POS** | 10 | 0 | 10 | 0 |
| L15 | 55 | M | Lung | Bronchioloalve olar carcinoma | - | 100 | 100 | | | | | | | **NEG** | 0 | 0 | 0 | 0 |
| L16 | 64 | F | Lung | Bronchioloalve olar carcinoma | - | 100 | 100 | | | | | | | **NEG** | 0 | 0 | 0 | 0 |
| M2 | 50 | F | Lung | Mucinous bronchioloalveolar carcinoma (sparse) | - | | | | | | | | | | | | | |
| M3 | 27 | M | Lung | Mucinous bronchioloalveolar carcinoma | - | 100 | 100 | | | | | | | **NEG** | 0 | 0 | 0 | 0 |

(continued)

| USBiomax LC20813 Lung TMA | | | | | | | | | | | | | | | | | | | |
|---|---|---|---|---|---|---|---|---|---|---|---|---|---|---|---|---|---|---|---|
| | | | | | | Apical & Cytoplasmic Staining (Percent) | | | | | | | | POS / | % POS | % POS | H | H |
| Array Position | Age | Sex | Organ | Pathology | Grade | 0C | 0A | 1+ C | 1+ A | 2+ C | 2+ A | 3+ C | 3+ A | NEG | Cyto | Apical | Cyto | Apical |
| M4 | 48 | M | Lung | Mucinous bronchioloalveolar carcinoma | - | 100 | 100 | | | | | | | **NEG** | 0 | 0 | 0 | 0 |
| M5 | 56 | M | Lung | Mucoepidermoid carcinoma | - | 90 | 100 | 10 | | | | | | **POS** | 10 | 0 | 10 | 0 |
| M6 | 51 | M | Lung | Mucoepidermoid carcinoma | - | | | | | | | | | | | | | |
| M7 | 48 | M | Lung | Mucoepidermoid carcinoma | - | 100 | 100 | | | | | | | **NEG** | 0 | 0 | 0 | 0 |
| M8 | 58 | M | Lung | Mucoepidermoid carcinoma | - | 90 | 100 | 10 | | | | | | **POS** | 10 | 0 | 10 | 0 |
| M9 | 49 | M | Lung | Atypical carcinoid | - | 100 | 100 | | | | | | | **NEG** | 0 | 0 | 0 | 0 |
| M10 | 47 | M | Lung | Atypical carcinoid | - | 100 | 100 | | | | | | | **NEG** | 0 | 0 | 0 | 0 |
| M11 | 67 | M | Lung | Atypical carcinoid | - | 100 | 100 | | | | | | | **NEG** | 0 | 0 | 0 | 0 |
| M12 | 65 | M | Lung | Large cell neuroendocrine carcinoma | - | 100 | 100 | | | | | | | **NEG** | 0 | 0 | 0 | 0 |
| M13 | 43 | M | Lung | Mixed large cell neuroendocrine carcinoma | - | 100 | 100 | | | | | | | **NEG** | 0 | 0 | 0 | 0 |
| M14 | 58 | M | Lung | Giant cell carcinoma | - | 100 | 100 | | | | | | | **NEG** | 0 | 0 | 0 | 0 |
| M15 | 36 | F | Lung | Basal cell carcinoma | - | 100 | 100 | | | | | | | **NEG** | 0 | 0 | 0 | 0 |
| M16 | 66 | M | Lung | Pleomorphic carcinoma | - | 100 | 100 | | | | | | | **NEG** | 0 | 0 | 0 | 0 |

**Table 28B: Summary of GCC Staining in US BioMax LC20813 Lung TMA**

| AVG % POS | AVG % POS | AVGH | AVGH | Total | Total | Pct |
|---|---|---|---|---|---|---|
| Cyto | Apical | Cyto | Apical | Pos | N | Pos |
| 14 | 1 | 15 | 3 | 56 | 199 | 28% |
| 11 | 0 | 12 | 0 | 15 | 74 | 20% |
| 23 | 3 | 26 | 7 | 36 | 82 | 44% |
| 0 | 0 | 0 | 0 | 0 | 21 | 0% |
| 3 | 0 | 3 | 0 | 5 | 22 | 23% |

**Table 29A: GCC IHC Staining in Leiomyosarcoma/Rhabdomyosarcoma Tumor MicroArray**

| | | | | | | Apical & Cytoplasmic Staining (Percent) | | | | | | | | | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | POS/NEG | Cyto | Apical | Cyto | Apical |
| A1 | M | 85 | Fibrous tissue | High malignant leiomyosarcoma | IIIB | 100 | 100 | | | | | | | **NEG** | 0 | 0 | 0 | 0 |
| A2 | M | 85 | Fibrous tissue | High malignant leiomyosarcoma | IIIB | 100 | 100 | | | | | | | **NEG** | 0 | 0 | 0 | 0 |
| A3 | M | 26 | Fibrous tissue | Moderate malignant leiomyosarcoma | IA | 100 | 100 | | | | | | | **NEG** | 0 | 0 | 0 | 0 |
| A4 | M | 26 | Fibrous tissue | Moderate malignant leiomyosarcoma | IA | 100 | 100 | | | | | | | **NEG** | 0 | 0 | 0 | 0 |
| A5 | F | 56 | Fibrous tissue | Moderate malignant leiomyosarcoma | IIB | 100 | 100 | | | | | | | **NEG** | 0 | 0 | 0 | 0 |
| A6 | F | 56 | Fibrous tissue | Moderate malignant leiomyosarcoma | IIB | 100 | 100 | | | | | | | **NEG** | 0 | 0 | 0 | 0 |
| A7 | F | 74 | Fibrous tissue | Low malignant leiomyosarcoma | IA | 100 | 100 | | | | | | | **NEG** | 0 | 0 | 0 | 0 |
| A8 | F | 74 | Fibrous tissue | Low malignant leiomyosarcoma | IA | 100 | 100 | | | | | | | **NEG** | 0 | 0 | 0 | 0 |
| A9 | M | 34 | Fibrous tissue | Moderate malignant leiomyosarcoma | IIA | 100 | 100 | | | | | | | **NEG** | 0 | 0 | 0 | 0 |
| B1 | M | 34 | Fibrous tissue | Moderate malignant leiomyosarcoma | IIA | | 100 | 100 | | | | | | **POS** | 100 | 0 | 100 | 0 |
| B2 | F | 20 | Smooth muscle | Moderate malignant leiomyosarcoma | IIIA | 100 | 100 | | | | | | | **NEG** | 0 | 0 | 0 | 0 |
| B3 | F | 20 | Smooth muscle | Moderate malignant leiomyosarcoma | IIIA | 100 | 100 | | | | | | | **NEG** | 0 | 0 | 0 | 0 |
| B4 | F | 49 | Fatty tissue | Moderate malignant leiomyosarcoma | IIB | 100 | 100 | | | | | | | **NEG** | 0 | 0 | 0 | 0 |
| B5 | F | 49 | Fatty tissue | Moderate malignant leiomyosarcoma | IIB | NET | 100 | | | | | | | **n/a** | | | | |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| B6 | F | 34 | Fatty tissue | Moderate malignant epithelioid leiomyosarcoma | IIB | 100 | 100 | | | | | | | **NEG** | 0 | 0 | 0 | 0 |
| B7 | F | 34 | Fatty tissue | Moderate malignant epithelioid leiomyosarcoma | IIB | | 100 | 100 | | | | | | **POS** | 100 | 0 | 100 | 0 |
| B8 | N | 38 | Smooth muscle | Low malignant leiomyosarcoma | IA | 100 | 100 | | | | | | | **NEG** | 0 | 0 | 0 | 0 |
| B9 | N | 38 | Smooth muscle | Low malignant leiomyosarcoma | IA | 80 | 100 | 20 | | | | | | **POS** | 20 | 0 | 20 | 0 |
| C1 | F | 44 | Ligament | Low malignant leiomyosarcoma | IB | 100 | 100 | | | | | | | **NEG** | 0 | 0 | 0 | 0 |
| C2 | F | 44 | Ligament | Low malignant leiomyosarcoma | IB | 100 | 100 | | | | | | | **NEG** | 0 | 0 | 0 | 0 |
| C3 | F | 69 | Smooth muscle | Moderate malignant leiomyosarcoma | IIB | 100 | 100 | | | | | | | **NEG** | 0 | 0 | 0 | 0 |
| C4 | F | 69 | Smooth muscle | Moderate malignant leiomyosarcoma | IIB | | 100 | 100 | | | | | | **POS** | 100 | 0 | 100 | 0 |
| C5 | F | 88 | Smooth muscle | Low malignant leiomyosarcoma | IA | 100 | 100 | | | | | | | **NEG** | 0 | 0 | 0 | 0 |
| C6 | F | 88 | Smooth muscle | Low malignant leiomyosarcoma | IA | 100 | 100 | | | | | | | **NEG** | 0 | 0 | 0 | 0 |
| C7 | F | 54 | Smooth muscle | Malignant pleomorphic leiomyosarcoma | IIIB | 100 | 100 | | | | | | | **NEG** | 0 | 0 | 0 | 0 |
| C8 | F | 54 | Smooth muscle | Malignant pleomorphic leiomyosarcoma | IIIB | 100 | 100 | | | | | | | **NEG** | 0 | 0 | 0 | 0 |
| C9 | F | 58 | Smooth muscle | Low malignant leiomyosarcoma | IIB | | 100 | 100 | | | | | | **POS** | 100 | 0 | 100 | 0 |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| D1 | F | 58 | Smooth muscle | Low malignant leiomyosarcoma | IIB | 100 | 100 | | | | | | | **NEG** | 0 | 0 | 0 | 0 |
| D2 | F | 57 | Smooth muscle | Low malignant leiomyosarcoma | IB | 90 | 100 | 10 | | | | | | **POS** | 10 | 0 | 10 | 0 |
| D3 | F | 57 | Smooth muscle | Low malignant leiomyosarcoma | IB | 50 | 100 | 50 | | | | | | **POS** | 50 | 0 | 50 | 0 |
| D4 | M | 78 | Smooth muscle | Moderate malignant leiomyosarcoma | IIA | NET | 100 | | | | | | | **n/a** | | | | |
| D5 | M | 78 | Smooth muscle | Moderate malignant leiomyosarcoma | IIA | NET | 100 | | | | | | | **n/a** | | | | |
| D6 | F | 52 | Smooth muscle | Moderate malignant leiomyosarcoma | IIA | 100 | 100 | | | | | | | **NEG** | 0 | 0 | 0 | 0 |
| D7 | F | 52 | Smooth muscle | Moderate malignant leiomyosarcoma | IIA | 50 | 100 | 50 | | | | | | **POS** | 50 | 0 | 50 | 0 |
| D8 | M | 61 | Smooth muscle | Moderate malignant leiomyosarcoma | IIA | | 100 | 100 | | | | | | **POS** | 100 | 0 | 100 | 0 |
| D9 | M | 61 | Smooth muscle | Moderate malignant leiomyosarcoma (fibrous tissue, blood | IIA | | 100 | 100 | | | | | | **POS** | 100 | 0 | 100 | 0 |
| | | | | vessel and smooth muscle) | | | | | | | | | | | | | | |
| E1 | F | 33 | Fibrous tissue | Pleomorphic rhabdomyosarcoma | IIA | 100 | 100 | | | | | | | **NEG** | 0 | 0 | 0 | 0 |
| E2 | F | 33 | Fibrous tissue | Pleomorphic rhabdomyosarcoma | IIA | 100 | 100 | | | | | | | **NEG** | 0 | 0 | 0 | 0 |
| E3 | M | 21 | Retroperitoneum | Embryonic rhabdomyosarcoma | IIIA | | 100 | 100 | | | | | | **POS** | 100 | 0 | 100 | 0 |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| E4 | M | 21 | Retrope ritoneum | Embryonic rhabdomyosarcoma | IIIA | | 100 | 100 | | | | | | **POS** | 100 | 0 | 100 | 0 |
| E5 | F | 51 | Uterine cervix | Pleomorphic rhabdomyosarcoma | IIIB | 100 | 100 | | | | | | | **NEG** | 0 | 0 | 0 | 0 |
| E6 | F | 51 | Uterine cervix | Pleomorphic rhabdomyosarcoma | IIIB | 100 | 100 | | | | | | | **NEG** | 0 | 0 | 0 | 0 |
| E7 | M | 16 | Testis | Spindle cell rhabdomyosarcoma | IIA | 100 | 100 | | | | | | | **NEG** | 0 | 0 | 0 | 0 |
| E8 | M | 16 | Testis | Spindle cell rhabdomyosarcoma | IIA | | 100 | 100 | | | | | | **POS** | 100 | 0 | 100 | 0 |
| E9 | F | 49 | Uterus | Pleomorphic rhabdomyosarcoma | IIIB | 100 | 100 | | | | | | | **NEG** | 0 | 0 | 0 | 0 |
| F1 | F | 49 | Uterus | Pleomorphic rhabdomyosarcoma | IIIB | 100 | 100 | | | | | | | **NEG** | 0 | 0 | 0 | 0 |
| F2 | M | 30 | Striated muscle | Rhabdomyosarcoma | IIB | 100 | 100 | | | | | | | **NEG** | 0 | 0 | 0 | 0 |
| F3 | M | 30 | Striated muscle | Rhabdomyosarcoma | IIB | 70 | 100 | 30 | | | | | | **POS** | 30 | 0 | 30 | 0 |
| F4 | M | 18 | Tongue | Embryonic rhabdomyosarcoma | IIIA | 100 | 100 | | | | | | | **NEG** | 0 | 0 | 0 | 0 |
| F5 | M | 18 | Tongue | Embryonic rhabdomyosarcoma | IIIA | 100 | 100 | | | | | | | **NEG** | 0 | 0 | 0 | 0 |
| F6 | M | 91 | Striated muscle | Pleomorphic rhabdomyosarcoma | IIIB | | 100 | 100 | | | | | | **POS** | 100 | 0 | 100 | 0 |
| F7 | M | 91 | Striated muscle | Pleomorphic rhabdomyosarcoma | IIIB | | 100 | 100 | | | | | | **POS** | 100 | 0 | 100 | 0 |
| F8 | F | 74 | Bladder | Pleomorphic rhabdomyosarcoma | IIIB | 100 | 100 | | | | | | | **NEG** | 0 | 0 | 0 | 0 |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| F9 | F | 74 | Bladder | Pleomorphic rhabdomyosarcoma | IIIB | 100 | 100 | | | | | | | **NEG** | 0 | 0 | 0 | 0 |
| G1 | F | 67 | Uterine cervix | Pleomorphic rhabdomyosarcoma | IIIB | 100 | 100 | | | | | | | **NEG** | 0 | 0 | 0 | 0 |
| G2 | F | 67 | Uterine cervix | Pleomorphic rhabdomyosarcoma | IIIB | 100 | 100 | | | | | | | **NEG** | 0 | 0 | 0 | 0 |
| G3 | F | 40 | Abdominal cavity | Embryonic rhabdomyosarcoma | IIIB | 100 | 100 | | | | | | | **NEG** | 0 | 0 | 0 | 0 |
| G4 | F | 40 | Abdominal cavity | Embryonic rhabdomyosarcoma | IIIB | 100 | 100 | | | | | | | **NEG** | 0 | 0 | 0 | 0 |
| G5 | F | 23 | Pelvic cavity | Embryonic rhabdomyosarcoma | IIIB | 100 | 100 | | | | | | | **NEG** | 0 | 0 | 0 | 0 |
| G6 | F | 23 | Pelvic cavity | Embryonic rhabdomyosarcoma | IIIB | 100 | 100 | | | | | | | **NEG** | 0 | 0 | 0 | 0 |
| G7 | N | 50 | Striated muscle | Pleomorphic rhabdomyosarcoma | IIA | 100 | 100 | | | | | | | **NEG** | 0 | 0 | 0 | 0 |
| G8 | M | 50 | Striated muscle | Pleomorphic rhabdomyosarcoma | IIA | | 100 | 100 | | | | | | **POS** | 100 | 0 | 100 | 0 |
| G9 | M | 10 | Striated muscle | Alveolus rhabdomyos arcoma | IIB | | 100 | 70 | | 30 | | | | **POS** | 100 | 0 | 130 | 0 |
| H1 | M | 10 | Striated muscle | Alveolus rhabdomyos arcoma | IIB | 50 | 100 | 50 | | | | | | **POS** | 50 | 0 | 50 | 0 |
| H2 | F | 32 | Uterine cervix | Pleomorphic rhabdomyosarcoma | IIIB | 70 | 100 | 30 | | | | | | **POS** | 30 | 0 | 30 | 0 |
| H3 | F | 32 | Uterine cervix | Pleomorphic rhabdomyosarcoma | IIIB | 100 | 100 | | | | | | | **NEG** | 0 | 0 | 0 | 0 |
| H4 | M | 48 | Soft tissue | Alveolus rhabdomyos arcoma | IIB | 80 | 100 | 20 | | | | | | **POS** | 20 | 0 | 20 | 0 |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| H5 | M | 48 | Soft tissue | Alveolus rhabdomyos arcoma | IIB | 100 | 100 | | | | | | | **NEG** | 0 | 0 | 0 | 0 |
| H6 | M | 40 | Soft tissue | Spindle cell rhabdomyosarcoma | IA | 80 | 100 | 20 | | | | | | **POS** | 20 | 0 | 20 | 0 |
| H7 | M | 40 | Soft tissue | Spindle cell rhabdomyosarcoma | IA | 80 | 100 | 20 | | | | | | **POS** | 20 | 0 | 20 | 0 |
| H8 | M | 49 | Testis | Embryonic rhabdomyosarcoma | IIB | | 100 | 100 | | | | | | **POS** | 100 | 0 | 100 | 0 |
| H9 | M | 49 | Testis | Embryonic rhabdomyosarcoma | IIB | | 100 | 100 | | | | | | **POS** | 100 | 0 | 100 | 0 |
| I1 | F | 48 | Uterus | Smooth muscle | - | 100 | 100 | | | | | | | **NEG** | 0 | 0 | 0 | 0 |
| I2 | F | 8 | Heart | Cardiac muscle | - | 100 | 100 | | | | | | | **NEG** | 0 | 0 | 0 | 0 |
| I3 | F | 14 | Heart | Cardiac muscle | - | 100 | 100 | | | | | | | **NEG** | 0 | 0 | 0 | 0 |
| n/a | | | Bladder | Normal Bladder | n/a | 100 | 100 | | | | | | | **NEG** | 0 | 0 | 0 | 0 |
| n/a | | | 1007 (GCC29 3); Xenogr aft | Human Embryonic Kidney Xenograft - GCC Transfected | n/a | | 100 | | | | | 100 | | **POS** | 100 | 0 | 300 | 0 |
| n/a | | | Kidney Cell Line - 293 HEK-GCC#2 | Human Embryonic Kidney Cell Line - GCC Transfected | n/a | | 100 | | | | | 100 | | **POS** | 100 | 0 | 300 | 0 |
| n/a | | | Kidney Cell Line - 293 [HEK-293] | Human Embryonic Kidney Cell Line | n/a | 100 | 100 | | | | | | | **NEG** | 0 | 0 | 0 | 0 |
| n/a | | | Tissue 1 | Colon Cancer MTB | n/a | 100 | | | | | 10 | | 90 | **POS** | 0 | 100 | 0 | 290 |
| n/a | | | Tissue 2 | Colon Cancer MTB | n/a | NET | | | | | | | | **n/a** | | | | |
| n/a | | | Tissue 3 | Colon Cancer MTB | n/a | 80 | 60 | 20 | 10 | | 20 | | 10 | **POS** | 20 | 40 | 20 | 80 |
| n/a | | | Tissue 4 | Colon Cancer MTB | n/a | 90 | 90 | 10 | 10 | | | | | **POS** | 10 | 10 | 10 | 10 |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| n/a | | | Tissue 5 | Colon Cancer MTB | n/a | NET | | | | | | | | **n/a** | | | | |
| n/a | | | Tissue 6 | Colon Cancer MTB | n/a | NET | | | | | | | | **n/a** | | | | |
| n/a | | | Tissue 7 | Colon Cancer MTB | n/a | 40 | | 40 | | 20 | 50 | | 50 | **POS** | 60 | 100 | 80 | 250 |
| n/a | | | Tissue 8 | Colon Cancer MTB | n/a | 80 | | 20 | 20 | | 30 | | 50 | **POS** | 20 | 100 | 20 | 230 |

Table 29B: Negative Control Ab Scoring Results in Leiomyosarcoma/Rhabdomyosarcoma Tumor MicroArray

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| A1 | M | 85 | Fibrous tissue | High malignant leiomyosarcoma | IIIB | 100 | 100 | | | | | | | **NEG** | 0 | 0 | 0 | 0 |
| A2 | M | 85 | Fibrous tissue | High malignant leiomyosarcoma | IIIB | 100 | 100 | | | | | | | **NEG** | 0 | 0 | 0 | 0 |
| A3 | M | 26 | Fibrous tissue | Moderate malignant leiomyosarcoma | IA | 100 | 100 | | | | | | | **NEG** | 0 | 0 | 0 | 0 |
| A4 | M | 26 | Fibrous tissue | Moderate malignant leiomyosarcoma | IA | 100 | 100 | | | | | | | **NEG** | 0 | 0 | 0 | 0 |
| A5 | F | 56 | Fibrous tissue | Moderate malignant leiomyosarcoma | IIB | 100 | 100 | | | | | | | **NEG** | 0 | 0 | 0 | 0 |
| A6 | F | 56 | Fibrous tissue | Moderate malignant leiomyosarcoma | IIB | 100 | 100 | | | | | | | **NEG** | 0 | 0 | 0 | 0 |
| A7 | F | 74 | Fibrous tissue | Low malignant leiomyosarcoma | IA | 100 | 100 | | | | | | | **NEG** | 0 | 0 | 0 | 0 |
| A8 | F | 74 | Fibrous tissue | Low malignant leiomyosarcoma | IA | 100 | 100 | | | | | | | **NEG** | 0 | 0 | 0 | 0 |
| A9 | M | 34 | Fibrous tissue | Moderate malignant leiomyosarcoma | IIA | 100 | 100 | | | | | | | **NEG** | 0 | 0 | 0 | 0 |
| B1 | M | 34 | Fibrous tissue | Moderate malignant leiomyosarcoma | IIA | 100 | 100 | | | | | | | **NEG** | 0 | 0 | 0 | 0 |
| B2 | F | 20 | Smooth muscle | Moderate malignant leiomyosarcoma | IIIA | 100 | 100 | | | | | | | **NEG** | 0 | 0 | 0 | 0 |
| B3 | F | 20 | Smooth muscle | Moderate malignant leiomyosarcoma | IIIA | 100 | 100 | | | | | | | **NEG** | 0 | 0 | 0 | 0 |
| B4 | F | 49 | Fatty tissue | Moderate malignant leiomyosarcoma | IIB | 100 | 100 | | | | | | | **NEG** | 0 | 0 | 0 | 0 |
| B5 | F | 49 | Fatty tissue | Moderate malignant leiomyosarcoma | IIB | 100 | 100 | | | | | | | **n/a** | | | | |

(continued)

| | | | | | | Apical & Cytoplasmic Staining (Percent) | | | | | | | | | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | POS/NEG | Cyto | Apical | Cyto | Apical |
| B6 | F | 34 | Fatty tissue | Moderate malignant epithelioid leiomyosarcoma | IIB | 100 | 100 | | | | | | | **NEG** | 0 | 0 | 0 | 0 |
| B7 | F | 34 | Fatty tissue | Moderate malignant epithelioid leiomyosarcoma | IIB | 100 | 100 | | | | | | | **NEG** | 0 | 0 | 0 | 0 |
| B8 | M | 38 | Smooth muscle | Low malignant leiomyosarcoma | IA | 100 | 100 | | | | | | | **NEG** | 0 | 0 | 0 | 0 |
| B9 | M | 38 | Smooth muscle | Low malignant leiomyosarcoma | IA | 100 | 100 | | | | | | | **NEG** | 0 | 0 | 0 | 0 |
| C1 | F | 44 | Ligame nt | Low malignant leiomyosarcoma | IB | 100 | 100 | | | | | | | **NEG** | 0 | 0 | 0 | 0 |
| C2 | F | 44 | Ligame nt | Low malignant leiomyosarcoma | IB | 100 | 100 | | | | | | | **NEG** | 0 | 0 | 0 | 0 |
| C3 | F | 69 | Smooth muscle | Moderate malignant leiomyosarcoma | IIB | 100 | 100 | | | | | | | **NEG** | 0 | 0 | 0 | 0 |
| C4 | F | 69 | Smooth muscle | Moderate malignant leiomyosarcoma | IIB | 100 | 100 | | | | | | | **NEG** | 0 | 0 | 0 | 0 |
| C5 | F | 88 | Smooth muscle | Low malignant leiomyosarcoma | IA | 100 | 100 | | | | | | | **NEG** | 0 | 0 | 0 | 0 |
| C6 | F | 88 | Smooth muscle | Low malignant leiomyosarcoma | IA | 100 | 100 | | | | | | | **NEG** | 0 | 0 | 0 | 0 |
| C7 | F | 54 | Smooth muscle | Malignant pleomorphic leiomyosarcoma | IIIB | 100 | 100 | | | | | | | **NEG** | 0 | 0 | 0 | 0 |
| C8 | F | 54 | Smooth muscle | Malignant pleomorphic leiomyosarcoma | IIIB | 100 | 100 | | | | | | | **NEG** | 0 | 0 | 0 | 0 |
| C9 | F | 58 | Smooth muscle | Low malignant leiomyosarcoma | IIB | 100 | 100 | | | | | | | **NEG** | 0 | 0 | 0 | 0 |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| D1 | F | 58 | Smooth muscle | Low malignant leiomyosarcoma | IIB | 100 | 100 | | | | | | | NEG | 0 | 0 | 0 | 0 |
| D2 | F | 57 | Smooth muscle | Low malignant leiomyosarcoma | IB | 100 | 100 | | | | | | | NEG | 0 | 0 | 0 | 0 |
| D3 | F | 57 | Smooth muscle | Low malignant leiomyosarcoma | IB | 100 | 100 | | | | | | | NEG | 0 | 0 | 0 | 0 |
| D4 | M | 78 | Smooth muscle | Moderate malignant leiomyosarcoma | IIA | 100 | 100 | | | | | | | n/a | | | | |
| D5 | M | 78 | Smooth muscle | Moderate malignant leiomyosarcoma | IIA | 100 | 100 | | | | | | | n/a | | | | |
| D6 | F | 52 | Smooth muscle | Moderate malignant leiomyosarcoma | IIA | 100 | 100 | | | | | | | NEG | 0 | 0 | 0 | 0 |
| D7 | F | 52 | Smooth muscle | Moderate malignant leiomyosarcoma | IIA | 100 | 100 | | | | | | | NEG | 0 | 0 | 0 | 0 |
| D8 | M | 61 | Smooth muscle | Moderate malignant leiomyosarcoma | IIA | 100 | 100 | | | | | | | NEG | 0 | 0 | 0 | 0 |
| D9 | M | 61 | Smooth muscle | Moderate malignant leiomyosarcoma (fibrous tissue, blood vessel and smooth muscle) | IIA | 100 | 100 | | | | | | | NEG | 0 | 0 | 0 | 0 |
| E1 | F | 33 | Fibrous tissue | Pleomorphic rhabdomyosarcoma | IIA | 100 | 100 | | | | | | | NEG | 0 | 0 | 0 | 0 |
| E2 | F | 33 | Fibrous tissue | Pleomorphic rhabdomyosarcoma | IIA | 100 | 100 | | | | | | | NEG | 0 | 0 | 0 | 0 |
| E3 | M | 21 | Retroperitoneum | Embryonic rhabdomyosarcoma | IIIA | 100 | 100 | | | | | | | NEG | 0 | 0 | 0 | 0 |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| E4 | M | 21 | Retroperitoneum | Embryonic rhabdomyosarcoma | IIIA | 100 | 100 | | | | | | | NEG | 0 | 0 | 0 | 0 |
| E5 | F | 51 | Uterine cervix | Pleomorphic rhabdomyosarcoma | IIIB | 100 | 100 | | | | | | | NEG | 0 | 0 | 0 | 0 |
| E6 | F | 51 | Uterine cervix | Pleomorphic rhabdomyosarcoma | IIIB | 100 | 100 | | | | | | | NEG | 0 | 0 | 0 | 0 |
| E7 | M | 16 | Testis | Spindle cell rhabdomyosarcoma | IIA | 100 | 100 | | | | | | | NEG | 0 | 0 | 0 | 0 |
| E8 | M | 16 | Testis | Spindle cell rhabdomyosarcoma | IIA | 100 | 100 | | | | | | | NEG | 0 | 0 | 0 | 0 |
| E9 | F | 49 | Uterus | Pleomorphic rhabdomyosarcoma | IIIB | 100 | 100 | | | | | | | NEG | 0 | 0 | 0 | 0 |
| F1 | F | 49 | Uterus | Pleomorphic rhabdomyosarcoma | IIIB | 100 | 100 | | | | | | | NEG | 0 | 0 | 0 | 0 |
| F2 | M | 30 | Striated muscle | Rhabdomyosarcoma | IIB | 100 | 100 | | | | | | | NEG | 0 | 0 | 0 | 0 |
| F3 | M | 30 | Striated muscle | Rhabdomyosarcoma | IIB | 100 | 100 | | | | | | | NEG | 0 | 0 | 0 | 0 |
| F4 | M | 18 | Tongue | Embryonic rhabdomyosarcoma | IIIA | 100 | 100 | | | | | | | NEG | 0 | 0 | 0 | 0 |
| F5 | M | 18 | Tongue | Embryonic rhabdomyosarcoma | IIIA | 100 | 100 | | | | | | | NEG | 0 | 0 | 0 | 0 |
| F6 | M | 91 | Striated muscle | Pleomorphic rhabdomyosarcoma | IIIB | 100 | 100 | | | | | | | NEG | 0 | 0 | 0 | 0 |
| F7 | M | 91 | Striated muscle | Pleomorphic rhabdomyosarcoma | IIIB | 100 | 100 | | | | | | | NEG | 0 | 0 | 0 | 0 |
| F8 | F | 74 | Bladder | Pleomorphic rhabdomyosarcoma | IIIB | 100 | 100 | | | | | | | NEG | 0 | 0 | 0 | 0 |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| F9 | F | 74 | Bladder | Pleomorphic rhabdomyosarcoma | IIIB | 100 | 100 | | | | | | | NEG | 0 | 0 | 0 | 0 |
| G1 | F | 67 | Uterine cervix | Pleomorphic rhabdomyosarcoma | IIIB | 100 | 100 | | | | | | | NEG | 0 | 0 | 0 | 0 |
| G2 | F | 67 | Uterine cervix | Pleomorphic rhabdomyosarcoma | IIIB | 100 | 100 | | | | | | | NEG | 0 | 0 | 0 | 0 |
| G3 | F | 40 | Abdominal cavity | Embryonic rhabdomyosarcoma | IIIB | 100 | 100 | | | | | | | NEG | 0 | 0 | 0 | 0 |
| G4 | F | 40 | Abdominal cavity | Embryonic rhabdomyosarcoma | IIIB | 100 | 100 | | | | | | | NEG | 0 | 0 | 0 | 0 |
| G5 | F | 23 | Pelvic cavity | Embryonic rhabdomyosarcoma | IIIB | 100 | 100 | | | | | | | NEG | 0 | 0 | 0 | 0 |
| G6 | F | 23 | Pelvic cavity | Embryonic rhabdomyosarcoma | IIIB | 100 | 100 | | | | | | | NEG | 0 | 0 | 0 | 0 |
| G7 | M | 50 | Striated muscle | Pleomorphic rhabdomyosarcoma | IIA | 100 | 100 | | | | | | | NEG | 0 | 0 | 0 | 0 |
| G8 | M | 50 | Striated muscle | Pleomorphic rhabdomyosarcoma | IIA | 100 | 100 | | | | | | | NEG | 0 | 0 | 0 | 0 |
| G9 | M | 10 | Striated muscle | Alveolus rhabdomyosarcoma | IIB | 100 | 100 | | | | | | | NEG | 0 | 0 | 0 | 0 |
| H1 | M | 10 | Striated muscle | Alveolus rhabdomyosarcoma | IIB | 100 | 100 | | | | | | | NEG | 0 | 0 | 0 | 0 |
| H2 | F | 32 | Uterine cervix | Pleomorphic rhabdomyosarcoma | IIIB | 100 | 100 | | | | | | | NEG | 0 | 0 | 0 | 0 |
| H3 | F | 32 | Uterine cervix | Pleomorphic rhabdomyosarcoma | IIIB | 100 | 100 | | | | | | | NEG | 0 | 0 | 0 | 0 |
| H4 | M | 48 | Soft tissue | Alveolus rhabdomyosarcoma | IIB | 100 | 100 | | | | | | | NEG | 0 | 0 | 0 | 0 |

(continued)

| | | | | | | Apical & Cytoplasmic Staining (Percent) | | | | | | | | | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | POS/NEG | Cyto | Apical | Cyto | Apical |
| H5 | M | 48 | Soft tissue | Alveolus rhabdomyosarcoma | IIB | 100 | 100 | | | | | | | **NEG** | 0 | 0 | 0 | 0 |
| H6 | M | 40 | Soft tissue | Spindle cell rhabdomyosarcoma | IA | 100 | 100 | | | | | | | **NEG** | 0 | 0 | 0 | 0 |
| H7 | M | 40 | Soft tissue | Spindle cell rhabdomyosarcoma | IA | 100 | 100 | | | | | | | **NEG** | 0 | 0 | 0 | 0 |
| H8 | M | 49 | Testis | Embryonic rhabdomyosarcoma | IIB | 100 | 100 | | | | | | | **NEG** | 0 | 0 | 0 | 0 |
| H9 | M | 49 | Testis | Embryonic rhabdomyosarcoma | IIB | 100 | 100 | | | | | | | **NEG** | 0 | 0 | 0 | 0 |
| I1 | F | 48 | Uterus | Smooth muscle | - | 100 | 100 | | | | | | | **NEG** | 0 | 0 | 0 | 0 |
| I2 | F | 8 | Heart | Cardiac muscle | - | 100 | 100 | | | | | | | **NEG** | 0 | 0 | 0 | 0 |
| I3 | F | 14 | Heart | Cardiac muscle | - | 100 | 100 | | | | | | | **NEG** | 0 | 0 | 0 | 0 |
| n/a | | | Bladder | Normal Bladder | n/a | 100 | 100 | | | | | | | **NEG** | 0 | 0 | 0 | 0 |
| n/a | | | 1007 (GCC29 3); Xenogr aft | Human Embryonic Kidney Xenograft - GCC Transfected | n/a | 100 | 100 | | | | | | | **NEG** | 0 | 0 | 0 | 0 |
| n/a | | | Kidney Cell Line - 293 HEK-GCC#2 | Human Embryonic Kidney Cell Line - GCC Transfected | n/a | 100 | 100 | | | | | | | **NEG** | 0 | 0 | 0 | 0 |
| n/a | | | Kidney Cell Line - 293 [HEK-293] | Human Embryonic Kidney Cell Line | n/a | 100 | 100 | | | | | | | **NEG** | 0 | 0 | 0 | 0 |
| n/a | | | Tissue 1 | Colon Cancer MTB | n/a | 100 | 100 | | | | | | | **NEG** | 0 | 0 | 0 | 0 |
| n/a | | | Tissue 2 | Colon Cancer MTB | n/a | NET | | | | | | | | **n/a** | | | | |
| n/a | | | Tissue 3 | Colon Cancer MTB | n/a | 100 | 100 | | | | | | | **NEG** | 0 | 0 | 0 | 0 |
| n/a | | | Tissue 4 | Colon Cancer MTB | n/a | 100 | 100 | | | | | | | **NEG** | 0 | 0 | 0 | 0 |

(continued)

| | | | | | | Apical & Cytoplasmic Staining (Percent) | | | | | | | | POS/NEG | % POS | % POS | H | H |
|---|---|---|---|---|---|---|---|---|---|---|---|---|---|---|---|---|---|---|
| Core Position | sex | age | Organ/Tissue | Pathology Diagnosis/ Tissue Description | Type | 0C | 0A | 1+ C | 1+ A | 2+ C | 2+ A | 3+ C | 3+ A | | Cyto | Apical | Cyto | Apical |
| n/a | | | Tissue 5 | Colon Cancer MTB | n/a | NET | | | | | | | | **n/a** | | | | |
| n/a | | | Tissue 6 | Colon Cancer MTB | n/a | NET | | | | | | | | **n/a** | | | | |
| n/a | | | Tissue 7 | Colon Cancer MTB | n/a | 100 | 100 | | | | | | | **NEG** | 0 | 0 | 0 | 0 |
| n/a | | | Tissue 8 | Colon Cancer MTB | n/a | 100 | 100 | | | | | | | **NEG** | 0 | 0 | 0 | 0 |

**Table 30A: GCC IHC Staining in Non-Colorectal Cancer Human Clinical Samples from a CRO (QualTek) Tissue Database**

| QualTek Tissue Database Samples | | | | | | | | | | | | | | | | |
|---|---|---|---|---|---|---|---|---|---|---|---|---|---|---|---|---|
| | QML Slide | | Apical & Cytoplasmic Staining (Percent) | | | | | | | | POS / | % POS | % POS | H | H |
| Sample | No. | Grade | 0C | 0A | 1+ C | 1+ A | 2+ C | 2+ A | 3+ C | 3+ A | NEG | Cyto | Apical | Cyto | Apical |
| **Pancreas Ca** | **MIL#7** | **2** | 80 | 100 | 20 | | | | | | **POS** | 20 | 0 | 20 | 0 |
| **Pancreas Ca** | **MIL#7** | **3** | 100 | 100 | | | | | | | **NEG** | 0 | 0 | 0 | 0 |
| **Pancreas Ca** | **MIL#7** | **2** | 60 | 80 | 30 | | 10 | 10 | | 10 | **POS** | 40 | 20 | 50 | 50 |
| **Gastric Adeno** | **MIL#8** | **3** | 60 | 80 | 20 | 0 | 20 | 10 | 10 | 10 | **POS** | 50 | 20 | 90 | 50 |
| **Gastric Adeno** | **MIL#9** | **3** | 100 | 100 | | | | | | | **NEG** | 0 | 0 | 0 | 0 |
| **Gastric Adeno** | **MIIL#10** | **2-3** | 80 | 100 | 20 | | | | | | **POS** | 20 | 0 | 20 | 0 |
| **Esophageal Sq Ca** | **MIL#33** | **2** | 90 | 100 | 10 | | | | | | **POS** | 10 | 0 | 10 | 0 |
| **Esophageal Sq Ca** | **MIL#12** | **2** | 30 | | 50 | | 20 | | | | **POS** | 70 | 0 | 90 | 0 |
| **Esophageal Sq Ca** | **MIL#13** | **2** | 100 | 100 | | | | | | | **NEG** | 0 | 0 | 0 | 0 |

**Table 30B: GCC IHC Staining in Human Clinical Samples of Normal Colon and Colorectal Cancer (Tumor Grade 1, 2, 2&3 or 3) from a CRO (QualTek) Tissue Database**

| QualTek Sample No. | MLNM Sample No. | Sample Type | GCC Apical & Cytoplasmic Staining (Percent) | | | | | | | | | | H-Score | H-Score | Tissue |
|---|---|---|---|---|---|---|---|---|---|---|---|---|---|---|---|
| | | | 0C | 0A | 0.5+ C | 0.5+ A | 1+ C | 1+ A | 2+ C | 2+ A | 3+ C | 3+ A | Cytoplasm | Apical | Grade |
| Q34 | PRGNX000525 | Colon | | | | | | | 100 | 30 | | 70 | 200 | 270 | Normal |
| Q35 | PRGNX000526 | Colon | | | 50 | | 50 | | | | | 100 | 75 | 300 | 2 |
| Q36 | PRGNX000527 | Colon | | | 50 | | 50 | | | 50 | | 50 | 75 | 250 | Normal |
| Q37 | PRGNX000528 | Colon | | | 50 | | 20 | | 30 | | | 100 | 105 | 300 | 2 |
| Q38 | PRGNX000529 | Colon | | | 50 | | 50 | | | 20 | | 80 | 75 | 280 | Normal |
| Q39 | PRGNX000530 | Colon | | | | | 50 | | | 50 | 50 | 50 | 200 | 250 | 3 |
| Q40 | PRGNX000531 | Colon | | | 50 | | 50 | | | 50 | | 50 | 75 | 250 | Normal |
| Q41 | PRGNX000532 | Colon | | 30 | 100 | | | 10 | | 30 | | 30 | 50 | 160 | 2 |
| Q42 | PRGNX000533 | Colon | | | | | 50 | | 50 | 20 | | 80 | 150 | 280 | Normal |
| Q43 | PRGNX000534 | Colon | | | | | 50 | | 50 | 30 | | 70 | 150 | 270 | 2 |
| Q44 | PRGNX000535 | Colon | | 80 | 100 | | | 20 | | | | | 50 | 20 | Normal |
| Q45 | PRGNX000536 | Colon | | | | | 50 | | 50 | | | 100 | 150 | 300 | 2 |
| Q46 | PRGNX000537 | Colon | | | | | | | 100 | 30 | | 70 | 200 | 270 | Normal |
| Q47 | PRGNX000538 | Colon | | | 80 | | 20 | | | | | 100 | 60 | 300 | 2 |
| Q48 | PRGNX000539 | Colon | | | 50 | | 50 | | | 50 | | 50 | 75 | 250 | Normal |
| Q49 | PRGNX000540 | Colon | | | | | | | 100 | 80 | | 20 | 200 | 220 | 2 |
| Q50 | PRGNX000541 | Colon | | | 50 | | 50 | 50 | | 50 | | | 75 | 150 | Normal |
| Q51 | PRGNX000542 | Colon | | | | | | | 100 | | | 100 | 200 | 300 | 2 |
| Q52 | PRGNX000543 | Colon | | | | | 70 | | 30 | | | 100 | 130 | 300 | Normal |
| Q53 | PRGNX000544 | Colon | | | | | 100 | | | | | 100 | 100 | 300 | 2 |
| Q54 | PRGNX000545 | Colon | | | 50 | | 50 | | | | | 100 | 75 | 300 | Normal |
| Q55 | PRGNX000546 | Colon | | 20 | 90 | | 10 | | | 30 | | 50 | 55 | 210 | 2 |
| Q56 | PRGNX000547 | Colon | | | 100 | 50 | | 50 | | | | | 50 | 75 | Normal |

(continued)

| QualTek Sample No. | MLNM Sample No. | Sample Type | GCC Apical & Cytoplasmic Staining (Percent) | | | | | | | | | | H-Score | H-Score | Tissue |
|---|---|---|---|---|---|---|---|---|---|---|---|---|---|---|---|
| | | | 0C | 0A | 0.5+ C | 0.5+ A | 1+ C | 1+ A | 2+ C | 2+ A | 3+ C | 3+ A | Cytoplasm | Apical | Grade |
| Q57 | PRGNX000548 | Colon | | | 40 | | 30 | | 30 | | | 100 | 110 | 300 | 2 |
| Q58 | PRGNX000549 | Colon | | | 100 | | | 30 | | 40 | | 30 | 50 | 200 | Normal |
| Q59 | PRGNX000550 | Colon | | | 30 | | 50 | | 20 | | | 100 | 105 | 300 | 2 |
| Q60 | PRGNX000551 | Colon | | | | | 80 | | 10 | 50 | 10 | 50 | 130 | 250 | Normal |
| Q61 | PRGNX000552 | Colon | 80 | 80 | | | 10 | | 10 | | | 20 | 30 | 60 | 2 & 3 |
| Q62 | PRGNX000553 | Colon | | | 50 | | 50 | | | 50 | | 50 | 75 | 250 | Normal |
| Q63 | PRGNX000554 | Colon | | | | | 20 | | 80 | | | 100 | 180 | 300 | 2 |
| Q64 | PRGNX000555 | Colon | | | | | 100 | | | 50 | | 50 | 100 | 250 | Normal |
| Q65 | PRGNX000556 | Colon | | | | | 90 | | 10 | | | 100 | 110 | 300 | 2 |
| Q66 | PRGNX000557 | Colon | | | 30 | | 50 | | 20 | 50 | | 50 | 105 | 250 | Normal |
| *Q67* | *PRGNX000558* | *Colon* | | | | | | | | | | | | | |
| Q68 | PRGNX000559 | Colon | | | 50 | | 50 | | | | | 100 | 75 | 300 | Normal |
| Q69 | PRGNX000560 | Colon | 50 | | 50 | | | 20 | | 30 | | 50 | 25 | 230 | 2 |
| Q70 | PRGNX000561 | Colon | | | 50 | | 50 | 20 | | 30 | | 50 | 75 | 230 | Normal |
| *Q71* | *PRGNX000562* | *Colon* | | | | | | | | | | | | | |
| Q72 | PRGNX000563 | Colon | | 40 | 70 | | 30 | 20 | | 20 | | 20 | 65 | 120 | Normal |
| Q73 | PRGNX000564 | Colon | | | 100 | | | | | | | 100 | 50 | 300 | 2 |
| Q74 | PRGNX000565 | Colon | | | 50 | | 50 | | | | | 100 | 75 | 300 | Normal |
| Q75 | PRGNX000566 | Colon | | | 100 | | | 10 | | 20 | | 70 | 50 | 260 | 2 |
| Q76 | PRGNX000567 | Colon | | | 50 | | 50 | | | 50 | | 50 | 75 | 250 | Normal |
| Q77 | PRGNX000568 | Colon | 20 | | | | 70 | | 10 | | | 100 | 90 | 300 | 2 |
| Q78 | PRGNX000569 | Colon | | 30 | 100 | | | 20 | | 30 | | 20 | 50 | 140 | Normal |
| Q79 | PRGNX000570 | Colon | | | 100 | | | | | | | 100 | 50 | 300 | 2 |

(continued)

| QualTek Sample No. | MLNM Sample No. | Sample Type | GCC Apical & Cytoplasmic Staining (Percent) | | | | | | | | | | H-Score | H-Score | Tissue |
|---|---|---|---|---|---|---|---|---|---|---|---|---|---|---|---|
| | | | 0C | 0A | 0.5+ C | 0.5+ A | 1+ C | 1+ A | 2+ C | 2+ A | 3+ C | 3+ A | Cytoplasm | Apical | Grade |
| Q80 | PRGNX000571 | Colon | | | | | 50 | | 50 | | | 100 | 150 | 300 | Normal |
| Q81 | PRGNX000572 | Colon | | | 80 | | 20 | | | | | 100 | 60 | 300 | 2 |
| Q82 | PRGNX000573 | Colon | | | 50 | | 50 | | | | | 100 | 75 | 300 | Normal |
| Q83 | PRGNX000574 | Colon | | | 50 | | 50 | | | 20 | | 80 | 75 | 280 | 2 |
| Q84 | PRGNX000575 | Colon | | | | | 100 | | | 20 | | 80 | 100 | 280 | Normal |
| Q85 | PRGNX000576 | Colon | | | 50 | | 20 | | 30 | 20 | | 80 | 105 | 280 | 2 |
| Q86 | PRGNX000577 | Colon | | | 50 | | 50 | | | 50 | | 50 | 75 | 250 | Normal |
| Q87 | PRGNX000578 | Colon | | | 20 | | 60 | | 20 | | | 100 | 110 | 300 | 2 |
| Q88 | PRGNX000579 | Colon | | | | | 50 | | 50 | | | 100 | 150 | 300 | Normal |
| Q89 | PRGNX000580 | Colon | | | | | 50 | | 50 | | | 100 | 150 | 300 | 3 |
| Q90 | PRGNX000581 | Colon | | | 50 | | 50 | | | 50 | | 50 | 75 | 250 | Normal |
| Q91 | PRGNX000582 | Colon | | | 30 | | 50 | | 20 | 20 | | 80 | 105 | 280 | 2 |
| Q92 | PRGNX000583 | Colon | | | 50 | | 50 | | | 50 | | 50 | 75 | 250 | Normal |
| Q93 | PRGNX000584 | Colon | | 20 | 100 | | | 30 | | | | 50 | 50 | 180 | 2 |
| Q94 | PRGNX000585 | Colon | | | 50 | | 50 | | | 20 | | 80 | 75 | 280 | Normal |
| Q95 | *PRGNX000586* | *Colon* | | | | | | | | | | | | | |
| Q96 | PRGNX000587 | Colon | | | 50 | | 50 | | | 50 | | 50 | 75 | 250 | Normal |
| Q97 | PRGNX000588 | Colon | | | | | 20 | | 80 | | | 100 | 180 | 300 | 2 |
| Q98 | PRGNX000589 | Colon | | | 50 | | 50 | | | | | 100 | 75 | 300 | Normal |
| Q99 | PRGNX000590 | Colon | | | 50 | | 30 | | 20 | 50 | | 50 | 95 | 250 | 2 |
| Q100 | PRGNX000591 | Colon | | | 50 | | 50 | | | 50 | | 50 | 75 | 250 | Normal |
| Q101 | PRGNX000592 | Colon | | 20 | 100 | | | | | 50 | | 30 | 50 | 190 | 2 |
| Q102 | PRGNX000593 | Colon | | | 50 | | 50 | | | | | 100 | 75 | 300 | Normal |

(continued)

| QualTek Sample No. | MLNM Sample No. | Sample Type | GCC Apical & Cytoplasmic Staining (Percent) | | | | | | | | | | H-Score | H-Score | Tissue |
|---|---|---|---|---|---|---|---|---|---|---|---|---|---|---|---|
| | | | 0C | 0A | 0.5+ C | 0.5+ A | 1+ C | 1+ A | 2+ C | 2+ A | 3+ C | 3+ A | Cytoplasm | Apical | Grade |
| Q103 | PRGNX000594 | Colon | 100 | 100 | | | | | | | | | 0 | 0 | 3 |
| Q104 | PRGNX000595 | Colon | | | 50 | | 50 | | | 90 | | 10 | 75 | 210 | Normal |
| Q105 | PRGNX000596 | Colon | | | 60 | | 40 | 20 | | 30 | | 50 | 70 | 230 | 2 |
| Q106 | PRGNX000597 | Colon | | | | | | | 100 | | | 100 | 200 | 300 | Normal |
| Q107 | PRGNX000598 | Colon | 10 | 10 | 10 | | 30 | | 30 | 40 | 20 | 50 | 155 | 230 | 2 & 3 |
| Q108 | PRGNX000599 | Colon | | | | | | | 100 | | | 100 | 200 | 300 | Normal |
| Q109 | PRGNX000600 | Colon | 80 | 100 | | | 20 | | | | | | 20 | 0 | 2 |
| Q110 | PRGNX000601 | Colon | | | 50 | | 50 | | | | | 100 | 75 | 300 | Normal |
| Q111 | PRGNX000602 | Colon | 10 | | 20 | | 60 | | 10 | | | 100 | 90 | 300 | 2 |
| Q112 | PRGNX000603 | Colon | | | 50 | | 50 | | | 50 | | 50 | 75 | 250 | Normal |
| Q113 | PRGNX000604 | Colon | | | 50 | | 50 | | | 20 | | 80 | 75 | 280 | 2 |
| Q114 | PRGNX000605 | Colon | | | 50 | | 50 | | | 50 | | 50 | 75 | 250 | Normal |
| Q115 | PRGNX000606 | Colon | | | 50 | | | | 20 | | 30 | 100 | 155 | 300 | 2 |
| Q116 | PRGNX000607 | Colon | | | | | 100 | | | 50 | | 50 | 100 | 250 | Normal |
| Q117 | PRGNX000608 | Colon | | 100 | 70 | | 30 | | | | | | 65 | 0 | 3 |
| Q118 | PRGNX000609 | Colon | | | | | 100 | | | 50 | | 50 | 100 | 250 | Normal |
| Q119 | PRGNX000610 | Colon | | | 30 | | 50 | | 20 | | | 100 | 105 | 300 | 2 |
| Q120 | PRGNX000611 | Colon | | | | | 100 | | | 50 | | 50 | 100 | 250 | Normal |
| Q121 | PRGNX000612 | Colon | | | 30 | | 60 | | 10 | 20 | | 80 | 95 | 280 | 2 |
| Q122 | PRGNX000613 | Colon | | | | | 100 | | | | | 100 | 100 | 300 | Normal |
| Q123 | PRGNX000614 | Colon | | | 30 | | 50 | | 20 | | | 100 | 105 | 300 | 2 |
| Q124 | PRGNX000615 | Colon | | 60 | | | 100 | 20 | | | | 20 | 100 | 80 | Normal |
| Q125 | PRGNX000616 | Colon | | | 100 | 20 | | 20 | | 30 | | 30 | 50 | 180 | 2 |

(continued)

| QualTek Sample No. | MLNM Sample No. | Sample Type | GCC Apical & Cytoplasmic Staining (Percent) | | | | | | | | | | H-Score | H-Score | Tissue |
|---|---|---|---|---|---|---|---|---|---|---|---|---|---|---|---|
| | | | 0C | 0A | 0.5+ C | 0.5+ A | 1+ C | 1+ A | 2+ C | 2+ A | 3+ C | 3+ A | Cytoplasm | Apical | Grade |
| Q126 | PRGNX000617 | Colon | | | | | 100 | | | 30 | | 70 | 100 | 270 | Normal |
| Q127 | PRGNX000618 | Colon | | | | | 80 | | 20 | | | 100 | 120 | 300 | 2 |
| Q128 | PRGNX000619 | Colon | | | 50 | | 50 | 40 | | 40 | | 20 | 75 | 180 | Normal |
| Q129 | PRGNX000620 | Colon | 100 | | | | | | | | | 100 | 0 | 300 | 2 |
| Q130 | PRGNX000621 | Colon | | | | | 80 | | 20 | | | 100 | 120 | 300 | Normal |
| Q131 | PRGNX000622 | Colon | | | 20 | | 30 | | 50 | | | 100 | 140 | 300 | 2 |
| Q132 | PRGNX000623 | Colon | | | | | 70 | | 30 | | | 100 | 130 | 300 | Normal |
| Q133 | PRGNX000624 | Colon | | | | | 70 | | 30 | | | 100 | 130 | 300 | 2 |

**Table 31: Clinical Samples from C260001 Phase I Dose Escalation Study of GCC Targeted Therapeutic MLN0264**

| Subject # | Cancer Type | Sex | Collection Date | Sample Type/ Quantity | Rcv'd Date | Apical & Cytoplasmic Staining (Percent) | | | | | | | | POS /NEG | % POS | % POS | H | H |
|---|---|---|---|---|---|---|---|---|---|---|---|---|---|---|---|---|---|---|
| | | | | | | 0C | 0A | 1+ C | 1+ A | 2+ C | 2+ A | 3+ C | 3+ A | | Cyto | Apical | Cyto | Apical |
| 58001-102** | CRC | M | 03-SEP-2008 | 5 unstained slides | 5/22/2012 | 100 | 0 | 0 | 0 | 0 | 0 | 0 | 100 | **POS** | 0 | 100 | 0 | 300 |
| 58001-103** | CRC | F | 17-FEB-2010 | 5 unstained slides | 5/22/2012 | 100 | 20 | 0 | 30 | 0 | 30 | 0 | 20 | **POS** | 0 | 80 | 0 | 150 |
| 58001-101** | Pancreatic | F | 15-MAY-2012 | 1 paraffin block | 5/22/2012 | 0 | 0 | 0 | 0 | 0 | 0 | 10 0 | 100 | **POS** | 100 | 100 | 300 | 300 |
| 58001-104 | CRC | M | 12-APR-2011 | 5 unstained slides | 6/6/2012 | 10 | 0 | 50 | 0 | 20 | 0 | 20 | 100 | **POS** | 90 | 100 | 150 | 300 |
| 58001-105 | Pancreatic | M | 23-AUG-2006 | 20 unstained slides | 7/3/2012 | 90 | 100 | 10 | 0 | 0 | 0 | 0 | 0 | **POS** | 10 | 0 | 10 | 0 |
| 58002-103 | Esophageal | M | 17-AUG-2010 | 5 unstained slides | 7/3/2012 | 90 | 100 | 0 | 0 | 10 | 0 | 0 | 0 | **POS** | 10 | 0 | 20 | 0 |
| 58002-101 | Pancreatic | M | 20-JAN-2011 | 5 unstained slides | 7/3/2012 | 90 | 100 | 10 | 0 | 0 | 0 | 0 | 0 | **POS** | 10 | 0 | 10 | 0 |
| 58002-102 | CRC | M | 09-AUG-2007 | 1 FFPE block | 7/3/2012 | 100 | 100 | 0 | 0 | 0 | 0 | 0 | 0 | **NEG** | 0 | 0 | 0 | 0 |
| 58002-106 | CRC | F | 22-JUN-2011 | 5 unstained slides | 7/11/2012 | 100 | 100 | 0 | 0 | 0 | 0 | 0 | 0 | **NEG** | 0 | 0 | 0 | 0 |
| 58001-107 | CRC | F | 28-NOV-2008 | 5 unstained slides | 7/24/2012 | 80 | 20 | 20 | 0 | 0 | 20 | 0 | 60 | **POS** | 20 | 80 | 20 | 220 |
| 58001-107 | CRC | F | 28-NOV-2008 | 5 unstained slides | 7/24/2012 | 90 | 10 | 10 | 10 | 0 | 30 | 0 | 50 | **POS** | 10 | 90 | 10 | 220 |
| 58001-107 | CRC | F | 28-NOV-2008 | 5 unstained slides | 7/24/2012 | 40 | 0 | 50 | 0 | 10 | 20 | 0 | 80 | **POS** | 60 | 100 | 70 | 280 |
| 58002-111 | CRC | F | 23-OCT-2009 | 5 unstained slides | 7/25/2012 | 0 | 0 | 100 | 0 | 0 | 0 | 0 | 100 | **POS** | 100 | 100 | 100 | 300 |

(continued)

| Subject # | Cancer Type | Sex | Collection Date | Sample Type/ Quantity | Rcv'd Date | Apical & Cytoplasmic Staining (Percent) | | | | | | | | POS /NEG | % POS | % POS | H | H |
|---|---|---|---|---|---|---|---|---|---|---|---|---|---|---|---|---|---|---|
| | | | | | | 0C | 0A | 1+ C | 1+ A | 2+ C | 2+ A | 3+ C | 3+ A | | Cyto | Apical | Cyto | Apical |
| 58002-112 | CRC | F | 01-DEC-2011 | 5 unstained slides | 7/25/2012 | 0 | 0 | 80 | 0 | 20 | 20 | 0 | 80 | **POS** | 100 | 100 | 120 | 280 |
| 58002-107 | CRC | F | 12-FEB-2007 | 5 unstained slides | 7/26/2012 | 80 | 50 | 10 | 0 | 10 | 50 | 0 | 0 | **POS** | 20 | 50 | 30 | 100 |
| 58002-108 | Gastric | M | 27-JUL-2012 | 5 unstained slides | 7/31/2012 | 20 | 100 | 20 | 0 | 50 | 0 | 10 | 0 | **POS** | 80 | 0 | 150 | 0 |
| 58002-113 | CRC | M | 09-JUL-2008 | 5 unstained slides | 7/31/2012 | 50 | 20 | 30 | 10 | 20 | 20 | 0 | 50 | **POS** | 50 | 80 | 70 | 200 |
| 58001-108 | Esophageal | M | 12-MAY-2010 | 20 unstained slides | 8/8/2012 | 100 | 100 | | | | | | | **NEG** | 0 | 0 | 0 | 0 |
| 58002-114 | CRC | F | 12-SEP-2005 | 5 unstained slides | 8/10/2012 | 100 | | | 10 | | 80 | | 10 | **POS** | 0 | 100 | 0 | 200 |
| 58002-105 | CRC | M | 19-APR-2010 | 1 FFPE block | 8/16/2012 | 10 | | 70 | | 20 | 20 | | 80 | **POS** | 90 | 100 | 110 | 280 |
| 58001-109 | Esophageal | M | 20-SEP-2011 | 6 unstained slides | 8/21/2012 | 100 | 100 | | | | | | | **NEG** | 0 | 0 | 0 | 0 |
| 58002-115 | CRC | M | 24-AUG-2010 | 5 unstained slides | 8/21/2012 | 100 | | | 10 | | 20 | | 70 | **POS** | 0 | 100 | 0 | 260 |
| 58002-109 | Pancreatic | M | 08-AUG-2012 | 5 unstained slides | 8/22/2012 | 20 | 10 | 40 | 30 | 20 | 30 | 20 | 30 | **POS** | 80 | 90 | 140 | 180 |
| 58002-117 | Pancreatic | M | 28-FEB-2011 | 5 unstained slides | 8/22/2012 | 40 | 10 | 40 | 50 | 20 | 30 | | 10 | **POS** | 60 | 90 | 80 | 140 |
| 58002-118 | CRC | M | 22-NOV-2010 | 1 FFPE block | 9/7/2012 | | 10 | | 10 | 70 | 10 | 30 | 70 | **POS** | 100 | 90 | 230 | 240 |
| 58001-110 | Small Intestine | F | 31-AUG-2012 | 1 FFPE block | 9/11/2012 | | 80 | | | 30 | | 70 | 20 | **POS** | 100 | 20 | 270 | 60 |

(continued)

| Subject # | Cancer Type | Sex | Collection Date | Sample Type/ Quantity | Rcv'd Date | Apical & Cytoplasmic Staining (Percent) | | | | | | | | POS /NEG | % POS | % POS | H | H |
|---|---|---|---|---|---|---|---|---|---|---|---|---|---|---|---|---|---|---|
| | | | | | | 0C | 0A | 1+ C | 1+ A | 2+ C | 2+ A | 3+ C | 3+ A | | Cyto | Apical | Cyto | Apical |
| 58002-120 | CRC | F | 12-MAY-2010 | 1 FFPE block | 9/12/2012 | | 70 | 30 | | 40 | | 30 | 30 | **POS** | 100 | 30 | 200 | 90 |
| 58002-121 | Gastric | F | 03-MAY-2012 | 1 FFPE block | 9/12/2012 | 40 | 100 | 10 | | 40 | | 10 | | **POS** | 60 | 0 | 120 | 0 |
| 58002-123 | Pancreatic | F | 20-MAY-2011 | 5 unstained slides | 9/13/2012 | 100 | 80 | | | | | | 20 | **POS** | 0 | 20 | 0 | 60 |
| 51001-101 | CRC | M | 03-AUG-2010 | 5 unstained slides | 9/17/2012 | | | | | 100 | | | 100 | **POS** | 100 | 100 | 200 | 300 |
| 58002-119 | CRC | M | 09-NOV-2005 | 5 unstained slides + 2 H&E's | 9/19/2012 | 40 | 20 | 40 | 20 | 20 | 20 | | 40 | **POS** | 60 | 80 | 80 | 180 |
| 58002-122 | Esophageal | M | 10-MAY-2010 | 1 FFPE block and 2 H&E's | 9/19/2012 | 20 | 100 | 20 | | 30 | | 30 | | **POS** | 80 | 0 | 170 | 0 |
| 51001-102 | CRC | M | 23-NOV-2010 | 5 unstained slides | 10/10/2012 | | | | | 50 | | 50 | 100 | **POS** | 100 | 100 | 250 | 300 |
| 51001-103 | Pancreatic | F | 05-AUG-2009 | 5 unstained slides | 10/15/2012 | 50 | 80 | 50 | | | 10 | | 10 | **POS** | 50 | 20 | 50 | 50 |
| 51001-104 | CRC | F | 24-MAR-2010 | 5 unstained slides | 10/22/2012 | 70 | | 30 | | | 10 | | 90 | **POS** | 30 | 100 | 30 | 290 |
| 58002-116 | CRC | M | 19-OCT-2010 | 5 unstained slides | 10/24/2012 | | | | | 50 | | 50 | 100 | **POS** | 100 | 100 | 250 | 300 |
| 51001-105 | Pancreatic | M | 29-DEC-2010 | 5 unstained slides | 10/24/2012 | 90 | 90 | 10 | 10 | | | | | **POS** | 10 | 10 | 10 | 10 |
| 51001-106 | CRC | F | 17-SEP-2010 | 5 unstained slides | 10/25/2012 | 80 | | 20 | | | 20 | | 80 | **POS** | 20 | 100 | 20 | 280 |

(continued)

| Subject # | Cancer Type | Sex | Collection Date | Sample Type/ Quantity | Rcv'd Date | Apical & Cytoplasmic Staining (Percent) | | | | | | | | POS /NEG | % POS | % POS | H | H |
|---|---|---|---|---|---|---|---|---|---|---|---|---|---|---|---|---|---|---|
| | | | | | | 0C | 0A | 1+ C | 1+ A | 2+ C | 2+ A | 3+ C | 3+ A | | Cyto | Apical | Cyto | Apical |
| 58001-111 | CRC | F | 05-FEB-2005 | 10 unstained slides | 10/31/2012 | 90 | | 10 | 10 | | 10 | | 80 | **POS** | 10 | 100 | 10 | 270 |
| 58003-105 | CRC | F | 09-JUN-2010 | 5 unstained slides | 11/6/2012 | 70 | | | | | | 30 | 100 | **POS** | 30 | 100 | 90 | 300 |
| 51001-107 | CRC | M | 31-AUG-2009 | 5 unstained slides | 11/7/2012 | 80 | | 20 | | | | | 100 | **POS** | 20 | 100 | 20 | 300 |
| 58002-127 | Gastric | F | 03-AUG-2011 | 5 unstained slides | 11/8/2012 | 100 | 100 | | | | | | | **NEG** | 0 | 0 | 0 | 0 |
| 58003-101 | CRC | F | 13-SEP-2010 | 1 paraffin block | 11/8/2012 | | 10 | 70 | 10 | 30 | 10 | | 70 | **POS** | 100 | 90 | 130 | 240 |
| 58003-107 | Pancreatic | F | 29-FEB-2012 | 5 unstained slides | 11/8/2012 | 100 | 100 | | | | | | | **NEG** | 0 | 0 | 0 | 0 |
| 58003-106 | CRC | F | 25-JUN-2009 | 5 unstained slides | 11/9/2012 | 100 | | | 10 | | 10 | | 80 | **POS** | 0 | 100 | 0 | 270 |
| 58003-108 | CRC | F | 28-JUL-2011 | 5 unstained slides | 11/9/2012 | 100 | | | | | 20 | | 80 | **POS** | 0 | 100 | 0 | 280 |
| 51001-108 | CRC | M | UK-UK-UK | 5 unstained slides | 11/9/2012 | | | 30 | 0 | 30 | 30 | 40 | 70 | **POS** | 100 | 100 | 210 | 270 |
| 51001-109 | CRC | M | 09-OCT-2007 | 5 unstained slides | 11/12/2012 | 70 | | 30 | | | 30 | | 70 | **POS** | 30 | 100 | 30 | 270 |
| 58003-103 | Esophageal | M | 09-MAR-2011 | 5 unstained slides | 11/13/2012 | 100 | 100 | | | | | | | **NEG** | 0 | 0 | 0 | 0 |
| 58001-112 | CRC | M | 11-JUN-2012 | 20 unstained slides | 11/13/2012 | 30 | | 30 | | 30 | | 10 | 100 | **POS** | 70 | 100 | 120 | 300 |

(continued)

| Subject # | Cancer Type | Sex | Collection Date | Sample Type/ Quantity | Rcv'd Date | Apical & Cytoplasmic Staining (Percent) | | | | | | | | POS /NEG | % POS | % POS | H | H |
|---|---|---|---|---|---|---|---|---|---|---|---|---|---|---|---|---|---|---|
| | | | | | | 0C | 0A | 1+ C | 1+ A | 2+ C | 2+ A | 3+ C | 3+ A | | Cyto | Apical | Cyto | Apical |
| 58003-110 | CRC | F | 30-MAY-2012 | 5 unstained slides | 11/14/2012 | | 100 | | | 30 | | 70 | | POS | 100 | 0 | 270 | 0 |
| 58003-102 | CRC | M | 17-MAR-2010 | 1 paraffin block | 11/16/2012 | 20 | | 30 | 10 | 40 | 20 | 10 | 70 | POS | 80 | 100 | 140 | 260 |
| 58002-126 | Pancreatic | F | 04-AUG-2011 | 5 unstained slides | 11/16/2012 | | | 30 | | 70 | | | | POS | 100 | | 170 | |
| 58003-112 | Esophageal | M | 21-OCT-2011 | 5 unstained slides | 11/29/2012 | 40 | 100 | 50 | | 10 | | | | POS | 60 | 0 | 70 | 0 |
| 58001-114 | Pancreatic | M | 30-NOV-2011 | 5 unstained slides | 11/29/2012 | 100 | 100 | | | | | | | NEG | 0 | 0 | 0 | 0 |
| 58003-104 | Pancreatic | M | 16-MAR-2012 | 5 unstained slides | 12/3/2012 | 20 | 100 | 40 | | 20 | | 20 | | POS | 80 | 0 | 140 | 0 |
| 51001-110 | CRC | F | 10-FEB-2009 | 5 unstained slides | 12/3/2012 | | 20 | 70 | 10 | 30 | 20 | | 50 | POS | 100 | 80 | 130 | 200 |
| 51001-111 | CRC | M | 16-SEP-2009 | 5 unstained slides | 12/7/2012 | | 30 | 100 | 10 | | 10 | | 50 | POS | 100 | 70 | 100 | 180 |
| 58003-113 | Gastric | F | 07-FEB-2011 | 5 unstained slides | 12/13/2012 | 100 | 100 | | | | | | | NEG | 0 | 0 | 0 | 0 |
| 58001-113 | Esophageal | F | 06-JUN-2012 | 5 unstained slides | 12/21/2012 | | | 100 | | | | | | POS | 100 | | 100 | |
| 58002-128 | Pancreatic | M | 09-NOV-2011 | 1 paraffin block | 12/21/2012 | | | | | | | | | | | | | |
| 58002-130 | Pancreatic | M | 10-DEC-2008 | 1 paraffin block | 12/21/2012 | | | 50 | | 50 | | | 100 | POS | 100 | 100 | 150 | 300 |
| 58002-130 | Pancreatic | M | 10-DEC-2008 | 1 paraffin block | 12/21/2012 | | | 50 | | 50 | 25 | | 75 | POS | 100 | 100 | 150 | 275 |

(continued)

| Subject # | Cancer Type | Sex | Collection Date | Sample Type/ Quantity | Rcv'd Date | Apical & Cytoplasmic Staining (Percent) | | | | | | | | POS /NEG | % POS | % POS | H | H |
|---|---|---|---|---|---|---|---|---|---|---|---|---|---|---|---|---|---|---|
| | | | | | | 0C | 0A | 1+ C | 1+ A | 2+ C | 2+ A | 3+ C | 3+ A | | Cyto | Apical | Cyto | Apical |
| 58002-130 | Pancreatic | M | 10-DEC-2008 | 1 paraffin block | 12/21/2012 | | | 50 | | 50 | | | 100 | **POS** | 100 | 100 | 150 | 300 |
| 58002-130 | Pancreatic | M | 13-APR-2011 | 1 paraffin block | 12/21/2012 | | | 25 | | 75 | 25 | | 75 | **POS** | 100 | 100 | 175 | 275 |
| 58003-114 | CRC | M | 29-MAR-2010 | 5 unstained slides | 12/26/2012 | 25 | | 50 | 25 | 25 | 25 | | 50 | **POS** | 75 | 100 | 100 | 225 |
| 58003-117 | CRC | F | 20-MAY-2011 | 5 unstained slides | 12/28/2012 | | 100 | 70 | | 30 | | | | **POS** | 100 | 0 | 130 | 0 |
| 58001-116 | Pancreatic | M | 26-JAN-2006 | 5 unstained slides | 1/4/2013 & 1/18/2013 ** | 100 | 100 | | | | | | | **NEG** | 0 | 0 | 0 | 0 |
| 58001-115 | Esophageal | M | 14-DEC-2010 | 5 unstained slides | 1/9/2013 | 50 | | 40 | | 10 | | | 100 | **POS** | 50 | 100 | 60 | 300 |
| 58003-119 | Esophageal | M | 27-DEC-2011 | 9 unstained slides | 1/14/2013 | 70 | 10 | 30 | | | 10 | | 80 | **POS** | 30 | 90 | 30 | 260 |
| 58002-132 | Esophageal | M | 23-NOV-2010 | 5 unstained slides & 1 FFPE block | 1/16/2013 | | 50 | 40 | | 30 | 20 | 30 | 30 | **POS** | 100 | 50 | 190 | 130 |
| 51001-112 | Gastric | M | 26-JAN-2009 | 5 unstained slides | 1/16/2013 | 70 | 70 | 10 | | 20 | | | 30 | **POS** | 30 | 30 | 50 | 90 |
| 58003-115 | CRC | M | 08-SEP-2009 | 5 unstained slides | 1/17/2013 | 20 | | 80 | | | | | 100 | **POS** | 80 | 100 | 80 | 300 |
| 58003-120 | Esophageal | M | 15-NOV-2011 | 5 unstained slides | 1/25/2013 | 10 | 10 | 60 | 10 | 30 | 10 | | 70 | **POS** | 90 | 90 | 120 | 240 |
| 51001-113 | CRC | M | 20-JUL-2009 | 5 unstained slides | 1/28/2013 | 50 | | 50 | | | | | 100 | **POS** | 50 | 100 | 50 | 300 |
| 51001-114 | Pancreatic | F | 08-NOV-2007 | 5 unstained slides | 1/28/2013 | 80 | 70 | 20 | 20 | | 10 | | | **POS** | 20 | 30 | 20 | 40 |

(continued)

| Subject # | Cancer Type | Sex | Collection Date | Sample Type/ Quantity | Rcv'd Date | Apical & Cytoplasmic Staining (Percent) | | | | | | | | POS /NEG | % POS | % POS | H | H |
|---|---|---|---|---|---|---|---|---|---|---|---|---|---|---|---|---|---|---|
| | | | | | | 0C | 0A | 1+ C | 1+ A | 2+ C | 2+ A | 3+ C | 3+ A | | Cyto | Apical | Cyto | Apical |
| 58001-117 | Gastric | M | 15-SEP-2012 | 5 unstained slides | 1/29/2013 | | 80 | 90 | | 10 | 10 | | 10 | **POS** | 100 | 20 | 110 | 50 |
| 58003-118 | Gastric | M | 28-MAY-2010 | 5 unstained slides | 2/4/2013 | 100 | | | 10 | | 40 | | 50 | **POS** | 0 | 100 | 0 | 240 |
| 58003-123 | CRC | M | 01-NOV-2011 | 5 unstained slides | 2/6/2013 | | | 30 | | 70 | | | 100 | **POS** | 100 | 100 | 170 | 300 |
| 58003-121 | Pancreatic | M | 07-SEP-2012 | 5 unstained slides | 2/6/2013 | 100 | 95 | | | | 5 | | | **POS** | 0 | 5 | 0 | 10 |
| 58003-125 | Pancreatic | M | 04-SEP-2012 | 5 unstained slides | 2/11/2013 | | 100 | 100 | | | | | | **POS** | 100 | 0 | 100 | 0 |
| 58003-127 | Intestinal | M | 11-APR-2011 | 5 unstained slides | 2/11/2013 | | 20 | 100 | | | | | 80 | **POS** | 100 | 80 | 100 | 240 |
| 58002-135 | | F | 19-DEC-2012 | 5 unstained slides | 2/14/2013 | 100 | 20 | | 30 | | 50 | | | **POS** | 0 | 80 | 0 | 130 |
| 58001-119 | Gastric | M | 29-OCT - 2012 | 5 unstained slides | 2/15/2013 | 100 | 95 | | | | 5 | | | **POS** | 0 | 5 | 0 | 10 |
| 58001-120 | CRC | F | 22-MAY-2012 | 16 unstained slides | 2/15/2013 | 100 | 20 | | | | 40 | | 40 | **POS** | 0 | 80 | 0 | 200 |
| 58003-126 | CRC | M | 29-DEC-2010 | 5 unstained slides | 2/18/2013 | 70 | | 30 | | | | | 100 | **POS** | 30 | 100 | 30 | 300 |
| 58003-129 | Pancreatic | M | 23-MAR-2012 | 5 unstained slides | 2/18/2013 | | 90 | 100 | 10 | | | | | **POS** | 100 | 10 | 100 | 10 |
| 58003-131 | CRC | M | 10-MAY-2012 | 5 unstained slides | 2/18/2013 | 100 | 100 | | | | | | | **NEG** | 0 | 0 | 0 | 0 |
| 58002-133 | Pancreatic | M | 03-MAR-2011 | 5 unstained slides | 2/20/2013 | | | | | | | | | | | | | |

(continued)

| Subject # | Cancer Type | Sex | Collection Date | Sample Type/ Quantity | Rcv'd Date | Apical & Cytoplasmic Staining (Percent) | | | | | | | | POS /NEG | % POS | % POS | H | H |
|---|---|---|---|---|---|---|---|---|---|---|---|---|---|---|---|---|---|---|
| | | | | | | 0C | 0A | 1+ C | 1+ A | 2+ C | 2+ A | 3+ C | 3+ A | | Cyto | Apical | Cyto | Apical |
| 58002-133 | Pancrea tic | M | 03-MAR-2011 | 5 unstained slides | 2/20/2013 | | | | | | | | | | | | | |
| 58003-122 | CRC | M | 24-MAR-2009 | 5 unstained slides | 2/25/2013 | | | 90 | | 10 | | | 100 | **POS** | 100 | 100 | 110 | 300 |
| 58003-128 | Pancrea tic | M | 21-DEC-2011 | 5 unstained slides | 2/25/2013 | 100 | 100 | | | | | | | **NEG** | 0 | 0 | 0 | 0 |
| 58001-121 | CRC | F | 22-JUN-2010 | 20 unstained slides | 2/25/2013 | 100 | 90 | | 10 | | | | | **POS** | 0 | 10 | 0 | 10 |
| 58001-115 | Esopha geal | F | 25-FEB-2011 | 5 unstained slides | 2/25/2013 | 100 | 55 | | 30 | | 10 | | 5 | **POS** | 0 | 45 | 0 | 65 |
| 58003-124 | Gastric | M | 07-JUN-2012 | 5 unstained slides | 2/28/2013 | | 100 | 20 | | 20 | | 60 | | **POS** | 100 | 0 | 240 | 0 |
| 58002-133 | Pancrea tic | M | 03-MAR-2011 | 10 unstained slides | 2/28/2013 | | | | | | | | | | | | | |
| 58002-129 | Esopha geal | M | 20-MAR-2012 | 5 unstained slides | 2/28/2013 | | | 70 | | 30 | | | 100 | **POS** | 100 | 100 | 130 | 300 |
| 58002-129 | Esopha geal | M | 08-MAR-2012* | 5 unstained slides | 2/28/2013 | | 70 | 80 | | 20 | | | 30 | **POS** | 100 | 30 | 120 | 90 |
| 58002-137 | | F | 03-OCT-2011 | 1 paraffin block | 3/1/2013 | 20 | 100 | 40 | | 30 | | 10 | | **POS** | 80 | 0 | 130 | 0 |

**Table 32: GCC IHC Expression in Primary and Metastatic Colorectal Tumor Samples**

| Sample # | Slide # | Apical & Cytoplasmic Staining (Percent) | | | | | | | | Comments | POS /NEG | % POS | % POS | H | H |
|---|---|---|---|---|---|---|---|---|---|---|---|---|---|---|---|
| | | 0C | 0A | 1+ C | 1+ A | 2+ C | 2+ A | 3+ C | 3+ A | | | Cyto | Apical | Cyto | Apical |
| 1A | 1 | | | 20 | | 80 | | | 100 | | POS | 100 | 100 | 180 | 300 |
| 1B | 2 | | | 80 | | 20 | | | 100 | | POS | 100 | 100 | 120 | 300 |
| 2A | 3 | | 50 | 100 | 20 | | 20 | | 10 | | POS | 100 | 50 | 100 | 90 |
| 2B | 4 | | 100 | 60 | | 40 | | | | | POS | 100 | 0 | 140 | 0 |
| 3A | 5 | | | 20 | | 80 | | | 100 | | POS | 100 | 100 | 180 | 300 |
| 3B | 6 | | | 80 | | 20 | | | 100 | | POS | 100 | 100 | 120 | 300 |
| 4A | 7 | 50 | | 50 | 50 | | 20 | | 30 | | POS | 50 | 100 | 50 | 180 |
| 4B | 8 | | 50 | 100 | 20 | | 30 | | | | POS | 100 | 50 | 100 | 80 |
| 5A | 9 | | | 80 | | 20 | 20 | | 80 | | POS | 100 | 100 | 120 | 280 |
| 5B | 10 | 50 | 40 | 30 | 20 | 20 | 20 | | 20 | | POS | 50 | 60 | 70 | 120 |
| 6A | 11 | | | 80 | 10 | 20 | 30 | | 60 | | POS | 100 | 100 | 120 | 250 |
| 6B | 12 | | | 50 | 10 | 50 | 20 | | 70 | | POS | 100 | 100 | 150 | 260 |
| 7A | 13 | 30 | | 50 | | 20 | 20 | | 80 | | POS | 70 | 100 | 90 | 280 |
| 7B | 14 | 30 | | 50 | | 20 | 20 | | 80 | | POS | 70 | 100 | 90 | 280 |
| 8A | 15 | | | | | 100 | | | 100 | | POS | 100 | 100 | 200 | 300 |
| 8B | 16 | | | 50 | | 50 | | | 100 | | POS | 100 | 100 | 150 | 300 |
| 9A | 17 | | 100 | | | 50 | | 50 | | High grade - staining in vacuoles | POS | 100 | 0 | 250 | 0 |
| 9B | 18 | | 60 | 100 | 20 | | 20 | | | | POS | 100 | 40 | 100 | 60 |
| 10A | 19 | | | 80 | | 20 | | | 100 | | POS | 100 | 100 | 120 | 300 |
| 10B | 20 | | | 50 | | 50 | | | 100 | | POS | 100 | 100 | 150 | 300 |
| 11A | 21 | 50 | 10 | 50 | 30 | | 30 | | 30 | | POS | 50 | 90 | 50 | 180 |
| 11B | 22 | 50 | 60 | 50 | 20 | | 10 | | 10 | | POS | 50 | 40 | 50 | 70 |
| 12A | 23 | | | 80 | | 20 | 50 | | 50 | | POS | 100 | 100 | 120 | 250 |

(continued)

| Sample # | Slide # | Apical & Cytoplasmic Staining (Percent) | | | | | | | | Comments | POS /NEG | % POS | % POS | H | H |
|---|---|---|---|---|---|---|---|---|---|---|---|---|---|---|---|
| | | 0C | 0A | 1+C | 1+ A | 2+C | 2+ A | 3+C | 3+A | | | Cyto | Apical | Cyto | Apical |
| 12B | 24 | | | | 20 | 10 | 30 | 90 | 50 | **High grade - staining in vacuoles mainly - a few lumens present** | **POS** | 100 | 100 | 290 | 230 |
| 13A | 25 | 50 | 20 | 50 | 10 | | 20 | | 50 | | **POS** | 50 | 80 | 50 | 200 |
| 13B | 26 | | 80 | 100 | 10 | | 10 | | | | **POS** | 100 | 20 | 100 | 30 |
| 14A | 27 | | | 80 | | 20 | | | 100 | **Signet Ring cell component not scored** | **POS** | 100 | 100 | 120 | 300 |
| 14B | 28 | | | 50 | | 50 | | | 100 | | **POS** | 100 | 100 | 150 | 300 |
| 15A | 29 | 50 | 50 | 40 | 10 | 10 | 30 | | 10 | | POS | 50 | 50 | 60 | 100 |
| 15B | 31 | 50 | 100 | 50 | | | | | | | POS | 50 | 0 | 50 | 0 |
| 16A | 32 | 60 | 20 | 30 | 20 | 10 | 30 | | 30 | **Mucinous** | POS | 40 | 80 | 50 | 170 |
| 16B | 33 | | | 70 | | 30 | | | 100 | | POS | 100 | 100 | 130 | 300 |
| 17A | 34 | | | 90 | | 10 | | | 100 | | POS | 100 | 100 | 110 | 300 |
| 17B | 35 | 50 | 20 | 50 | 20 | | 20 | | 40 | | POS | 50 | 80 | 50 | 180 |
| 18A | 36 | | | 70 | 20 | 30 | 30 | | 50 | | POS | 100 | 100 | 130 | 230 |
| 18B | 37 | 10 | 50 | 80 | 10 | 10 | 20 | | 20 | | POS | 90 | 50 | 100 | 110 |
| 19A | 38 | | | 80 | | 20 | | | 100 | | POS | 100 | 100 | 120 | 300 |
| 19B | 39 | 70 | 70 | 30 | | | 20 | | 10 | | POS | 30 | 30 | 30 | 70 |
| 20A | 40 | 80 | 100 | 20 | | | | | | **Neuroendocrine** | POS | 20 | 0 | 20 | 0 |
| 20B | 41 | 50 | 90 | 20 | | 30 | 10 | | | | POS | 50 | 10 | 80 | 20 |
| 21A | 42 | 50 | | 50 | | | | | 100 | | POS | 50 | 100 | 50 | 300 |
| 21B | 43 | NET | | | | | | | | **No Evidence of Tumor** | | | | | |
| 22A | 44 | 60 | 10 | 30 | 10 | 10 | 20 | | 60 | | POS | 40 | 90 | 50 | 230 |
| 22B | 45 | | 10 | 100 | | | 10 | | 80 | | POS | 100 | 90 | 100 | 260 |

(continued)

| Sample # | Slide # | Apical & Cytoplasmic Staining (Percent) | | | | | | | | Comments | POS /NEG | % POS | % POS | H | H |
|---|---|---|---|---|---|---|---|---|---|---|---|---|---|---|---|
| | | 0C | 0A | 1+C | 1+ A | 2+C | 2+ A | 3+C | 3+A | | | Cyto | Apical | Cyto | Apical |
| 23A | 46 | 50 | 10 | 50 | | | | | 90 | | POS | 50 | 90 | 50 | 270 |
| 23B | 47 | | | 70 | | 30 | | | 100 | | POS | 100 | 100 | 130 | 300 |
| 24A | 48 | | 10 | 80 | | 20 | 40 | | 50 | | POS | 100 | 90 | 120 | 230 |
| 24B | 49 | 80 | 40 | 10 | | 10 | 30 | | 30 | **Only small amt of tumor** | **POS** | 20 | 60 | 30 | 150 |
| 25A | 50 | 50 | 30 | 50 | 20 | | 20 | | 30 | | **POS** | 50 | 70 | 50 | 150 |
| 25B | 51 | | | 80 | | 20 | | | 100 | | **POS** | 100 | 100 | 120 | 300 |
| 26A | 52 | | | 80 | | 20 | 50 | | 50 | | **POS** | 100 | 100 | 120 | 250 |
| 26B | 53 | Net | | | | | | | | **No Evidence of Tumor** | | | | | |
| 27A | 54 | 40 | 70 | 20 | 10 | 20 | 10 | 20 | 10 | | **POS** | 60 | 30 | 120 | 60 |
| 27B | 55 | | | 60 | | 20 | 30 | 20 | 70 | | **POS** | 100 | 100 | 160 | 270 |
| 28A | 56 | | | 50 | | 50 | | | 100 | | **POS** | 100 | 100 | 150 | 300 |
| 28B | 57 | | | 30 | | 50 | | 20 | 100 | | **POS** | 100 | 100 | 190 | 300 |
| 29A | 58 | | | 50 | | 50 | | | 100 | | **POS** | 100 | 100 | 150 | 300 |
| 29B | 59 | 50 | | 40 | 20 | 10 | 30 | | 50 | | **POS** | 50 | 100 | 60 | 230 |
| 30A | 61 | 10 | | 70 | | 20 | 20 | | 80 | | **POS** | 90 | 100 | 110 | 280 |
| 30B | 62 | | | 50 | | 50 | 50 | | 50 | | **POS** | 100 | 100 | 150 | 250 |
| 31A | 63 | | | | | 50 | | 50 | 100 | | **POS** | 100 | 100 | 250 | 300 |
| 31B | 64 | | | 50 | | 50 | 50 | | 50 | | **POS** | 100 | 100 | 150 | 250 |
| 32A | 65 | | | 80 | | 20 | | | 100 | | **POS** | 100 | 100 | 120 | 300 |
| 32B | 66 | 50 | 50 | 50 | | | | | 50 | | **POS** | 50 | 50 | 50 | 150 |
| 33A | 67 | | | 80 | | 20 | 50 | | 50 | | **POS** | 100 | 100 | 120 | 250 |
| 33B | 68 | | | 20 | | 80 | | | 100 | | **POS** | 100 | 100 | 180 | 300 |
| 34A | 69 | | | 100 | | | 30 | | 70 | | **POS** | 100 | 100 | 100 | 270 |

(continued)

| Sample # | Slide # | Apical & Cytoplasmic Staining (Percent) | | | | | | | | Comments | POS /NEG | % POS | % POS | H | H |
|---|---|---|---|---|---|---|---|---|---|---|---|---|---|---|---|
| | | 0C | 0A | 1+C | 1+ A | 2+C | 2+ A | 3+C | 3+A | | | Cyto | Apical | Cyto | Apical |
| 34B | 70 | | | 100 | 20 | | 40 | | 40 | | POS | 100 | 100 | 100 | 220 |
| 35A | 71 | | | 70 | | 20 | | 10 | 100 | | POS | 100 | 100 | 140 | 300 |
| 35B | 72 | | | 70 | | 20 | | 10 | 100 | | POS | 100 | 100 | 140 | 300 |
| 36A | 73 | | | 50 | 40 | 40 | 50 | 10 | 10 | | POS | 100 | 100 | 160 | 170 |
| 36B | 74 | | | 30 | 40 | 40 | 30 | 30 | 30 | | POS | 100 | 100 | 200 | 190 |
| 37A | 75 | | 20 | 100 | 10 | | 20 | | 50 | | POS | 100 | 80 | 100 | 200 |
| 37B | 76 | 50 | 90 | 50 | | | | | 10 | | POS | 50 | 10 | 50 | 30 |
| 38A | 77 | 30 | | 70 | | | 50 | | 50 | | POS | 70 | 100 | 70 | 250 |
| 38B | 78 | 80 | | 20 | | | | | 100 | | POS | 20 | 100 | 20 | 300 |
| 39A | 79 | | | 100 | | | 50 | | 50 | | POS | 100 | 100 | 100 | 250 |
| 39B | 80 | | | 100 | | | | | 100 | | POS | 100 | 100 | 100 | 300 |
| 40A | 81 | | | 40 | | 60 | 20 | | 80 | | POS | 100 | 100 | 160 | 280 |
| 40B | 82 | NET | | | | | | | | No Evidence of Tumor | | | | | |
| 41A | 83 | 20 | 60 | 80 | 20 | | 10 | | 10 | | POS | 80 | 40 | 80 | 70 |
| 41B | 84 | 50 | 40 | 50 | 20 | | 20 | | 20 | | POS | 50 | 60 | 50 | 120 |
| 42A | 85 | | 10 | 80 | 10 | 20 | 40 | | 40 | | POS | 100 | 90 | 120 | 210 |
| 42B | 86 | | 10 | 80 | 10 | 20 | 30 | | 50 | | POS | 100 | 90 | 120 | 220 |
| 43A | 87 | | 10 | 30 | 30 | 50 | 30 | 20 | 30 | | POS | 100 | 90 | 190 | 180 |
| 43B | 88 | | | 50 | | 40 | 30 | 10 | 70 | | POS | 100 | 100 | 160 | 270 |
| 44A | 89 | | | 80 | | 20 | | | 100 | | POS | 100 | 100 | 120 | 300 |
| 44B | 91 | 60 | 100 | 30 | | 10 | | | | | POS | 40 | 0 | 50 | 0 |
| 45A | 92 | | | 30 | | 50 | | 20 | 100 | | POS | 100 | 100 | 190 | 300 |
| 45B | 93 | | | 70 | | 20 | | 10 | 100 | Golgi-like staining in cytoplasm | POS | 100 | 100 | 140 | 300 |

(continued)

| Sample # | Slide # | Apical & Cytoplasmic Staining (Percent) | | | | | | | | Comments | POS /NEG | % POS | % POS | H | H |
|---|---|---|---|---|---|---|---|---|---|---|---|---|---|---|---|
| | | 0C | 0A | 1+C | 1+ A | 2+C | 2+ A | 3+C | 3+A | | | Cyto | Apical | Cyto | Apical |
| 46A | 94 | 90 | 90 | 10 | 10 | | | | | | POS | 10 | 10 | 10 | 10 |
| 46B | 95 | 20 | 80 | 80 | | | 10 | | 10 | | POS | 80 | 20 | 80 | 50 |
| 47A | 96 | 50 | 100 | 50 | | | | | | Adenoma-no tumor | POS | 50 | 0 | 50 | 0 |
| 47B | 97 | | 40 | 80 | 20 | 20 | 20 | | 20 | | POS | 100 | 60 | 120 | 120 |
| 48A | 98 | 30 | | 70 | | | 20 | | 80 | | POS | 70 | 100 | 70 | 280 |
| 48B | 99 | 90 | | 10 | | | 20 | | 80 | | POS | 10 | 100 | 10 | 280 |
| 49A | 100 | 30 | | 40 | | 30 | 30 | | 70 | | POS | 70 | 100 | 100 | 270 |
| 49B | 101 | 30 | 70 | 50 | | 20 | | | 30 | | POS | 70 | 30 | 90 | 90 |
| 50A | 102 | | 10 | 90 | 30 | 10 | | | 60 | | POS | 100 | 90 | 110 | 210 |
| 50B | 103 | 100 | 70 | | 10 | | | | 20 | | POS | 0 | 30 | 0 | 70 |
| 51A | 104 | 50 | 80 | 50 | | | 20 | | | | POS | 50 | 20 | 50 | 40 |
| 51B | 105 | NET | | | | | | | | No Evidence of Tumor | | | | | |
| 52A | 106 | 50 | 90 | 50 | | | 10 | | | | POS | 50 | 10 | 50 | 20 |
| 52B | 107 | 90 | 90 | 10 | 10 | | | | | | POS | 10 | 10 | 10 | 10 |
| 53A | 108 | | 40 | 80 | 20 | 20 | 20 | | 20 | | POS | 100 | 60 | 120 | 120 |
| 53B | 109 | 50 | 80 | 50 | 20 | | | | | | POS | 50 | 20 | 50 | 20 |
| 54A | 110 | | 50 | 70 | 20 | 30 | 20 | | 10 | | POS | 100 | 50 | 130 | 90 |
| 54B | 111 | | 20 | 80 | | 20 | 20 | | 60 | | POS | 100 | 80 | 120 | 220 |
| 55A | 112 | 100 | 100 | | | | | | | | NEG | 0 | 0 | 0 | 0 |
| 55B | 113 | 100 | 100 | | | | | | | | NEG | 0 | 0 | 0 | 0 |
| 56A | 114 | 50 | 70 | 50 | 30 | | | | | | POS | 50 | 30 | 50 | 30 |
| 56B | 115 | 100 | 100 | | | | | | | | NEG | 0 | 0 | 0 | 0 |
| 57A | 116 | 50 | 70 | 50 | 10 | | 10 | | 10 | | POS | 50 | 30 | 50 | 60 |

(continued)

| Sample # | Slide # | Apical & Cytoplasmic Staining (Percent) | | | | | | | | Comments | POS /NEG | % POS | % POS | H | H |
|---|---|---|---|---|---|---|---|---|---|---|---|---|---|---|---|
| | | 0C | 0A | 1+C | 1+ A | 2+C | 2+ A | 3+C | 3+A | | | Cyto | Apical | Cyto | Apical |
| 57B | 117 | | 80 | 50 | | 50 | 10 | | 10 | **Small amount of tumor** | **POS** | 100 | 20 | 150 | 50 |
| 58A | 118 | 10 | | 80 | 20 | 10 | 40 | | 40 | | **POS** | 90 | 100 | 100 | 220 |
| 58B | 119 | | 40 | 100 | 20 | | 20 | | 20 | **Small amount of tumor** | **POS** | 100 | 60 | 100 | 120 |
| 59A | 121 | | | 70 | | 30 | 30 | | 70 | | **POS** | 100 | 100 | 130 | 270 |
| 59B | 122 | | | 30 | | 70 | | | 100 | | **POS** | 100 | 100 | 170 | 300 |
| 60A | 123 | 50 | 70 | 50 | 10 | | 10 | | 10 | | **POS** | 50 | 30 | 50 | 60 |
| 60B | 124 | 10 | 30 | 70 | 20 | 20 | 20 | | 30 | | **POS** | 90 | 70 | 110 | 150 |
| 61A | 125 | 50 | | 30 | | 20 | 50 | | 50 | | **POS** | 50 | 100 | 70 | 250 |
| 61B | 126 | 100 | | | 20 | | 30 | | 50 | | **POS** | 0 | 100 | 0 | 230 |
| 62A | 127 | | 80 | 100 | | | 10 | | 10 | | **POS** | 100 | 20 | 100 | 50 |
| 62B | 128 | | 90 | 80 | | 20 | 10 | | | | **POS** | 100 | 10 | 120 | 20 |
| 63A | 129 | 10 | 50 | 80 | | 10 | 20 | | 30 | | **POS** | 90 | 50 | 100 | 130 |
| 63B | 130 | | 100 | 90 | | 10 | | | | | **POS** | 100 | 0 | 110 | 0 |
| 64A | 131 | | 90 | 80 | | 20 | 10 | | | | **POS** | 100 | 10 | 120 | 20 |
| 64B | 132 | | 60 | 70 | 10 | 30 | 20 | | 10 | | **POS** | 100 | 40 | 130 | 80 |
| 65A | 133 | 50 | 90 | 50 | | | 10 | | | | **POS** | 50 | 10 | 50 | 20 |
| 65B | 134 | 70 | 100 | 30 | | | | | | **Tumor high Grade** | **POS** | 30 | 0 | 30 | 0 |
| 66A | 135 | | 30 | 80 | | 20 | 50 | | 20 | | **POS** | 100 | 70 | 120 | 160 |
| 66B | 136 | | 100 | 100 | | | | | | **Small amount of tumor** | **POS** | 100 | 0 | 100 | 0 |
| 67A | 137 | 10 | 20 | 80 | | 10 | | | 80 | | **POS** | 90 | 80 | 100 | 240 |
| 67B | 138 | 30 | 20 | 70 | | | 40 | | 40 | | **POS** | 70 | 80 | 70 | 200 |
| 68A | 139 | 20 | 90 | | | 20 | | 60 | 10 | **Vacuolar staining** | **POS** | 80 | 10 | 220 | 30 |
| 68B | 140 | | 100 | 70 | | | | 30 | | | **POS** | 100 | 0 | 160 | 0 |

(continued)

| Sample # | Slide # | Apical & Cytoplasmic Staining (Percent) | | | | | | | | Comments | POS /NEG | % POS | % POS | H | H |
|---|---|---|---|---|---|---|---|---|---|---|---|---|---|---|---|
| | | 0C | 0A | 1+C | 1+ A | 2+C | 2+ A | 3+C | 3+A | | | Cyto | Apical | Cyto | Apical |
| 69A | 141 | | 40 | 20 | | 80 | 30 | | 30 | | **POS** | 100 | 60 | 180 | 150 |
| 69B | 142 | | 20 | 20 | 20 | 50 | 30 | 30 | 30 | | **POS** | 100 | 80 | 210 | 170 |
| 70A | 143 | | | 50 | | 50 | | | 100 | | **POS** | 100 | 100 | 150 | 300 |
| 70B | 144 | | 10 | 40 | 30 | 40 | 30 | 20 | 30 | | **POS** | 100 | 90 | 180 | 180 |
| 71A | 145 | | | | | | | | | **Only a few tumor cells-cannot score** | | | | | |
| 7IB | 71-2 | 60 | 50 | 20 | | 20 | 20 | | 30 | | **POS** | 40 | 50 | 60 | 130 |
| 72A | 72-1 | | 50 | 50 | | 30 | 20 | 20 | 30 | **Higher grade-apicically negative** | **POS** | 100 | 50 | 170 | 130 |
| 72B | 72-2 | 100 | 100 | | | | | | | | **NEG** | 0 | 0 | 0 | 0 |
| 73A | 73-1 | 20 | 80 | 80 | | | | | 20 | | **POS** | 80 | 20 | 80 | 60 |
| 73B | 73-2 | 40 | 50 | 40 | 10 | 10 | 20 | 10 | 20 | | **POS** | 60 | 50 | 90 | 110 |
| 74A | 74-1 | | | | | | | 100 | 100 | **Vacuolar staining** | **POS** | 100 | 100 | 300 | 300 |
| 74B | 74-2 | | 20 | 50 | 10 | 50 | 30 | | 40 | | **POS** | 100 | 80 | 150 | 190 |
| 75A | 75-1 | | | 70 | | 30 | 30 | | 70 | | **POS** | 100 | 100 | 130 | 270 |
| 75B | 75-2 | 10 | 50 | 90 | 10 | | 20 | | 20 | | **POS** | 90 | 50 | 90 | 110 |
| 76A | 76-1 | | 10 | 60 | 10 | 40 | 20 | | 60 | | **POS** | 100 | 90 | 140 | 230 |
| 76B | 76-2 | | | 50 | 10 | 50 | 30 | | 60 | | **POS** | 100 | 100 | 150 | 250 |
| 77A | 77-1 | | 20 | 90 | 20 | 10 | 20 | | 40 | | **POS** | 100 | 80 | 110 | 180 |
| 77B | 77-2 | 10 | 20 | 90 | 10 | | 20 | | 50 | | **POS** | 90 | 80 | 90 | 200 |
| 78A | 78-1 | | | 100 | | | | | 100 | | **POS** | 100 | 100 | 100 | 300 |
| 78B | 78-2 | | | 100 | | | | | 100 | | **POS** | 100 | 100 | 100 | 300 |
| 79A | 79-1 | | | 80 | | 20 | | | 100 | | **POS** | 100 | 100 | 120 | 300 |
| 79B | 79-2 | | | 50 | | 50 | | | 100 | | **POS** | 100 | 100 | 150 | 300 |
| 80A | 80-1 | 90 | | 10 | | | | | 100 | | **POS** | 10 | 100 | 10 | 300 |

(continued)

| Sample # | Slide # | Apical & Cytoplasmic Staining (Percent) | | | | | | | | Comments | POS /NEG | % POS | % POS | H | H |
|---|---|---|---|---|---|---|---|---|---|---|---|---|---|---|---|
| | | 0C | 0A | 1+C | 1+ A | 2+C | 2+ A | 3+C | 3+A | | | Cyto | Apical | Cyto | Apical |
| 80B | 80-2 | | | 20 | | 50 | | 30 | 100 | | **POS** | 100 | 100 | 210 | 300 |
| 81A | 81-1 | | | 80 | | 20 | 30 | | 70 | | **POS** | 100 | 100 | 120 | 270 |
| 81B | 81-2 | | 10 | 90 | 10 | 10 | 20 | | 60 | | **POS** | 100 | 90 | 110 | 230 |
| 82A | 82-1 | | | 50 | | 50 | | | 100 | | **POS** | 100 | 100 | 150 | 300 |
| 82B | 82-2 | 20 | | 80 | | | | | 100 | | **POS** | 80 | 100 | 80 | 300 |
| 83A | 83-1 | 30 | | 70 | 50 | | 20 | | 30 | | **POS** | 70 | 100 | 70 | 180 |
| 83B | 83-2 | | | 80 | | 20 | 20 | | 80 | **Golgi-like staining in cytoplasm** | **POS** | 100 | 100 | 120 | 280 |
| 84A | 84-1 | 80 | | 20 | | | | | 100 | | **POS** | 20 | 100 | 20 | 300 |
| 84B | 84-2 | 100 | 60 | | 20 | | 10 | | 10 | | **POS** | 0 | 40 | 0 | 70 |
| 85A | 85-1 | | | 100 | | | | | 100 | | **POS** | 100 | 100 | 100 | 300 |
| 85B | 85-2 | 100 | | | | | | | 100 | | **POS** | 0 | 100 | 0 | 300 |
| 86A | 86-1 | 10 | 40 | 80 | | 10 | | | 60 | | **POS** | 90 | 60 | 100 | 180 |
| 86B | 86-2 | | | 50 | | 50 | | | 100 | | **POS** | 100 | 100 | 150 | 300 |
| 87A | 87-1 | 10 | 10 | 80 | 20 | 10 | 20 | | 50 | | **POS** | 90 | 90 | 100 | 210 |
| 87B | 87-2 | 10 | 10 | 50 | 30 | 30 | 30 | 10 | 30 | | **POS** | 90 | 90 | 140 | 180 |
| 88A | 88-1 | | 30 | 80 | 20 | 20 | 20 | | 30 | | **POS** | 100 | 70 | 120 | 150 |
| 88B | 88-2 | | 100 | 50 | | 50 | | | | | **POS** | 100 | 0 | 150 | 0 |
| 89A | 89-1 | 100 | 100 | | | | | | | **Renal Cell** | **NEG** | 0 | 0 | 0 | 0 |
| 89B | 89-2 | 100 | 100 | | | | | | | | **NEG** | 0 | 0 | 0 | 0 |
| 90A | 90-1 | 50 | 100 | 20 | | | | 30 | | **Pancreas - Neuroendocrine** | **POS** | 50 | 0 | 110 | 0 |
| 90B | 90-2 | | 100 | | | 20 | | 80 | | **Vacuolar staining** | **POS** | 100 | 0 | 280 | 0 |
| 91A | 91-1 | 100 | 100 | | | | | | | **Peritoneal-Liposarcoma** | **NEG** | 0 | 0 | 0 | 0 |
| 91B | 91-2 | 100 | 100 | | | | | | | | **NEG** | 0 | 0 | 0 | 0 |

(continued)

| Sample # | Slide # | Apical & Cytoplasmic Staining (Percent) | | | | | | | | Comments | POS /NEG | % POS | % POS | H | H |
|---|---|---|---|---|---|---|---|---|---|---|---|---|---|---|---|
| | | 0C | 0A | 1+ C | 1+ A | 2+ C | 2+ A | 3+ C | 3+ A | | | Cyto | Apical | Cyto | Apical |
| 92A | 92-1 | 100 | 100 | | | | | | | **Gastric GIST** | **NEG** | 0 | 0 | 0 | 0 |
| 92B | 92-2 | 100 | 100 | | | | | | | | **NEG** | 0 | 0 | 0 | 0 |
| 93A | 93-1 | 50 | | 30 | | 20 | | | 100 | **Golgi-like staining in cytoplasm** | **POS** | 50 | 100 | 70 | 300 |
| 93B | 93-2 | 50 | | 30 | | 20 | | | 100 | | **POS** | 50 | 100 | 70 | 300 |
| 94A | 94-1 | 50 | | 50 | | | | | 100 | | **POS** | 50 | 100 | 50 | 300 |
| 94B | 94-2 | | | 80 | | 20 | | | 100 | | **POS** | 100 | 100 | 120 | 300 |
| 95A | 95-1 | | | 40 | | 50 | | 10 | 100 | | **POS** | 100 | 100 | 170 | 300 |
| 95B | 95-2 | 50 | | 50 | | | | | 100 | | **POS** | 50 | 100 | 50 | 300 |
| 96A | 96-1 | | | | | 100 | | | 100 | | **POS** | 100 | 100 | 200 | 300 |
| 96B | 96-2 | 100 | 40 | | 20 | | 20 | | 20 | | **POS** | 0 | 60 | 0 | 120 |
| 97A | 97-1 | 100 | 100 | | | | | | | **Pancreatic** | **NEG** | 0 | 0 | 0 | 0 |
| 97B | 97-2 | 100 | 100 | | | | | | | | **NEG** | 0 | 0 | 0 | 0 |
| 98A | 98-1 | | 100 | | | | | 100 | | **Uterus - Leiomyosarcoma** | **POS** | 100 | 0 | 300 | 0 |
| 98B | 98-2 | | 100 | | | | | 100 | | | **POS** | 100 | 0 | 300 | 0 |
| 99A | 99-1 | 10 | 100 | | | | | 90 | | **Pancreas - Neuroendocrine** | **POS** | 90 | 0 | 270 | 0 |
| 99B | 99-2 | 100 | 100 | | | | | | | | **NEG** | 0 | 0 | 0 | 0 |
| 100A | 100-1 | | | 100 | | | 50 | | 50 | **Gastric** | **POS** | 100 | 100 | 100 | 250 |
| 100B | 100-2 | | 20 | 60 | | 40 | 40 | | 40 | | **POS** | 100 | 80 | 140 | 200 |

**Summary of GCC IHC Staining Results**

The GCC IHC staining results shown in Examples 3 and 4 demonstrate that GCC is expressed in a variety of gastrointestinal malignancies, including colorectal, stomach, small intestine and esophageal tumors, as well as non-gastrointestinal tumors including pancreatic tumors and lung adenocarcinomas, lung squamous cell carcinomas, leiomyosarcomas and rhabdomyosarcomas. A summary of the various tumor microarray analyses is shown in Table 33 below.

**Table 33: TMA analysis shows GCC is expressed in a variety of maligancies**

| | | **% GCC Positive Staining*** | | |
|---|---|---|---|---|
| **Tumor Type** | **N Tested** | **Apical/Membranous** | **Cytoplasmic** | **% positive with either** |
| Colorectal | 298 | 76 | 90 | 95 |
| Gastric | 154 | 33.1 | 63 | 79 |
| Pancreatic | 221 | 15.4 | 43.4 | 63 |
| Esophageal | 138 | 1.4 | 30 | 30 |
| Lung Adenocarcinoma | 81 | 2.4 | 25.6 | 44 |
| Lung Squamous | 74 | 0 | 10.8 | 10.8 |
| Leiomyosarcoma | 18 | 0 | 44.4 | 44.4 |
| Rhabdomyosarcoma | 18 | 0 | 55.6 | 55.6 |
| *(% positive is defined by H score >10) | | | | |

Human clinical samples of gastrointestinal and GI-related malignancies also tested positive for varying levels of GCC expression, as summarized in Table 34 below.

**Table 34: Percent of Tumors Screend in C26001 positive**

| | **N** | **% any positive** | **% greater 400 (on combined/aggregate apical and cytoplasmic H score)** |
|---|---|---|---|
| Colorectal | 46 | 93.5 | 26.1 |
| Gastric | 9 | 77.8 | 0 |
| Esophageal | 14 | 78.6 | 7.1 |
| Pancreatic | 22 | 81.8 | 22.7 |
| Small Intestine | 2 | 100 | 0 |
| Total | 93 | 86.0 | |
| (Positive is defined as an H score > = to 10 in either apical or cytoplasmic) | | | |

The results summarized in Table 34 are similar to the results observed throughout the TMA screenings summarized in Table 33.

The combined/aggregate H Score distribution of GCC expression across tumor types from patient enrollment screening for the the C26001 trial is depicted in Figures 2A-2D.

The combined/aggregate H score distribute of GCC expression from the various colorectal, gastric, and pancreatic tumor microarrays screened is depicted in Figures 3A-3C.

## Claims

1. An anti-GCC antibody molecule comprising three heavy chain complementarity determining regions (HCDR1, HCDR2, and HCDR3) comprising amino acid sequences of SEQ ID NOs: 21, 22 and 23, respectively; and three light chain complementarity determining regions (LCDR1, LCDR2, and LCDR3) comprising amino acid sequences of SEQ ID NOs: 27, 28 and 29, respectively.

2. The anti-GCC antibody molecule of claim 1, comprising a heavy chain variable region comprising an amino acid sequence of SEQ ID NO: 11 and a light chain variable region comprising an amino acid sequence of SEQ ID NO: 13.

3. The anti-GCC antibody molecule of claim 1 or claim 2, wherein the antibody molecule is:

   (a) a monoclonal antibody; and/or
   (b) a rabbit or rabbit-derived antibody, optionally a rabbit monoclonal antibody or a humanized rabbit monoclonal antibody.

4. The anti-GCC antibody molecule of any one of claims 1 to 3, wherein the antibody molecule is conjugated to a detectable label, wherein the detectable label is selected from a biologically active enzyme, a ligand, a prosthetic group, a fluorescent material, a luminescent material, a chemiluminescent material, a bioluminescent material, a chromophoric material, an electron dense material, a paramagnetic material, and a radioactive material.

5. Isolated nucleic acid sequences that encode an antibody molecule of any one of claims 1 to 3.

6. A vector comprising a nucleic acid encoding the light chain and the heavy chain of an antibody molecule of any one of claims 1 to 3, or comprising the isolated nucleic acid sequences of claim 5.

7. A method of producing an antibody molecule of any one of claims 1 to 3, comprising culturing a cell comprising the isolated nucleic acid sequences of claim 5 or the vector of claim 6 under conditions that allow production of an antibody molecule, thereby producing the antibody molecule.

8. A method of detecting a GCC molecule in a biological sample comprising contacting the biological sample with an antibody molecule of any one of claims 1 to 3 and determining if said antibody molecule binds to said GCC molecule.

9. The method of claim 8, wherein detecting a GCC molecule comprises an immunohistochemistry assay.

10. The method of claim 8 or claim 9, wherein said biological sample is a tumor biopsy derived from a patient suspected of having a GCC-expressing cancer, wherein the cancer is optionally selected from colorectal cancer, gastric cancer, small intestine cancer, esophageal cancer, pancreatic cancer, lung cancer, a soft tissue sarcoma, a neuroectodermal tumor, and a neuroendocrine tumor.

11. The method of any one of claims 8 to 10, further comprising quantifying apical and/or cytoplasmic GCC expression in said biological sample.

12. The method of claim 11, wherein the quantifying comprises an H-score approach.

13. A kit comprising the anti-GCC antibody molecule of any one of claims 1 to 4 and instructions for use.

14. A method of determining sensitivity of cancer cells to a GCC-targeted therapy and/or evaluating whether a subject is a potential candidate for a GCC-targeted therapy, comprising:

i) contacting a sample of cancer cells obtained from a patient that has cancer with the antibody molecule of any one of claims 1 to 4; and
ii) detecting formation of a complex between the antibody molecule and GCC protein in the sample, thereby determining the sensitivity of the cancer cells to a GCC-targeted therapy, and/or determining if a subject is a candidate for treatment with a GCC-targeted therapy.

**15.** A reaction mixture comprising a biological sample and an anti-GCC antibody molecule of any one of claims 1 to 4.

**16.** A method for generating a personalized cancer treatment report using the anti-GCC antibody molecule of any one of claims 1 to 4, comprising:

i) contacting a biological sample comprising one or more cancer cells obtained from a cancer patient suspected of having a GCC-expressing cancer with the antibody molecule of any one of claims 1 to 4;
ii) detecting formation of a complex between the antibody molecule and GCC protein in the biological sample;
iii) quantifying GCC expression in the biological sample from the complex;
iv) comparing the GCC expression level against a database comprising GCC-targeted therapy; and
v) selecting a GCC-targeted therapy and, optionally, a dosing regimen based on the GCC expression level determined in the sample.

**17.** The method of claim 14 or claim 16, wherein the cancer is selected from colorectal cancer, gastric cancer, small intestine cancer, esophageal cancer, pancreatic cancer, lung cancer, a soft-tissue sarcoma, a neuroectodermal tumor, and a neuroendocrine tumor.

## Patentansprüche

**1.** Anti-GCC-Antikörpermolekül, umfassend drei Schwerketten-Komplementaritätsbestimmungsregionen (HCDR1, HCDR2 und HCDR3), umfassend die Aminosäuresequenzen von SEQ ID Nr. 21, 22 bzw. 23; und drei Leichtketten-Komplementaritätsbestimmungsregionen (LCDR1, LCDR2 und LCDR3), umfassend die Aminosäuresequenzen von SEQ ID Nr. 27, 28 bzw. 29.

**2.** Anti-GCC-Antikörpermolekül nach Anspruch 1, umfassend eine variable Schwerketten-Region, umfassend eine Aminosäuresequenz von SEQ ID Nr. 11, und eine variable Leichtketten-Region, umfassend eine Aminosäuresequenz von SEQ ID Nr. 13.

**3.** Anti-GCC-Antikörpermolekül nach Anspruch 1 oder 2, wobei das Antikörpermolekül ist:

(a) ein monoklonaler Antikörper; und/oder
(b) ein Kaninchen- oder von Kaninchen abgeleiteter Antikörper, optional ein monoklonaler Kaninchen-Antikörper oder ein humanisierter monoklonaler Kaninchen-Antikörper.

**4.** Anti-GCC-Antikörpermolekül nach einem der Ansprüche 1 bis 3, wobei das Antikörpermolekül an eine detektierbare Markierung konjugiert ist, wobei die detektierbare Markierung aus einem biologisch aktiven Enzym, einem Liganden, einer prosthetischen Gruppe, einem fluoreszierenden Material, einem lumineszierenden Material, einem chemilumineszierenden Material, einem biolumineszierenden Material, einem chromophoren Material, einem elektronendichten Material, einem paramagnetischen Material und einem radioaktiven Material ausgewählt ist.

**5.** Isolierte Nukleinsäuresequenzen, die ein Antikörpermolekül nach einem der Ansprüche 1 bis 3 codieren.

**6.** Vektor, umfassend eine Nukleinsäure, welche die Leichtkette und die Schwerkette eines Antikörpermoleküls nach einem der Ansprüche 1 bis 3 codiert, oder umfassend die isolierten Nukleinsäuresequenzen nach Anspruch 5.

**7.** Verfahren zur Herstellung eines Antikörpermoleküls nach einem der Ansprüche 1 bis 3, umfassend das Kultivieren einer Zelle, umfassend die isolierten Nukleinsäuresequenzen nach Anspruch 5 oder den Vektor nach Anspruch 6, unter Bedingungen, welche die Herstellung eines Antikörpermoleküls erlauben, wodurch das Antikörpermolekül hergestellt wird.

**8.** Verfahren zur Detektion eines GCC-Moleküls in einer biologischen Probe, umfassend das Inkontaktbringen der

biologischen Probe mit einem Antikörpermolekül nach einem der Ansprüche 1 bis 3 und das Bestimmen, ob das Antikörpermolekül an das GCC-Molekül bindet.

**9.** Verfahren nach Anspruch 8, wobei das Detektieren eines GCC-Moleküls einen Immunhistochemie-Assay umfasst.

**10.** Verfahren nach Anspruch 8 oder 9, wobei die biologische Probe eine Tumorbiopsie ist, die von einem Patienten abgeleitet ist, der im Verdacht steht, einen GCC-exprimierenden Krebs zu haben, wobei der Krebs optional aus Kolorektalkrebs, Magenkrebs, Dünndarmkrebs, Speiseröhrenkrebs, Bauchspeicheldrüsenkrebs, Lungenkrebs, einem Weichgewebssarkom, einem neuroektodermalen Tumor und einem neuroendokrinen Tumor ausgewählt ist.

**11.** Verfahren nach einem der Ansprüche 8 bis 10, ferner umfassend das Quantifizieren der apikalen und/oder zytoplasmatischen GCC-Expression in der biologischen Probe.

**12.** Verfahren nach Anspruch 11, wobei das Quantifizieren einen H-Score-Ansatz umfasst.

**13.** Kit, umfassend das Anti-GCC-Antikörpermolekül nach einem der Ansprüche 1 bis 4 und Gebrauchsanweisungen.

**14.** Verfahren zur Bestimmung des Ansprechvermögens von Krebszellen gegenüber einer GCC-gezielten Therapie und/oder zur Evaluierung, ob ein Individuum ein potenzieller Kandidat für eine GCC-gezielte Therapie ist, umfassend:

i) Inkontaktbringen einer Probe von Krebszellen, die von einem Patienten gewonnen wurden, der Krebs hat, mit dem Antikörpermolekül nach einem der Ansprüche 1 bis 4; und
ii) Detektieren der Bildung eines Komplexes zwischen dem Antikörpermolekül und dem GCC-Protein in der Probe, wodurch das Ansprechvermögen der Krebszellen gegenüber einer GCC-gezielten Therapie bestimmt wird, und/oder Bestimmen, ob ein Individuum ein Kandidat für eine Behandlung mit einer GCC-gezielten Therapie ist.

**15.** Reaktionsgemisch, umfassend eine biologische Probe und ein Anti-GCC-Antikörpermolekül nach einem der Ansprüche 1 bis 4.

**16.** Verfahren zur Generierung eines personalisierten Krebsbehandlungsberichts unter Verwendung des Anti-GCC-Antikörpermoleküls nach einem der Ansprüche 1 bis 4, umfassend:

i) Inkontaktbringen einer biologischen Probe, umfassend eine oder mehrere Krebszellen, die von einem Krebspatienten gewonnen wurden, der im Verdacht steht, einen GCC-exprimierenden Krebs zu haben, mit dem Antikörpermolekül nach einem der Ansprüche 1 bis 4;
ii) Detektieren der Bildung eines Komplexes zwischen dem Antikörpermolekül und dem GCC-Protein in der biologischen Probe;
iii) Quantifizieren der GCC-Expression in der biologischen Probe von dem Komplex;
iv) Vergleichen des GCC-Expressionsniveaus mit einer Datenbank, umfassend eine GCC-gezielte Therapie; und
v) Auswählen einer GCC-gezielten Therapie und optional eines Dosierungsschemas basierend auf dem in der Probe bestimmten GCC-Expressionsniveau.

**17.** Verfahren nach Anspruch 14 oder 16, wobei der Krebs aus kolorektalem Krebs, Magenkrebs, Dünndarmkrebs, Speiseröhrenkrebs, Bauchspeicheldrüsenkrebs, Lungenkrebs, einem Weichgewebssarkom, einem neuroektodermalen Tumor und einem neuroendokrinen Tumor ausgewählt ist.

## Revendications

**1.** Molécule d'anticorps anti-GCC comprenant trois régions déterminant la complémentarité de chaîne lourde (HCDR1, HCDR2 et HCDR3) qui comprennent les séquences d'acides aminés de SEQ ID n° : 21, 22 et 23 respectivement ; et trois régions déterminant la complémentarité de chaîne légère (LCDR1, LCDR2 et LCDR3) qui comprennent les séquences d'acides aminés de SEQ ID n° : 27, 28 et 29 respectivement.

**2.** Molécule d'anticorps anti-GCC de la revendication 1, comprenant une région variable de chaîne lourde, qui comprend une séquence d'acides aminés de SEQ ID n° : 11, et une région variable de chaîne légère qui comprend une

séquence d'acides aminés de SEQ ID n° : 13.

3. Molécule d'anticorps anti-GCC de la revendication 1 ou la revendication 2, la molécule d'anticorps étant :

   (a) un anticorps monoclonal ; et/ou
   (b) un anticorps de lapin ou dérivé d'un lapin, en option un anticorps monoclonal de lapin ou un anticorps monoclonal de lapin humanisé.

4. Molécule d'anticorps anti-GCC de l'une quelconque des revendications 1 à 3, la molécule d'anticorps étant conjuguée avec un marqueur détectable, le marqueur détectable étant choisi parmi une enzyme biologiquement active, un ligand, un groupe prosthétique, un matériau fluorescent, un matériau luminescent, un matériau chimiluminescent, un matériau bioluminescent, un matériau chromophore, un matériau électronique dense, un matériau paramagnétique et un matériau radioactif.

5. Séquences d'acides nucléiques isolées qui codent pour une molécule d'anticorps de l'une quelconque des revendications 1 à 3.

6. Vecteur comprenant un acide nucléique qui code pour la chaîne légère et la chaîne lourde d'une molécule d'anticorps de l'une quelconque des revendications 1 à 3, ou comprenant les séquences d'acides nucléiques isolées de la revendication 5.

7. Procédé de production d'une molécule d'anticorps de l'une quelconque des revendications 1 à 3, comprenant l'étape consistant à cultiver une cellule comprenant les séquences d'acides nucléiques isolées de la revendication 5 ou le vecteur de la revendication 6, dans des conditions qui permettent de réaliser la production d'une molécule d'anticorps, en produisant de ce fait la molécule d'anticorps.

8. Procédé de détection d'une molécule de GCC dans un échantillon biologique comprenant les étapes consistant à mettre en contact l'échantillon biologique avec une molécule d'anticorps de l'une quelconque des revendications 1 à 3 et déterminer si ladite molécule d'anticorps se lie à ladite molécule de GCC.

9. Procédé de la revendication 8, dans lequel la détection d'une molécule de GCC comprend un dosage par immunohistochimie.

10. Procédé de la revendication 8 ou la revendication 9, dans lequel ledit échantillon biologique est une biopsie de tumeur dérivée d'un patient qui est suspecté d'avoir un cancer exprimant une GCC, le cancer étant, en option, choisi parmi un cancer colorectal, un cancer de l'estomac, un cancer de l'intestin grêle, un cancer de l'oesophage, un cancer du pancréas, un cancer du poumon, un sarcome des tissus mous, une tumeur neuro-ectodermique et une tumeur neuroendocrine.

11. Procédé de l'une quelconque des revendications 8 à 10, comprenant en outre une quantification de l'expression d'une GCC apicale et/ou cytoplasmique dans ledit échantillon biologique.

12. Procédé de la revendication 11, dans lequel la quantification comprend une approche par score H.

13. Trousse comprenant la molécule d'anticorps anti-GCC de l'une quelconque des revendications 1 à 4 ainsi que des instructions d'utilisation.

14. Procédé de détermination de la sensibilité de cellules cancéreuses à une thérapie ciblée sur GCC et/ou d'évaluation de si un sujet est un candidat potentiel pour une thérapie ciblée sur GCC, comprenant les étapes consistant à :

   i) mettre en contact un échantillon de cellules cancéreuses, obtenu à partir d'un patient qui a un cancer, avec la molécule d'anticorps de l'une quelconque des revendications 1 à 4 ; et
   ii) détecter la formation d'un complexe entre la molécule d'anticorps et une protéine GCC dans l'échantillon, en entraînant une détermination de la sensibilité des cellules cancéreuses à une thérapie ciblée sur GCC et/ou une détermination de si un sujet est un candidat pour un traitement avec une thérapie ciblée sur GCC.

15. Mélange réactionnel comprenant un échantillon biologique et une molécule d'anticorps anti-GCC de l'une quelconque des revendications 1 à 4.

**16.** Procédé destiné à générer un rapport personnalisé de traitement d'un cancer en utilisant la molécule d'anticorps anti-GCC de l'une quelconque des revendications 1 à 4, comprenant les étapes consistant à :

i) mettre en contact un échantillon biologique comprenant une ou plusieurs cellule (s) cancéreuse (s), obtenu à partir d'un patient atteint d'un cancer étant suspecté d'avoir un cancer exprimant une GCC, avec la molécule d'anticorps de l'une quelconque des revendications 1 à 4 ;
ii) détecter la formation d'un complexe entre la molécule d'anticorps et une protéine GCC dans l'échantillon biologique ;
iii) quantifier l'expression d'une GCC dans l'échantillon biologique à partir du complexe ;
iv) comparer le niveau d'expression d'une GCC avec une base de données comprenant une thérapie ciblée sur GCC ; et
v) sélectionner une thérapie ciblée sur GCC et, en option, un régime posologique basé sur le niveau d'expression d'une GCC déterminé dans l'échantillon.

**17.** Procédé de la revendication 14 ou la revendication 16, dans lequel le cancer est choisi parmi un cancer colorectal, un cancer de l'estomac, un cancer de l'intestin grêle, un cancer de l'oesophage, un cancer du pancréas, un cancer du poumon, un sarcome des tissus mous, une tumeur neuro-ectodermique et une tumeur neuroendocrine.

FIG. 1

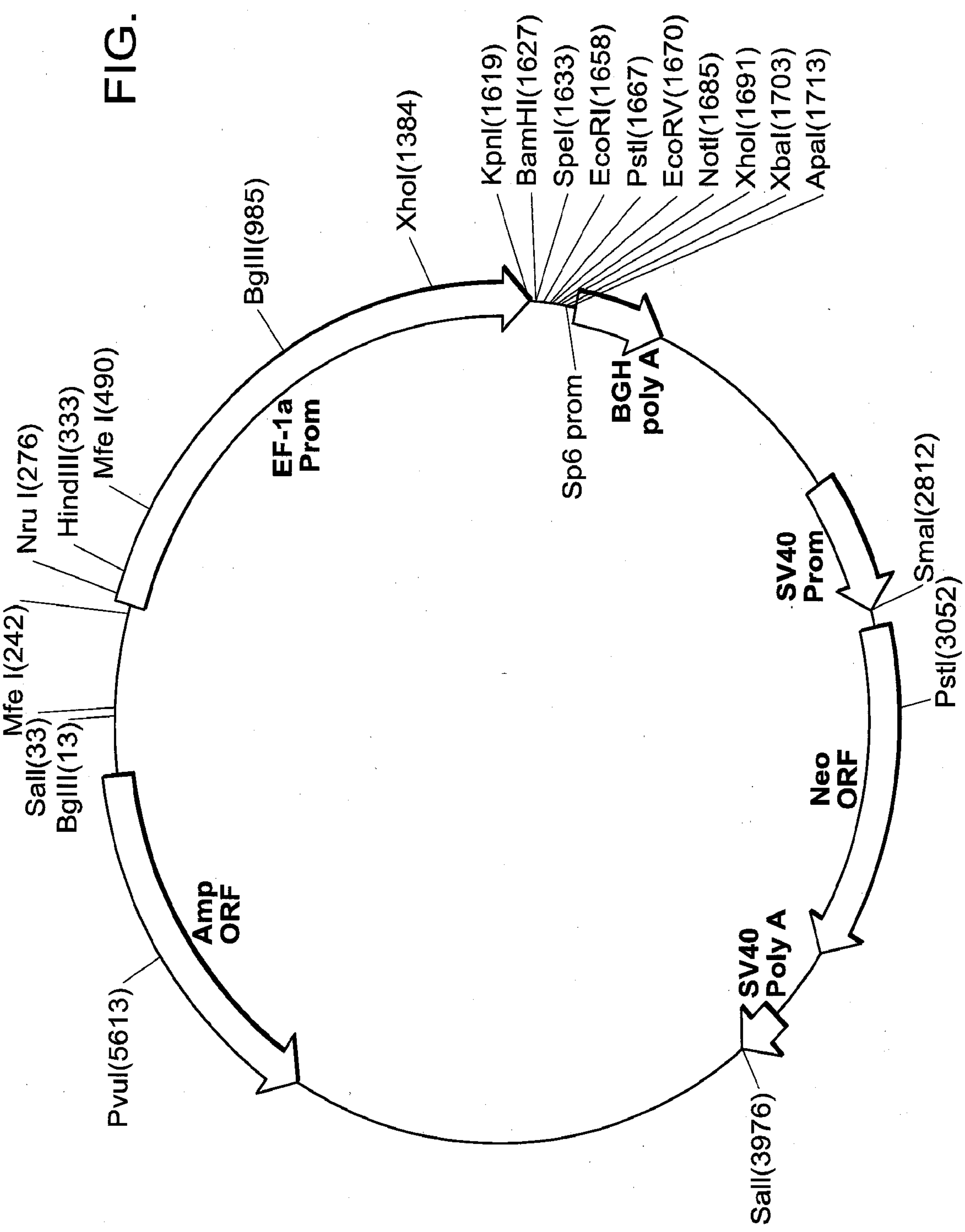
KpnI(1619)
BamHI(1627)
SpeI(1633)
EcoRI(1658)
PstI(1667)
EcoRV(1670)
NotI(1685)
XhoI(1691)
XbaI(1703)
ApaI(1713)
XhoI(1384)
BglII(985)
Mfe I(490)
HindIII(333)
Nru I(276)
Mfe I(242)
SalI(33)
BglII(13)
PvuI(5613)
SalI(3976)
PstI(3052)
SmaI(2812)
Sp6 prom
BGH
poly A
EF-1a
Prom
SV40
Prom
Neo
ORF
SV40
Poly A
Amp
ORF

FIG. 2A

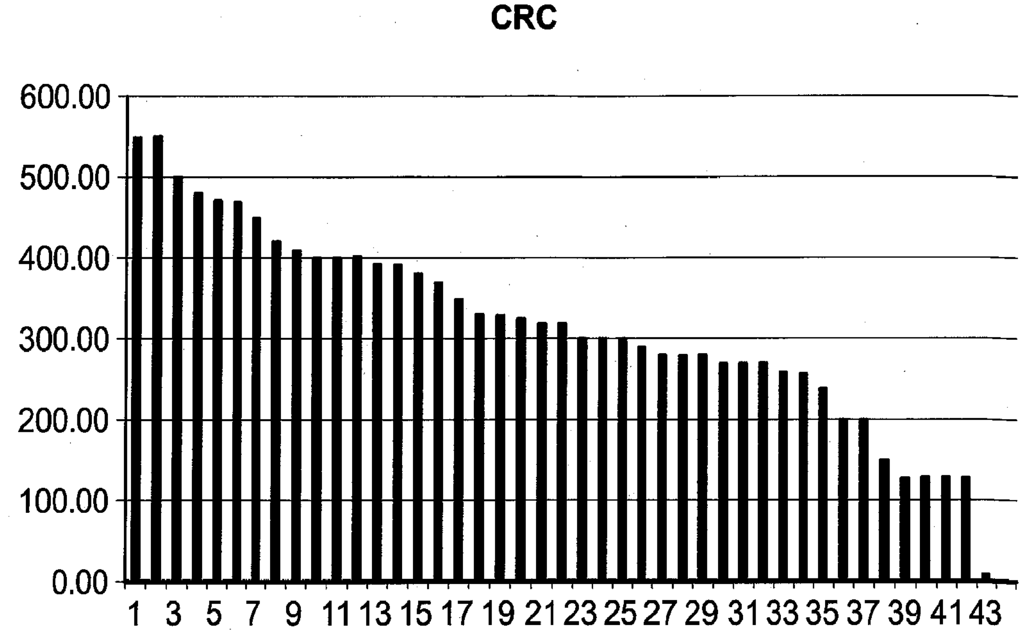
CRC
600.00
500.00
400.00
300.00
200.00
100.00
0.00
1 3 5 7 9 11 13 15 17 19 21 23 25 27 29 31 33 35 37 39 41 43

FIG. 2B

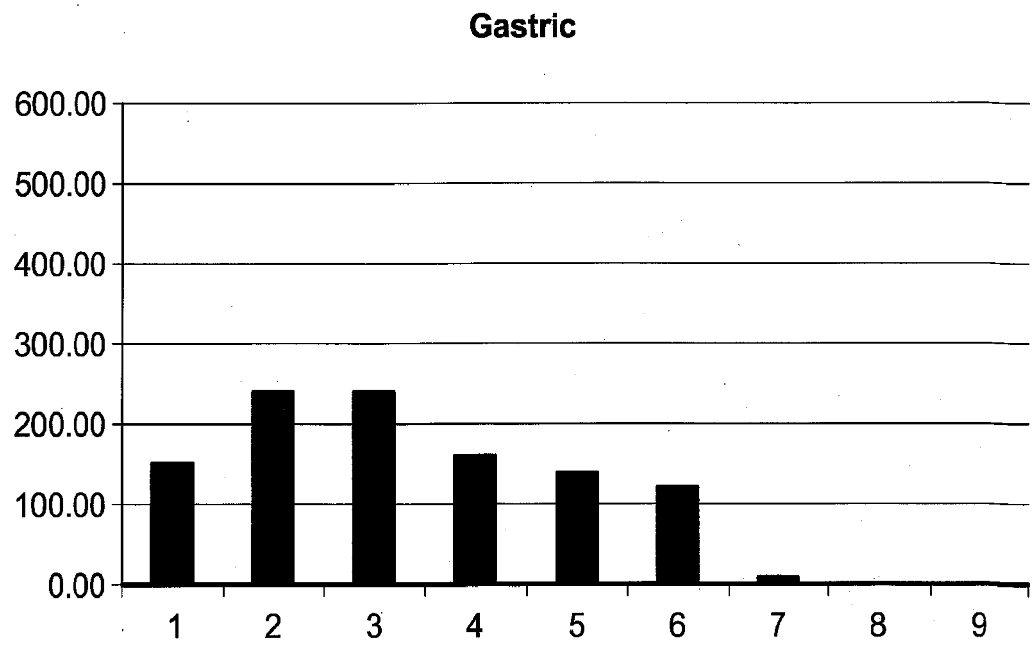
Gastric
600.00
500.00
400.00
300.00
200.00
100.00
0.00
1 2 3 4 5 6 7 8 9

FIG. 2C

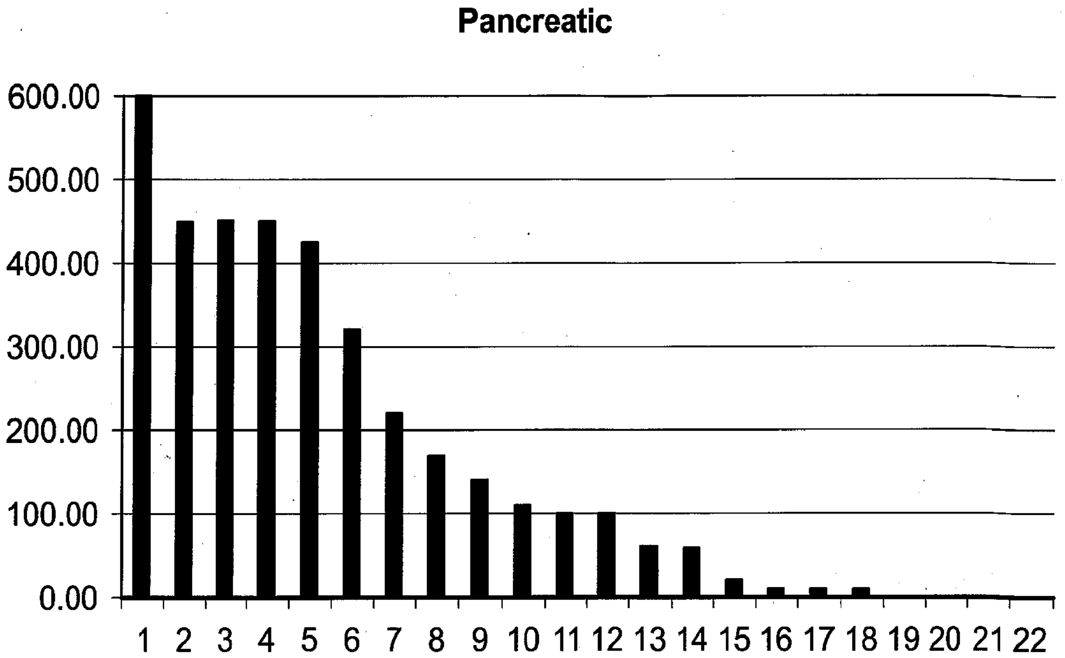
Pancreatic
600.00
500.00
400.00
300.00
200.00
100.00
0.00
1 2 3 4 5 6 7 8 9 10 11 12 13 14 15 16 17 18 19 20 21 22

FIG. 2D

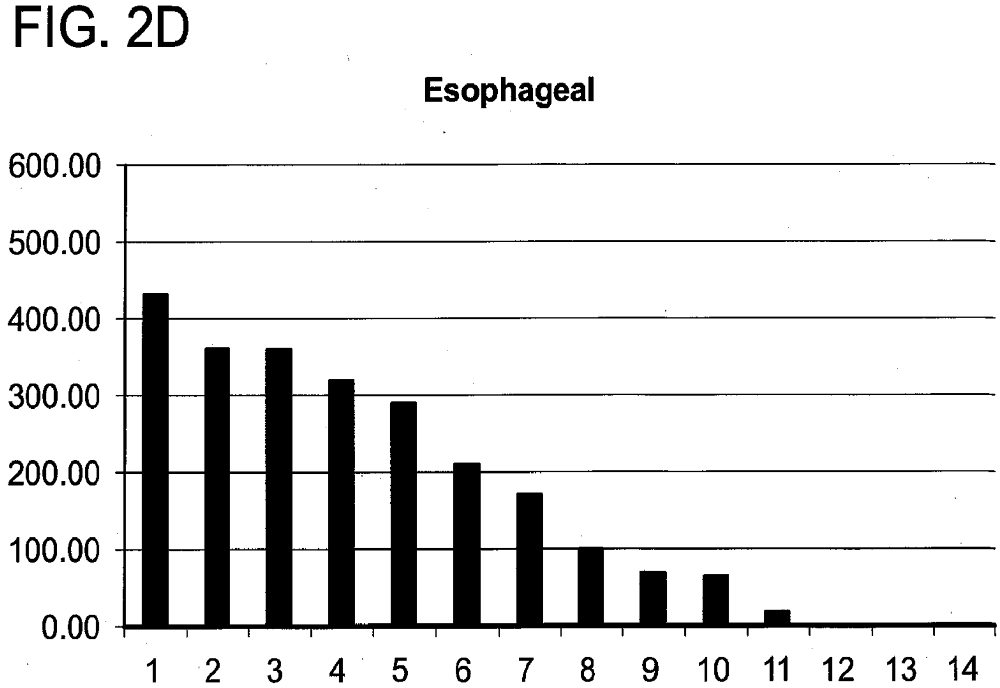
Esophageal
600.00
500.00
400.00
300.00
200.00
100.00
0.00
1 2 3 4 5 6 7 8 9 10 11 12 13 14

FIG. 3A

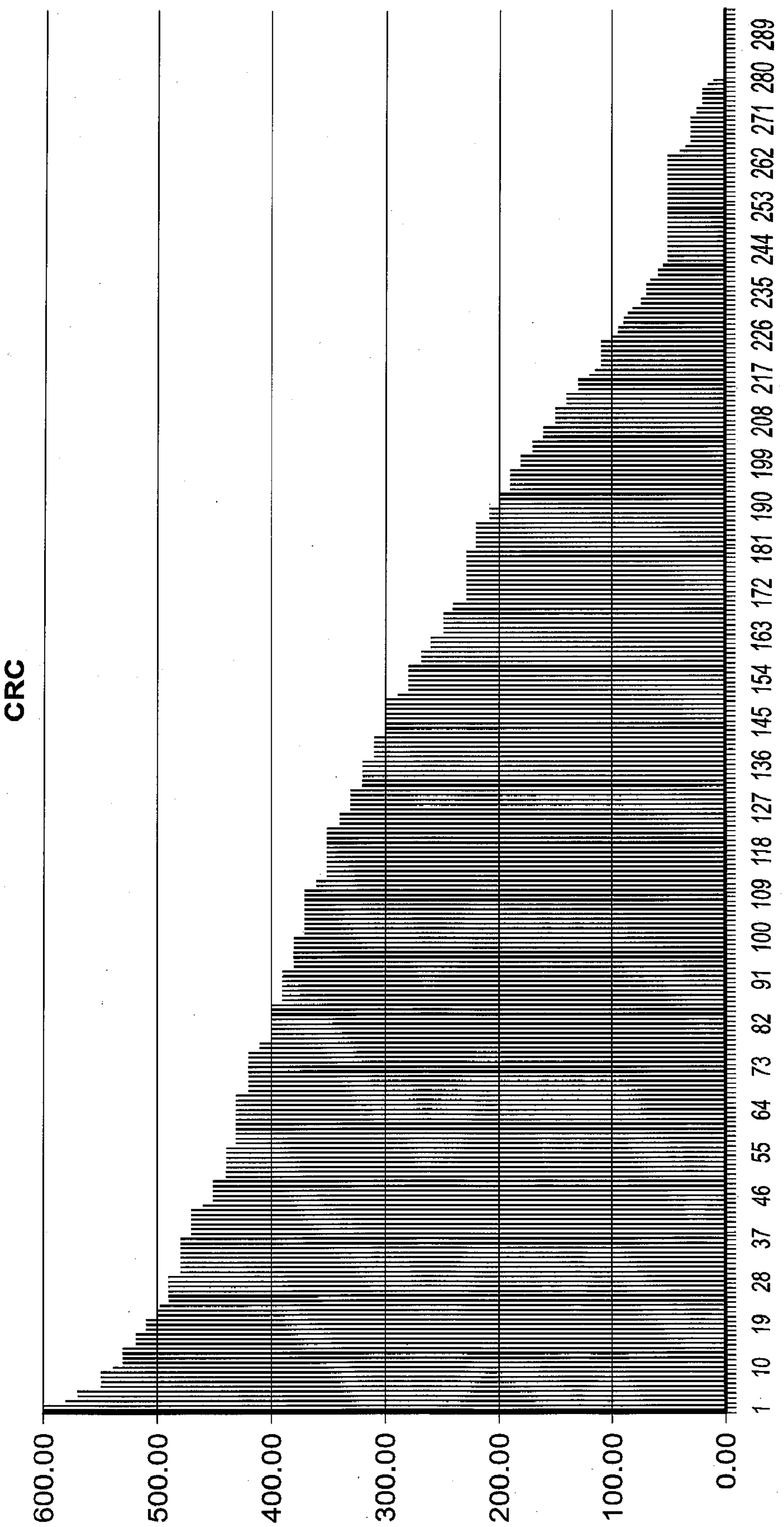
CRC
600.00
500.00
400.00
300.00
200.00
100.00
0.00
1
10
19
28
37
46
55
64
73
82
91
100
109
118
127
136
145
154
163
172
181
190
199
208
217
226
235
244
253
262
271
280
289

FIG. 3B

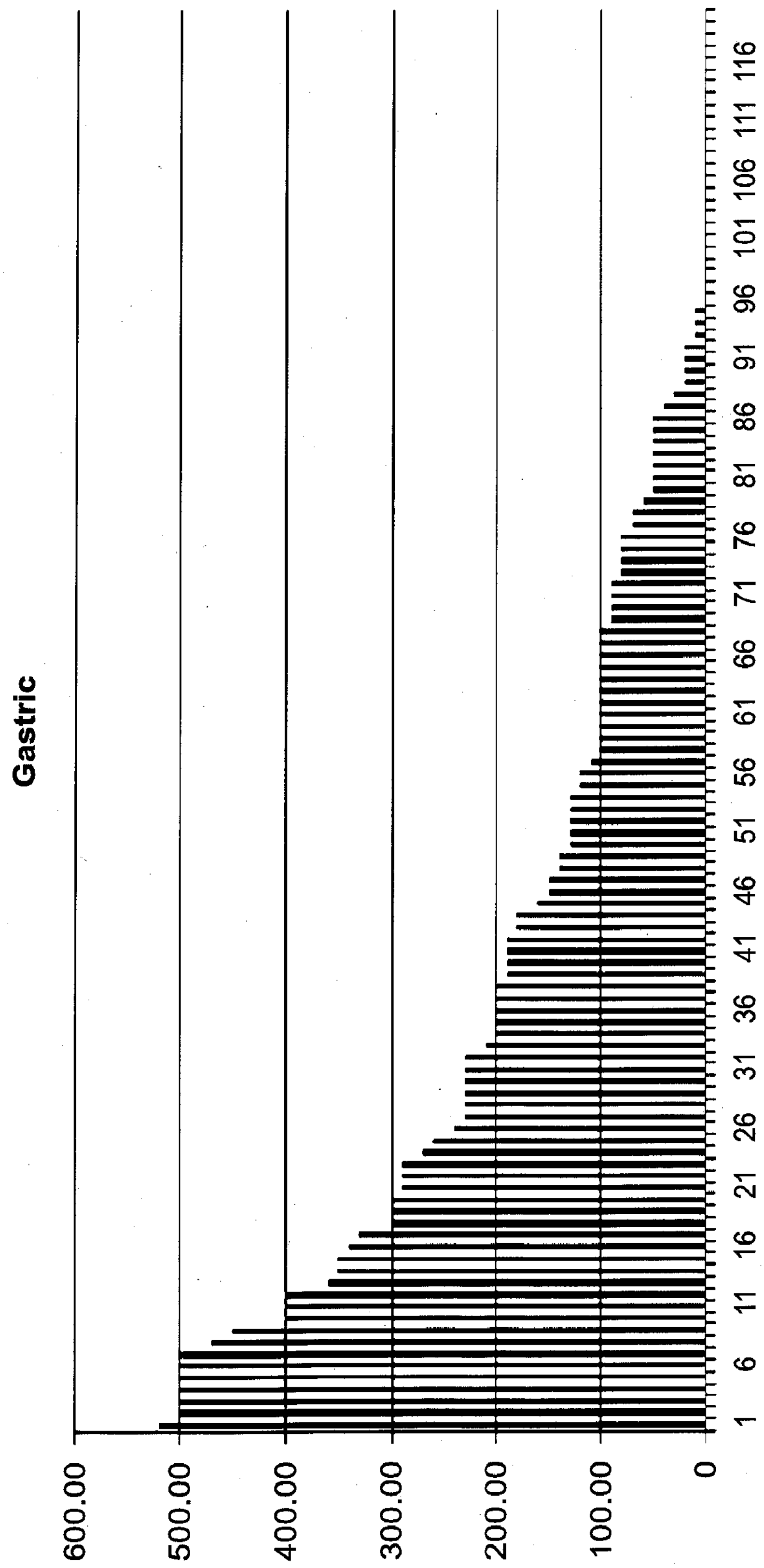
Gastric
600.00
500.00
400.00
300.00
200.00
100.00
0
1
6
11
16
21
26
31
36
41
46
51
56
61
66
71
76
81
86
91
96
101
106
111
116

FIG. 3C

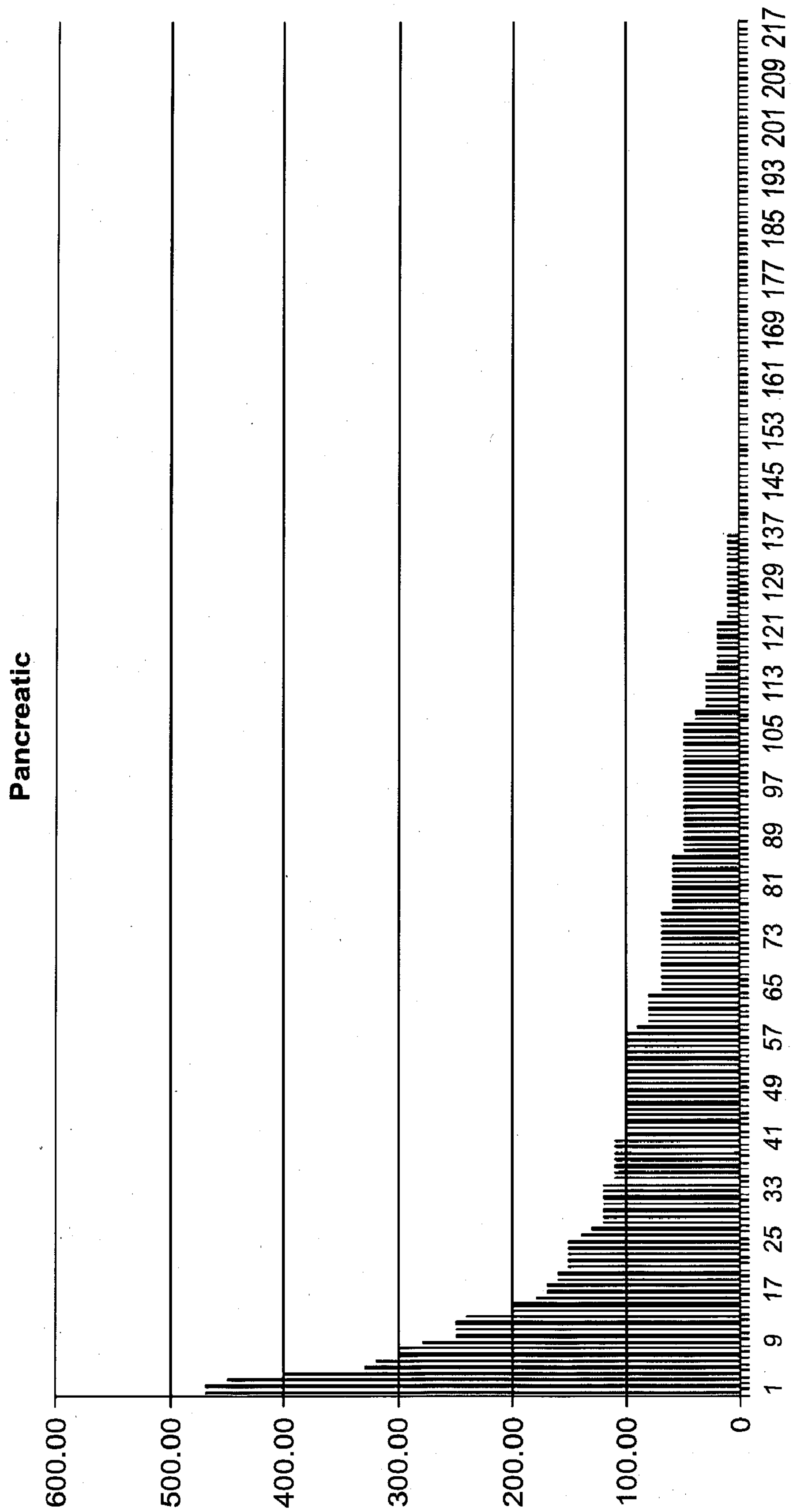
Pancreatic
600.00
500.00
400.00
300.00
200.00
100.00
0
1 9 17 25 33 41 49 57 65 73 81 89 97 105 113 121 129 137 145 153 161 169 177 185 193 201 209 217

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61639376 A
- WO 2011050242 A
- US 6767704 B
- WO 9404678 A
- US 20050037421 A
- WO 03014161 A
- US 20110110936 A
- US 20060035852 A
- US 20040258687 A
- US 4703004 A
- GB 2209757 B, Winter
- WO 8907142 A, Morrison
- WO 9429351 A, Morgan
- US 6020153 A
- US 6331415 B
- US 7402409 B
- US 7429487 B
- US 7462697 B
- US 7575896 B
- US 7732168 B
- US 8062867 B
- US 6197582 B, Trakht
- WO 9202190 A
- US 5530101 A
- US 5585089 A
- US 5693761 A
- US 5693792 A
- US 5714350 A
- US 5777085 A
- US 4683195 A
- US 4683202 A
- US 5565332 A
- US 5573905 A
- US 6300064 B, Knappik
- US 7317091 B
- US 5624821 A
- WO 0042072 A
- US 5648260 A, Winter
- US 5834597 A, Tso
- US 20030157108 A, Presta
- US 20040093621 A
- EP 1176195 A, Hang
- WO 03035835 A, Presta
- WO 9954342 A, Umana
- US 4816567 A
- US 5225539 A
- US 5859205 A
- EP 519596 A1, Padlan
- WO 9852976 A
- WO 0034317 PCT
- US 6818749 B
- US 4179337 A
- US 5612460 A
- WO 9506058 A
- WO 0026256 PCT
- US 20030026805 A
- US 5733743 A
- US 4816397 A
- US 5916771 A
- US 5837821 A
- US 20060147445 A
- WO 9203918 A
- EP 0216846 A
- EP 0256055 A
- EP 0323997 A
- EP 89303964 A
- US 5827690 A
- US 5756687 A
- US 5750172 A
- US 5741957 A
- US 5194594 A
- US 4681581 A
- US 4735210 A
- US 5101827 A
- US 5102990 A
- US 35500 A
- US 5648471 A
- US 5697902 A
- US 6214345 B
- US 5122368 A
- US 5824805 A
- US 5622929 A
- US 4880935 A
- US 20050238649 A
- WO 2004010957 A
- US 20060074008 A
- US 20060024317 A
- WO 0138318 A
- US 5208020 A
- US 6333410 B
- US 7498298 B
- US 7091186 B
- US 6884869 B
- US 6323315 B
- US 6239104 B
- US 6034065 A
- US 5780588 A
- US 5665860 A

- US 5663149 A
- US 5635483 A
- US 5599902 A
- US 5554725 A
- US 5530097 A
- US 5521284 A
- US 5504191 A
- US 5410024 A
- US 5138036 A
- US 5076973 A
- US 4986988 A
- US 4978744 A
- US 4879278 A
- US 4816444 A
- US 4486414 A
- US 20090010945 A
- US 20080300192 A
- US 20050009751 A
- US 20030083236 A
- WO 04010957 A
- WO 02088172 A
- US 4737456 A
- US 3940475 A
- US 4289747 A
- US 4376110 A
- US 5631169 A, Lakowicz
- US 4868103 A, Stavrianopoulos
- WO 2013016662 A
- WO 04071436 A
- US 7053202 B
- US 61639376 B

**Non-patent literature cited in the description**


- **CARRITHERS et al.** *Proc. Natl. Acad. Sci. USA,* 2003, vol. 100, 3018-3020
- **CARRITHERS et al.** *Dis Colon Rectum,* 1996, vol. 39, 171-181
- **BUC et al.** *Eur J Cancer,* 2005, vol. 41, 1618-1627
- **CARRITHERS et al.** *Gastroenterology,* 1994, vol. 107, 1653-1661
- **CARRITHERS et al.** *Proc Natl Acad Sci USA,* 2003, vol. 100, 3018-3020
- **MANN et al.** *Biochem Biophys Res Commun,* 1997, vol. 239, 463-466
- **PITARI et al.** *Proc Natl Acad Sci USA,* 2003, vol. 100, 2695-2699
- **WIEGAND et al.** *FEBS Lett.,* 1992, vol. 311, 150-154
- **RAO, M. C.** *Ciba Found. Symp.,* 1985, vol. 112, 74-93
- **KNOOP F. C. ; OWENS, M.** *J. Pharmacol. Toxicol. Methods,* 1992, vol. 28, 67-72
- **DEBRUYNE et al.** *Gastroenterology,* 2006, vol. 130, 1191-1206
- **ALMENOFF et al.** *Mol Microbiol,* vol. 8, 865-873
- **GUARINO et al.** *Dig Dis Sci,* 1987, vol. 32, 1017-1026
- **URBANSKI et al.** *Biochem Biophys Acta,* 1995, vol. 1245, 29-36
- **NANDI et al.** *Protein Expr. Purif.,* 1996, vol. 8, 151-159
- **NANDI et al.** *J. Cell. Biochem.,* 1997, vol. 66, 500-511
- **VIJAYACHANDRA et al.** *Biochemistry,* 2000, vol. 39, 16075-16083
- **BAKRE et al.** *Eur. J. Biochem.,* 2000, vol. 267, 179-187
- **NANDI et al.** *Protein Sci.,* 1998, vol. 7, 2175-2183
- **BHANDARI et al.** *Biochemistry,* 2001, vol. 40, 9196-9206
- **SAMBROOK et al.** Molecular Cloning: A Laboratory Manual. Cold Spring Harbor Laboratory Press, 2000
- **HARLOW, E. ; LANE, D.** Antibodies: A Laboratory Manual. Cold Spring Harbor Laboratory Press, 1988
- **BIRD et al.** *Science,* 1988, vol. 242, 423-426
- **HUSTON et al.** *Proc. Natl. Acad. Sci. USA,* 1988, vol. 85, 5879-5883
- **NEEDLEMAN ; WUNSCH.** *J. Mol. Biol.,* 1970, vol. 48, 444-453
- Current Protocols in Molecular Biology. John Wiley & Sons, 1989, 6.3.1-6.3.6
- **BOWIE, J U et al.** *Science,* 1990, vol. 247, 1306-1310
- **PADLAN et al.** *FASEB J.,* 1995, vol. 9, 133-139
- **SJOLANDER, S ; URBANICZKY, C.** *Anal. Chem.,* 1991, vol. 63, 2338-2345
- **SZABO et al.** *Curr. Opin. Struct. Biol.,* 1995, vol. 5, 699-705
- **PACE et al.** *Protein Science,* 1995, vol. 4, 2411
- **PACE ; GRIMSLEY.** *Current Protocols in Protein Science,* 2003, 3.1.1-3.1.9
- **MYSZKA ; MORTON.** *Trends Biochem. Sci.,* 1998, vol. 23, 149-150
- Fundamental Immunology. Raven Press, 1989
- **KABAT.** Sequences of Proteins of Immunological Interest. National Institutes of Health, Bethesda, 1987
- **CHOTHIA ; LESK.** *J. Mol. Biol.,* 1987, vol. 196, 901-917

- **CHOTHIA et al.** *Nature,* 1989, vol. 342, 878-883
- **HOMBACH et al.** *Eur. J. Immunol.,* 1990, vol. 20, 2795-2799
- **KOHLER ; MILSTEIN.** *Nature,* 1975, vol. 256, 495
- **NIEDBALA et al.** *Hybridoma,* 1998, vol. 17, 299-304
- **ZANELLA et al.** *J Immunol Methods,* 1992, vol. 156, 205-215
- **GUSTAFSSON et al.** *Hum Antibodies Hybridomas,* 1991, vol. 2, 26-32
- **WINTER ; HARRIS.** *Immunol Today,* 1993, vol. 14, 43-46
- **WRIGHT et al.** *Crit. Reviews in Immunol.,* 1992, 12125-168
- **LIU et al.** *Proc Natl Acad Sci USA.,* 1987, vol. 84, 3439
- *J. Immunol.,* 1987, vol. 139, 3521
- **MCCAFFERTY et al.** *Nature,* 1990, vol. 348, 552-553
- **JOHNSON ; CHISWELL.** *Current Opinion in Structural Biology,* 1993, vol. 3, 564-571
- **CLACKSON et al.** *Nature,* 1991, vol. 352, 624-628
- **MARKS et al.** *J. Mol. Biol.,* 1991, vol. 222, 581-597
- **GRIFFITH et al.** *EMBO J.,* 1993, vol. 12, 725-734
- **KABAT et al.** Sequences of Proteins of Immunological Interest. N.I.H, 1991
- **SHIELDS et al.** *J. Biol. Chem.,* 2001, vol. 276, 6591-6604
- **LAZAR et al.** *Proc. Natl. Acad. Sci. U.S.A.,* 2006, vol. 103, 4005-4010
- **SATOH et al.** *Expert Opin Biol. Ther.,* 2006, vol. 6, 1161-1173
- **SHIELDS, R. L. et al.** *J. Biol. Chem.,* 2002, vol. 277, 26733-26740
- **UMANA et al.** *Nat. Biotech.,* 1999, vol. 17, 176-180
- **KABAT, E. A. et al.** Sequences of Proteins of Immunological Interest. U.S. Department of Health and Human Services, NIH, 1991
- **CHOTHIA, C. et al.** *J. Mol. Biol.,* 1987, vol. 196, 901-917
- **THOMPSON J. D. et al.** *Nucleic Acids Res.,* 1994, vol. 22, 4673-4680
- **KABAT, E. A. et al.** Sequences of Proteins of Immunological Interest. U.S. Department of Health and Human Services, U.S. Government Printing Office, 1991
- **JOHNSON, G. ; WU, T. T.** *Nucleic Acids Research,* 2001, vol. 29, 205-206
- **TOMLINSON, I. A. et al.** *J. Mol. Biol.,* 1992, vol. 227, 776-798
- **COOK, G. P. et al.** *Immunol. Today,* 1995, vol. 16 (5), 237-242
- **CHOTHIA, D. et al.** *J. Mol. Bio.,* 1992, vol. 227, 799-817
- **TOMLINSON, I. A. et al.** *MRC Centre for Protein Engineering*
- **KASHMIRI et al.** *Methods,* 2005, vol. 36, 25-34
- **TAN et al.** *J. Immunol.,* 2006, vol. 169, 1119-1125
- **HIGGINS D. G. et al.** *Meth. Enzymol.,* 1996, vol. 266, 383-402
- **WARD et al.** *Nature,* 1989, vol. 341, 544-546
- **CORTEZ-RETAMOZO et al.** *Cancer Res.,* 2004, vol. 64, 2853-2857
- **LINDENBAUM et al.** *Nucleic Acids Res.,* 2004, vol. 32, e172
- **KENNARD et al.** *Biotechnol. Bioeng. Online,* 20 May 2009
- **KIM ; BALDWIN.** Specific Intermediates in the Folding Reactions of Small Proteins and the Mechanism of Protein Folding. *Ann. Rev. Biochem.,* 1982, vol. 51, 459-89
- **HANES ; PLUCTHAU.** *PNAS USA,* 1997, vol. 94, 4937-4942
- **PARMLEY ; SMITH.** *Gene,* 1988, vol. 73, 305-318
- **SCOTT.** *TIBS,* 1992, vol. 17, 241-245
- **CWIRLA et al.** *Proc Natl Acad Sci USA,* 1990, vol. 87, 6378-6382
- **RUSSEL et al.** *Nucl. Acids Research,* 1993, vol. 21, 1081-1085
- **HOGANBOOM et al.** *Immunol. Reviews,* 1992, vol. 130, 43-68
- **CHISWELL ; MCCAFFERTY.** *TIBTECH,* 1992, vol. 10, 80-84
- **FANGER et al.** *Immunomethods,* 1994, vol. 4, 72-81
- **TRAUNECKER et al.** *Int. J. Cancer,* 1992, vol. 7, 51-52
- **SONGSIVILAI ; LACHMANN.** *Clin. Exp. Immunol.,* 1990, vol. 79, 315-321
- **KOSTELNY et al.** *J. Immunol.,* 1992, vol. 148, 1547-1553
- **ILL. et al.** Design and construction of a hybrid immunoglobulin domain with properties of both heavy and light chain variable regions. *Protein Eng,* 1997, vol. 10, 949-57
- **MARTIN et al.** *EMBO J.,* 1994, vol. 13, 5303-9
- **HOLLIGER et al.** *Proc Natl Acad Sci USA,* 1993, vol. 90, 6444-6448
- **TRAUNECKER et al.** *EMBO J.,* 1991, vol. 10, 3655-3659
- **TRAUNECKER et al.** *Int J Cancer,* 1992, vol. 7, 51-52
- **GOLDMAN, L. A. et al.** *Biotechniques,* 1996, vol. 21, 1013-1015
- **MIZUSHIMA, S. et al.** *Nucleic Acids Res.,* 1990, vol. 18, 5322

- Current Protocols in Molecular Biology. 1991, vol. 2, 16.4.1-16.7.8
- *Vitetta Immunol Today,* 1993, vol. 14, 252
- **JUNGHANS et al.** Cancer Chemotherapy and Biotherapy. Lippincott Raven, 1996, 655-686
- **CHARI et al.** *Cancer Research,* 1992, vol. 52, 127-131
- **DUBOWCHIK ; WALKER.** *Pharm. Therapeutics,* 1999, vol. 83, 67-123
- **NEVILLE et al.** *Biol. Chem.,* 1989, vol. 264, 14653-14661
- **THORPE et al.** *Cancer Res.,* 1987, vol. 47, 5924-5931
- **WAWRZYNCZAK et al.** Immunoconjugates: Antibody Conjugates in Radioimagery and Therapy of Cancer. Oxford U. Press, 1987
- **JOHNSON et al.** *Anticancer Res.,* 1995, vol. 15, 1387-93
- **LAU et al.** *Bioorg Med. Chem.,* 1995, vol. 3 (10), 1299-1304
- **LAU et al.** *Bioorg-Med-Chem.,* 1995, vol. 3 (10), 1305-12
- **CHARI et al.** *Cancer Res.,* 1992, vol. 52, 127-131
- **WIDDISON et al.** *J. Med. Chem.,* 2006, vol. 49, 4392-4408
- **DORONINA et al.** *Nature Biotech.,* 2003, vol. 21, 778-784
- **HAMBLETT et al.** *Clin. Cancer Res.,* 2004, vol. 10, 7063-7070
- **CARTER ; SENTER.** *Cancer J.,* 2008, vol. 14, 154-169
- **MAGERSTADT, M.** Antibody Conjugates And Malignant Disease. CRC Press, 1991
- **BARCHEL, S. W. ; RHODES, B. H.** Radioimaging and Radiotherapy. Elsevier, 1983
- **STRYER.** *Science,* 1968, vol. 162, 526
- **BRAND, L. et al.** *Annual Review of Biochemistry,* 1972, vol. 41, 843-868
- **WENSEL ; MEARES.** Radioimmunoimaging and Radioimmunotherapy. Elsevier, 1983
- **D. COLCHER et al.** *Meth. Enzymol.,* 1986, vol. 121, 802-816
- Developments in Antibody Imaging. **A. R. BRADWELL et al.** Monoclonal Antibodies for Cancer Detection and Therapy. Academic Press, 1985, 65-85

SEQUENCE LISTING

<110> MILLENNIUM PHARMACEUTICALS, INC.

<120> ANTI-GCC ANTIBODY MOLECULES AND USE OF SAME TO TEST FOR SUSCEPTIBILITY TO GCC-TARGETED THERAPY

<130> MPI12-018P1RNWOM

<140> US 61/639,376
<141> 2012-04-27

<160> 89

<170> PatentIn version 3.5

<210> 1
<211> 18
<212> PRT
<213> Escherichia coli

<400> 1

**Asn Thr Phe Tyr Cys Cys Glu Leu Cys Cys Asn Pro Ala Cys Ala Gly**
**1 5 10 15**

Cys Tyr

<210> 2
<211> 3360
<212> DNA
<213> Homo sapiens

<400> 2

**gaccagagag aagcgtgggg aagagtgggc tgagggactc cactagaggc tgtccatctg 60**

**gattccctgc ctccctagga gcccaacaga gcaaagcaag tgggcacaag gagtatggtt 120**

**ctaacgtgat tggggtcatg aagacgttgc tgttggactt ggctttgtgg tcactgctct 180**

**tccagcccgg gtggctgtcc tttagttccc aggtgagtca gaactgccac aatggcagct 240**

**atgaaatcag cgtcctgatg atgggcaact cagcctttgc agagcccctg aaaaacttgg 300**

**aagatgcggt gaatgagggg ctggaaatag tgagaggacg tctgcaaaat gctggcctaa 360**

**atgtgactgt gaacgctact ttcatgtatt cggatggtct gattcataac tcaggcgact 420**

**gccggagtag cacctgtgaa ggcctcgacc tactcaggaa aatttcaaat gcacaacgga 480**

**tgggctgtgt cctcataggg ccctcatgta catactccac cttccagatg taccttgaca 540**

**cagaattgag ctaccccatg atctcagctg gaagttttgg attgtcatgt gactataaag 600**

**aaaccttaac caggctgatg tctccagcta gaaagttgat gtacttcttg gttaactttt 660**

**ggaaaaccaa cgatctgccc ttcaaaactt attcctggag cacttcgtat gtttacaaga 720**

**atggtacaga aactgaggac tgtttctggt accttaatgc tctggaggct agcgtttcct 780**

**atttctccca cgaactcggc tttaaggtgg tgttaagaca agataaggag tttcaggata 840**

tcttaatgga ccacaacagg aaaagcaatg tgattattat gtgtggtggt ccagagttcc 900
tctacaagct gaagggtgac cgagcagtgg ctgaagacat tgtcattatt ctagtggatc 960
ttttcaatga ccagtacttt gaggacaatg tcacagcccc tgactatatg aaaaatgtcc 1020
ttgttctgac gctgtctcct gggaattccc ttctaaatag ctctttctcc aggaatctat 1080
caccaacaaa acgagacttt gctcttgcct atttgaatgg aatcctgctc tttggacata 1140
tgctgaagat atttcttgaa aatggagaaa atattaccac ccccaaattt gctcatgctt 1200
tcaggaatct cacttttgaa gggtatgacg gtccagtgac cttggatgac tggggggatg 1260
ttgacagtac catggtgctt ctgtatacct ctgtggacac caagaaatac aaggttcttt 1320
tgacctatga tacccacgta aataagacct atcctgtgga tatgagcccc acattcactt 1380
ggaagaactc taaacttcct aatgatatta caggccgggg ccctcagatc ctgatgattg 1440
cagtcttcac cctcactgga gctgtggtgc tgctcctgct cgtcgctctc ctgatgctca 1500
gaaaatatag aaaagattat gaacttcgtc agaaaaaatg gtcccacatt cctcctgaaa 1560
atatctttcc tctggagacc aatgagacca atcatgttag cctcaagatc gatgatgaca 1620
aaagacgaga tacaatccag agactacgac agtgcaaata cgacaaaaag cgagtgattc 1680
tcaaagatct caagcacaat gatggtaatt tcactgaaaa acagaagata gaattgaaca 1740
agttgcttca gattgactat tacaacctga ccaagttcta cggcacagtg aaacttgata 1800
ccatgatctt cggggtgata gaatactgtg agagaggatc cctccgggaa gttttaaatg 1860
acacaatttc ctaccctgat ggcacattca tggattggga gtttaagatc tctgtcttgt 1920
atgacattgc taagggaatg tcatatctgc actccagtaa gacagaagtc catggtcgtc 1980
tgaaatctac caactgcgta gtggacagta gaatggtggt gaagatcact gattttggct 2040
gcaattccat tttacctcca aaaaaggacc tgtggacagc tccagagcac ctccgccaag 2100
ccaacatctc tcagaaagga gatgtgtaca gctatgggat catcgcacag gagatcatcc 2160
tgcggaaaga aaccttctac actttgagct gtcgggaccg gaatgagaag attttcagag 2220
tggaaaattc caatggaatg aaacccttcc gcccagattt attcttggaa acagcagagg 2280
aaaaagagct agaagtgtac ctacttgtaa aaaactgttg ggaggaagat ccagaaaaga 2340
gaccagattt caaaaaaatt gagactacac ttgccaagat atttggactt tttcatgacc 2400
aaaaaaatga aagctatatg gataccttga tccgacgtct acagctatat tctcgaaacc 2460
tggaacatct ggtagaggaa aggacacagc tgtacaaggc agagagggac agggctgaca 2520
gacttaactt tatgttgctt ccaaggctag tggtaaagtc tctgaaggag aaaggctttg 2580
tggagccgga actatatgag gaagttacaa tctacttcag tgacattgta ggtttcacta 2640
ctatctgcaa atacagcacc cccatggaag tggtggacat gcttaatgac atctataaga 2700

gttttgacca cattgttgat catcatgatg tctacaaggt ggaaaccatc ggtgatgcgt 2760
acatggtggc tagtggtttg cctaagagaa atggcaatcg gcatgcaata gacattgcca 2820
agatggcctt ggaaatcctc agcttcatgg ggacctttga gctggagcat cttcctggcc 2880
tcccaatatg gattcgcatt ggagttcact ctggtccctg tgctgctgga gttgtgggaa 2940
tcaagatgcc tcgttattgt ctatttggag atacggtcaa cacagcctct aggatggaat 3000
ccactggcct ccctttgaga attcacgtga gtggctccac catagccatc ctgaagagaa 3060
ctgagtgcca gttcctttat gaagtgagag gagaaacata cttaaaggga agaggaaatg 3120
agactaccta ctggctgact gggatgaagg accagaaatt caacctgcca acccctccta 3180
ctgtggagaa tcaacagcgt ttgcaagcag aattttcaga catgattgcc aactctttac 3240
agaaaagaca ggcagcaggg ataagaagcc aaaaacccag acgggtagcc agctataaaa 3300
aaggcactct ggaatacttg cagctgaata ccacagacaa ggagagcacc tatttttaaa 3360

<210> 3
<211> 1073
<212> PRT
<213> Homo sapiens

<400> 3

```
Met Lys Thr Leu Leu Leu Asp Leu Ala Leu Trp Ser Leu Leu Phe Gln
1               5                   10                  15

Pro Gly Trp Leu Ser Phe Ser Ser Gln Val Ser Gln Asn Cys His Asn
            20                  25                  30

Gly Ser Tyr Glu Ile Ser Val Leu Met Met Gly Asn Ser Ala Phe Ala
        35                  40                  45

Glu Pro Leu Lys Asn Leu Glu Asp Ala Val Asn Glu Gly Leu Glu Ile
    50                  55                  60

Val Arg Gly Arg Leu Gln Asn Ala Gly Leu Asn Val Thr Val Asn Ala
65                  70                  75                  80

Thr Phe Met Tyr Ser Asp Gly Leu Ile His Asn Ser Gly Asp Cys Arg
                85                  90                  95

Ser Ser Thr Cys Glu Gly Leu Asp Leu Leu Arg Lys Ile Ser Asn Ala
            100                 105                 110

Gln Arg Met Gly Cys Val Leu Ile Gly Pro Ser Cys Thr Tyr Ser Thr
        115                 120                 125

Phe Gln Met Tyr Leu Asp Thr Glu Leu Ser Tyr Pro Met Ile Ser Ala
    130                 135                 140
```

```
Gly Ser Phe Gly Leu Ser Cys Asp Tyr Lys Glu Thr Leu Thr Arg Leu
145                 150                 155                 160

Met Ser Pro Ala Arg Lys Leu Met Tyr Phe Leu Val Asn Phe Trp Lys
                165                 170                 175

Thr Asn Asp Leu Pro Phe Lys Thr Tyr Ser Trp Ser Thr Ser Tyr Val
            180                 185                 190

Tyr Lys Asn Gly Thr Glu Thr Glu Asp Cys Phe Trp Tyr Leu Asn Ala
        195                 200                 205

Leu Glu Ala Ser Val Ser Tyr Phe Ser His Glu Leu Gly Phe Lys Val
    210                 215                 220

Val Leu Arg Gln Asp Lys Glu Phe Gln Asp Ile Leu Met Asp His Asn
225                 230                 235                 240

Arg Lys Ser Asn Val Ile Ile Met Cys Gly Gly Pro Glu Phe Leu Tyr
                245                 250                 255

Lys Leu Lys Gly Asp Arg Ala Val Ala Glu Asp Ile Val Ile Ile Leu
            260                 265                 270

Val Asp Leu Phe Asn Asp Gln Tyr Phe Glu Asp Asn Val Thr Ala Pro
        275                 280                 285

Asp Tyr Met Lys Asn Val Leu Val Leu Thr Leu Ser Pro Gly Asn Ser
    290                 295                 300

Leu Leu Asn Ser Ser Phe Ser Arg Asn Leu Ser Pro Thr Lys Arg Asp
305                 310                 315                 320

Phe Ala Leu Ala Tyr Leu Asn Gly Ile Leu Leu Phe Gly His Met Leu
                325                 330                 335

Lys Ile Phe Leu Glu Asn Gly Glu Asn Ile Thr Thr Pro Lys Phe Ala
            340                 345                 350

His Ala Phe Arg Asn Leu Thr Phe Glu Gly Tyr Asp Gly Pro Val Thr
        355                 360                 365

Leu Asp Asp Trp Gly Asp Val Asp Ser Thr Met Val Leu Leu Tyr Thr
    370                 375                 380

Ser Val Asp Thr Lys Lys Tyr Lys Val Leu Leu Thr Tyr Asp Thr His
```

385 390 395 400

Val Asn Lys Thr Tyr Pro Val Asp Met Ser Pro Thr Phe Thr Trp Lys
405 410 415

Asn Ser Lys Leu Pro Asn Asp Ile Thr Gly Arg Gly Pro Gln Ile Leu
420 425 430

Met Ile Ala Val Phe Thr Leu Thr Gly Ala Val Val Leu Leu Leu Leu
435 440 445

Val Ala Leu Leu Met Leu Arg Lys Tyr Arg Lys Asp Tyr Glu Leu Arg
450 455 460

Gln Lys Lys Trp Ser His Ile Pro Pro Glu Asn Ile Phe Pro Leu Glu
465 470 475 480

Thr Asn Glu Thr Asn His Val Ser Leu Lys Ile Asp Asp Asp Lys Arg
485 490 495

Arg Asp Thr Ile Gln Arg Leu Arg Gln Cys Lys Tyr Asp Lys Lys Arg
500 505 510

Val Ile Leu Lys Asp Leu Lys His Asn Asp Gly Asn Phe Thr Glu Lys
515 520 525

Gln Lys Ile Glu Leu Asn Lys Leu Leu Gln Ile Asp Tyr Tyr Asn Leu
530 535 540

Thr Lys Phe Tyr Gly Thr Val Lys Leu Asp Thr Met Ile Phe Gly Val
545 550 555 560

Ile Glu Tyr Cys Glu Arg Gly Ser Leu Arg Glu Val Leu Asn Asp Thr
565 570 575

Ile Ser Tyr Pro Asp Gly Thr Phe Met Asp Trp Glu Phe Lys Ile Ser
580 585 590

Val Leu Tyr Asp Ile Ala Lys Gly Met Ser Tyr Leu His Ser Ser Lys
595 600 605

Thr Glu Val His Gly Arg Leu Lys Ser Thr Asn Cys Val Val Asp Ser
610 615 620

Arg Met Val Val Lys Ile Thr Asp Phe Gly Cys Asn Ser Ile Leu Pro
625 630 635 640

```
Pro Lys Lys Asp Leu Trp Thr Ala Pro Glu His Leu Arg Gln Ala Asn
                645                 650                 655

Ile Ser Gln Lys Gly Asp Val Tyr Ser Tyr Gly Ile Ile Ala Gln Glu
            660                 665                 670

Ile Ile Leu Arg Lys Glu Thr Phe Tyr Thr Leu Ser Cys Arg Asp Arg
        675                 680                 685

Asn Glu Lys Ile Phe Arg Val Glu Asn Ser Asn Gly Met Lys Pro Phe
    690                 695                 700

Arg Pro Asp Leu Phe Leu Glu Thr Ala Glu Glu Lys Glu Leu Glu Val
705                 710                 715                 720

Tyr Leu Leu Val Lys Asn Cys Trp Glu Glu Asp Pro Glu Lys Arg Pro
                725                 730                 735

Asp Phe Lys Lys Ile Glu Thr Thr Leu Ala Lys Ile Phe Gly Leu Phe
            740                 745                 750

His Asp Gln Lys Asn Glu Ser Tyr Met Asp Thr Leu Ile Arg Arg Leu
        755                 760                 765

Gln Leu Tyr Ser Arg Asn Leu Glu His Leu Val Glu Glu Arg Thr Gln
    770                 775                 780

Leu Tyr Lys Ala Glu Arg Asp Arg Ala Asp Arg Leu Asn Phe Met Leu
785                 790                 795                 800

Leu Pro Arg Leu Val Val Lys Ser Leu Lys Glu Lys Gly Phe Val Glu
                805                 810                 815

Pro Glu Leu Tyr Glu Glu Val Thr Ile Tyr Phe Ser Asp Ile Val Gly
            820                 825                 830

Phe Thr Thr Ile Cys Lys Tyr Ser Thr Pro Met Glu Val Val Asp Met
        835                 840                 845

Leu Asn Asp Ile Tyr Lys Ser Phe Asp His Ile Val Asp His His Asp
    850                 855                 860

Val Tyr Lys Val Glu Thr Ile Gly Asp Ala Tyr Met Val Ala Ser Gly
865                 870                 875                 880

Leu Pro Lys Arg Asn Gly Asn Arg His Ala Ile Asp Ile Ala Lys Met
                885                 890                 895
```

Ala Leu Glu Ile Leu Ser Phe Met Gly Thr Phe Glu Leu Glu His Leu
900 905 910

Pro Gly Leu Pro Ile Trp Ile Arg Ile Gly Val His Ser Gly Pro Cys
915 920 925

Ala Ala Gly Val Val Gly Ile Lys Met Pro Arg Tyr Cys Leu Phe Gly
930 935 940

Asp Thr Val Asn Thr Ala Ser Arg Met Glu Ser Thr Gly Leu Pro Leu
945 950 955 960

Arg Ile His Val Ser Gly Ser Thr Ile Ala Ile Leu Lys Arg Thr Glu
965 970 975

Cys Gln Phe Leu Tyr Glu Val Arg Gly Glu Thr Tyr Leu Lys Gly Arg
980 985 990

Gly Asn Glu Thr Thr Tyr Trp Leu Thr Gly Met Lys Asp Gln Lys Phe
995 1000 1005

Asn Leu Pro Thr Pro Pro Thr Val Glu Asn Gln Gln Arg Leu Gln
1010 1015 1020

Ala Glu Phe Ser Asp Met Ile Ala Asn Ser Leu Gln Lys Arg Gln
1025 1030 1035

Ala Ala Gly Ile Arg Ser Gln Lys Pro Arg Arg Val Ala Ser Tyr
1040 1045 1050

Lys Lys Gly Thr Leu Glu Tyr Leu Gln Leu Asn Thr Thr Asp Lys
1055 1060 1065

Glu Ser Thr Tyr Phe
1070

<210> 4
<211> 1380
<212> DNA
<213> Artificial Sequence

<220>
<223> Description of Artificial Sequence: Synthetic polynucleotide

<400> 4

atggagactg ggctgcgctg gcttctcctg gtcgctgtgc tcaaaggtgt ccagtgtcag 60

tcagtgaagg agtccggggg aggcctcttc aagccaacgg ataccctgac actcacctgc 120

accgtctctg gattctccct cagtagtcat agaatgaact gggtccgcca gactccaggg 180
aaggggctgg aatggatcgc aatcattact cataatagta tcacatacta cgcgagctgg 240
gcgaaaagcc gatccaccat caccagaaac accagcgaga acacggtgac tctgaaaatg 300
accagtctga cagccgcgga cacggccact tatttctgtg ccagagagga tagtatgggg 360
tattattttg acttgtgggg cccaggcacc ctggtcacca tctcctcagg gcaacctaag 420
gctccatcag tcttcccact ggcccctgc tgcggggaca cacccagctc cacggtgacc 480
ctgggctgcc tggtcaaagg gtacctcccg gagccagtga ccgtgacctg gaactcgggc 540
accctcacca atggggtacg caccttcccg tccgtccggc agtcctcagg cctctactcg 600
ctgagcagcg tggtgagcgt gacctcaagc agccagcccg tcacctgcaa cgtggcccac 660
ccagccacca acaccaaagt ggacaagacc gttgcgccct cgacatgcag caagcccacg 720
tgcccacccc ctgaactcct ggggggaccg tctgtcttca tcttcccccc aaaacccaag 780
gacaccctca tgatctcacg caccccgag gtcacatgcg tggtggtgga cgtgagccag 840
gatgaccccg aggtgcagtt cacatggtac ataaacaacg agcaggtgcg caccgcccgg 900
ccgccgctac gggagcagca gttcaacagc acgatccgcg tggtcagcac cctccccatc 960
gcgcaccagg actggctgag gggcaaggag ttcaagtgca aagtccacaa caaggcactc 1020
ccggcccca tcgagaaaac catctccaaa gccagagggc agcccctgga gccgaaggtc 1080
tacaccatgg gccctcccg ggaggagctg agcagcaggt cggtcagcct gacctgcatg 1140
atcaacggct tctacccttc cgacatctcg gtggagtggg agaagaacgg gaaggcagag 1200
gacaactaca agaccacgcc ggccgtgctg gacagcgacg gctcctactt cctctacagc 1260
aagctctcag tgcccacgag tgagtggcag cggggcgacg tcttcacctg ctccgtgatg 1320
cacgaggcct tgcacaacca ctacacgcag aagtccatct cccgctctcc gggtaaatga 1380

<210> 5
<211> 711
<212> DNA
<213> Artificial Sequence

<220>
<223> Description of Artificial Sequence: Synthetic polynucleotide

<400> 5

```
atggacacga gggcccccac tcagctgctg gggctcctgc tgctctggct cccaggtgcc      60
agatgtgcct atgatatgac ccagactcca gcctctgtgg aggtagctgt gggaggcaca     120
gtcaccatca agtgccaggc cagtcagagc attagtaact ggttagcctg gtatcagcag     180
aaaccagggc agtctcccaa gcccctgatc tacagggcat ccactctggc atctggggtc     240
tcatcgcggt tcagaggcag tggatctggg acacagttca ctctcaccat cagtggcgtg     300
gagtgtgccg atgctgccac ttactactgt cagcagactt atactaataa tcatcttgat     360
aatggtttcg gcggagggac cgaggtggtg gtcaaaggtg atccagttgc acctactgtc     420
ctcatcttcc caccagctgc tgatcaggtg gcaactggaa cagtcaccat cgtgtgtgtg     480
gcgaataaat actttcccga tgtcaccgtc acctgggagg tggatggcac cacccaaaca     540
actggcatcg agaacagtaa aacaccgcag aattctgcag attgtaccta caacctcagc     600
agcactctga cactgaccag cacacagtac aacagccaca aagagtacac ctgcagggtg     660
acccagggca cgacctcagt cgtccagagc ttcaataggg gtgactgtta g              711
```

<210> 6
<211> 1377
<212> DNA
<213> Artificial Sequence

<220>
<223> Description of Artificial Sequence: Synthetic polynucleotide

<400> 6

atggagactg ggctgcgctg gcttctcctg gtcgctgtgc tcaaaggtgt ccagtgtcag 60
tcggtggagg agtccggggg tcgcctggtc acgcctggga cacccctgac actcacctgc 120
acagcctctg gatccgacat cagtaactat gcaatatcct gggtccgcca ggctccaggg 180
aaggggctgg aattcatcgg atatattagt tatggtaaaa gtatatacta cgcgagctgg 240
gcgaaaggcc ggttcgccat ctccaaaacc tcgtcgacca cggtggatct ggaaatcacc 300
agtccgacaa ccgaggacac ggccacctat ttttgtgcca gagaggatag tgctacttat 360
agtcctaact tgtggggccc aggcaccctg gtcaccgtct cctcagggca acctaaggct 420
ccatcagtct tcccactggc cccctgctgc ggggacacac ccagctccac ggtgaccctg 480
ggctgcctgg tcaaagggta cctcccggag ccagtgaccg tgacctggaa ctcgggcacc 540
ctcaccaatg ggtacgcac cttcccgtcc gtccggcagt cctcaggcct ctactcgctg 600
agcagcgtgg tgagcgtgac ctcaagcagc cagcccgtca cctgcaacgt ggcccaccca 660
gccaccaaca ccaaagtgga caagaccgtt gcgccctcga catgcagcaa gcccacgtgc 720
ccaccccctg aactcctggg gggaccgtct gtcttcatct tcccccaaa acccaaggac 780
accctcatga tctcacgcac ccccgaggtc acatgcgtgg tggtggacgt gagccaggat 840
gaccccgagg tgcagttcac atggtacata aacaacgagc aggtgcgcac cgcccggccg 900
ccgctacggg agcagcagtt caacagcacg atccgcgtgg tcagcaccct ccccatcgcg 960
caccaggact ggctgagggg caaggagttc aagtgcaaag tccacaacaa ggcactcccg 1020
gcccccatcg agaaaaccat ctccaaagcc agagggcagc ccctggagcc gaaggtctac 1080
accatgggcc ctccccggga ggagctgagc agcaggtcgg tcagcctgac ctgcatgatc 1140
aacggcttct acccttccga catctcggtg gagtgggaga agaacgggaa ggcagaggac 1200
aactacaaga ccacgccggc cgtgctggac agcgacggct cctacttcct ctacagcaag 1260
ctctcagtgc ccacgagtga gtggcagcgg ggcgacgtct tcacctgctc cgtgatgcac 1320
gaggccttgc acaaccacta cacgcagaag tccatctccc gctctccggg taaatga 1377

<210> 7
<211> 711
<212> DNA
<213> Artificial Sequence

<220>
<223> Description of Artificial Sequence: Synthetic polynucleotide

<400> 7

atggacacga gggcccccac tcagctgctg gggctcctgc tgctctggct cccaggtgcc 60
agatgtgcct atgatatgac ccagactcca gcctctgtgg aggtagctgt gggaggcaca 120
gtcaccatca agtgccaggc cagtcagagt attaacacct acttagcctg gtatcagcag 180
aaaccagggc agcgtcccaa gctcctgatc tacagggcat ccactctggc atctggggtc 240
tcatcgcggt tcaaaggcag tggatctggg acagagttca ctctcaccat cagcggcgtg 300
gagtgtgccg atgctgccac ttactactgt caacagggtt atagttataa taatcttgat 360
cgtgctttcg gcggagggac cgaggtggtg gtcacaggtg atccagttgc acctactgtc 420
ctcatcttcc caccagctgc tgatcaggtg gcaactggaa cagtcaccat cgtgtgtgtg 480
gcgaataaat actttcccga tgtcaccgtc acctgggagg tggatggcac cacccaaaca 540
actggcatcg agaacagtaa aacaccgcag aattctgcag attgtaccta caacctcagc 600
agcactctga cactgaccag cacacagtac aacagccaca aagagtacac ctgcaaggtg 660
acccagggca cgacctcagt cgtccagagc ttcaataggg gtgactgtta g 711

<210> 8
<211> 30
<212> PRT
<213> Homo sapiens

<400> 8

Ile Leu Val Asp Leu Phe Asn Asp Gln Tyr Phe Glu Asp Asn Val Thr
1 5 10 15

Ala Pro Asp Tyr Met Lys Asn Val Leu Val Leu Thr Leu Ser
20 25 30

<210> 9
<211> 25
<212> PRT
<213> Homo sapiens

<400> 9

Phe Ala His Ala Phe Arg Asn Leu Thr Phe Glu Gly Tyr Asp Gly Pro
1 5 10 15

Val Thr Leu Asp Asp Trp Gly Asp Val
20 25

<210> 10
<211> 351
<212> DNA
<213> Artificial Sequence

<220>
<223> Description of Artificial Sequence: Synthetic polynucleotide

<400> 10

```
cagtcagtga aggagtccgg gggaggcctc ttcaagccaa cggataccct gacactcacc      60
tgcaccgtct ctggattctc cctcagtagt catagaatga actgggtccg ccagactcca     120
gggaaggggc tggaatggat cgcaatcatt actcataata gtatcacata ctacgcgagc     180
tgggcgaaaa gccgatccac catcaccaga aacaccagcg agaacacggt gactctgaaa     240
atgaccagtc tgacagccgc ggacacggcc acttatttct gtgccagaga ggatagtatg     300
gggtattatt ttgacttgtg gggcccaggc accctggtca ccatctcctc a              351
```

<210> 11
<211> 117
<212> PRT
<213> Artificial Sequence

<220>
<223> Description of Artificial Sequence: Synthetic polypeptide

<400> 11

```
Gln Ser Val Lys Glu Ser Gly Gly Gly Leu Phe Lys Pro Thr Asp Thr
1               5                   10                  15

Leu Thr Leu Thr Cys Thr Val Ser Gly Phe Ser Leu Ser Ser His Arg
            20                  25                  30

Met Asn Trp Val Arg Gln Thr Pro Gly Lys Gly Leu Glu Trp Ile Ala
        35                  40                  45

Ile Ile Thr His Asn Ser Ile Thr Tyr Tyr Ala Ser Trp Ala Lys Ser
    50                  55                  60

Arg Ser Thr Ile Thr Arg Asn Thr Ser Glu Asn Thr Val Thr Leu Lys
65                  70                  75                  80

Met Thr Ser Leu Thr Ala Ala Asp Thr Ala Thr Tyr Phe Cys Ala Arg

                85                  90                  95

Glu Asp Ser Met Gly Tyr Tyr Phe Asp Leu Trp Gly Pro Gly Thr Leu
            100                 105                 110

Val Thr Ile Ser Ser
        115
```

<210> 12
<211> 330
<212> DNA

<213> Artificial Sequence

<220>
<223> Description of Artificial Sequence: Synthetic polynucleotide

<400> 12

```
gcctatgata tgacccagac tccagcctct gtggaggtag ctgtgggagg cacagtcacc      60
atcaagtgcc aggccagtca gagcattagt aactggttag cctggtatca gcagaaacca     120
gggcagtctc ccaagcccct gatctacagg gcatccactc tggcatctgg ggtctcatcg     180
cggttcagag gcagtggatc tgggacacag ttcactctca ccatcagtgg cgtggagtgt     240
gccgatgctg ccacttacta ctgtcagcag acttatacta ataatcatct tgataatggt     300
ttcggcggag ggaccgaggt ggtggtcaaa                                      330
```

<210> 13
<211> 110
<212> PRT
<213> Artificial Sequence

<220>
<223> Description of Artificial Sequence: Synthetic polypeptide

<400> 13

```
Ala Tyr Asp Met Thr Gln Thr Pro Ala Ser Val Glu Val Ala Val Gly
1               5                   10                  15

Gly Thr Val Thr Ile Lys Cys Gln Ala Ser Gln Ser Ile Ser Asn Trp
            20                  25                  30

Leu Ala Trp Tyr Gln Gln Lys Pro Gly Gln Ser Pro Lys Pro Leu Ile
        35                  40                  45

Tyr Arg Ala Ser Thr Leu Ala Ser Gly Val Ser Ser Arg Phe Arg Gly
    50                  55                  60

Ser Gly Ser Gly Thr Gln Phe Thr Leu Thr Ile Ser Gly Val Glu Cys
65                  70                  75                  80

Ala Asp Ala Ala Thr Tyr Tyr Cys Gln Gln Thr Tyr Thr Asn Asn His
                85                  90                  95

Leu Asp Asn Gly Phe Gly Gly Gly Thr Glu Val Val Val Lys
            100                 105                 110
```

<210> 14
<211> 348
<212> DNA
<213> Artificial Sequence

<220>
<223> Description of Artificial Sequence: Synthetic polynucleotide

<400> 14

```
cagtcggtgg aggagtccgg gggtcgcctg gtcacgcctg ggacacccct gacactcacc      60
tgcacagcct ctggatccga catcagtaac tatgcaatat cctgggtccg ccaggctcca     120
gggaaggggc tggaattcat cggatatatt agttatggta aaagtatata ctacgcgagc     180
tgggcgaaag gccggttcgc catctccaaa acctcgtcga ccacggtgga tctggaaatc     240
accagtccga caaccgagga cacggccacc tatttttgtg ccagagagga tagtgctact     300
tatagtccta acttgtgggg cccaggcacc ctggtcaccg tctcctca                 348
```

<210> 15
<211> 116
<212> PRT
<213> Artificial Sequence

<220>
<223> Description of Artificial Sequence: Synthetic polypeptide

<400> 15

```
Gln Ser Val Glu Glu Ser Gly Gly Arg Leu Val Thr Pro Gly Thr Pro
1               5                   10                  15

Leu Thr Leu Thr Cys Thr Ala Ser Gly Ser Asp Ile Ser Asn Tyr Ala
            20                  25                  30

Ile Ser Trp Val Arg Gln Ala Pro Gly Lys Gly Leu Glu Phe Ile Gly
        35                  40                  45

Tyr Ile Ser Tyr Gly Lys Ser Ile Tyr Tyr Ala Ser Trp Ala Lys Gly
    50                  55                  60

Arg Phe Ala Ile Ser Lys Thr Ser Ser Thr Thr Val Asp Leu Glu Ile
65                  70                  75                  80

Thr Ser Pro Thr Thr Glu Asp Thr Ala Thr Tyr Phe Cys Ala Arg Glu

                85                  90                  95

Asp Ser Ala Thr Tyr Ser Pro Asn Leu Trp Gly Pro Gly Thr Leu Val
            100                 105                 110

Thr Val Ser Ser
        115
```

<210> 16
<211> 330
<212> DNA
<213> Artificial Sequence

<220>
<223> Description of Artificial Sequence: Synthetic polynucleotide

<400> 16

```
gcctatgata tgacccagac tccagcctct gtggaggtag ctgtgggagg cacagtcacc      60
atcaagtgcc aggccagtca gagtattaac acctacttag cctggtatca gcagaaacca     120
gggcagcgtc ccaagctcct gatctacagg gcatccactc tggcatctgg ggtctcatcg     180
cggttcaaag gcagtggatc tgggacagag ttcactctca ccatcagcgg cgtggagtgt     240
gccgatgctg ccacttacta ctgtcaacag ggttatagtt ataataatct tgatcgtgct     300
ttcggcggag ggaccgaggt ggtggtcaca                                      330
```

<210> 17
<211> 110
<212> PRT
<213> Artificial Sequence

<220>
<223> Description of Artificial Sequence: Synthetic polypeptide

<400> 17

```
Ala Tyr Asp Met Thr Gln Thr Pro Ala Ser Val Glu Val Ala Val Gly
1               5                   10                  15

Gly Thr Val Thr Ile Lys Cys Gln Ala Ser Gln Ser Ile Asn Thr Tyr
            20                  25                  30

Leu Ala Trp Tyr Gln Gln Lys Pro Gly Gln Arg Pro Lys Leu Leu Ile
        35                  40                  45

Tyr Arg Ala Ser Thr Leu Ala Ser Gly Val Ser Ser Arg Phe Lys Gly
    50                  55                  60

Ser Gly Ser Gly Thr Glu Phe Thr Leu Thr Ile Ser Gly Val Glu Cys
65                  70                  75                  80

Ala Asp Ala Ala Thr Tyr Tyr Cys Gln Gln Gly Tyr Ser Tyr Asn Asn
                85                  90                  95

Leu Asp Arg Ala Phe Gly Gly Gly Thr Glu Val Val Val Thr
            100                 105                 110
```

<210> 18

<211> 15
<212> DNA
<213> Artificial Sequence

<220>
<223> Description of Artificial Sequence: Synthetic oligonucleotide

<400> 18
agtcatagaa tgaac 15

<210> 19
<211> 48
<212> DNA
<213> Artificial Sequence

<220>
<223> Description of Artificial Sequence: Synthetic oligonucleotide

<400> 19
atcattactc ataatagtat cacatactac gcgagctggg cgaaaagc 48

<210> 20
<211> 30
<212> DNA
<213> Artificial Sequence

<220>
<223> Description of Artificial Sequence: Synthetic oligonucleotide

<400> 20
gaggatagta tggggtatta ttttgacttg 30

<210> 21
<211> 5
<212> PRT
<213> Artificial Sequence

<220>
<223> Description of Artificial Sequence: Synthetic peptide

<400> 21

```
Ser His Arg Met Asn
1               5
```

<210> 22
<211> 16
<212> PRT
<213> Artificial Sequence

<220>
<223> Description of Artificial Sequence: Synthetic peptide

<400> 22

```
Ile Ile Thr His Asn Ser Ile Thr Tyr Tyr Ala Ser Trp Ala Lys Ser
1               5                   10                  15
```

<210> 23
<211> 10
<212> PRT
<213> Artificial Sequence

<220>
<223> Description of Artificial Sequence: Synthetic peptide

<400> 23

```
Glu Asp Ser Met Gly Tyr Tyr Phe Asp Leu
1               5                   10
```

<210> 24
<211> 33
<212> DNA
<213> Artificial Sequence

<220>
<223> Description of Artificial Sequence: Synthetic oligonucleotide

<400> 24
caggccagtc agagcattag taactggtta gcc 33

<210> 25
<211> 21
<212> DNA
<213> Artificial Sequence

<220>
<223> Description of Artificial Sequence: Synthetic oligonucleotide

<400> 25
agggcatcca ctctggcatc t 21

<210> 26
<211> 36
<212> DNA
<213> Artificial Sequence

<220>
<223> Description of Artificial Sequence: Synthetic oligonucleotide

<400> 26
cagcagactt atactaataa tcatcttgat aatggt 36

<210> 27
<211> 11
<212> PRT
<213> Artificial Sequence

<220>
<223> Description of Artificial Sequence: Synthetic peptide

<400> 27

```
Gln Ala Ser Gln Ser Ile Ser Asn Trp Leu Ala
1               5                   10
```

<210> 28
<211> 7
<212> PRT
<213> Artificial Sequence

<220>
<223> Description of Artificial Sequence: Synthetic peptide

<400> 28

Arg Ala Ser Thr Leu Ala Ser
1 5

<210> 29
<211> 12
<212> PRT
<213> Artificial Sequence

<220>
<223> Description of Artificial Sequence: Synthetic peptide

<400> 29

Gln Gln Thr Tyr Thr Asn Asn His Leu Asp Asn Gly
1 5 10

<210> 30
<211> 15
<212> DNA
<213> Artificial Sequence

<220>
<223> Description of Artificial Sequence: Synthetic oligonucleotide

<400> 30
aactatgcaa tatcc 15

<210> 31
<211> 48
<212> DNA
<213> Artificial Sequence

<220>
<223> Description of Artificial Sequence: Synthetic oligonucleotide

<400> 31
tatattagtt atggtaaaag tatatactac gcgagctggg cgaaaggc 48

<210> 32
<211> 12
<212> DNA
<213> Artificial Sequence

<220>
<223> Description of Artificial Sequence: Synthetic oligonucleotide

<400> 32
agtcctaact tg 12

<210> 33
<211> 5
<212> PRT
<213> Artificial Sequence

<220>
<223> Description of Artificial Sequence: Synthetic peptide

<400> 33

Asn Tyr Ala Ile Ser
1 5

<210> 34
<211> 16
<212> PRT
<213> Artificial Sequence

<220>
<223> Description of Artificial Sequence: Synthetic peptide

<400> 34

Tyr Ile Ser Tyr Gly Lys Ser Ile Tyr Tyr Ala Ser Trp Ala Lys Gly
1 5 10 15

<210> 35
<211> 10
<212> PRT
<213> Artificial Sequence

<220>
<223> Description of Artificial Sequence: Synthetic peptide

<400> 35

Glu Asp Ser Ala Thr Tyr Ser Pro Asn Leu
1 5 10

<210> 36
<211> 33
<212> DNA
<213> Artificial Sequence

<220>
<223> Description of Artificial Sequence: Synthetic oligonucleotide

<400> 36
caggccagtc agagtattaa cacctactta gcc 33

<210> 37
<211> 21
<212> DNA
<213> Artificial Sequence

<220>
<223> Description of Artificial Sequence: Synthetic oligonucleotide

<400> 37
agggcatcca ctctggcatc t 21

<210> 38
<211> 36
<212> DNA
<213> Artificial Sequence

<220>
<223> Description of Artificial Sequence: Synthetic oligonucleotide

<400> 38
caacagggtt atagttataa taatcttgat cgtgct 36

<210> 39
<211> 11
<212> PRT
<213> Artificial Sequence

<220>
<223> Description of Artificial Sequence: Synthetic peptide

<400> 39

```
Gln Ala Ser Gln Ser Ile Asn Thr Tyr Leu Ala
1               5                   10
```

<210> 40
<211> 7
<212> PRT
<213> Artificial Sequence

<220>
<223> Description of Artificial Sequence: Synthetic peptide

<400> 40

```
Arg Ala Ser Thr Leu Ala Ser
1               5
```

<210> 41
<211> 12
<212> PRT
<213> Artificial Sequence

<220>
<223> Description of Artificial Sequence: Synthetic peptide

<400> 41

```
Gln Gln Gly Tyr Ser Tyr Asn Asn Leu Asp Arg Ala
1               5                   10
```

<210> 42
<211> 459
<212> PRT
<213> Artificial Sequence

<220>
<223> Description of Artificial Sequence: Synthetic polypeptide

<400> 42

```
Met Glu Thr Gly Leu Arg Trp Leu Leu Leu Val Ala Val Leu Lys Gly
1               5                   10                  15

Val Gln Cys Gln Ser Val Lys Glu Ser Gly Gly Gly Leu Phe Lys Pro
            20                  25                  30

Thr Asp Thr Leu Thr Leu Thr Cys Thr Val Ser Gly Phe Ser Leu Ser
        35                  40                  45

Ser His Arg Met Asn Trp Val Arg Gln Thr Pro Gly Lys Gly Leu Glu
    50                  55                  60

Trp Ile Ala Ile Ile Thr His Asn Ser Ile Thr Tyr Tyr Ala Ser Trp
65                  70                  75                  80

Ala Lys Ser Arg Ser Thr Ile Thr Arg Asn Thr Ser Glu Asn Thr Val
                85                  90                  95

Thr Leu Lys Met Thr Ser Leu Thr Ala Ala Asp Thr Ala Thr Tyr Phe
            100                 105                 110

Cys Ala Arg Glu Asp Ser Met Gly Tyr Tyr Phe Asp Leu Trp Gly Pro
        115                 120                 125

Gly Thr Leu Val Thr Ile Ser Ser Gly Gln Pro Lys Ala Pro Ser Val
    130                 135                 140
```

```
Phe Pro Leu Ala Pro Cys Cys Gly Asp Thr Pro Ser Ser Thr Val Thr
145                 150                 155                 160

Leu Gly Cys Leu Val Lys Gly Tyr Leu Pro Glu Pro Val Thr Val Thr
                165                 170                 175

Trp Asn Ser Gly Thr Leu Thr Asn Gly Val Arg Thr Phe Pro Ser Val
            180                 185                 190

Arg Gln Ser Ser Gly Leu Tyr Ser Leu Ser Ser Val Val Ser Val Thr
        195                 200                 205

Ser Ser Ser Gln Pro Val Thr Cys Asn Val Ala His Pro Ala Thr Asn
    210                 215                 220

Thr Lys Val Asp Lys Thr Val Ala Pro Ser Thr Cys Ser Lys Pro Thr
225                 230                 235                 240

Cys Pro Pro Pro Glu Leu Leu Gly Gly Pro Ser Val Phe Ile Phe Pro
                245                 250                 255

Pro Lys Pro Lys Asp Thr Leu Met Ile Ser Arg Thr Pro Glu Val Thr
            260                 265                 270

Cys Val Val Val Asp Val Ser Gln Asp Asp Pro Glu Val Gln Phe Thr
        275                 280                 285

Trp Tyr Ile Asn Asn Glu Gln Val Arg Thr Ala Arg Pro Pro Leu Arg
    290                 295                 300

Glu Gln Gln Phe Asn Ser Thr Ile Arg Val Val Ser Thr Leu Pro Ile
305                 310                 315                 320

Ala His Gln Asp Trp Leu Arg Gly Lys Glu Phe Lys Cys Lys Val His
                325                 330                 335

Asn Lys Ala Leu Pro Ala Pro Ile Glu Lys Thr Ile Ser Lys Ala Arg
            340                 345                 350

Gly Gln Pro Leu Glu Pro Lys Val Tyr Thr Met Gly Pro Pro Arg Glu
        355                 360                 365

Glu Leu Ser Ser Arg Ser Val Ser Leu Thr Cys Met Ile Asn Gly Phe
    370                 375                 380

Tyr Pro Ser Asp Ile Ser Val Glu Trp Glu Lys Asn Gly Lys Ala Glu
385                 390                 395                 400
```

Asp Asn Tyr Lys Thr Thr Pro Ala Val Leu Asp Ser Asp Gly Ser Tyr
405 410 415

Phe Leu Tyr Ser Lys Leu Ser Val Pro Thr Ser Glu Trp Gln Arg Gly
420 425 430

Asp Val Phe Thr Cys Ser Val Met His Glu Ala Leu His Asn His Tyr
435 440 445

Thr Gln Lys Ser Ile Ser Arg Ser Pro Gly Lys
450 455

<210> 43
<211> 236
<212> PRT
<213> Artificial Sequence

<220>
<223> Description of Artificial Sequence: Synthetic polypeptide

<400> 43

Met Asp Thr Arg Ala Pro Thr Gln Leu Leu Gly Leu Leu Leu Leu Trp
1 5 10 15

Leu Pro Gly Ala Arg Cys Ala Tyr Asp Met Thr Gln Thr Pro Ala Ser
20 25 30

Val Glu Val Ala Val Gly Gly Thr Val Thr Ile Lys Cys Gln Ala Ser
35 40 45

Gln Ser Ile Ser Asn Trp Leu Ala Trp Tyr Gln Gln Lys Pro Gly Gln
50 55 60

Ser Pro Lys Pro Leu Ile Tyr Arg Ala Ser Thr Leu Ala Ser Gly Val
65 70 75 80

Ser Ser Arg Phe Arg Gly Ser Gly Ser Gly Thr Gln Phe Thr Leu Thr
85 90 95

Ile Ser Gly Val Glu Cys Ala Asp Ala Ala Thr Tyr Tyr Cys Gln Gln
100 105 110

Thr Tyr Thr Asn Asn His Leu Asp Asn Gly Phe Gly Gly Gly Thr Glu
115 120 125

Val Val Val Lys Gly Asp Pro Val Ala Pro Thr Val Leu Ile Phe Pro
130 135 140

Pro Ala Ala Asp Gln Val Ala Thr Gly Thr Val Thr Ile Val Cys Val
145 150 155 160

Ala Asn Lys Tyr Phe Pro Asp Val Thr Val Thr Trp Glu Val Asp Gly
165 170 175

Thr Thr Gln Thr Thr Gly Ile Glu Asn Ser Lys Thr Pro Gln Asn Ser
180 185 190

Ala Asp Cys Thr Tyr Asn Leu Ser Ser Thr Leu Thr Leu Thr Ser Thr
195 200 205

Gln Tyr Asn Ser His Lys Glu Tyr Thr Cys Arg Val Thr Gln Gly Thr
210 215 220

Thr Ser Val Val Gln Ser Phe Asn Arg Gly Asp Cys
225 230 235

<210> 44
<211> 458
<212> PRT
<213> Artificial Sequence

<220>
<223> Description of Artificial Sequence: Synthetic polypeptide

<400> 44

Met Glu Thr Gly Leu Arg Trp Leu Leu Leu Val Ala Val Leu Lys Gly
1 5 10 15

Val Gln Cys Gln Ser Val Glu Glu Ser Gly Gly Arg Leu Val Thr Pro
20 25 30

Gly Thr Pro Leu Thr Leu Thr Cys Thr Ala Ser Gly Ser Asp Ile Ser
35 40 45

Asn Tyr Ala Ile Ser Trp Val Arg Gln Ala Pro Gly Lys Gly Leu Glu
50 55 60

Phe Ile Gly Tyr Ile Ser Tyr Gly Lys Ser Ile Tyr Tyr Ala Ser Trp
65 70 75 80

Ala Lys Gly Arg Phe Ala Ile Ser Lys Thr Ser Ser Thr Thr Val Asp
85 90 95

Leu Glu Ile Thr Ser Pro Thr Thr Glu Asp Thr Ala Thr Tyr Phe Cys
100 105 110

Ala Arg Glu Asp Ser Ala Thr Tyr Ser Pro Asn Leu Trp Gly Pro Gly
        115                 120                 125

Thr Leu Val Thr Val Ser Ser Gly Gln Pro Lys Ala Pro Ser Val Phe
    130                 135                 140

Pro Leu Ala Pro Cys Cys Gly Asp Thr Pro Ser Ser Thr Val Thr Leu
145                 150                 155                 160

Gly Cys Leu Val Lys Gly Tyr Leu Pro Glu Pro Val Thr Val Thr Trp
                165                 170                 175

Asn Ser Gly Thr Leu Thr Asn Gly Val Arg Thr Phe Pro Ser Val Arg
            180                 185                 190

Gln Ser Ser Gly Leu Tyr Ser Leu Ser Ser Val Val Ser Val Thr Ser
        195                 200                 205

Ser Ser Gln Pro Val Thr Cys Asn Val Ala His Pro Ala Thr Asn Thr
    210                 215                 220

Lys Val Asp Lys Thr Val Ala Pro Ser Thr Cys Ser Lys Pro Thr Cys
225                 230                 235                 240

Pro Pro Pro Glu Leu Leu Gly Gly Pro Ser Val Phe Ile Phe Pro Pro
                245                 250                 255

Lys Pro Lys Asp Thr Leu Met Ile Ser Arg Thr Pro Glu Val Thr Cys
            260                 265                 270

Val Val Val Asp Val Ser Gln Asp Asp Pro Glu Val Gln Phe Thr Trp
        275                 280                 285

Tyr Ile Asn Asn Glu Gln Val Arg Thr Ala Arg Pro Pro Leu Arg Glu
    290                 295                 300

Gln Gln Phe Asn Ser Thr Ile Arg Val Val Ser Thr Leu Pro Ile Ala
305                 310                 315                 320

His Gln Asp Trp Leu Arg Gly Lys Glu Phe Lys Cys Lys Val His Asn
                325                 330                 335

Lys Ala Leu Pro Ala Pro Ile Glu Lys Thr Ile Ser Lys Ala Arg Gly
            340                 345                 350

Gln Pro Leu Glu Pro Lys Val Tyr Thr Met Gly Pro Pro Arg Glu Glu
        355                 360                 365

Leu Ser Ser Arg Ser Val Ser Leu Thr Cys Met Ile Asn Gly Phe Tyr
370 375 380

Pro Ser Asp Ile Ser Val Glu Trp Glu Lys Asn Gly Lys Ala Glu Asp
385 390 395 400

Asn Tyr Lys Thr Thr Pro Ala Val Leu Asp Ser Asp Gly Ser Tyr Phe
405 410 415

Leu Tyr Ser Lys Leu Ser Val Pro Thr Ser Glu Trp Gln Arg Gly Asp
420 425 430

Val Phe Thr Cys Ser Val Met His Glu Ala Leu His Asn His Tyr Thr
435 440 445

Gln Lys Ser Ile Ser Arg Ser Pro Gly Lys
450 455

<210> 45
<211> 236
<212> PRT
<213> Artificial Sequence

<220>
<223> Description of Artificial Sequence: Synthetic polypeptide

<400> 45

Met Asp Thr Arg Ala Pro Thr Gln Leu Leu Gly Leu Leu Leu Leu Trp
1 5 10 15

Leu Pro Gly Ala Arg Cys Ala Tyr Asp Met Thr Gln Thr Pro Ala Ser
20 25 30

Val Glu Val Ala Val Gly Gly Thr Val Thr Ile Lys Cys Gln Ala Ser
35 40 45

Gln Ser Ile Asn Thr Tyr Leu Ala Trp Tyr Gln Gln Lys Pro Gly Gln
50 55 60

Arg Pro Lys Leu Leu Ile Tyr Arg Ala Ser Thr Leu Ala Ser Gly Val
65 70 75 80

Ser Ser Arg Phe Lys Gly Ser Gly Ser Gly Thr Glu Phe Thr Leu Thr
85 90 95

Ile Ser Gly Val Glu Cys Ala Asp Ala Ala Thr Tyr Tyr Cys Gln Gln
100 105 110

```
Gly Tyr Ser Tyr Asn Asn Leu Asp Arg Ala Phe Gly Gly Gly Thr Glu
        115                 120                 125

Val Val Val Thr Gly Asp Pro Val Ala Pro Thr Val Leu Ile Phe Pro
    130                 135                 140

Pro Ala Ala Asp Gln Val Ala Thr Gly Thr Val Thr Ile Val Cys Val
145                 150                 155                 160

Ala Asn Lys Tyr Phe Pro Asp Val Thr Val Thr Trp Glu Val Asp Gly
                165                 170                 175

Thr Thr Gln Thr Thr Gly Ile Glu Asn Ser Lys Thr Pro Gln Asn Ser
            180                 185                 190

Ala Asp Cys Thr Tyr Asn Leu Ser Ser Thr Leu Thr Leu Thr Ser Thr
        195                 200                 205

Gln Tyr Asn Ser His Lys Glu Tyr Thr Cys Lys Val Thr Gln Gly Thr
    210                 215                 220

Thr Ser Val Val Gln Ser Phe Asn Arg Gly Asp Cys
225                 230                 235
```

<210> 46
<211> 420
<212> PRT
<213> Homo sapiens

<400> 46

Met Lys Thr Leu Leu Leu Asp Leu Ala Leu Trp Ser Leu Leu Phe Gln
1 5 10 15

Pro Gly Trp Leu Ser Phe Ser Ser Gln Val Ser Gln Asn Cys His Asn
20 25 30

Gly Ser Tyr Glu Ile Ser Val Leu Met Met Gly Asn Ser Ala Phe Ala
35 40 45

Glu Pro Leu Lys Asn Leu Glu Asp Ala Val Asn Glu Gly Leu Glu Ile
50 55 60

Val Arg Gly Arg Leu Gln Asn Ala Gly Leu Asn Val Thr Val Asn Ala
65 70 75 80

Thr Phe Met Tyr Ser Asp Gly Leu Ile His Asn Ser Gly Asp Cys Arg
85 90 95

Ser Ser Thr Cys Glu Gly Leu Asp Leu Leu Arg Lys Ile Ser Asn Ala

```
            100                 105                 110

Gln Arg Met Gly Cys Val Leu Ile Gly Pro Ser Cys Thr Tyr Ser Thr
        115                 120                 125

Phe Gln Met Tyr Leu Asp Thr Glu Leu Ser Tyr Pro Met Ile Ser Ala
    130                 135                 140

Gly Ser Phe Gly Leu Ser Cys Asp Tyr Lys Glu Thr Leu Thr Arg Leu
145                 150                 155                 160

Met Ser Pro Ala Arg Lys Leu Met Tyr Phe Leu Val Asn Phe Trp Lys
                165                 170                 175

Thr Asn Asp Leu Pro Phe Lys Thr Tyr Ser Trp Ser Thr Ser Tyr Val
            180                 185                 190

Tyr Lys Asn Gly Thr Glu Thr Glu Asp Cys Phe Trp Tyr Leu Asn Ala
        195                 200                 205

Leu Glu Ala Ser Val Ser Tyr Phe Ser His Glu Leu Gly Phe Lys Val
    210                 215                 220

Val Leu Arg Gln Asp Lys Glu Phe Gln Asp Ile Leu Met Asp His Asn
225                 230                 235                 240

Arg Lys Ser Asn Val Ile Ile Met Cys Gly Gly Pro Glu Phe Leu Tyr
                245                 250                 255

Lys Leu Lys Gly Asp Arg Ala Val Ala Glu Asp Ile Val Ile Ile Leu
            260                 265                 270

Val Asp Leu Phe Asn Asp Gln Tyr Phe Glu Asp Asn Val Thr Ala Pro
        275                 280                 285

Asp Tyr Met Lys Asn Val Leu Val Leu Thr Leu Ser Pro Gly Asn Ser
    290                 295                 300

Leu Leu Asn Ser Ser Phe Ser Arg Asn Leu Ser Pro Thr Lys Arg Asp
305                 310                 315                 320

Phe Ala Leu Ala Tyr Leu Asn Gly Ile Leu Leu Phe Gly His Met Leu
                325                 330                 335

Lys Ile Phe Leu Glu Asn Gly Glu Asn Ile Thr Thr Pro Lys Phe Ala
            340                 345                 350
```

```
His Ala Phe Arg Asn Leu Thr Phe Glu Gly Tyr Asp Gly Pro Val Thr
        355                 360                 365

Leu Asp Asp Trp Gly Asp Val Asp Ser Thr Met Val Leu Leu Tyr Thr
    370                 375                 380

Ser Val Asp Thr Lys Lys Tyr Lys Val Leu Leu Thr Tyr Asp Thr His
385                 390                 395                 400

Val Asn Lys Thr Tyr Pro Val Asp Met Ser Pro Thr Phe Thr Trp Lys
                405                 410                 415

Asn Ser Lys Leu
            420
```

<210> 47
<211> 2028
<212> DNA
<213> Artificial Sequence

<220>
<223> Description of Artificial Sequence: Synthetic polynucleotide

<400> 47

```
cgcggatccc tcaccatgaa gacgttgctg ttggacttgg ctttgtggtc actgctcttc      60
cagcccgggt ggctgtcctt tagttcccag gtgagtcaga actgccacaa tggcagctat     120
gaaatcagcg tcctgatgat gggcaactca gcctttgcag agcccctgaa aaacttggaa     180
gatgcggtga atgaggggct ggaaatagtg agaggacgtc tgcaaaatgc tggcctaaat     240
gtgactgtga acgctacttt catgtattcg gatggtctga ttcataactc aggcgactgc     300
cggagtagca cctgtgaagg cctcgaccta ctcaggaaaa tttcaaatgc acaacggatg     360
ggctgtgtcc tcatagggcc ctcatgtaca tactccacct tccagatgta ccttgacaca     420
gaattgagct accccatgat ctcagctgga agttttggat tgtcatgtga ctataaagaa     480
accttaacca ggctgatgtc tccagctaga aagttgatgt acttcttggt taacttttgg     540
aaaaccaacg atctgccctt caaaacttat tcctggagca cttcgtatgt ttacaagaat     600
ggtacagaaa ctgaggactg tttctggtac cttaatgctc tggaggctag cgtttcctat     660
ttctcccacg aactcggctt taaggtggtg ttaagacaag ataaggagtt tcaggatatc     720
ttaatggacc acaacaggaa aagcaatgtg attattatgt gtggtggtcc agagttcctc     780
tacaagctga agggtgaccg agcagtggct gaagacattg tcattattct agtggatctt     840
ttcaatgacc agtacttgga ggacaatgtc acagcccctg actatatgaa aaatgtcctt     900
gttctgacgc tgtctcctgg gaattccctt ctaaatagct ctttctccag gaatctatca     960
ccaacaaaac gagactttgc tcttgcctat ttgaatggaa tcctgctctt tggacatatg    1020
```

```
ctgaagatat ttcttgaaaa tggagaaaat attaccaccc ccaaatttgc tcatgctttc    1080
aggaatctca cttttgaagg gtatgacggt ccagtgacct tggatgactg gggggatgtt    1140
gacagtacca tggtgcttct gtatacctct gtggacacca agaaatacaa ggttcttttg    1200
acctatgata cccacgtaaa taagacctat cctgtggata tgagccccac attcacttgg    1260
aagaactcta aacttcctaa tgatattaca ggccggggcc ctcagcccag agtgcccata    1320
acacagaacc cctgtcctcc actcaaagag tgtcccccat gcgcagctcc agacctcgca    1380
ggtgcaccat ccgtcttcat cttccctcca aagatcaagg atgtactcat gatctccctg    1440
agccccatgg tcacatgtgt ggtggtggat gtgagcgagg atgacccaga cgtccagatc    1500
agctggtttg tgaacaacgt ggaagtacac acagctcaga cacaaaccca tagagaggat    1560
tacaacagta ctctccgggt ggtcagtgcc ctccccatcc agcaccagga ctggatgagt    1620
ggcaaggcat tcaaatgcaa ggtcaacaac agagccctcc catcccccat cgagaaaacc    1680
atctcaaaac ccagagggcc agtaagagct ccacaggtat atgtcttgcc tccaccagca    1740
gaagagatga ctaagaaaga gttcagtctg acctgcatga tcacaggctt cttacctgcc    1800
gaaattgctg tggactggac cagcaatggg cgtacagagc aaaactacaa gaacaccgca    1860
acagtcctgg actctgatgg ttcttacttc atgtacagca agctcagagt acaaaagagc    1920
acttgggaaa gaggaagtct tttcgcctgc tcagtggtcc acgagggtct gcacaatcac    1980
cttacgacta agaccatctc ccggtctctg ggtaaataat ctagagca                 2028
```

<210> 48
<211> 667
<212> PRT
<213> Artificial Sequence

<220>
<223> Description of Artificial Sequence: Synthetic polypeptide

<400> 48

```
Met Lys Thr Leu Leu Leu Asp Leu Ala Leu Trp Ser Leu Leu Phe Gln
1               5                   10                  15

Pro Gly Trp Leu Ser Phe Ser Ser Gln Val Ser Gln Asn Cys His Asn
            20                  25                  30

Gly Ser Tyr Glu Ile Ser Val Leu Met Met Gly Asn Ser Ala Phe Ala
        35                  40                  45

Glu Pro Leu Lys Asn Leu Glu Asp Ala Val Asn Glu Gly Leu Glu Ile
    50                  55                  60

Val Arg Gly Arg Leu Gln Asn Ala Gly Leu Asn Val Thr Val Asn Ala
```

65 70 75 80

Thr Phe Met Tyr Ser Asp Gly Leu Ile His Asn Ser Gly Asp Cys Arg
85 90 95

Ser Ser Thr Cys Glu Gly Leu Asp Leu Leu Arg Lys Ile Ser Asn Ala
100 105 110

Gln Arg Met Gly Cys Val Leu Ile Gly Pro Ser Cys Thr Tyr Ser Thr
115 120 125

Phe Gln Met Tyr Leu Asp Thr Glu Leu Ser Tyr Pro Met Ile Ser Ala
130 135 140

Gly Ser Phe Gly Leu Ser Cys Asp Tyr Lys Glu Thr Leu Thr Arg Leu
145 150 155 160

Met Ser Pro Ala Arg Lys Leu Met Tyr Phe Leu Val Asn Phe Trp Lys
165 170 175

Thr Asn Asp Leu Pro Phe Lys Thr Tyr Ser Trp Ser Thr Ser Tyr Val
180 185 190

Tyr Lys Asn Gly Thr Glu Thr Glu Asp Cys Phe Trp Tyr Leu Asn Ala
195 200 205

Leu Glu Ala Ser Val Ser Tyr Phe Ser His Glu Leu Gly Phe Lys Val
210 215 220

Val Leu Arg Gln Asp Lys Glu Phe Gln Asp Ile Leu Met Asp His Asn
225 230 235 240

Arg Lys Ser Asn Val Ile Ile Met Cys Gly Gly Pro Glu Phe Leu Tyr
245 250 255

Lys Leu Lys Gly Asp Arg Ala Val Ala Glu Asp Ile Val Ile Ile Leu
260 265 270

Val Asp Leu Phe Asn Asp Gln Tyr Leu Glu Asp Asn Val Thr Ala Pro
275 280 285

Asp Tyr Met Lys Asn Val Leu Val Leu Thr Leu Ser Pro Gly Asn Ser
290 295 300

Leu Leu Asn Ser Ser Phe Ser Arg Asn Leu Ser Pro Thr Lys Arg Asp
305 310 315 320

Phe Ala Leu Ala Tyr Leu Asn Gly Ile Leu Leu Phe Gly His Met Leu
325 330 335

Lys Ile Phe Leu Glu Asn Gly Glu Asn Ile Thr Thr Pro Lys Phe Ala
340 345 350

His Ala Phe Arg Asn Leu Thr Phe Glu Gly Tyr Asp Gly Pro Val Thr
355 360 365

Leu Asp Asp Trp Gly Asp Val Asp Ser Thr Met Val Leu Leu Tyr Thr
370 375 380

Ser Val Asp Thr Lys Lys Tyr Lys Val Leu Leu Thr Tyr Asp Thr His
385 390 395 400

Val Asn Lys Thr Tyr Pro Val Asp Met Ser Pro Thr Phe Thr Trp Lys
405 410 415

Asn Ser Lys Leu Pro Asn Asp Ile Thr Gly Arg Gly Pro Gln Pro Arg
420 425 430

Val Pro Ile Thr Gln Asn Pro Cys Pro Pro Leu Lys Glu Cys Pro Pro
435 440 445

Cys Ala Ala Pro Asp Leu Ala Gly Ala Pro Ser Val Phe Ile Phe Pro
450 455 460

Pro Lys Ile Lys Asp Val Leu Met Ile Ser Leu Ser Pro Met Val Thr
465 470 475 480

Cys Val Val Val Asp Val Ser Glu Asp Asp Pro Asp Val Gln Ile Ser
485 490 495

Trp Phe Val Asn Asn Val Glu Val His Thr Ala Gln Thr Gln Thr His
500 505 510

Arg Glu Asp Tyr Asn Ser Thr Leu Arg Val Val Ser Ala Leu Pro Ile
515 520 525

Gln His Gln Asp Trp Met Ser Gly Lys Ala Phe Lys Cys Lys Val Asn
530 535 540

Asn Arg Ala Leu Pro Ser Pro Ile Glu Lys Thr Ile Ser Lys Pro Arg
545 550 555 560

Gly Pro Val Arg Ala Pro Gln Val Tyr Val Leu Pro Pro Pro Ala Glu
565 570 575

```
Glu Met Thr Lys Lys Glu Phe Ser Leu Thr Cys Met Ile Thr Gly Phe
            580                 585                 590

Leu Pro Ala Glu Ile Ala Val Asp Trp Thr Ser Asn Gly Arg Thr Glu
        595                 600                 605

Gln Asn Tyr Lys Asn Thr Ala Thr Val Leu Asp Ser Asp Gly Ser Tyr
    610                 615                 620

Phe Met Tyr Ser Lys Leu Arg Val Gln Lys Ser Thr Trp Glu Arg Gly
625                 630                 635                 640

Ser Leu Phe Ala Cys Ser Val Val His Glu Gly Leu His Asn His Leu
                645                 650                 655

Thr Thr Lys Thr Ile Ser Arg Ser Leu Gly Lys
            660                 665
```

<210> 49
<211> 41
<212> DNA
<213> Artificial Sequence

<220>
<223> Description of Artificial Sequence: Synthetic primer

<400> 49
cgcggatccc tcaccatgaa gacgttgctg ttggacttgg c 41

<210> 50
<211> 42
<212> DNA
<213> Artificial Sequence

<220>
<223> Description of Artificial Sequence: Synthetic primer

<400> 50
tgggcactct gggctgaggg ccccggcctg taatatcatt ag 42

<210> 51
<211> 49
<212> DNA
<213> Artificial Sequence

<220>
<223> Description of Artificial Sequence: Synthetic primer

<400> 51
caggccgggg ccctcagccc agagtgccca taacacagaa cccctgtcc 49

<210> 52
<211> 40
<212> DNA

<213> Artificial Sequence

<220>
<223> Description of Artificial Sequence: Synthetic primer

<400> 52
tgctctagat tatttaccca gagaccggga gatggtctta 40

<210> 53
<211> 20
<212> DNA
<213> Artificial Sequence

<220>
<223> Description of Artificial Sequence: Synthetic primer

<400> 53
acctgtggag ctcttactgg 20

<210> 54
<211> 20
<212> DNA
<213> Artificial Sequence

<220>
<223> Description of Artificial Sequence: Synthetic primer

<400> 54
catttcaggt gtcgtgagga 20

<210> 55
<211> 18
<212> DNA
<213> Artificial Sequence

<220>
<223> Description of Artificial Sequence: Synthetic primer

<400> 55
atttaggtga cactatag 18

<210> 56
<211> 17
<212> DNA
<213> Artificial Sequence

<220>
<223> Description of Artificial Sequence: Synthetic primer

<400> 56
gttttcccag tcacgac 17

<210> 57
<211> 16
<212> DNA
<213> Artificial Sequence

<220>

<223> Description of Artificial Sequence: Synthetic primer

<400> 57
aacagctatg accatg 16

<210> 58
<211> 8
<212> PRT
<213> Mus sp.

<400> 58

Pro Arg Val Pro Ile Thr Glu Asn
1 5

<210> 59
<211> 4
<212> PRT
<213> Mus sp.

<400> 59

Leu Leu Gly Gly
1

<210> 60
<211> 4
<212> PRT
<213> Artificial Sequence

<220>
<223> Description of Artificial Sequence: Synthetic peptide

<400> 60

Leu Ala Gly Ala
1

<210> 61
<211> 4
<212> PRT
<213> Mus sp.

<400> 61

Lys Lys Gly Gly
1

<210> 62
<211> 4
<212> PRT
<213> Artificial Sequence

<220>
<223> Description of Artificial Sequence: Synthetic peptide

<400> 62

Lys Ala Gly Ala
1

<210> 63
<211> 5
<212> PRT
<213> Mus sp.

<400> 63

Glu Phe Lys Cys Lys
1               5

<210> 64
<211> 5
<212> PRT
<213> Artificial Sequence

<220>
<223> Description of Artificial Sequence: Synthetic peptide

<400> 64

Ala Phe Lys Cys Lys
1               5

<210> 65
<211> 4
<212> PRT
<213> Artificial Sequence

<220>
<223> Description of Artificial Sequence: Synthetic peptide

<400> 65

Phe Lys Cys Lys
1

<210> 66
<211> 5
<212> PRT
<213> Homo sapiens

<400> 66

Gly Arg Gly Pro Gln
1               5

<210> 67
<211> 5
<212> PRT
<213> Artificial Sequence

<220>
<223> Description of Artificial Sequence: Synthetic peptide

<400> 67

Gly Tyr Tyr Trp Ser
1 5

<210> 68
<211> 16
<212> PRT
<213> Artificial Sequence

<220>
<223> Description of Artificial Sequence: Synthetic peptide

<400> 68

Glu Ile Asn His Arg Gly Asn Thr Asn Asp Asn Pro Ser Leu Lys Ser
1 5 10 15

<210> 69
<211> 11
<212> PRT
<213> Artificial Sequence

<220>
<223> Description of Artificial Sequence: Synthetic peptide

<400> 69

Glu Arg Gly Tyr Thr Tyr Gly Asn Phe Asp His
1 5 10

<210> 70
<211> 11
<212> PRT
<213> Artificial Sequence

<220>
<223> Description of Artificial Sequence: Synthetic peptide

<400> 70

Arg Ala Ser Gln Ser Val Ser Arg Asn Leu Ala
1 5 10

<210> 71
<211> 7
<212> PRT
<213> Artificial Sequence

<220>
<223> Description of Artificial Sequence: Synthetic peptide

<400> 71

Gly Ala Ser Thr Arg Ala Thr

1 5

<210> 72
<211> 9
<212> PRT
<213> Artificial Sequence

<220>
<223> Description of Artificial Sequence: Synthetic peptide

<400> 72

```
Gln Gln Tyr Lys Thr Trp Pro Arg Thr
1               5
```

<210> 73
<211> 15
<212> DNA
<213> Artificial Sequence

<220>
<223> Description of Artificial Sequence: Synthetic oligonucleotide

<400> 73
ggttactact ggagc 15

<210> 74
<211> 45
<212> DNA
<213> Artificial Sequence

<220>
<223> Description of Artificial Sequence: Synthetic oligonucleotide

<400> 74
gaaatcaatc atcgtggaaa caccaacgac aacccgtccc tcaag 45

<210> 75
<211> 33
<212> DNA
<213> Artificial Sequence

<220>
<223> Description of Artificial Sequence: Synthetic oligonucleotide

<400> 75
gaacgtggat acacctatgg taactttgac cac 33

<210> 76
<211> 33
<212> DNA
<213> Artificial Sequence

<220>
<223> Description of Artificial Sequence: Synthetic oligonucleotide

<400> 76
agggccagtc agagtgttag cagaaactta gcc 33

<210> 77

<211> 21
<212> DNA
<213> Artificial Sequence

<220>
<223> Description of Artificial Sequence: Synthetic oligonucleotide

<400> 77
ggtgcatcca ccagggccac t 21

<210> 78
<211> 27
<212> DNA
<213> Artificial Sequence

<220>
<223> Description of Artificial Sequence: Synthetic oligonucleotide

<400> 78
cagcagtata aaacctggcc tcggacg 27

<210> 79
<211> 119
<212> PRT
<213> Artificial Sequence

<220>
<223> Description of Artificial Sequence: Synthetic polypeptide

<400> 79

```
Gln Val Gln Leu Gln Gln Trp Gly Ala Gly Leu Leu Lys Pro Ser Glu
1               5                   10                  15

Thr Leu Ser Leu Thr Cys Ala Val Phe Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Arg Gly Asn Thr Asn Asp Asn Pro Ser Leu Lys
    50                  55                  60

Ser Arg Val Thr Ile Ser Val Asp Thr Ser Lys Asn Gln Phe Ala Leu
65                  70                  75                  80

Lys Leu Ser Ser Val Thr Ala Ala Asp Thr Ala Val Tyr Tyr Cys Ala
```

85 90 95

Arg Glu Arg Gly Tyr Thr Tyr Gly Asn Phe Asp His Trp Gly Gln Gly
100 105 110

Thr Leu Val Thr Val Ser Ser
115

<210> 80
<211> 107
<212> PRT
<213> Artificial Sequence

<220>
<223> Description of Artificial Sequence: Synthetic polypeptide

<400> 80

Glu Ile Val Met Thr Gln Ser Pro Ala Thr Leu Ser Val Ser Pro Gly
1 5 10 15

Glu Arg Ala Thr Leu Ser Cys Arg Ala Ser Gln Ser Val Ser Arg Asn
20 25 30

Leu Ala Trp Tyr Gln Gln Lys Pro Gly Gln Ala Pro Arg Leu Leu Ile
35 40 45

Tyr Gly Ala Ser Thr Arg Ala Thr Gly Ile Pro Ala Arg Phe Ser Gly
50 55 60

Ser Gly Ser Gly Thr Glu Phe Thr Leu Thr Ile Gly Ser Leu Gln Ser
65 70 75 80

Glu Asp Phe Ala Val Tyr Tyr Cys Gln Gln Tyr Lys Thr Trp Pro Arg
85 90 95

Thr Phe Gly Gln Gly Thr Asn Val Glu Ile Lys
100 105

<210> 81
<211> 357
<212> DNA
<213> Artificial Sequence

<220>
<223> Description of Artificial Sequence: Synthetic polynucleotide

<400> 81

caggtgcagc tacagcagtg gggcgcagga ctgttgaagc cttcggagac cctgtccctc 60

acctgcgctg tctttggtgg gtccttcagt ggttactact ggagctggat ccgccagccc 120

ccagggaagg ggctggagtg gattggggaa atcaatcatc gtggaaacac caacgacaac 180

ccgtccctca agagtcgagt caccatatca gtagacacgt ccaagaacca gttcgccctg 240

aagctgagtt ctgtgaccgc cgcggacacg gctgtttatt actgtgcgag agaacgtgga 300

tacacctatg gtaactttga ccactggggc cagggaaccc tggtcaccgt ctcctca 357

<210> 82
<211> 321
<212> DNA
<213> Artificial Sequence

<220>
<223> Description of Artificial Sequence: Synthetic polynucleotide

<400> 82

gaaatagtga tgacgcagtc tccagccacc ctgtctgtgt ctccagggga aagagccacc 60

ctctcctgca gggccagtca gagtgttagc agaaacttag cctggtatca gcagaaacct 120

ggccaggctc ccaggctcct catctatggt gcatccacca gggccactgg aatcccagcc 180

aggttcagtg gcagtgggtc tgggacagag ttcactctca ccatcggcag cctgcagtct 240

gaagattttg cagtttatta ctgtcagcag tataaaacct ggcctcggac gttcggccaa 300

gggaccaacg tggaaatcaa a 321

<210> 83
<211> 1444
<212> DNA
<213> Homo sapiens

<400> 83

gaattcctca ccatgggatg gagctgtatc atcctcttct tggtagcaac agctacaggt 60

gtccactccc aggtgcagct acagcagtgg ggcgcaggac tgttgaagcc ttcggagacc 120

ctgtccctca cctgcgctgt ctttggtggg tctttcagtg gttactactg gagctggatc 180

cgccagcccc cagggaaggg gctggagtgg attggggaaa tcaatcatcg tggaaacacc 240

aacgacaacc cgtccctcaa gagtcgagtc accatatcag tagacacgtc caagaaccag 300

ttcgccctga agctgagttc tgtgaccgcc gcggacacgg ctgtttatta ctgtgcgaga 360

gaacgtggat acacctatgg taactttgac cactggggcc agggaaccct ggtcaccgtc 420

agctcagcct ccaccaaggg cccatcggtc ttccccctgg caccctcctc caagagcacc 480

tctgggggca cagcggccct gggctgcctg gtcaaggact acttccccga accggtgacg 540

gtgtcgtgga actcaggcgc cctgaccagc ggcgtgcaca ccttcccggc tgtcctacag 600

tcctcaggac tctactccct cagcagcgtg gtgaccgtgc cctccagcag cttgggcacc 660

cagacctaca tctgcaacgt gaatcacaag cccagcaaca ccaaggtgga caagaaagtt 720

gagcccaaat cttgtgacaa aactcacaca tgcccaccgt gcccagcacc tgaactcctg 780

gggggaccgt cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccgcg ggaggagcag 960

tacaacagca cgtaccgtgt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaagggca gccccgagaa ccacaggtgt acaccctgcc cccatcccgg 1140

gatgagctga ccaagaacca ggtcagcctg acctgcctgg tcaaaggctt ctatcccagc 1200

gacatcgccg tggagtggga gagcaatggg cagccggaga acaactacaa gaccacgcct 1260

cccgtgctgg actccgacgg ctccttcttc ctctacagca agctcaccgt ggacaagagc 1320

aggtggcagc aggggaacgt cttctcatgc tccgtgatgc atgaggctct gcacaaccac 1380

tacacgcaga agagcctctc cctgtctccg ggtaaataat agggataaca gggtaatact 1440

agag 1444

<210> 84
<211> 468
<212> PRT
<213> Homo sapiens

<400> 84

```
Met Gly Trp Ser Cys Ile Ile Leu Phe Leu Val Ala Thr Ala Thr Gly
1               5                   10                  15

Val His Ser Gln Val Gln Leu Gln Gln Trp Gly Ala Gly Leu Leu Lys
            20                  25                  30

Pro Ser Glu Thr Leu Ser Leu Thr Cys Ala Val Phe Gly Gly Ser Phe
        35                  40                  45

Ser Gly Tyr Tyr Trp Ser Trp Ile Arg Gln Pro Pro Gly Lys Gly Leu
    50                  55                  60

Glu Trp Ile Gly Glu Ile Asn His Arg Gly Asn Thr Asn Asp Asn Pro
65                  70                  75                  80

Ser Leu Lys Ser Arg Val Thr Ile Ser Val Asp Thr Ser Lys Asn Gln
                85                  90                  95

Phe Ala Leu Lys Leu Ser Ser Val Thr Ala Ala Asp Thr Ala Val Tyr
            100                 105                 110

Tyr Cys Ala Arg Glu Arg Gly Tyr Thr Tyr Gly Asn Phe Asp His Trp
        115                 120                 125
```

```
Gly Gln Gly Thr Leu Val Thr Val Ser Ser Ala Ser Thr Lys Gly Pro
    130                 135                 140

Ser Val Phe Pro Leu Ala Pro Ser Ser Lys Ser Thr Ser Gly Gly Thr
145                 150                 155                 160

Ala Ala Leu Gly Cys Leu Val Lys Asp Tyr Phe Pro Glu Pro Val Thr
                165                 170                 175

Val Ser Trp Asn Ser Gly Ala Leu Thr Ser Gly Val His Thr Phe Pro
            180                 185                 190

Ala Val Leu Gln Ser Ser Gly Leu Tyr Ser Leu Ser Ser Val Val Thr
        195                 200                 205

Val Pro Ser Ser Ser Leu Gly Thr Gln Thr Tyr Ile Cys Asn Val Asn
    210                 215                 220

His Lys Pro Ser Asn Thr Lys Val Asp Lys Lys Val Glu Pro Lys Ser
225                 230                 235                 240

Cys Asp Lys Thr His Thr Cys Pro Pro Cys Pro Ala Pro Glu Leu Leu
                245                 250                 255

Gly Gly Pro Ser Val Phe Leu Phe Pro Pro Lys Pro Lys Asp Thr Leu
            260                 265                 270

Met Ile Ser Arg Thr Pro Glu Val Thr Cys Val Val Val Asp Val Ser
        275                 280                 285

His Glu Asp Pro Glu Val Lys Phe Asn Trp Tyr Val Asp Gly Val Glu
    290                 295                 300

Val His Asn Ala Lys Thr Lys Pro Arg Glu Glu Gln Tyr Asn Ser Thr
305                 310                 315                 320

Tyr Arg Val Val Ser Val Leu Thr Val Leu His Gln Asp Trp Leu Asn
                325                 330                 335

Gly Lys Glu Tyr Lys Cys Lys Val Ser Asn Lys Ala Leu Pro Ala Pro
            340                 345                 350

Ile Glu Lys Thr Ile Ser Lys Ala Lys Gly Gln Pro Arg Glu Pro Gln
        355                 360                 365

Val Tyr Thr Leu Pro Pro Ser Arg Asp Glu Leu Thr Lys Asn Gln Val
    370                 375                 380
```

Ser Leu Thr Cys Leu Val Lys Gly Phe Tyr Pro Ser Asp Ile Ala Val
385 390 395 400

Glu Trp Glu Ser Asn Gly Gln Pro Glu Asn Asn Tyr Lys Thr Thr Pro
405 410 415

Pro Val Leu Asp Ser Asp Gly Ser Phe Phe Leu Tyr Ser Lys Leu Thr
420 425 430

Val Asp Lys Ser Arg Trp Gln Gln Gly Asn Val Phe Ser Cys Ser Val
435 440 445

Met His Glu Ala Leu His Asn His Tyr Thr Gln Lys Ser Leu Ser Leu
450 455 460

Ser Pro Gly Lys
465

<210> 85
<211> 722
<212> DNA
<213> Homo sapiens

<400> 85

gcggccgcct caccatggga tggagctgta tcatcctctt cttggtagca acagctacag 60
gtgtccactc cgaaatagtg atgacgcagt ctccagccac cctgtctgtg tctccagggg 120
aaagagccac cctctcctgc agggccagtc agagtgttag cagaaactta gcctggtatc 180
agcagaaacc tggccaggct cccaggctcc tcatctatgg tgcatccacc agggccactg 240
gaatcccagc caggttcagt ggcagtgggt ctgggacaga gttcactctc accatcggca 300
gcctgcagtc tgaagatttt gcagtttatt actgtcagca gtataaaacc tggcctcgga 360
cgttcggcca agggaccaac gtggaaatca aacgtacggt ggctgcacca tctgtcttca 420
tcttcccgcc atctgatgag cagttgaaat ctggaactgc ctctgttgtg tgcctgctga 480
ataacttcta tcccagagag gccaaagtac agtggaaggt ggataacgcc ctccaatcgg 540
gtaactccca ggagagtgtc acagagcagg acagcaagga cagcacctac agcctcagca 600
gcaccctgac cctgagcaaa gcagactacg agaaacacaa agtctacgcc tgcgaagtca 660
cccatcaggg cctgagctcg cccgtcacaa agagcttcaa caggggagag tgttagtcta 720
ga 722

<210> 86
<211> 233
<212> PRT
<213> Homo sapiens

<400> 86

```
Met Gly Trp Ser Cys Ile Ile Leu Phe Leu Val Ala Thr Ala Thr Gly
1               5                   10                  15

Val His Ser Glu Ile Val Met Thr Gln Ser Pro Ala Thr Leu Ser Val
            20                  25                  30

Ser Pro Gly Glu Arg Ala Thr Leu Ser Cys Arg Ala Ser Gln Ser Val
        35                  40                  45

Ser Arg Asn Leu Ala Trp Tyr Gln Gln Lys Pro Gly Gln Ala Pro Arg
    50                  55                  60

Leu Leu Ile Tyr Gly Ala Ser Thr Arg Ala Thr Gly Ile Pro Ala Arg
65                  70                  75                  80

Phe Ser Gly Ser Gly Ser Gly Thr Glu Phe Thr Leu Thr Ile Gly Ser
                85                  90                  95

Leu Gln Ser Glu Asp Phe Ala Val Tyr Tyr Cys Gln Gln Tyr Lys Thr
            100                 105                 110

Trp Pro Arg Thr Phe Gly Gln Gly Thr Asn Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230
```

<210> 87
<211> 14
<212> PRT
<213> Artificial Sequence

<220>
<223> Description of Artificial Sequence: Synthetic peptide

<220>
<223> N-term H

<220>
<221> misc_feature
<222> (1)..(6)
<223> Disulfide bond between residues

<220>
<221> misc_feature
<222> (2)..(10)
<223> Disulfide bond between residues

<220>
<221> misc_feature
<222> (5)..(13)
<223> Disulfide bond between residues

<220>
<223> C-term OH

<400> 87

```
Cys Cys Glu Tyr Cys Cys Asn Pro Ala Cys Thr Gly Cys Tyr
1               5                   10
```

<210> 88
<211> 6
<212> PRT
<213> Artificial Sequence

<220>
<223> Description of Artificial Sequence: Synthetic His tag

<220>
<221> misc_feature
<222> (1)..(6)
<223> This sequence may encompass 2, 3, 4, 5, or 6 residues

<400> 88

```
His His His His His His
1               5
```

<210> 89
<211> 4
<212> PRT
<213> Artificial Sequence

<220>

<223> Description of Artificial Sequence: Synthetic peptide

<400> 89

```
Gly Phe Leu Gly
1
```